# EUROPEAN PATENT APPLICATION

(11) **EP 1 577 305 A1**
(43) Date of publication of application: **21.09.2005**
(21) Application number: 05104581.3
(22) Date of filing: 15.07.2002
(51) Int. Cl.: C07D 263/32, A61K 31/422, A61P 3/10

(54) **Oral antidiabetic agents**

(30) Priority: 29.08.2001 US 315728 P
(62) Divisional of application: 02745739.9
(71) Applicant: Warner-Lambert Company LLC, New Jersey 07950 (US)
(72) Inventor: Bigge, Christopher F., Ann Arbor, MI Michigan 48105 (US); Sexton, Karen Elaine, Ann Arbor, MI Michigan 48105 (US); Schlosser, Kevin Matthew, Ann Arbor, MI Michigan 48105 (US); Schaum, Robert Philipp, Ann Arbor, MI Michigan 48105 (US); Lee, Helen Tsenwhei, Ann Arbor, MI Michigan 48105 (US); Reed, Jessica Elizabeth, Ann Arbor, MI Michigan 48105 (US); Fakhoury, Stephen Alan, Ann Arbor, MI Michigan 48105 (US); Casimiro-Garcia, Agustin, Ann Arbor, MI Michigan 48105 (US); Zhou, Hairong, Ann Arbor, MI Michigan 48105 (US); Bridges, Alexander, James, Ann Arbor, MI Michigan 48105 (US)
(74) Representative: Rutt, Jason Edward

(57) **Abstract**

The present invention provides compounds of formula (I) 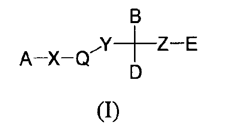 or a pharmaceutically acceptable salt thereof, wherein:
A is 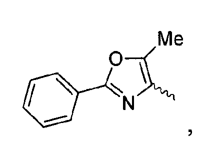 X is 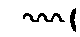 CH₂-CH₂-O 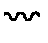 or -CH₂CH₂CH₂-;
Q is 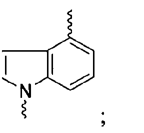 Y is CH₂;
Z is absent;
B is H;
D is H, 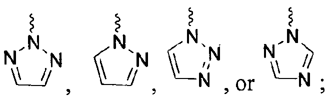 and
E is CO₂H;
that are useful as antidiabetic agents. Also disclosed are pharmaceutical compositions comprising compounds of formula (I).

## Description

### CROSS REFERENCE TO RELATED APPLICATIONS

This application claims benefit of priority from United States Provisional Application Number 60/315,728 filed on August 29, 2001, United States Provisional Application Number 60/322,123 filed on September 14, 2001, and United States Provisional Application Number 60/369,788 filed on April 3, 2002.

### FIELD OF THE INVENTION

The present invention relates to compounds that are useful as antidiabetic agents.

### BACKGROUND OF THE INVENTION

Type II diabetes, or non-insulin dependent diabetes (NIDDM) is a significant healthcare problem whose incidence is on the rise. Between 1990 and 1998, the prevalence of NIDDM in the United States increased by 33 percent, to about 13 million persons. An additional 5 million persons are presumed to have undiagnosed NIDDM, while another 14 million persons have impaired glucose tolerance. Direct medical costs associated with diabetes were $44 billion in 1997, due mainly to hyperglycemia-related diabetic complications, including diabetic angiopathy, atherosclerosis, diabetic nephropathy, diabetic neuropathy, and diabetic ocular complications such as retinopathy, cataract formation, and glaucoma.

NIDDM is one of a number of disease states associated with the phenomenon of insulin resistance. Insulin resistance is defined as the reduced sensitivity to the actions of insulin in the whole body or individual tissues, such as skeletal muscle tissue, myocardial tissue, fat tissue or liver tissue. Insulin resistance occurs in many individuals with or without diabetes mellitus. Insulin resistance syndrome (hereinafter IRS) refers to the cluster of manifestations that include insulin resistance; hyperinsulinemia; non insulin dependent diabetes mellitus (NIDDM); arterial hypertension; central (visceral) obesity; and dyslipidemia.

The primary goal of IRS therapy and thus diabetes therapy is to lower blood glucose levels so as to prevent acute and long-term disease complications. For some persons, modified diet and increased exercise may be a successful therapeutic option for achieving the goal of glucose control. When modified diet and increased exercise are not successful, drug therapy using oral antidiabetic agents is initiated.

To date, a number of oral antidiabetic agents have been developed. For instance, sulfonylureas are generally used to stimulate insuln. The biguanide metformin is generally used to improve insulin sensitivity and to decrease hepatic glucose output. Acarbose is used to limit postprandial hyperglycemia, Thiazolidine 2,4 diones are used to enhance insulin action.

New drug therapies for the treatment of NIDDM have focused in part on the discovery of new Peroxisome Proliferator Activation Recpetor (PPAR) agonists. PPARs are members of the nuclear receptor superfamily of transcription factors that includes steroid, thyroid, and vitamin D receptors. PPARs play a role in controlling expression of proteins that regulate lipid metabolism. There are three PPAR subtypes: PPAR α, PPAR δ, and PPAR γ.

Each PPAR receptor shows a different pattern of tissue expression, and differences in activation by structurally diverse compounds. PPAR γ, for instance, is expressed most abundantly in adipose tissue and at lower levels in skeletal muscle, heart, liver, intestine, kidney, vascular endothelial and smooth muscle cells as well as macrophages. Two isoforms of PPAR γ exist, identified as γ₁ and γ₂, respectively. PPAR γ mediates adipocyte signalling, lipid storage, and fat metabolism. Evidence gathered to date support the conclusion that PPAR γ is the primary, and perhaps the only, molecular target mediating the insulin sensitizing action of one class of antidiabetic agents, the thiazolidine 2,4 diones.

In a monotherapeutic or combination therapy context, new and established oral antidiabetic agents are still considered to have non-uniform and even limited effectiveness. The effectivieness of oral antidiabetic therapies may be limited, in part, because of poor or limited glycemic control, or poor patient compliance due to unacceptable side effects. These side effects include edema weight gain, or even more serious complications. For instance, hypoglycemia is observed in some patients taking sulfonylureas. Metformin, a substituted biguanide, can cause diarrhea and gastrointestinal discomfort. Finally, edema, weight gain, and in some cases, hepatoxicity, have been linked to the administration of some thiazolidine 2,4 dione antidiabetic agents. Combination therapy using two or more of the above agents is common, but generally only leads to incremental improvements in glycemic control.

As a result, there is a need for oral antidiabetic agents that can be used alone or in combination, and that do not give rise to side effects such as fluid retention, peripheral edema, weight gain, or more severe complications.

### SUMMARY OF THE INVENTION

These and other needs are met by the current invention which is a compound of formula (I) 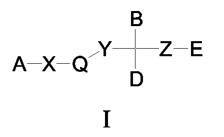 or a pharmaceutically acceptable salt thereof, wherein:
A is aryl and substituted aryl,
   heteroaryl and substituted heteroaryl,
   fused heteroaryl and substituted fused heteroaryl,
   (C₃-C₇)cycloalkyl and substituted cycloalkyl, or
   heterocycloalkyl and substituted heterocycloalkyl;
X is a tether 2 to 5 atoms in length selected from 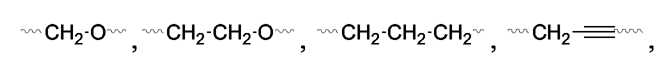 or 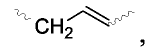 wherein " 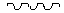 " indicates a point of attachment;
Q is aryl or substituted aryl;
   heteroaryl or substituted heteroaryl;
   fused heteroaryl, or substituted fused heteroaryl; provided that when q is a five-membered heteroaryl, the point of attachment from X to Q is not at the heteroatom of the heteroaryl;
Y and Z are independently absent or are (CR₁R₂)ₙ and (CR₃R₄)ₘ respectively,
   wherein R₁-R₄ are each independently H, halo, (C₁-C₆)alkyl, hydroxy, (C₁-C₆)alkoxy, and m and n are each independently 1, 2, or 3;
B is H, halo, (C₁-C₆)alkyl, halo(C₁-C₆)alkyl, or (C₁-C₆)alkoxy;
D is H, -NH-aryl or -NH-substituted aryl,
   (C₁-C₆)alkanoyl and substituted alkanoyl,
   benzoyl and substituted benzoyl,
   aryl and substituted aryl,
   heteroaryl and substituted heteroaryl,
   (C₃-C₇)cycloalkyl and substituted cycloalkyl, or
   heterocycloalkyl and substituted heterocycloalkyl;
E is COR₅, wherein R₅ is (C₁-C₆)alkyl, OH, (C₁-C₆)alkoxy, NR₆R₇, wherein R₆ and R₇ are each independently H or (C₁-C₆)alkyl, or one of R₆ and R₇ is H or (C₁-C₆)alkyl and the other is SO₂R₈, wherein R₈ is H or (C₁-C₆)alkyl, or E is substituted heteroaryl or 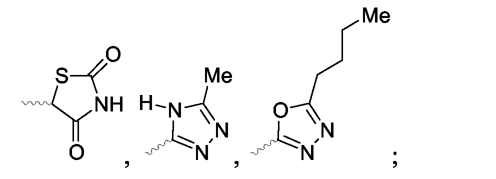 provided that when A is 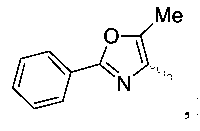 X is 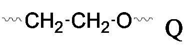 is phenyl, Y and Z are CH₂, B is H, and E is CO₂H, D is not pyrrolyl.

The invention also provides a compound of formula (II) 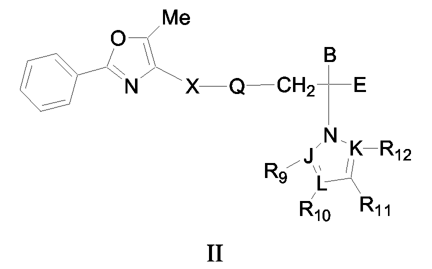 or a pharmaceutically acceptable salt thereof, wherein:
X is a tether 2 to 5 atoms in length selected from 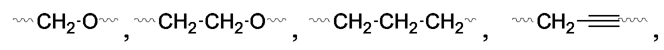 or 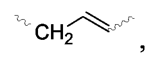 wherein " 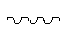 " indicates a point of attachment;
Q is aryl or substituted aryl;
   heteroaryl or substituted heteroaryl;
   fused heteroaryl, or substituted fused heteroaryl; provided that when q is a five-membered heteroaryl, the point of attachment from X to Q is not at the heteroatom of the heteroaryl;
B is H, halo, (C₁-C₆)alkyl, halo(C₁-C₆)alkyl, or (C₁-C₆)alkoxy;
E is COR₅, wherein R₅ is (C₁-C₆)alkyl, OH, (C₁-C₆)alkoxy, NR₆R₇, wherein R₆ and R₇ are each independently H or (C₁-C₆)alkyl, or one of R₆ and R₇ is H or (C₁-C₆)alkyl and the other is SO₂R₈, wherein R₈ is H or (C₁-C₆)alkyl, or E is substituted heteroaryl or 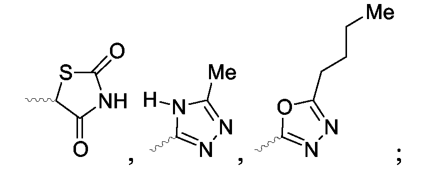
R₉-R₁₂ are each independently H, halo, aryl, (C₁-C₆)alkyl, halo(C₁-C₆)alkyl, (C₁-C₆)alkanoyl, halo(C₁-C₆)alkanoyl, (C₃-C₇)cycloalkylcarbonyl, benzoyl, or halo(C₂-C₆)alkanoyl; and
1 or 2 of J, K, and L are N,
   provided that when J, K, or L are N, R₉, R₁₀, R₁₁, or R₁₂ is absent at that position.

The invention also provides a compound of formula (III): 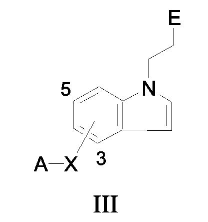 or a pharmaceutically acceptable salt thereof, wherein:
A is aryl and substituted aryl,
   heteroaryl and substituted heteroaryl,
   fused heteroaryl and substituted fused heteroaryl,
   (C₃-C₇)cycloalkyl and substituted cycloalkyl, or
   heterocycloalkyl and substituted heterocycloalkyl;
X is a tether 2 to 5 atoms in length selected from 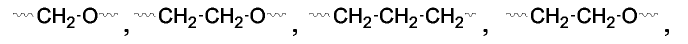 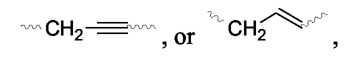 wherein " 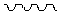 " indicates a point of attachment, and wherein -XA can be attached, to the 3, 4, 5, or 6 position of the indolinyl core; and
E is COR₅, wherein R₅ is (C₁-C₆)alkyl, OH, (C₁-C₆)alkoxy, NR₆R₇, wherein R₆ and R₇ are each independently H or (C₁-C₆)alkyl, or one of R₆ and R₇ is H or (C₁-C₆)alkyl and the other is SO₂R₈, wherein R₈ is H or (C₁-C₆)alkyl, or E is substituted heteroaryl or

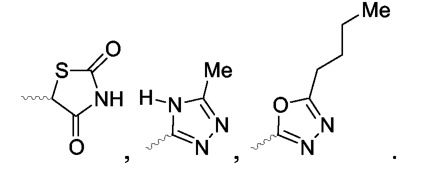

The invention also provides a compound which is:

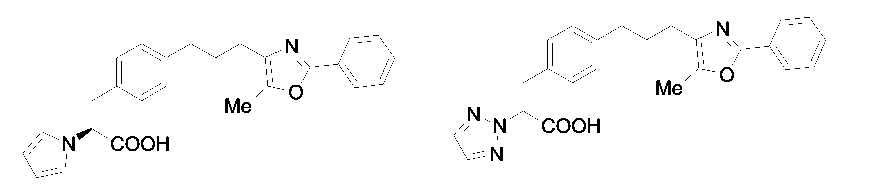

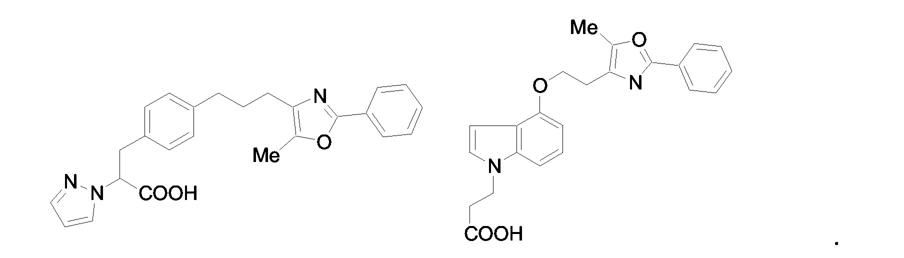

The invention also provides a compound which is:

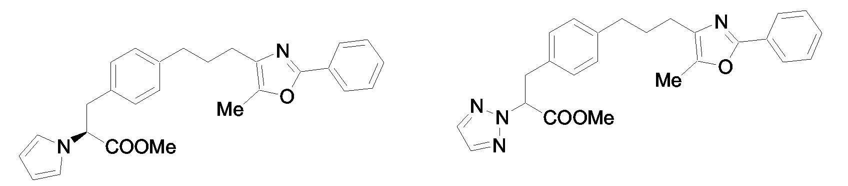

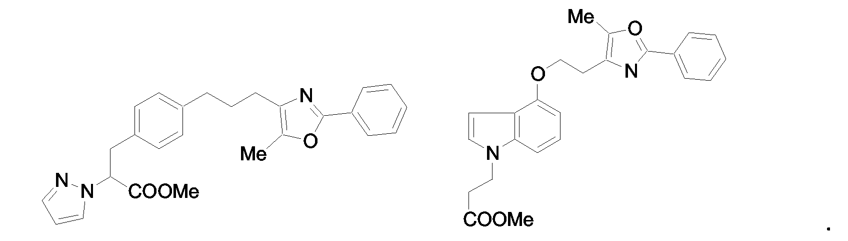

The invention also provides a compound which is:
(R)-2-(2-{4-[2-(5-Methyl-2-phenyl-oxazol-4-yl)-ethoxy]-phenyl}-1-pyrrol-1-yl-ethyl)-5-propyl-1,3,4-oxadiazole;
(R)-2-Ethyl-5-(2-{4-[2-(5-methyl-2-phenyl-oxazol-4-yl)-ethoxy]-phenyl}-1-pyrrol-1-yl-ethyl)-1,3,4-oxadiazole;
(R)-2-{4-[2-(5-Methyl-2-phenyl-oxazol-4-yl)-ethoxy]-phenyl}-1-pyrrol-1-yl-ethyl)-1,3,4-oxadiazole;
(R)-2-Methyl-3-{4-[2-(5-methyl-2-phenyl-oxazol-4-yl)-ethoxy]-phenyl}-2-pyrrol-1-yl-propionic acid;
(R)-3-Methyl-5-(2-{4-[2-(5-methyl-2-phenyl-oxazol-4-yl)-ethoxy]-phenyl}-1-(S)-pyrrol-1-yl-ethyl)-4H-1,2,4-triazole;
(R)-2-(3-Bromo-pyrrol-1-yl)-2-methyl-3-{4-[2-(5-methyl-2-phenyl-oxazol-4-yl)-ethoxy]-phenyl}-propionic acid;
(S)-3,3,3-Trifluoro-2-{4-[2-(5-methyl-2-phenyl-oxazol-4-yl)-ethoxy]-benzyl}-2-pyrrol-1-yl-propionic acid;
(R)-3,3,3-Trifluoro-2-{4-[2-(5-methyl-2-phenyl-oxazol-4-yl)-ethoxy]-benzyl}-2-pyrrol-1-yl-propionic acid;
(R)-2-ethyl-3- {4-[2-(5-methyl-2-phenyl-oxazol-4-yl)-ethoxy]-phenyl -2-pyrrol-1-yl-propionic acid;
(S)-2-ethyl-3-{4-[2-(5-methyl-2-phenyl-oxazol-4-yl)-ethoxy]-phenyl}-2-pyrrol-1-yl-propionic acid;
(R)-2-propyl-3-{4-[2-(5-methyl-2-phenyl-oxazol-4-yl)-ethoxy]-phenyl}-2-pyrrol-1-yl-propionic acid;
(S)-2-propyl-3- {4-[2-(5-methyl-2-phenyl-oxazol-4-yl)-ethoxy]-phenyl}-2-pyrrol-1-yl-propionic acid;
(S)-3,3,3-Trifluoroethyl-2-{4-[2-(5-methyl-2-phenyl-oxazol-4-yl)-ethoxy]-benzyl}-2-pyrrol-1-yl-propionic acid;
(R)-3,3,3-Trifluoroethyl-2-{4-[2-(5-methyl-2-phenyl-oxazol-4-yl)-ethoxy]-benzyl}-2-pyrrol-1-yl-propionic acid;
(R) 2-Fluoromethyl-2-{4-[2-(5-methyl-2-phenyl-oxazol-4-yl)-ethoxy]-benzyl}-2-pyrrol-1-yl-propionic acid;
(S) 2-Fluoromethyl-2-{4-[2-(5-methyl-2-phenyl-oxazol-4-yl)-ethoxy]-benzyl}-2-pyrrol-1-yl-propionic acid;
(S)-3-{4-[2-(5-Ethyl-pyridin-2-yl)-ethoxy]-phenyl}-2-fluoromethyl-2-pyrrol-1-yl-propionic acid;
(R)-3-{4-[2-(5-Ethyl-pyridin-2-yl)-ethoxy]-phenyl}-2-fluoromethyl-2-pyrrol-1-yl-propionic acid;
(S)-3-{4-[2-(5-Ethyl-pyridin-2-yl)-ethoxy]-phenyl}-1-pyrrol-1-yl-ethyl)-5-propyl-1,3,4-oxadiazole;
(R)-3-{4-[2-(5-Ethyl-pyridin-2-yl)-ethoxy]-phenyl}-1-pyrrol-1-yl-ethyl)-5-propyl-1,3,4-oxadiazole;
(S)-3-{4-[2-(5-Ethyl-pyridin-2-yl)-ethoxy]-phenyl}-1-pyrrol-1-yl-ethyl)-5-propyl-1,3,4-oxadiazole;
(R)-3-{4-[2-(5-Ethyl-pyridin-2-yl)-ethoxy]-phenyl}-1-pyrrol-1-yl-ethyl)-1,3,4-oxadiazole;
(S)-3-{4-[2-(5-Ethyl-pyridin-2-yl)-ethoxy]-phenyl}-1-pyrrol-1-yl-ethyl)-1,3,4-oxadiazole;
(R)-3-{4-[2-(5-Ethyl-pyridin-2-yl)-ethoxy]-phenyl}-2-pyrrol-1-yl-propionic acid;
(S)-3-{4-[2-(5-Ethyl-pyridin-2-yl)-ethoxy]-phenyl}-2-pyrrol-1-yl-propionic acid;
(R)-3-{4-[2-(5-Ethyl-pyridin-2-yl)-ethoxy]-phenyl}-1-(S)-pyrrol-1-yl-ethyl)-4H-1,2,4-triazole;
(S)-3-{4-[2-(5-Ethyl-pyridin-2-yl)-ethoxy]-phenyl}-1-(S)-pyrrol-1-yl-ethyl)-4H-1,2,4-triazole;
(R)-2-(3-Bromo-pyrrol-1-yl)-3-{4-[2-(5-Ethyl-pyridin-2-yl)-ethoxy]-phenyl}-propionic acid;
(S)-2-(3Bromo-pyrrol-1-yl)-3-{4-[2-(5-Ethyl-pyridin-2-yl)-ethoxy]-phenyl}-propionic acid;
(S)-3,3,3-Trifluoro-3-{4-[2-(5-Ethyl-pyridin-2-yl)-ethoxy]-phenyl}-2-pyrrol-1-yl-propionic acid;
(R)-3,3,3-Trifluoro-3-{4-[2-(5-Ethyl-pyridin-2-yl)-ethoxy]-phenyl}-2-pyrrol-1-yl-propionic acid;
(R)-2-ethyl-3-{4-[2-(5-Ethyl-pyridin-2-yl)-ethoxy]-phenyl}-2-pyrrol-1-yl-propionic acid;
(S)-2-ethyl-3-{4-[2-(5-Ethyl-pyridin-2-yl)-ethoxy]-phenyl]-2-pyrrol-1-yl-propionic acid;
(R)-2-propyl-3-{4-[2-(5-Ethyl-pyridin-2-yl)-ethoxy]-phenyl}-2-pyrrol-1-yl-propionic acid;
(S)-2-propyl-3- {4-[2-(5-Ethyl-pyridin-2-yl)-ethoxy]-phenyl}-2-pyrrol-1-yl-propionic acid;
(S)-3,3,3-Trifluoroethyl-3-{4-[2-(5-Ethyl-pyridin-2-yl)-ethoxy]-phenyl}-2-pyrrol-1-yl-propionic acid;
(R)-3,3,3-Trifluoroethyl-3-{4-[2-(5-Ethyl-pyridin-2-yl)-ethoxy]-phenyl}-2-pyrrol-1-yl-propionic acid;
(S)-2-Pyrrol-1-yl-3-[4-(4'-trifluoromethyl-biphenyl-4-ylmethoxy)-phenyl]-propionic acid;
(R)-2-Pyrrol-1-yl-3-[4-(4'-trifluoromethyl-biphenyl-4-ylmethoxy)-phenyl]-propionic acid;
(R)-2-(2-[4-(4'-trifluoromethyl-biphenyl-4-ylmethoxy)-phenyl}]-1-pyrrol-1-yl-ethyl)-5-propyl-1,3,4-oxadiazole;
(S)-2-(2-[4-(4'-trifluoromethyl-biphenyl-4-ylmethoxy)-phenyl}]-1-pyrrol-1-yl-ethyl)-5-propyl-1,3,4-oxadiazole;
(R)-2-Ethyl-2-[4-(4'-trifluoromethyl-biphenyl-4-ylmethoxy)-phenyl}]-1-pyrrol-1-yl-ethyl)-1,3,4-oxadiazole;
(S)-2-Ethyl-2-[4-(4'-trifluoromethyl-biphenyl-4-ylmethoxy)-phenyl}]-1-pyrrol-1-yl-ethyl)-1,3,4-oxadiazole;
(R)-2-2-[4-(4'-trifluoromethyl-biphenyl-4-ylmethoxy)-phenyl]-1-pyrrol-1-yl-ethyl)-1,3,4-oxadiazole;
(S)-2-2-[4-(4'-trifluoromethyl-biphenyl-4-ylmethoxy)-phenyl]-1-pyrrol-1-yl-ethyl)-1,3,4-oxadiazole;
(R)-2-Methyl-3-[4-(4'-trifluoromethyl-biphenyl-4-ylmethoxy)-phenyl]-2-pyrrol-1-yl-propionic acid;
(S)-2-Methyl-3-[4-(4'-trifluoromethyl-biphenyl-4-ylmethoxy)-phenyl]-2-pyrrol-1-yl-propionic acid;
(R)-3-Methyl-5-(3-[4-(4'-trifluoromethyl-biphenyl-4-ylmethoxy)-phenyl]-1-(S)-pyrrol-1-yl-ethyl)-4H-1,2,4-triazole;
(S)-3-Methyl-5-(3-[4-(4'-trifluoromethyl-biphenyl-4-ylmethoxy)-phenyl]-1-(S)-pyrrol-1-yl-ethyl)-4H-1,2,4-triazole;
(R)-2-(3-Bromo-pyrrol-1-yl)-3-[4-(4'-trifluoromethyl-biphenyl-4-ylmethoxy)-phenyl]-propionic acid;
(R)-2-(3-Bromo-pyrrol-1-yl)-3-[4-(4'-trifluoromethyl-biphenyl-4-ylmethoxy)-phenyl]-propionic acid;
(S)-3,3,3-Trifluoro-2-[4-(4'-trifluoromethyl-biphenyl-4-ylmethoxy)-benzyl]-2-pyrrol-1-yl-propionic acid;
(R)-3,3,3-Trifluoro-2-[4-(4'-trifluoromethyl-biphenyl-4-ylmethoxy)-benzyl]-2-pyrrol-1-yl-propionic acid;
(R)-2-ethyl-3-[4-(4'-trifluoromethyl-biphenyl-4-ylmethoxy)-phenyl]-2-pyrrol-1-yl-propionic acid;
(S)-2-ethyl-3-[4-(4'-trifluoromethyl-biphenyl-4-ylmethoxy)-phenyl]-2-pyrrol-1-yl-propionic acid;
(R)-2-propyl-3-[4-(4'-trifluoromethyl-biphenyl-4-ylmethoxy)-phenyl]-2-pyrrol-1-yl-propionic acid;
(S)-2-propyl-3-[4-(4'-trifluoromethyl-biphenyl-4-ylmethoxy)-phenyl]-2-pyrrol-1-yl-propionic acid;
(S)-3,3,3-Trifluoroethyl-2-[4-(4'-trifluoromethyl-biphenyl-4-ylmethoxy)-benzyl]-2-pyrrol-1-yl-propionic acid;
(R)-3,3,3-Trifluoroethyl-2--[4-(4'-trifluoromethyl-biphenyl-4-ylmethoxy)-benzyl]-2-pyrrol-1-yl-propionic acid;
(R) 2-Fluoromethyl-2-[4-(4'-trifluoromethyl-biphenyl-4-ylmethoxy)-benzyl]-2-pyrrol-1-yl-propionic acid;
(S) 2-Fluoromethyl-2--[4-(4'-trifluoromethyl-biphenyl-4-ylmethoxy)-benzyl]-2-pyrrol-1-yl-propionic acid;
(S)-2-3-{4-[2-(Methyl-pyridin-2-yl-amino)-ethoxy]-phenyl}-1-pyrrol-1-yl-ethyl)-5-propyl-1,3,4-oxadiazole;
(R)-2-3-{4-[2-(Methyl-pyridin-2-yl-amino)-ethoxy]-phenyl}-1-pyrrol-1-yl-ethyl)-5-propyl-1,3,4-oxadiazole;
(S)-2-Ethyl-2- {4-[2-(Methyl-pyridin-2-yl-amino)-ethoxy]-phenyl}-1-pyrrol-1-yl-ethyl)-1,3,4-oxadiazole;
(R)-2-Ethyl-2-{4-[2-(Methyl-pyridin-2-yl-amino)-ethoxy]-phenyl}-1-pyrrol-1-yl-ethyl)-1,3,4-oxadiazole;
(R)-2-2- {4-[2-(Methyl-pyridin-2-yl-amino)-ethoxy]-phenyl}-1-pyrrol-1-yl-ethyl)-1,3,4-oxadiazole;
(S)-2-2- {4-[2-(Methyl-pyridin-2-yl-amino)-ethoxy]-phenyl}-1-pyrrol-1-yl-ethyl)-1,3,4-oxadiazole;
(R)-2-Methyl-3-14-[2-(Methyl-pyridin-2-yl-amino)-ethoxy]-phenyl}-2-pyrrol-1-yl-propionic acid;
(S)-2-Methyl-3--{4-[2-(Methyl-pyridin-2-yl-amino)-ethoxy]-phenyl}-2-pyrrol-1-yl-propionic acid;
(R)-3-Methyl-5-(3- {4-[2-(Methyl-pyridin-2-yl-amino)-ethoxy]-phenyl}-1-(S)-pyrrol-1-yl-ethyl)-4H-1,2,4-triazole;
(S)-3-Methyl-5-(3-{4-[2-(Methyl-pyridin-2-yl-amino)-ethoxy]-phenyl}-1-(S)-pyrrol-1-yl-ethyl)-4H-1,2,4-triazole;
(R)-2-(3-Bromo-pyrrol-1-yl)-3-{4-[2-(Methyl-pyridin-2-yl-amino)-ethoxy]-phenyl}-propionic acid;
(S)-2-(3-Bromo-pyrrol-1-yl)-3-{4-[2-(Methyl-pyridin-2-yl-amino)-ethoxy]-phenyl}-propionic acid;
(S)-3,3,3-Trifluoro-2-{4-[2-(Methyl-pyridin-2-yl-amino)-ethoxy]-benzyl}-2-pyrrol-1-yl-propionic acid;
(R)-3,3,3-Trifluoro-2-{4-[2-(Methyl-pyridin-2-yl-amino)-ethoxy]-benzyl}-2-pyrrol-1-yl-propionic acid;
(R)-2-ethyl-3-{4-[2-(Methyl-pyridin-2-yl-amino)-ethoxy]-phenyl}-2-pyrrol-1-yl-propionic acid;
(S)-2-ethyl-3-{4-[2-(Methyl-pyridin-2-yl-amino)-ethoxy]-phenyl}-2-pyrrol-1-yl-propionic acid;
(R)-2-propyl-3-{4-[2-(Methyl-pyridin-2-yl-amino)-ethoxy]-phenyl}-2-pyrrol-1-yl-propionic acid;
(S)-2-propyl-3-{4-[2-(Methyl-pyridin-2-yl-amino)-ethoxy]-phenyl-2-pyrrol-1-yl-propionic acid;
(S)-3,3,3-Trifluoroethyl-2-{4-[2-(Methyl-pyridin-2-yl-amino)-ethoxy]-benzyl}-2-pyrrol-1-yl-propionic acid;
(R)-3,3,3-Trifluoroethyl-2-{4-[2-(Methyl-pyridin-2-yl-amino)-ethoxy]-benzyl}-2-pyrrol-1-yl-propionic acid;
(R) 2-Fluoromethyl-2-{4-[2-(Methyl-pyridin-2-yl-amino)-ethoxy]-benzyl]-2-pyrrol-1-yl-propionic acid; or
(S) 2-Fluoromethyl-2-{4-[2-(Methyl-pyridin-2-yl-amino)-ethoxy]-benzyl}-2-pyrrol-1-yl-propionic acid.

The invention also provides a compound which is:
(S)-3-{4-[2-(5-Methyl-2-phenyl-oxazol-4-yl)-ethoxy]-phenyl}-2-pyrrol-1-yl-propionic acid;
(S)-2-(2-Benzoyl-pyrrol-1-yl)-3-{4-[2-(5-methyl-2-phenyl-oxazol-4-yl)-ethoxy]-phenyl}-propionic acid;
(S)-2-(3-Benzoyl-pyrrol-1-yl)-3-{4-[2-(5-methyl-2-phenyl-oxazol-4-yl)-ethoxy]-phenyl}-propionic acid;
(S)-3-{4-[2-(5-Methyl-2-phenyl-oxazol-4-yl)-ethoxy]-phenyl}-2-(3-phenyl-pyrrol-1-yl)-propionic acid;
(S)-3-{4-[2-(Benzooxazol-2-yl-methyl-amino)-ethoxy]-phenyl}-2-(3-phenyl-pyrrol-1-yl)-propionic acid;
N-(S)-(3-{4-[2-(5-Methyl-2-phenyl-oxazol-4-yl)-ethoxy]-phenyl}-2-pyrrol-1-yl-propionyl)-Benzenesulfonamide
N-(S)-(3-{4-[2-(5-Methyl-2-phenyl-oxazol-4-yl)-ethoxy]-phenyl}-2-pyrrol-1-yl-propionyl)-methanesulfonamide;
(S)-2-(3-Isobutyryl-pyrrol-1-yl)-3-{4-[2-(5-methyl-2-phenyl-oxazol-4-yl)-ethoxy]-phenyl}-propionic acid;
(S)-2-(3-Cyclohexanecarbonyl-pyrrol-1-yl)-3-{4-[2-(5-methyl-2-phenyl-oxazol-4-yl)-ethoxy]-phenyl}-propionic acid;
(S)-3-{4-[2-(Benzooxazol-2-yl-methyl-amino)-ethoxy]-phenyl}-2-(3-cyclohexanecarbonyl-pyrrol-1-yl)-propionic acid;
(S)-2-(3-Cyclohexanecarbonyl-pyrrol-1-yl)-3-[4-(2-fluoro-benzyloxy)-phenyl]-propionic acid;
(S)-3-{4-[2-(5-Methyl-2-phenyl-oxazol-4-yl)-ethoxy]-phenyl}-2-[2-(2,2,2-trifluoro-acetyl)-pyrrol-1-yl]-propionic acid;.
3-{4-[2-(5-Methyl-2-phenyl-oxazol-4-yl)-ethoxy]-phenyl}-2-pyridin-4-yl-propionic acid;
2-Methyl-5-(2-{4-[2-(5-methyl-2-phenyl-oxazol-4-yl)-ethoxy]-phenyl}-1-phenyl-ethyl)-2H-tetrazole
2-Methyl-3-{4-[2-(5-methyl-2-phenyl-oxazol-4-yl)-ethoxy]-phenyl}-2-thiophen-2-yl-propionic acid;
N-(2,2-Dimethyl-3-{4-[2-(5-methyl-2-phenyl-oxazol-4-yl)-ethoxy]-phenyl}-propionyl)-methanesulfonamide
N-(2-Fluoro-3-{4-[2-(5-methyl-2-phenyl-oxazol-4-yl)-ethoxy]-phenyl}-2-phenyl-propionyl)-methanesulfonamide
2-(5-Methyl-2-phenyl-oxazol-4-yl)-3-{4-[3-(5-methyl-2-phenyl-oxazol-4-yl)-propyl]-phenyl}-propionic acid;
2-(5-Methyl-2-phenyl-oxazol-4-yl)-3-{4-[2-(5-methyl-2-phenyl-oxazol-4-yl)-ethoxy]-phenyl}-propionic acid;
3-{4-[3-(5-Methyl-2-phenyl-oxazol-4-yl)-propyl]-phenyl]-2-phenyl-propionic acid;
(S)-2-(2-Acetyl-pyrrol-1-yl)-3-{4-[2-(5-methyl-2-phenyl-oxazol-4-yl)-ethoxy]-phenyl}-propionic acid;
3- {4-[2-(Benzothiazol-2-ylsulfanyl)-ethoxy]-phenyl}-2-pyrrol-1-yl-propionic acid;
(S)-2-Pyrrol-1-yl-3-{4-[2-(2-trifluoromethyl-phenyl)-ethoxy]-phenyl}-propionic acid;
(S)-3-{4-[2-(4-Phenyl-piperazin-1-yl)-ethoxy]-phenyl}-2-pyrrol-1-yl-propionic acid;
(S)-3-{4-[2-(5-Methyl-2-phenyl-thiazol-4-yl)-ethoxy]-phenyl}-2-pyrrol-1-yl-propionic acid;
(S)-2-(3-Chloro-2,5-dimethyl-pyrrol-1-yl)-3-{4-[2-(5-methyl-2-phenyl-oxazol-4-yl)-ethoxy]-phenyl}-propionic acid;
(S)-3-(4-{2-[(7-Chloro-quinolin-4-yl)-methyl-amino]-ethoxy]-phenyl)-2-pyrrol-1-yl-propionic acid;
(S)-2-(3-Acetyl-2,5-dimethyl-pyrrol-1-yl)-3-{4-[2-(4-methyl-2-phenyl-oxazol-5-yl)-ethoxy]-phenyl}-propionic acid;
(S)-2-[2,5-Dimethyl-3-(2,2,2-trifluoro-acetyl)-pyrrol-1-yl]-3-{4-[2-(5-methyl-2-phenyl-oxazol-4-yl)-ethoxy]-phenyl}-propionic acid;
3-{4-[2-(2-Phenyl-benzimidazol-1-yl)-ethoxy]-phenyl}-2-pyrrol-1-yl-propionic acid;
2-(3-Bromo-pyrrol-1-yl)-3-{4-[2-(5-methyl-2-phenyl-oxazol-4-yl)-ethoxy]-phenyl}-propionic acid;
2-(2-Bromo-pyrrol-1-yl)-3-{4-[2-(5-methyl-2-phenyl-oxazol-4-yl)-ethoxy]-phenyl}-propionic acid;
2-(2-Chloro-pyrrol-1-yl)-3-{4-[2-(5-methyl-2-phenyl-oxazol-4-yl)-ethoxy]-phenyl}-propionic acid;
(S)-2-(2-Butyryl-pyrrol-1-yl)-3-{4-[2-(5-methyl-2-phenyl-oxazol-4-yl)-ethoxy]-phenyl}-propionic acid;
(S)-2-(2,5-Dimethyl-pyrrol-1-yl)-3-[4-(2-fluoro-benzyloxy)-phenyl]-propionic acid;
(S)-3-{4-[2-(5-Methyl-2-phenyl-oxazol-4-yl)-ethoxy]-phenyl}-2-(2-methyl-5-phenyl-pyrrol-1-yl)-propionic acid;
(S)-2-(2,5-Dimethyl-pyrrol-1-yl)-3-{4-[2-(5-methyl-2-phenyl-oxazol-4-yl)-ethoxy]-phenyl}-propionic acid;
3- {4-[3-(5-Methyl-2-phenyl-oxazol-4-yl)-propionyl]-phenyl}-2-pyrrol-1-yl-propionic acid;
3-{4-[2-(5-Methyl-2-phenyl-oxazol-4-yl)-ethoxy]-phenyl}-2-thiophen-2-yl-propionic acid;
3-{4-[2-(5-Methyl-2-phenyl-oxazol-4-yl)-ethoxy]-phenyl}-2-pyridin-3-yl-propionic acid;
3-{4-[2-(5-Methyl-2-phenyl-oxazol-4-yl)-ethoxy]-phenyl}-2-phenylpropionic acid;
3- {4-[2-(5-Methyl-2-phenyl-oxazol-4-yl)-ethoxy]-phenyl}-2-(3-triflorophenyl)-propionic acid;
3-{4-[2-(5-Methyl-2-phenyl-oxazol-4-yl)-ethoxy]-phenyl]-2-phenylpropionic acid; (R)
2-(4-Methoxy-phenyl)-3-{4-[2-(5-methyl-2-phenyl-oxazol-4-yl)-ethoxy]-phenyl]-propionic acid;
3-{4-[2-(5-Methyl-2-phenyl-oxazol-4-yl)-ethylamino]-phenyl}-2-pyrrol-1-yl-propionic acid;
2-Methyl-3-{4-[2-(5-methyl-2-phenyl-oxazol-4-yl)-ethoxy]-phenyl}-2-phenyl-propionic acid;
2,2-Dimethyl-3-{4-[2-(5-methyl-2-phenyl-oxazol-4-yl)-ethoxy]-phenyl}-propionic acid;
2-Fluoro-3-{4-[2-(5-methyl-2-phenyl-oxazol-4-yl)-ethoxy]-phenyl}-2-phenyl-propionic acid;
3-{4-[2-(5-Methyl-2-phenyl-oxazol-4-yl)-ethoxy]-phenyl}-2-(2-oxo-pyrrolidin-1-yl)-propionic acid;
2-Ethyl-5-(2-{4-[2-(5-methyl-2-phenyl-oxazol-4-yl)-ethoxy]-phenyl}-1-phenyl-ethyl)-1,3,4-oxadiazole
3-{4-[3-(5-Methyl-2-phenyl-oxazol-4-yl)-propionyl]-phenyl}-2-pyrrol-1-yl-propionic acid;
3-{4-[2-(5-Methyl-2-phenyl-oxazol-4-yl)-ethoxy]-phenyl}-2-pyridin-3-yl-propionic acid;
3-{4-[2-(5-Methyl-2-phenyl-oxazol-4-yl)-.ethoxy]-phenyl}-2-phenyl-propionic acid;
3- {4-[2-(5-Methyl-2-phenyl-oxazol-4-yl)-ethoxy]-phenyl}-2-(3-triflorophenyl)-propionic acid;
(R)-3-{4-[2-(5-Methyl-2-phenyl-oxazol-4-yl)-ethoxy]-phenyl}-2-phenyl-propionic acid;
S)-2-Methyl-3-{4-[2-(5-methyl-2-phenyl-oxazol-4-yl)-ethoxy]-phenyl}-2-pyrrol-1-yl-propionic acid; methyl ester
3-{5-[2-(5-Methyl-2-phenyl-oxazol-4-yl)-ethoxy]-indol-1-yl]-propionic acid;
3- {4-[2-(5-Methyl-2-phenyl-oxazol-4-yl)-ethoxy]-indol-1-yl}-propionic acid;
(S)-2-(2-{4-[2-(5-Methyl-2-phenyl-oxazol-4-yl)-ethoxy]-phenyl}-1-pyrrol-1-yl-ethyl)-5-propyl-1,3,4-oxadiazole
(S)-2-Ethyl-5-(2-{4-[2-(5-methyl-2-phenyl-oxazol-4-yl)-ethoxy]-phenyl}-1-pyrrol-1-yl-ethyl)-1,3,4-oxadiazole
(S)-2-{4-[2-(5-Methyl-2-phenyl-oxazol-4-yl)-ethoxy]-phenyl}-1-pyrrol-1-yl-ethyl)-1,3,4-oxadiazole
(S)-3-Methyl-5-(2-{4-[2-(5-methyl-2-phenyl-oxazol-4-yl)-ethoxy]-phenyl}-1-(S)-pyrrol-1-yl-ethyl)-4H-1,2,4-triazole
2-(3-Bromo-pyrrol-1-yl)-2-methyl-3-{4-[2-(5-methyl-2-phenyl-oxazol-4-yl)-ethoxy]-phenyl}-propionic acid;
3,3,3-Trifluoro-2-{4-[2-(5-methyl-2-phenyl-oxazol-4-yl)-ethoxy]-benzyl}-2-pyrrol-1-yl-propionic acid;
2-(4-Benzyloxy-indol-1-yl)-propionic acid;
3-(1-{4-[3-(5-Methyl-2-phenyl-oxazol-4-yl)-propyl]-benzyl}-1H-pyrrol-2-yl)-propionic acid;
(1-[2-(5-Methyl-2-phenyl-oxazol-4-yl)-ethyl]-1H-indol-5-yloxy)-phenyl-acetic acid;
2-(1-Methyl-1H-indol-3-yl)-3-{4-[2-(5-methyl-2-phenyl-oxazol-4-yl)-ethoxy]-phenyl}-propionic acid;
3-(4-[3-(5-Methyl-2-phenyl-oxazol-4-yl)-propoxy]-indol-1-yl)-propionic acid;
2-(4-[2-(5-Methyl-2-phenyl-oxazol-4-yl)-ethoxy]-indol-1-yl)-propionic acid;
3-(4-[2-(2-Trifluoromethyl-phenyl)-ethoxy]-indol-1-yl)-propionic acid;
(4-[3-(5-Methyl-2-phenyl-oxazol-4-yl)-propoxy]-indol-1-yl)-acetic acid;
4-[2-(5-Methyl-2-phenyl-oxazol-4-yl)-ethoxy]-indol-1-yl} -acetic acid;
4-{4-[2-(5-Methyl-2-phenyl-oxazol-4-yl)-ethoxy]-indol-1-yl}-butyric acid;
3-(3-Fluoro-4-[2-(5-methyl-2-phenyl-oxazol-4-yl)-ethoxy]-phenyl}-2-(S)-pyrrol-1-yl-propionic acid;
3-{4-[2-(5-Methyl-2-phenyl-oxazol-4-yl)-ethoxy]-phenyl}-2-pyridin-4-yl-propionic acid;
2-Methyl-5-(2-{4-[2-(5-methyl-2-phenyl-oxazol-4-yl)-ethoxy]-phenyl}-1-phenyl-ethyl)-2H-tetrazole
1-Methyl-5-(2-{4-[2-(5-methyl-2-phenyl-oxazol-4-yl)-ethoxy]-phenyl}-1-phenyl-ethyl)-1H-tetrazole
2-Methyl-3-{4-[2-(5-methyl-2-phenyl-oxazol-4-yl)-ethoxy]-phenyl}-2-thiophen-2-yl-propionic acid;
2-Methyl-3-{4-[2-(5-methyl-2-phenyl-oxazol-4-yl)-ethoxy]-phenyl}-2-thiophen-3-yl-propionic acid;
N-(2,2-Dimethyl-3-{4-[2-(5-methyl-2-phenyl-oxazol-4-yl)-ethoxy]-phenyl}-propionyl)-methanesulfonamide
N-(2-Fluoro-3-{4-[2-(5-methyl-2-phenyl-oxazol-4-yl)-ethoxy]-phenyl}-2-phenyl-propionyl)-methanesulfonamide
3-{3-[2-(5-Methyl-2-phenyl-oxazol-4-yl)-ethoxy]-isoxazol-5-yl}-2-pyrrol-1-yl-propionic acid;
2-(5-Methyl-2-phenyl-oxazol-4-yl)-3-{4-[3-(5-methyl-2-phenyl-oxazol-4-yl)-propyl]-phenyl}-propionic acid;
2-(5-Methyl-2-phenyl-oxazol-4-yl)-3-{4-[2-(5-methyl-2-phenyl-oxazol-4-yl)-ethoxy]-phenyl}-propionic acid;
3-{4-[3-(5-Methyl-2-phenyl-oxazol-4-yl)-propyl]-phenyl]-2-phenyl-propionic acid;
3-{4-[3-(5-Methyl-2-phenyloxazol-4-yl)-propyl]-phenyl}-2-phenylpropionic acid;
3-{6-[2-(5-Methyl-2-phenyl-oxazol-4-yl)-ethoxy]-pyridin-3-yl}-2-pyrrol-1-yl-propionic acid;
3-{6-[2-(5-Methyl-2-phenyl-xoazol-4-yl)-ethoxy]-yridin-3-yl]-2-phenylpropionic acid;
3-{6-[2-(5-Methyl-2-phenyl-xoazol-4-yl)-ethoxy]-yridin-3-yl]-2-phenylpropionic acid;
3-{6-[2-(5-Methyl-2-phenyl-oxazol-4-yl)-ethoxy]-pyridin-3-yl]-2-thiophen-2-yl-propionic acid;
3-{4-[2-(5-Methyl-2-phenyl-oxazol-4-yl)-ethoxy]-phenyl}-2-phenyl-acrylic acid;
{4-[2-(5-methyl-2-phenyl-oxazol-4-yl)-ethoxy]benzylamino]phenylacetic acid
Methyl-{4-[2-(5-methyl-2-phenyl-oxazol-4-yl)-ethoxy]-benzylamino}-phenylacetic acid;
6-[2-(5-Methyl-2-phenyl-oxazol-4-yl)-ethoxy]-naphthalene-2-carboxylic acid;
3-{5-[3-(5-Methyl-2-phenyl-oxazol-4-yl)-propyl]-naphthalen-1-yl}-2-pyrrol-1-yl-propionic acid;
3-{4-[3-(5-Methyl-2-phenyl-oxazol-4-yl)-propyl]-phenyl}-2-1,2,3-triazol-1-yl-propionic acid; ethyl ester
3-{4-[3-(5-Methyl-2-phenyl-oxazol-4-yl)-propyl]-phenyl}-2-1,2,3-triazol-1-yl-propionic acid;
3-{4-[3-(5-Methyl-2-phenyl-oxazol-4-yl)-propyl]-phenyl}-2-pyrazol-1-yl-propionic acid;
3-{4-[3-(5-Methyl-2-phenyl-oxazol-4-yl)-propyl]-phenyl}-2-1,2,4-triazol-1-yl-propionic acid;
5-Methyl-2-phenyl-4-prop-2-enyloxazole
3-{4-[3-(5-Methyl-2-phenyl-oxazol-4-yl)-prop-1-ynyl]-phenyl}-2-pyridin-3-yl-propionic acid;
3-{4-[3-(5-Methyl-2-phenyl-oxazol-4-yl)-propyl]-phenyl}-2-pyridin-3-yl-propionic acid;
3-{4-[3-(5-Methyl-2-phenyl-oxazol-4-yl)-propyl]-phenyl}-2-pyridin-3-yl-propionic acid;
2-Biphenyl-4-yl-3-{4-[3-(5-methyl-2-phenyl-oxazol-4-yl)-propyl]-phenyl]-propionic acid;
2-Biphenyl-4-yl-3-{4-[3-(5-methyl-2-phenyl-oxazol-4-yl)-prop-1-ynyl]-phenyl]-propionic acid;
2-Biphenyl-3-yl-3-{4-[3-(5-methyl-2-phenyl-oxazol-4-yl)-prop-1-ynyl]-phenyl}-propionic acid;
2-Biphenyl-3-yl-3-{4-[3-(5-methyl-2-phenyl-oxazol-4-yl)-propyl]-phenyl]-propionic acid;
2-(5-Methyl-isoxazol-3-yl)-3-{4-[3-(5-methyl-2-phenyl-oxazol-4-yl)-propyl]-phenyl}-propionic acid;
2-(3-Methyl-isoxazol-5-yl)-3-{4-[3-(5-methyl-2-phenyl-oxazol-4-yl)-propyl]-phenyl}-propionic acid;
(*S*)-3-(4-{[(5-Methyl-2-phenyl-oxazol-4-ylmethyl)-amino]-methyl}-phenyl)-2-pyrrol-1-yl-propionic acid;
(*S*)-3-(4-{[Acetyl-(5-methyl-2-phenyl-oxazol-4-ylmethyl)-amino]-methyl}-phenyl)-2-pyrrol-1-yl-propionic acid;
(*S*)-3-(4-{[Methyl-(5-methyl-2-phenyl-oxazol-4-ylmethyl)-amino]-methyl}-phenyl)-2-pyrrol-1-yl-propionic acid;
(*S*)-3-(4-{[2-(5-Methyl-2-phenyl-oxazol-4-yl)-ethylamino]-methyl}-phenyl)-2-pyrrol-1-yl-propionic acid;
(*S*)-3-[4-({Benzyl-[2-(5-methyl-2-phenyl-oxazol-4-yl)-ethyl]-amino}-methyl)-phenyl]-2-pyrrol-1-yl-propionic acid;
(*S*)-3-[4-({Benzoyl-[2-(5-methyl-2-phenyl-oxazol-4-yl)-ethyl]-amino}-methyl)-phenyl]-2-pyrrol-1-yl-propionic acid;
(*S*)-3-[4-({Acetyl-[2-(5-methyl-2-phenyl-oxazol-4-yl)-ethyl]-amino}-methyl)-phenyl]-2-pyrrol-1-yl-propionic acid;
4-{4-[3-(5-Methyl-2-phenyl-oxazol-4-yl)-propyl]-phenyl}-2-pyrrol-1-yl-butyric acid;
3- {1-[3-(5-Methyl-2-phenyl-oxazol-4-yl)-propyl]-piperidin-4-yl]-2-pyrrol-1-yl-propionic acid;
3-{3-Methoxy-4-[3-(5-methyl-2-phenyl-oxazol-4-yl)-propenyl]-phenyl}-2-pyrrol-1-yl-propionic acid;
3-{3-Methoxy-4-[3-(5-methyl-2-phenyl-oxazol-4-yl)-propyl]-phenyl}-2-pyrrol-1-yl-propionic acid;
3-{3-Methoxy-4-[2-(5-methyl-2-phenyl-oxazol-4-yl)-ethoxy]-phenyl}-2-pyrrol-1-yl-propionic acid;
Butane-1-sulfonic acid; (3-{4-[3-(5-methyl-2-phenyl-oxazol-4-yl)-propyl]-phenyl}-2-pyrrol-1-yl-propionyl)-amide
N-(3-{4-[3-(5-Methyl-2-phenyl-oxazol-4-yl)-propyl]-phenyl}-2-pyrrol-1-yl-propionyl)-methanesulfonamide
3-{3-Iodo-4-[2-(5-methyl-2-phenyl-oxazol-4-yl)-ethoxy]-phenyl}-2-pyrrol-1-yl-propionic acid;
3-{4-[2-(5-Methyl-2-phenyl-oxazol-4-yl)-ethoxy]-3-prop-1-ynyl-phenyl}-2-pyrrol-1-yl-propionic acid;
3-{3-Ethyl-4-[2-(5-methyl-2-phenyl-oxazol-4-yl)-ethoxy]-phenyl]-2-pyrrol-1-yl-propionic acid;
3-{6-[2-(5-Methyl-2-phenyl-oxazol-4-yl)-ethoxy]-biphenyl-3-yl}-2-pyrrol-1-yl-propionic acid;
4-{4-[3-(5-Methyl-2-phenyl-oxazol-4-yl)-propyl]-phenyl-2-pyrrol-1-yl-butyric acid;
2-Pyrrol-1-yl-4-(4-trifluoromethanesulfonyl-phenyl)-butyric acid; methyl ester
3-{1-[3-(5-Methyl-2-phenyl-oxazol-4-yl)-propyl]-piperidin-4-yl}-2-pyrrol-1-yl-propionic acid;
3-{3-Methoxy-4-[3-(5-methyl-2-phenyl-oxazol-4-yl)-propenyl]-phenyl}-2-pyrrol-1-yl-propionic acid;
3-{3-Methoxy-4-[3-(5-methyl-2-phenyl-oxazol-4-yl)-propyl]-phenyl}-2-pyrrol-1-yl-propionic acid;
3-{3-Methoxy-4-[2-(5-methyl-2-phenyl-oxazol-4-yl)-ethoxy]-phenyl}-2-pyrrol-1-yl-propionic acid;
Butane-1-sulfonic acid; (3-{4-[3-(5-methyl-2-phenyl-oxazol-4-yl)-propyl]-phenyl}-2-pyrrol-1-yl-propionyl)-amide;
N-(3-{4-[3-(5-Methyl-2-phenyl-oxazol-4-yl)-propyl]-phenyl}-2-pyrrol-1-yl-propionyl)-methanesulfonamide;
3-{3-Iodo-4-[2-(5-methyl-2-phenyl-oxazol-4-yl)-ethoxy]-phenyl}-2-pyrrol-1-yl-propionic acid;
3-{4-[2-(5-Methyl-2-phenyl-oxazol-4-yl)-ethoxy]-3-prop-1-ynyl-phenyl}-2-pyrrol-1-yl-propionic acid;
3-{3-Ethyl-4-[2-(5-methyl-2-phenyl-oxazol-4-yl)-ethoxy]-phenyl}-2-pyrrol-1-yl-propionic acid;
3-{6-[2-(5-Methyl-2-phenyl-oxazol-4-yl)-ethoxy]-biphenyl-3-yl}-2-pyrrol-1-yl-propionic acid;

The invention also provides a compound which is:
(S)-3-{4-[2-(5-Methyl-2-phenyl-oxazol-4-yl)-ethoxy]-phenyl}-2-pyrrol-1-yl-propionic acid methyl ester;
(S)-2-(2-Benzoyl-pyrrol-1-yl)-3-{4-[2-(5-methyl-2-phenyl-oxazol-4-yl)-ethoxy]-phenyl}-propionic acid methyl ester;
(S)-2-(3-Benzoyl-pyrrol-1-yl)-3-{4-[2-(5-methyl-2-phenyl-oxazol-4-yl)-ethoxy]-phenyl}-propionic acid methyl ester;
(S)-3-{4-[2-(5-Methyl-2-phenyl-oxazol-4-yl)-ethoxy]-phenyl}-2-(3-phenyl-pyrrol-1-yl)-propionic acid methyl ester;
(S)-3-{4-[2-(Benzooxazol-2-yl-methyl-amino)-ethoxy]-phenyl}-2-(3-phenyl-pyrrol-1-yl)-propionic acid methyl ester;
N-(S)-(3-{4-[2-(5-Methyl-2-phenyl-oxazol-4-yl)-ethoxy]-phenyl}-2-pyrrol-1-yl-propionyl)-Benzenesulfonamide
N-(S)-(3-{4-[2-(5-Methyl-2-phenyl-oxazol-4-yl)-ethoxy]-phenyl}-2-pyrrol-1-yl-propionyl)-Methanesulfonamide
(S)-2-(3-Isobutyryl-pyrrol-1-yl)-3-{4-[2-(5-methyl-2-phenyl-oxazol-4-yl)-ethoxy]-phenyl}-propionic acid methyl ester;
(S)-2-(3-Cyclohexanecarbonyl-pyrrol-1-yl)-3-{4-[2-(5-methyl-2-phenyl-oxazol-4-yl)-ethoxy]-phenyl}-propionic acid methyl ester;
(S)-3-{4-[2-(Benzooxazol-2-yl-methyl-amino)-ethoxy]-phenyl}-2-(3-cyclohexanecarbonyl-pyrrol-1-yl)-propionic acid methyl ester;
(S)-2-(3-Cyclohexanecarbonyl-pyrrol-1-yl)-3-[4-(2-fluoro-benzyloxy)-phenyl]-propionic acid methyl ester;
(S)-3-{4-[2-(5-Methyl-2-phenyl-oxazol-4-yl)-ethoxy]-phenyl}-2-[2-(2,2,2-trifluoro-acetyl)-pyrrol-1-yl]-propionic acid methyl ester;.
3-{4-[2-(5-Methyl-2-phenyl-oxazol-4-yl)-ethoxy]-phenyl}-2-pyridin-4-yl-propionic acid methyl ester;
2-Methyl-5-(2-{4-[2-(5-methyl-2-phenyl-oxazol-4-yl)-ethoxy]-phenyl]-1-phenyl-ethyl)-2H-tetrazole;
2-Methyl-3-{4-[2-(5-methyl-2-phenyl-oxazol-4-yl)-ethoxy]-phenyl}-2-thiophen-2-yl-propionic acid methyl ester;
N-(2,2-Dimethyl-3-{4-[2-(5-methyl-2-phenyl-oxazol-4-yl)-ethoxy]-phenyl}-propionyl)-methanesulfonamide;
N-(2-Fluoro-3-{4-[2-(5-methyl-2-phenyl-oxazol-4-yl)-ethoxy]-phenyl}-2-phenyl-propionyl)-methanesulfonamide
2-(5-Methyl-2-phenyl-oxazol-4-yl)-3-{4-[3-(5-methyl-2-phenyl-oxazol-4-yl)-propyl]-phenyl}-propionic acid methyl ester;
2-(5-Methyl-2-phenyl-oxazol-4-yl)-3-{4-[2-(5-methyl-2-phenyl-oxazol-4-yl)-ethoxy]-phenyl}-propionic acid methyl ester;
3-{4-[3-(5-Methyl-2-phenyl-oxazol-4-yl)-propyl]-phenyl]-2-phenylpropionic acid methyl ester;
(S)-2-(2-Acetyl-pyrrol-1-yl)-3-{4-[2-(5-methyl-2-phenyl-oxazol-4-yl)-ethoxy]-phenyl}-propionic acid methyl ester;
3-{4-[2-(Benzothiazol-2-ylsulfanyl)-ethoxy]-phenyl}-2-pyrrol-1-yl-propionic acid methyl ester;
(S)-2-Pyrrol-1-yl-3-{4-[2-(2-trifluoromethyl-phenyl)-ethoxy]-phenyl}-propionic acid methyl ester;
(S)-3-{4-[2-(4-Phenyl-piperazin-1-yl)-ethoxy]-phenyl}-2-pyrrol-1-yl-propionic acid methyl ester;
(S)-3-{4-[2-(5-Methyl-2-phenyl-thiazol-4-yl)-ethoxy]-phenyl}-2-pyrrol-1-yl-propionic acid methyl ester;
(S)-2-(3-Chloro-2,5-dimethyl-pyrrol-1-yl)-3-{4-[2-(5-methyl-2-phenyl-oxazol-4-yl)-ethoxy]-phenyl}-propionic acid methyl ester;
(S)-3-(4-{2-[(7-Chloro-quinolin-4-yl)-methyl-amino]-ethoxy]-phenyl)-2-pyrrol-1-yl-propionic acid methyl ester;
(S)-2-(3-Acetyl-2,5-dimethyl-pyrrol-1-yl)-3-{4-[2-(4-methyl-2-phenyl-oxazol-5-yl)-ethoxy]-phenyl]-propionic acid methyl ester;
(S)-2-[2,5-Dimethyl-3-(2,2,2-trifluoro-acetyl)-pyrrol-1-yl]-3-{4-[2-(5-methyl-2-phenyl-oxazol-4-yl)-ethoxy]-phenyl]-propionic acid methyl ester;
3-{4-[2-(2-Phenyl-benzimidazol-1-yl)-ethoxy]-phenyl}-2-pyrrol-1-yl-propionic acid methyl ester;
2-(3-Bromo-pyrrol-1-yl)-3-{4-[2-(5-methyl-2-phenyl-oxazol-4-yl)-ethoxy]-phenyl}-propionic acid methyl ester;
2-(2-Bromo-pyrrol-1-yl)-3-{4-[2-(5-methyl-2-phenyl-oxazol-4-yl)-ethoxy]-phenyl}-propionic acid methyl ester;
2-(2-Chloro-pyrrol-1-yl)-3-{4-[2-(5-methyl-2-phenyl-oxazol-4-yl)-ethoxy]-phenyl}-propionic acid methyl ester;
(S)-2-(2-Butyryl-pyrrol-1-yl)-3-{4-[2-(5-methyl-2-phenyl-oxazol-4-yl)-ethoxy]-phenyl}-propionic acid methyl ester;
(S)-2-(2,5-Dimethyl-pyrrol-1-yl)-3-[4-(2-fluoro-benzyloxy)-phenyl]-propionic acid methyl ester;
(S)-3-{4-[2-(5-Methyl-2-phenyl-oxazol-4-yl)-ethoxy]-phenyl}-2-(2-methyl-5-phenyl-pyrrol-1-yl)-propionic acid methyl ester;
(S)-2-(2,5-Dimethyl-pyrrol-1-yl)-3-{4-[2-(5-methyl-2-phenyl-oxazol-4-yl)-ethoxy]-phenyl]-propionic acid methyl ester;
3-{4-[3-(5-Methyl-2-phenyl-oxazol-4-yl)-propionyl]-phenyl}-2-pyrrol-1-yl-propionic acid methyl ester;
3-{4-[2-(5-Methyl-2-phenyl-oxazol-4-yl)-ethoxy]-phenyl}-2-thiophen-2-yl-propionic acid methyl ester;
3-{4-[2-(5-Methyl-2-phenyl-oxazol-4-yl)-ethoxy]-phenyl}-2-pyridin-3-yl-propionic acid methyl ester;
3-{4-[2-(5-Methyl-2-phenyl-oxazol-4-yl)-ethoxy]-phenyl}-2-phenylpropionic acid methyl ester;
3- {4-[2-(5-Methyl-2-phenyl-oxazol-4-yl)-ethoxy]-phenyl}-2-(3-triflorophenyl)-propionic acid methyl ester;
3-{4-[2-(5-Methyl-2-phenyl-oxazol-4-yl)-ethoxy]-phenyl}-2-phenylpropionic acid methyl ester; (R)
2-(4-Methoxy-phenyl)-3-{4-[2-(5-methyl-2-phenyl-oxazol-4-yl)-ethoxy]-phenyl]-propionic acid methyl ester;
3-{4-[2-(5-Methyl-2-phenyl-oxazol-4-yl)-ethylamino]-phenyl}-2-pyrrol-1-yl-propionic acid methyl ester;
2-Methyl-3-{4-[2-(5-methyl-2-phenyl-oxazol-4-yl)-ethoxy]-phenyl}-2-phenyl-propionic acid methyl ester;
2,2-Dimethyl-3-{4-[2-(5-methyl-2-phenyl-oxazol-4-yl)-ethoxy]-phenyl}-propionic acid methyl ester;
2-Fluoro-3-{4-[2-(5-methyl-2-phenyl-oxazol-4-yl)-ethoxy]-phenyl}-2-phenyl-propionic acid methyl ester;
3-{4-[2-(5-Methyl-2-phenyl-oxazol-4-yl)-ethoxy]-phenyl}-2-(2-oxopyrrolidin-1-yl)-propionic acid methyl ester;
2-Ethyl-5-(2-{4-[2-(5-methyl-2-phenyl-oxazol-4-yl)-ethoxy]-phenyl}-1-phenyl-ethyl)-1,3,4-oxadiazole
3- {4-[3-(5-Methyl-2-phenyl-oxazol-4-yl)-propionyl]-phenyl}-2-pyrrol-1-yl-propionic acid methyl ester;
3-{4-[2-(5-Methyl-2-phenyl-oxazol-4-yl)-ethoxy]-phenyl}-2-pyridin-3-yl-propionic acid methyl ester;
3-{4-[2-(5-Methyl-2-phenyl-oxazol-4-yl)-.ethoxy]-phenyl}-2-phenylpropionic acid methyl ester;
3-{4-[2-(5-Methyl-2-phenyl-oxazol-4-yl)-ethoxy]-phenyl}-2-(3-triflorophenyl)-propionic acid methyl ester;
(R)-3-{4-[2-(5-Methyl-2-phenyl-oxazol-4-yl)-ethoxy]-phenyl}-2-phenyl-propionic acid methyl ester;
S)-2-Methyl-3-{4-[2-(5-methyl-2-phenyl-oxazol-4-yl)-ethoxy]-phenyl}-2-pyrrol-1-yl-propionic acid methyl ester; methyl ester
3-{5-[2-(5-Methyl-2-phenyl-oxazol-4-yl)-ethoxy]-indol-1-yl}-propionic acid methyl ester;
3-{4-[2-(5-Methyl-2-phenyl-oxazol-4-yl)-ethoxy]-indol-1-yl}-propionic acid methyl ester;
(S)-2-(2-{4-[2-(5-Methyl-2-phenyl-oxazol-4-yl)-ethoxy]-phenyl}-1-pyrrol-1-yl-ethyl)-5-propyl-1,3,4-oxadiazole;
(S)-2-Ethyl-5-(2-{4-[2-(5-methyl-2-phenyl-oxazol-4-yl)-ethoxy]-phenyl}-1-pyrrol-1-yl-ethyl)-1,3,4-oxadiazole;
(S)-2-{4-[2-(5-Methyl-2-phenyl-oxazol-4-yl)-ethoxy]-phenyl}-1-pyrrol-1-yl-ethyl)-1,3,4-oxadiazole;
(S)-3-Methyl-5-(2-{4-[2-(5-methyl-2-phenyl-oxazol-4-yl)-ethoxy]-phenyl}-1-(S)-pyrrol-1-yl-ethyl)-4H-1,2,4-triazole;
2-(3-Bromo-pyrrol-1-yl)-2-methyl-3-{4-[2-(5-methyl-2-phenyl-oxazol-4-yl)-ethoxy]-phenyl}-propionic acid methyl ester;
3,3,3-Trifluoro-2-{4-[2-(5-methyl-2-phenyl-oxazol-4-yl)-ethoxy]-benzyl}-2-pyrrol-1-yl-propionic acid methyl ester;
2-(4-Benzyloxy-indol-1-yl)-propionic acid methyl ester;
3-(1-{4-[3-(5-Methyl-2-phenyl-oxazol-4-yl)-propyl]-benzyl}-1H-pyrrol-2-yl)-propionic acid methyl ester;
(1-[2-(5-Methyl-2-phenyl-oxazol-4-yl)-ethyl]-1H-indol-5-yloxy)-phenyl-acetic acid methyl ester;
2-(1-Methyl-1H-indol-3-yl)-3-{4-[2-(5-methyl-2-phenyl-oxazol-4-yl)-ethoxy]-phenyl}-propionic acid methyl ester;
3-(4-[3-(5-Methyl-2-phenyl-oxazol-4-yl)-propoxy]-indol-1-yl)-propionic acid methyl ester;
2-(4-[2-(5-Methyl-2-phenyl-oxazol-4-yl)-ethoxy]-indol-1-yl)-propionic acid methyl ester;
3-(4-[2-(2-Trifluoromethyl-phenyl)-ethoxy]-indol-1-yl)-propionic acid methyl ester;
(4-[3-(5-Methyl-2-phenyl-oxazol-4-yl)-propoxy]-indol-1-yl)-acetic acid methyl ester;
4-[2-(5-Methyl-2-phenyl-oxazol-4-yl)-ethoxy]-indol-1-yl}-acetic acid methyl ester;
4-{4-[2-(5-Methyl-2-phenyl-oxazol-4-yl)-ethoxy]-indol-1-yl}-butyric acid methyl ester;
3-(3-Fluoro-4-[2-(5-methyl-2-phenyl-oxazol-4-yl)-ethoxy]-phenyl}-2-(S)-pyrrol-1-yl-propionic acid methyl ester;
3-{4-[2-(5-Methyl-2-phenyl-oxazol-4-yl)-ethoxy]-phenyl}-2-pyridin-4-yl-propionic acid methyl ester;
2-Methyl-5-(2-{4-[2-(5-methyl-2-phenyl-oxazol-4-yl)-ethoxy]-phenyl}-1-phenyl-ethyl)-2H-tetrazole
1-Methyl-5-(2-{4-[2-(5-methyl-2-phenyl-oxazol-4-yl)-ethoxy]-phenyl}-1-phenyl-ethyl)-1H-tetrazole
2-Methyl-3-{4-[2-(5-methyl-2-phenyl-oxazol-4-yl)-ethoxy]-phenyl}-2-thiophen-2-yl-propionic acid methyl ester;
2-Methyl-3-{4-[2-(5-methyl-2-phenyl-oxazol-4-yl)-ethoxy]-phenyl}-2-thiophen-3-yl-propionic acid methyl ester;
N-(2,2-Dimethyl-3-{4-[2-(5-methyl-2-phenyl-oxazol-4-yl)-ethoxy]-phenyl}-propionyl)-methanesulfonamide
N-(2-Fluoro-3-{4-[2-(5-methyl-2-phenyl-oxazol-4-yl)-ethoxy]-phenyl}-2-phenyl-propionyl)-methanesulfonamide
3-{3-[2-(5-Methyl-2-phenyl-oxazol-4-yl)-ethoxy]-isoxazol-5-yl}-2-pyrrol-1-yl-propionic acid methyl ester;
2-(5-Methyl-2-phenyl-oxazol-4-yl)-3-{4-[3-(5-methyl-2-phenyl-oxazol-4-yl)-propyl]-phenyl}-propionic acid methyl ester;
2-(5-Methyl-2-phenyl-oxazol-4-yl)-3-{4-[2-(5-methyl-2-phenyl-oxazol-4-yl)-ethoxy]-phenyl]-propionic acid methyl ester;
3-{4-[3-(5-Methyl-2-phenyl-oxazol-4-yl)-propyl]-phenyl]-2-phenyl-propionic acid methyl ester;
3-{4-[3-(5-Methyl-2-phenyloxazol-4-yl)-propyl]-phenyl}-2-phenylpropionic acid methyl ester;
3-{6-[2-(5-Methyl-2-phenyl-oxazol-4-yl)-ethoxy]-pyridin-3-yl}-2-pyrrol-1-yl-propionic acid methyl ester;
3-{6-[2-(5-Methyl-2-phenyl-xoazol-4-yl)-ethoxy]-yridin-3-yl}-2-phenylpropionic acid methyl ester;
3-{6-[2-(5-Methyl-2-phenyl-xoazol-4-yl)-ethoxy]-yridin-3-yl}-2-phenylpropionic acid methyl ester;
3-{6-[2-(5-Methyl-2-phenyl-oxazol-4-yl)-ethoxy]-pyridin-3-yl]-2-thiophen-2-yl-propionic acid methyl ester;
3-{4-[2-(5-Methyl-2-phenyl-oxazol-4-yl)-ethoxy]-phenyl}-2-phenyl-acrylic acid methyl ester;
{4-[2-(5-methyl-2-phenyl-oxazol-4-yl)-ethoxy]-benzylamino}-phenylacetic acid methyl ester;
{4-[2-(5-methyl-2-phenyl-oxazol-4-yl)-ethoxy]-benzylamino]-phenylacetic acid methyl ester; methyl ester
Methyl- {4-[2-(5-methyl-2-phenyl-oxazol-4-yl)-ethoxy]-benzylamino}-phenylacetic acid methyl ester;
6-[2-(5-Methyl-2-phenyl-oxazol-4-yl)-ethoxy]-naphthalene-2-carboxylic acid methyl ester;
3-{5-[3-(5-Methyl-2-phenyl-oxazol-4-yl)-propyl]-naphthalen-1-yl]-2-pyrrol-1-yl-propionic acid methyl ester;
3-{4-[3-(5-Methyl-2-phenyl-oxazol-4-yl)-propyl]-phenyl}-2-1,2,3-triazol-1-yl-propionic acid methyl ester; ethyl ester
3-{4-[3-(5-Methyl-2-phenyl-oxazol-4-yl)-propyl]-phenyl}-2-1,2,3-triazol-1-yl-propionic acid methyl ester;
3-{4-[3-(5-Methyl-2-phenyl-oxazol-4-yl)-propyl]-phenyl}-2-pyrazol-1-yl-propionic acid methyl ester;
3-{4-[3-(5-Methyl-2-phenyl-oxazol-4-yl)-propyl]-phenyl}-2-1,2,4-triazol-1-yl-propionic acid methyl ester;
5-Methyl-2-phenyl-4-prop-2-enyloxazole
3--{4-[3-(5-Methyl-2-phenyl-oxazol-4-yl)-prop-1-ynyl]-phenyl}-2-pyridin-3-yl-propionic acid methyl ester;
3-{4-[3-(5-Methyl-2-phenyl-oxazol-4-yl)-propyl]-phenyl}-2-pyridin-3-yl-propionic acid methyl ester;
3-{4-[3-(5-Methyl-2-phenyl-oxazol-4-yl)-propyl]-phenyl}-2-pyridin-3-yl-propionic acid methyl ester;
2-Biphenyl-4-yl-3-{4-[3-(5-methyl-2-phenyl-oxazol-4-yl)-propyl]-phenyl}-propionic acid methyl ester;
2-Biphenyl-4-yl-3-{4-[3-(5-methyl-2-phenyl-oxazol-4-yl)-prop-1-ynyl]-phenyl}-propionic acid methyl ester;
2-Biphenyl-3-yl-3-{4-[3-(5-methyl-2-phenyl-oxazol-4-yl)-prop-1-ynyl]-phenyl]-propionic acid methyl ester;
2-Biphenyl-3-yl-3-{4-[3-(5-methyl-2-phenyl-oxazol-4-yl)-propyl]-phenyl}-propionic acid methyl ester;
2-(5-Methyl-isoxazol-3-yl)-3-{4-[3-(5-methyl-2-phenyl-oxazol-4-yl)-propyl]-phenyl}-propionic acid methyl ester;
2-(3-Methyl-isoxazol-5-yl)-3-{4-[3-(5-methyl-2-phenyl-oxazol-4-yl)-propyl]-phenyl}-propionic acid methyl ester;
(*S*)-3-(4-{[(5-Methyl-2-phenyl-oxazol-4-ylmethyl)-amino]-methyl}-phenyl)-2-pyrrol-1-yl-propionic acid methyl ester;
(*S*)-3-(4-{[Acetyl-(5-methyl-2-phenyl-oxazol-4-ylmethyl)-amino]-methyl]-phenyl)-2-pyrrol-1-yl-propionic acid methyl ester;
(*S*)-3-(4-{[Methyl-(5-methyl-2-phenyl-oxazol-4-ylmethyl)-amino]-methyl}-phenyl)-2-pyrrol-1-yl-propionic acid methyl ester;
(*S*)-3-(4-{[2-(5-Methyl-2-phenyl-oxazol-4-yl)-ethylamino]-methyl}-phenyl)-2-pyrrol-1-yl-propionic acid methyl ester;
(*S*)-3-[4-({Benzyl-[2-(5-methyl-2-phenyl-oxazol-4-yl)-ethyl]-amino}-methyl)-phenyl]-2-pyrrol-1-yl-propionic acid methyl ester;
(*S*)-3-[4-({Benzoyl-[2-(5-methyl-2-phenyl-oxazol-4-yl)-ethyl]-amino}-methyl)-phenyl]-2-pyrrol-1-yl-propionic acid methyl ester;
(*S*)- 3-[4-({Acetyl-[2-(5-methyl-2-phenyl-oxazol-4-yl)-ethyl]-amino}-methyl)-phenyl]-2-pyrrol-1-yl-propionic acid methyl ester;
4-{4-[3-(5-Methyl-2-phenyl-oxazol-4-yl)-propyl]-phenyl}-2-pyrrol-1-yl-butyric acid methyl ester;
3- {1-[3-(5-Methyl-2-phenyl-oxazol-4-yl)-propyl]-piperidin-4-yl}-2-pyrrol-1-yl-propionic acid methyl ester;
3-{3-Methoxy-4-[3-(5-methyl-2-phenyl-oxazol-4-yl)-propenyl]-phenyl]-2-pyrrol-1-yl-propionic acid methyl ester;
3-{3-Methoxy-4-[3-(5-methyl-2-phenyl-oxazol-4-yl)-propyl]-phenyl}-2-pyrrol-1-yl-propionic acid methyl ester;
3-{3-Methoxy-4-[2-(5-methyl-2-phenyl-oxazol-4-yl)-ethoxy]-phenyl}-2-pyrrol-1-yl-propionic acid methyl ester;
Butane-1-sulfonic acid methyl ester; (3-{4-[3-(5-methyl-2-phenyl-oxazol-4-yl)-propyl]-phenyl}-2-pyrrol-1-yl-propionyl)-amide
N-(3-{4-[3-(5-Methyl-2-phenyl-oxazol-4-yl)-propyl]-phenyl}-2-pyrrol-1-yl-propionyl)-methanesulfonamide
3-{3-Iodo-4-[2-(5-methyl-2-phenyl-oxazol-4-yl)-ethoxy]-phenyl}-2-pyrrol-1-yl-propionic acid methyl ester;
3-{4-[2-(5-Methyl-2-phenyl-oxazol-4-yl)-ethoxy]-3-prop-1-ynyl-phenyl}-2-pyrrol-1-yl-propionic acid methyl ester;
3-{3-Ethyl-4-[2-(5-methyl-2-phenyl-oxazol-4-yl)-ethoxy]-phenyl]-2-pyrrol-1-yl-propionic acid methyl ester;
3-{6-[2-(5-Methyl-2-phenyl-oxazol-4-yl)-ethoxy]-biphenyl-3-yl}-2-pyrrol-1-yl-propionic acid methyl ester;
4-{4-[3-(5-Methyl-2-phenyl-oxazol-4-yl)-propyl]-phenyl}-2-pyrrol-1-yl-butyric acid methyl ester;
2-Pyrrol-1-yl-4-(4-trifluoromethanesulfonyl-phenyl)-butyric acid methyl ester; methyl ester
3-{1-[3-(5-Methyl-2-phenyl-oxazol-4-yl)-propyl]-piperidin-4-yl}-2-pyrrol-1-yl-propionic acid methyl ester;
3-{3-Methoxy-4-[3-(5-methyl-2-phenyl-oxazol-4-yl)-propenyl]-phenyl]-2-pyrrol-1-yl-propionic acid methyl ester;
3-{3-Methoxy-4-[3-(5-methyl-2-phenyl-oxazol-4-yl)-propyl]-phenyl}-2-pyrrol-1-yl-propionic acid methyl ester;
3-{3-Methoxy-4-[2-(5-methyl-2-phenyl-oxazol-4-yl)-ethoxy]-phenyl}-2-pyrrol-1-yl-propionic acid methyl ester;
Butane-1-sulfonic acid methyl ester; (3-{4-[3-(5-methyl-2-phenyl-oxazol-4-yl)-propyl]-phenyl}-2-pyrrol-1-yl-propionyl)-amide
N-(3-{4-[3-(5-Methyl-2-phenyl-oxazol-4-yl)-propyl]-phenyl}-2-pyrrol-1-yl-propionyl)-methanesulfonamide
3-{3-Iodo-4-[2-(5-methyl-2-phenyl-oxazol-4-yl)-ethoxy]-phenyl}-2-pyrrol-1-yl-propionic acid methyl ester;
3-{4-[2-(5-Methyl-2-phenyl-oxazol-4-yl)-ethoxy]-3-prop-1-ynyl-phenyl}-2-pyrrol-1-yl-propionic acid methyl ester;
3-{3-Ethyl-4-[2-(5-methyl-2-phenyl-oxazol-4-yl)-ethoxy]-phenyl}-2-pyrrol-1-yl-propionic acid methyl ester;
3-{6-[2-(5-Methyl-2-phenyl-oxazol-4-yl)-ethoxy]-biphenyl-3-yl}-2-pyrrol-1-yl-propionic acid methyl ester;

The invention also provides a pharmaceutical composition comprising a compound of formula I, II, or III admixed with a carrier, diluent, or excipient.

The invention also provides a method of treating, preventing or controlling non-insulin dependent diabetes mellitus in a mammal comprising administering to the mammal in need thereof an effective amount of a compound of formula I, II, or III.

The invention also provides a method of treating, preventing or controlling obesity in a mammal comprising administering to the mammal in need thereof an effective amount of a compound of formula I, II, or III.

The invention also provides a method of reducing body weight in an obese mammal comprising administering to the mammal in need thereof an effective amount of a compound of formula I, II, or III

The invention also provides a method of treating, preventing or controlling hyperglycemia in a mammal comprising administering to the mammal in need thereof an effective amount of a compound of formula I, II, or III

The invention also provides a method of treating, preventing or controlling hyperlipidemia in a mammal comprising administering to the mammal in need thereof an effective amount of a compound of formula I, II, or III.

The invention also provides a method of treating, preventing or controlling hypercholesteremia in a mammal comprising administering to the mammal in need thereof an effective amount of a compound of formula I, II, or III.

The invention also provides a method of treating, preventing or controlling atherosclerosis in a mammal comprising administering to the mammal in need thereof an effective amount of a compound of formula I, II, or III.

The invention also provides a method of treating, preventing or controlling hypertriglyceridemia in a mammal comprising administering to the mammal in need thereof an effective amount of a compound of formula I, II, or III.

The invention also provides a method of treating, preventing or controlling hyperinsulinemia in a mammal comprising administering to the mammal in need thereof an effective amount of a compound of formula I, II, or III.

The invention also provides a method of treating a patient suffering from abnormal insulin and/or evidence of glucose disorders associated with circulating glucocorticoids, growth hormone, catecholamines, glucagon, or parathyroid hormone, comprising administering to the patient a therapeutically effective amount of a compound of of formula I, II, or III.

The invention also provides a method of treating insulin resistance syndrome in humans comprising administering to a patient in need of treatment a composition of formula I, II, or III.

The invention also provides a method of modulating PPAR activity in a mammal, comprising administering to a mammal an effective amount of a PPAR modulator of formula I, II, or III.

The invention also provides a method of lowering blood glucose in a mammal, comprising administering to a mammal an effective amount of a compound of formula I, II, or III.

The invention also provides a method of modulating fat cell differentiation in a mammal, comprising administering to a mammal an effective amount of a compound of formula I, II, or III.

The invention also provides a process for preparing a compound of formula (IV) 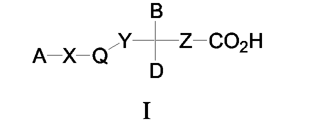 or a pharmaceutically acceptable salt thereof, wherein:
A is aryl and substituted aryl,
   heteroaryl and substituted heteroaryl,
   fused heteroaryl and substituted fused heteroaryl,
   (C₃-C₇)cycloalkyl and substituted cycloalkyl, or heterocycloalkyl and substituted heterocycloalkyl;
X is a tether 2 to 5 atoms in length selected from 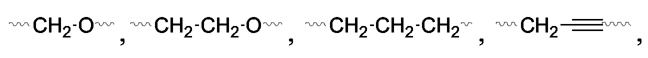 or 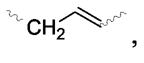 wherein " 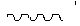 " indicates a point of attachment;
Q is aryl or substituted aryl;
   heteroaryl or substituted heteroaryl;
   fused heteroaryl, or substituted fused heteroaryl; provided that when q is a five-membered heteroaryl, the point of attachment from X to Q is not at the heteroatom of the heteroaryl;
Y and Z are independently absent or are (CR₁R₂)ₙ and (CR₃R₄)ₘ respectively,
   wherein R₁-R₄ are each independently H, halo, (C₁-C₆)alkyl, hydroxy,
   (C₁-C₆)alkoxy, and m and n are each independently 1, 2, or 3;
B is H, halo, (C₁-C₆)alkyl, halo(C₁-C₆)alkyl, or (C₁-C₆)alkoxy;
D is H, -NH-aryl or -NH-substituted aryl,
   (C₁-C₆)alkanoyl and substituted alkanoyl,
   benzoyl and substituted benzoyl,
   aryl and substituted aryl,
   heteroaryl and substituted heteroaryl,
   (C₃-C₇)cycloalkyl and substituted cycloalkyl, or
   heterocycloalkyl and substituted heterocycloalkyl;
   comprising:
   (a) hydrolyzing the ester moiety in the compound of formula I-A to an acid moiety under basic conditions.

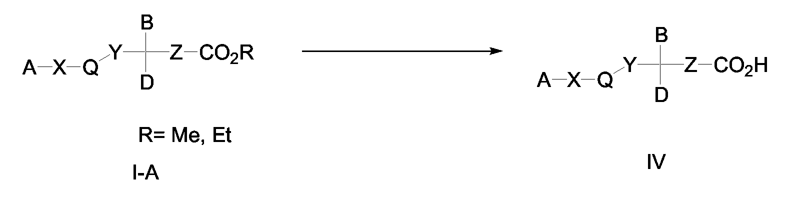

The invention also provides a process for preparing compound of formula (II) 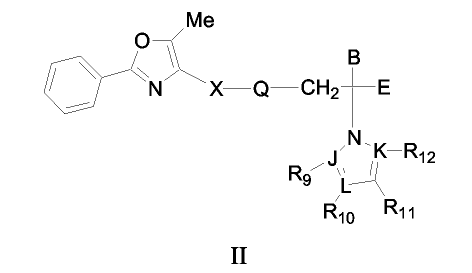 or a pharmaceutically acceptable salt thereof, wherein:
X is a tether 2 to 5 atoms in length selected from 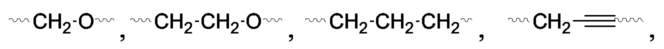 or 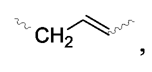 wherein " 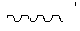 " indicates a point of attachment;
Q is aryl or substituted aryl;
   heteroaryl or substituted heteroaryl;
   fused heteroaryl, or substituted fused heteroaryl; provided that when q is a five-membered heteroaryl, the point of attachment from X to Q is not at the heteroatom of the heteroaryl;
B is H, halo, (C₁-C₆)alkyl, halo(C₁-C₆)alkyl, or (C₁-C₆)alkoxy;
E is COR₅, wherein R₅ is (C₁-C₆)alkyl, OH, (C₁-C₆)alkoxy, NR₆R₇, wherein R₆ and R₇ are each independently H or (C₁-C₆)alkyl, or one of R₆ and R₇ is H or (C₁-C₆)alkyl and the other is SO₂R₈, wherein R₈ is H or (C₁-C₆)alkyl, or E is substituted heteroaryl or 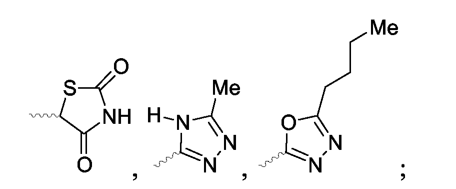
R₉-R₁₂ are each independently H, halo, aryl, (C₁-C₆)alkyl, halo(C₁-C₆)alkyl, (C₁-C₆)alkanoyl, halo(C₁-C₆)alkanoyl, (C₃-C₇)cycloalkylcarbonyl, benzoyl, or halo(C₂-C₆)alkanoyl; and
1 or 2 of J, K, and L are N,
   provided that when J, K, or L are N, R₉, R₁₀, R₁₁, or R₁₂ is absent at that position;
   comprising:
   (a) alkylating **1** with **2** and decarboxylating **2** when n=2; 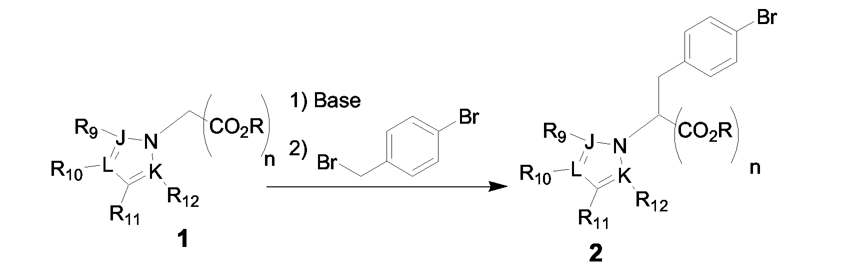 n= 1 or 2
   (b) coupling 2 with 3' to form 4; 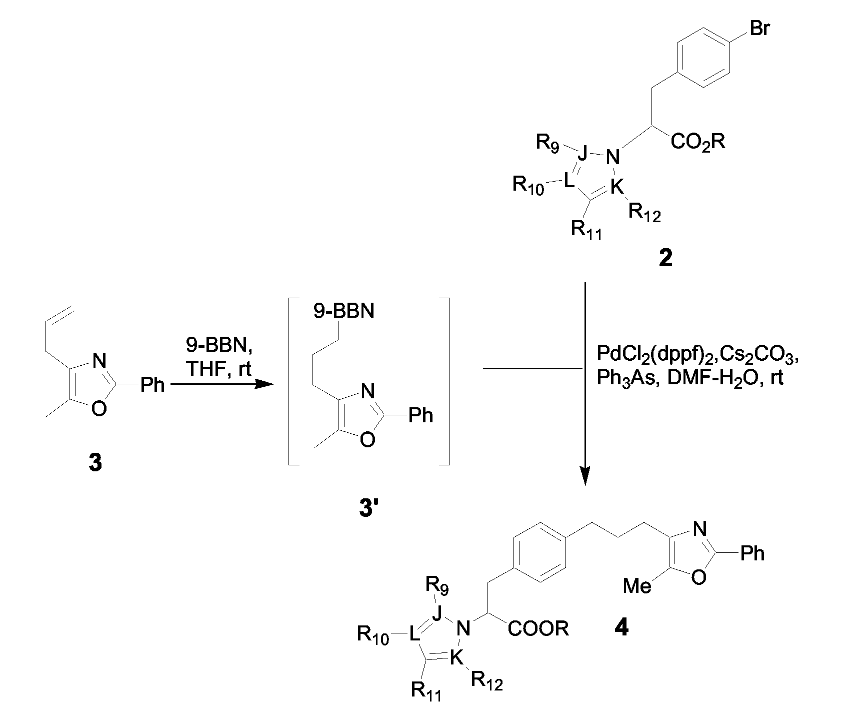
   (c) hydrolyzing the ester moiety in 4 to form a compound of the invention.

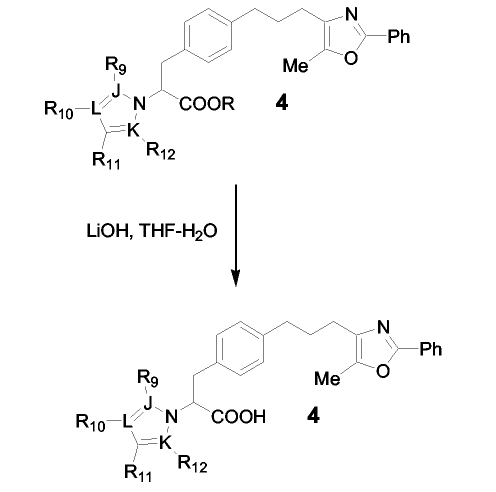

The invention also provides a process for preparing a compound of formula (III): 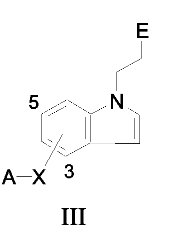 or a pharmaceutically acceptable salt thereof, wherein:
A is aryl and substituted aryl,
   heteroaryl and substituted heteroaryl,
   fused heteroaryl and substituted fused heteroaryl,
   (C₃-C₇)cycloalkyl and substituted cycloalkyl, or
   heterocycloalkyl and substituted heterocycloalkyl;
X is a tether 2 to 5 atoms in length selected from 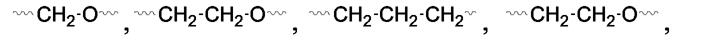 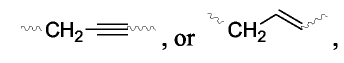 wherein " 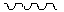 " indicates a point of attachment, and wherein -XA can be attached, to the 3, 4, 5, or 6 position of the indolinyl core; and
E is COR₅, wherein R₅ is (C₁-C₆)alkyl, OH, (C₁-C₆)alkoxy, NR₆R₇, wherein R₆ and R₇ are each independently H or (C₁-C₆)alkyl, or one of R₆ and R₇ is H or (C₁-C₆)alkyl and the other is SO₂R₈, wherein R₈ is H or (C₁-C₆)alkyl, or E is substituted heteroaryl or 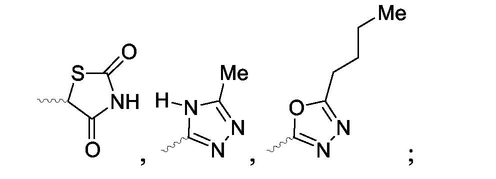 comprising:
   (a) preparing compound 6 by coupling beta hydroxy ester 6 with indole derivative 5: 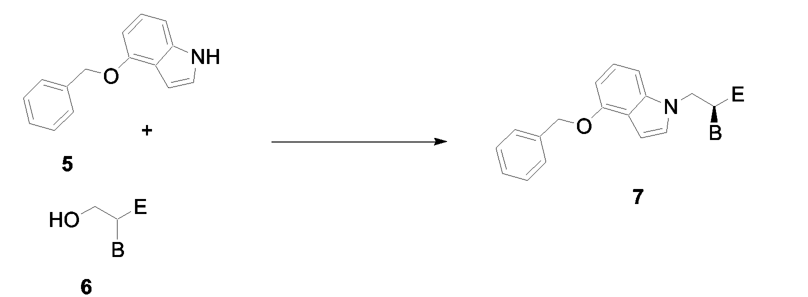
   (b) removing the benzyl group in 7 to provide 8; 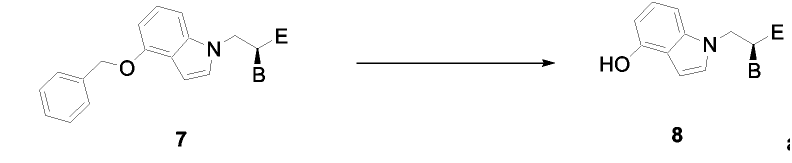 and
   (c) coupling 8 with 9 wherein XX is halo, OH, a mesylate, or a tosylate, to provide 10.

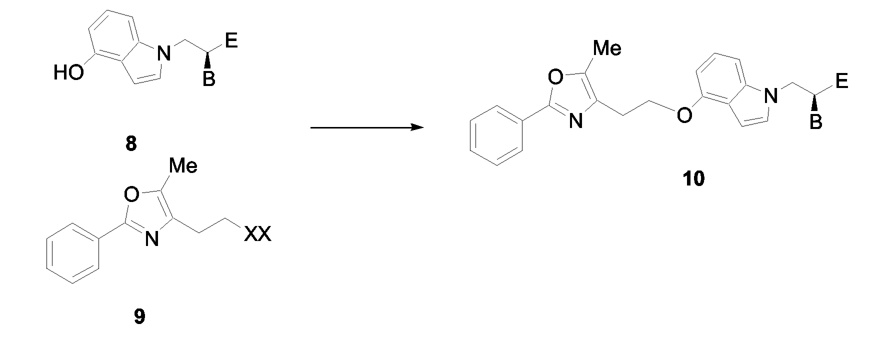

### DETAILED DESCRIPTION OF THE INVENTION

The following definitions are used, unless otherwise described: halo is fluoro, chloro, bromo, or iodo. Alkyl, alkoxy, alkenyl, alkynyl, etc. denote both straight and branched groups; but reference to an individual radical such as "propyl" embraces only the straight chain radical, a branched chain isomer such as "isopropyl" being specifically referred to.

The term "(C₁-C₆)alkyl" means a straight or branched hydrocarbon radical having from 1 to 6 carbon atoms and includes, for example, methyl, ethyl, n-propyl, isopropyl, n-butyl, sec-butyl, isobutyl, tert-butyl, n-pentyl, and n-hexyl.

The term "heterocycloalkyl" means a monocyclic, fused, bridged, or spiro bicyclic heterocyclic ring system. Monocyclic heterocyclic rings contain from about 3 to 12 ring atoms, with from 1 to 5 heteroatoms selected from N, O, and S, and preferably from 3 to 7 member atoms, in the ring. Bicyclic heterocyclics contain from 7 to 17 member atoms, preferably 7 to 12 member atoms, in the ring. Bicyclic heterocyclics contain from about 7 to about 17 ring atoms, preferably from 7 to 12 ring atoms. Bicyclic heterocyclics rings may be fused, spiro, or bridged ring systems. Examples of heterocyclic groups include cyclic ethers (oxiranes) such as ethyleneoxide, tetrahydrofuran, dioxane, and substituted cyclic ethers, wherein the substituents are those described above for the alkyl and cycloalkyl groups. Typical substituted cyclic ethers include propyleneoxide, phenyloxirane (styrene oxide), cis-2-butene-oxide (2,3-dimethyloxirane), 3-chlorotetrahydrofuran, 2,6-dimethyl-1,4-dioxane, and the like. Heterocycloalyl groups containing nitrogen are groups such as pyrrolidine, piperidine, piperazine, tetrahydrotriazine, tetrahydropyrazole, and substituted groups such as 3-aminopyrrolidine, 4-methylpiperazin-1-yl, and the like. Typical sulfur containing heterocycles include tetrahydrothiophene, dihydro-1,3-dithiol-2-yl, and hexahydrothiepin-4-yl. Other commonly employed heterocycles include dihydro-oxathiol-4-yl, tetrahydro-oxazolyl, tetrahydro-oxadiazolyl, tetrahydro-dioxazolyl, tetrahydro-oxathiazolyl, hexahydrotriazinyl, tetrahydro-oxazinyl, morpholinyl, thiomorpholinyl, tetrahydropyrimidinyl, dioxolinyl, octahydrobenzofuranyl, octahydrobenzimidazolyl, and octahydrobenzothiazolyl. For heterocycles containing sulfur, the oxidized sulfur heterocycles containing SO or SO₂ groups are also included. Examples include the sulfoxide and sulfone forms of tetrahydrothiophene.

The term "aryl" means a cyclic or polycyclic aromatic ring having from 5 to 12 carbon atoms, and being unsubstituted or substituted with up to 3 of the substituent groups recited above for alkyl, alkenyl, and alkynyl. Examples of aryl groups include phenyl, 2,6-dichlorophenyl, 3-methoxyphenyl, naphthyl, 4-thionaphthyl, tetralinyl, anthracinyl, phenanthrenyl, benzonaphthenyl, fluorenyl, 2-acetamidofluoren-9-yl, and 4'-bromobiphenyl.

The term "heteroaryl" means an aromatic cyclic or fused polycyclic ring system having from 1 to 8 heteroatoms selected from N, O, and S. The heteroaryl groups or fused heteroaryl groups may be unsubstituted or substituted by 1 to 3 substituents selected from those described above for alkyl, alkenyl, and alkynyl, for example, cyanothienyl and formylpyrrolyl.

Typical heteroaryl groups include 2- or 3-thienyl, 2- or 3-furyl, 2- or 3-pyrrolyl, 2-, 4-, or 5-imidazolyl, 3-, 4-, or 5-pyrazolyl, 2-, 4-, or 5-thiazolyl, 3-, 4-, or 5-isothiazolyl, 2-, 4-, or 5-oxazolyl, 3-, 4-, or 5-isoxazolyl, 3- or 5-1,2,4-triazolyl, 4- or 5-1,2,3-triazolyl, tetrazolyl, 2-, 3-, or 4-pyridyl, 3- or 4-pyridazinyl, 3-, 4-, or 5-pyrazinyl, 2-pyrazinyl, 2-, 4-, or 5-pyrimidinyl.

Aromatic fused heteroaryl groups of from 8 to 20 atoms include but are not limited to 1-, 2-, 3-, 5-, 6-, 7-, or 8-indolizinyl, 1-, 3-, 4-, 5-, 6-, or 7-isoindolyl, 2-, 3-, 4-, 5-, 6-, or 7-indolyl, 2-, 3-, 4-, 5-, 6-, or 7-indazolyl, 2-, 4-, 5-, 6-, 7-, or 8-purinyl, 1-, 2-, 3-, 4-, 6-, 7-, 8-, or 9-quinolizinyl, 2-, 3-, 4-, 5-, 6-, 7-, or 8-quinoliyl, 1-, 3-, 4-, 5-, 6-, 7-, or 8-isoquinoliyl, 1-, 4-, 5-, 6-, 7-, or 8-phthalazinyl, 2-, 3-, 4-, 5-, or 6-naphthyridinyl, 2-, 3-, 5-, 6-, 7-, or 8-quinazolinyl, 3-, 4-, 5-, 6-, 7-, or 8-cinnolinyl, 2-, 4-, 6-, or 7-pteridinyl, 1-, 2-, 3-, 4-, 5-, 6-, 7-, or 8-4a*H* carbazolyl, 1-, 2-, 3-, 4-, 5-, 6-, 7-, or 8-carbzaolyl, 1-, 3-, 4-, 5-, 6-, 7-, 8-, or 9-carbolinyl, 1-, 2-, 3-, 4-, 6-, 7-, 8-, 9-, or 10-phenanthridinyl, 1-, 2-, 3-, 4-, 5-, 6-, 7-, 8-, or 9-acridinyl, 1-, 2-, 4-, 5-, 6-, 7-, 8-, or 9-perimidinyl, 2-, 3-, 4-, 5-, 6-, 8-, 9-, or 10-phenathrolinyl, 1-, 2-, 3-, 4-, 6-, 7-, 8-, or 9-phenazinyl, 1-, 2-, 3-, 4-, 6-, 7-, 8-, 9-, or 10-phenothiazinyl, 1-, 2-, 3-, 4-, 6-, 7-, 8-, 9-, or 10-phenoxazinyl, 2-, 3-, 4-, 5-, 6-, or 1-, 3-, 4-, 5-, 6-, 7-, 8-, 9-, or 10-benzisoqinolinyl, 2-, 3-, 4-, or thieno[2,3-*b*]furanyl, 2-, 3-, 5-, 6-, 7-, 8-, 9-, 10-, or 11-*7H*-pyrazino[2,3-*c*]carbazolyl,2-, 3-, 5-, 6-, or 7-2*H*-furo[3,2-*b*]-pyranyl, 2-, 3-, 4-, 5-, 7-, or 8-5H-pyrido[2,3-*d*]-*o*-oxazinyl, 1-, 3-, or 5-1*H*-pyrazolo[4,3-*d*]-oxazolyl, 2-, 4-, or 5-4*H*-imidazo[4,5-*d*]thiazolyl, 3-, 5-, or 8-pyrazino[2,3-*d*]pyridazinyl, 2-, 3-, 5-, or 6-imidazo[2,1-*b*]thiazolyl, 1-, 3-, 6-, 7-, 8-, or 9-furo[3,4-c]cinnolinyl, 1-, 2-, 3-, 4-, 5-, 6-, 8-, 9-, 10, or 11-4*H*-pyrido[2,3-*c*]carbazolyl, 2-, 3-, 6-, or 7-imidazo[1,2-b][1,2,4]triazinyl, 7-benzo[b]thienyl, 2-, 4-, 5-, 6-, or 7-benzoxazolyl, 2-, 4-, 5-, 6-, or 7-benzimidazolyl, 2-, 4-, 5-, 6-, or 7-benzothiazolyl, 1-, 2-, 4-, 5-, 6-, 7-, 8-, or 9-benzoxapinyl, 2-, 4-, 5-, 6-, 7-, or 8-benzoxazinyl, 1-, 2-, 3-, 5-, 6-, 7-, 8-, 9-, 10-, or 11-1*H*-pyrrolo[1,2-b] [2]benzazapinyl. Typical fused heteroary groups include, but are not limited to 2-, 3-, 4-, 5-, 6-, 7-, or 8-quinolinyl, 1-, 3-, 4-, 5-, 6-, 7-, or 8-isoquinolinyl, 2-, 3-, 4-, 5-, 6-, or 7-indolyl, 2-, 3-, 4-, 5-, 6-, or 7-benzo[b]thienyl, 2-, 4-, 5-, 6-, or 7-benzoxazolyl, 2-, 4-, 5-, 6-, or 7-benzimidazolyl, 2-, 4-, 5-, 6-, or 7-benzothiazolyl.

Other heteroaryl groups include the following groups wherein " 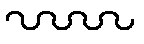 " indicates the point of attachment. 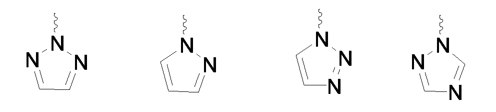 The alkyl, cycloalkyl, heterocycloalkyl, aryl, and heteroaryl can be substituted with 1 to 4 groups selected from halo, hydroxy, cyano, C₁-C₆ alkoxy, nitro, nitroso, amino, C₁-C₆ alkylamino, di-C₁-C₆ alkylamino, carboxy, C₁-C₆ C₁-C₆ alkanoyl, C₁-C₆ alkoxycarbonyl, aminocarbonyl, halomethyl, dihalomethyl, trihalomethyl, haloethyl, dihaloethyl, trihaloethyl, tetrahaloethyl, pentahaloethyl, thiol, (C₁-C₄)alkylsulfanyl, (C₁-C₄)alkylsulfinyl, and aminosulfonyl. Examples of substituted alkyl groups include fluoromethyl, tribromomethyl, hydroxymethyl, 3-methoxypropyl, 3-carboxypentyl, 3,5-dibromo-6-aminocarbonyldecyl, and 4-ethylsulfinyloctyl.

The term "prodrug" denotes a compound that is converted in vivo to an active compound. The term "prodrug group" denotes a moiety that is converted in vivo into the active compound of formula I wherein E is substituted heteroaryl or ―CO₂H. Such groups are generally known in the art and include ester forming groups, that form an ester prodrug, such as benzyloxy, di(C₁-C₆)alkylaminoethyloxy, acetoxymethyl, pivaloyloxymethyl, phthalidoyl, ethoxycarbonyloxyethyl, 5-methyl-2-oxo-1,3-dioxol-4-yl methyl, and (C₁-C₆)alkoxy optionally substituted by N-morpholino and amide-forming groups such as di(C₁-C₆)alkylamino. Other prodrug groups include C₁-C₆ alkoxy, and O-M⁺ where M⁺ represents a cation. Preferred cations include sodium, potassium, and ammonium. Other cations include magnesium and calcium. Further prodrug groups include O⁼M⁺⁺, where M⁺⁺ is a divalent cation such as magnesium or calcium.

The term "diabetes" refers to a metabolic disorder in which there is impaired glucose utilization inducing hyperglycemia. An overview of the pathogenesis and morphology of diabetes and its late complications, particularly NIDDM, is available to practitioners of the art, for instance, in Robins' Pathologic Basis of Disease (5^{th} Ed. pp. 910-922). Other metabolic disorders associated with impaired glucose utilization and insulin resistance include IRS, described previously. In addition to the major late-stage complications of NIDDM (diabetic angiopathy, atherosclerosis, diabetic nephropathy, diabetic neuropathy, and diabetic ocular complications such as retinopathy, cataract formation and glaucoma), many other conditions are linked to NIDDM, including dyslipidemia glucocortcoid induced insulin resistance, dyslipidemia, polycysitic ovarian syndrome, obesity, hyperglycemia, hyperlipidemia, hypercholerteremia, hypertriglyceridemia, hyperinsulinemia, and hypertension. Brief definitions of these conditions are available in any medical dictionary, for instance, Stedman's Medical Dictionary (Xth Ed.).

As used herein, the term "modulate" means to change (something such as an action or a process); to make it more suitable for its situation. Cambridge Dictionaries Online (http:// dictionary. cambridge. org/define. asp?key= modulate*2+0 *last visited* June 20, 2002).

It will be appreciated by those skilled in the art that compounds of the invention having a chiral center may exist in and be in optically active and racemic forms. Some compounds may exhibit polymorphism. It is to be understood that the present invention encompasses any racemic, optically-active, polymorphic, or stereoisomeric form, or mixtures thereof, of a compound of the invention, which possess the useful properties described herein, it being well known in the art how to prepare optically active forms (for example, by resolution of the racemic form by recrystallization techniques, by synthesis from optically-active starting materials, by chiral synthesis, or by chromatographic separation using a chiral stationary phase) and how to determine activity or cytotoxicity using the standard tests described herein, or using other similar tests which are well known in the art.

Specific and preferred values listed below for radicals, substituents, and ranges, are for illustration only; they do not exclude other defined values or other values within defined ranges for the radicals and substituents

Specifically, (C₁ -C₆)alkyl can be methyl, ethyl, propyl, isopropyl, butyl, iso-butyl, sec-butyl, pentyl, 3-pentyl, or hexyl; (C₁ -C₆)alkoxy can be methoxy, ethoxy, propoxy, isopropoxy, butoxy, iso-butoxy, sec-butoxy, pentoxy, 3-pentoxy, or hexyloxy; halo(C₁ -C₆)alkyl can be iodomethyl, bromomethyl, chloromethyl, fluoromethyl, trichloromethyl, trifluoromethyl, 2-chloroethyl, 2-fluoroethyl, 2,2,2-trifluoroethyl, or pentafluoroethyl; (C₃ -C₇)heterocycloalkyl can be pyrrolidinyl, piperidinyl, furanyl, pyranyl, thiofuranyl, and thiopyranyl; and heteroaryl can be furyl, imidazolyl, triazolyl, triazinyl, oxazoyl, isoxazoyl, thiazolyl, isothiazoyl, pyrazolyl, pyrrolyl, pyrazinyl, tetrazolyl, pyridyl, (or its N-oxide), thienyl, pyrimidinyl (or its N-oxide), indolyl, isoquinolyl (or its N-oxide) or quinolyl (or its N-oxide).

Referring to a compound of formula (I), a specific value for A is 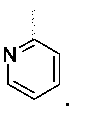 Another specific value for A is 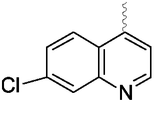 wherein " 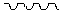 " indicates a point of attachment. Another specific value for A is 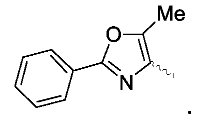 Another specific value for A is 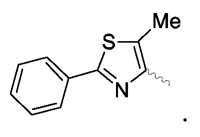 Another specific value for A is 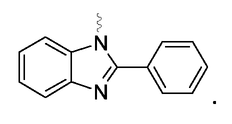 Another specific value for A is 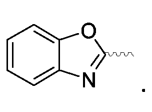 Another specific value for A is 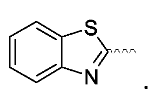 Another specific value for A is 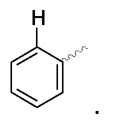 Another specific value for A is 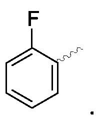 Another specific value for A is , 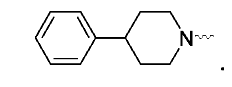 Still another specific value for A is

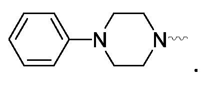

A specific value for X is 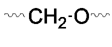 wherein " 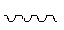 " indicates a point of attachment. Another specific value for X is 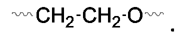 Another specific value for X is 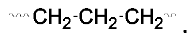 Another specific value for X is 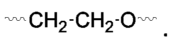 Another specific value for X is 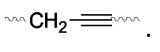 Still another specific value for X is

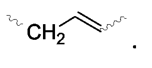

A specific value for Q is 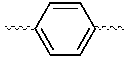 wherein " 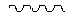 " indicates a point of attachment. Another specific value for Q is 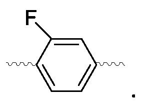 Another specific value for Q is 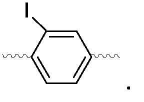 Another specific value for Q is 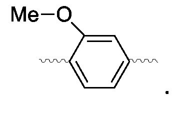 Another specific value for Q is 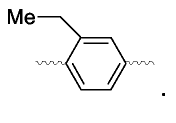 Another specific value for Q is 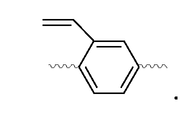 Another specific value for Q is 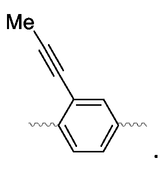 Another specific value for Q is 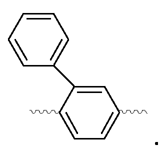 Another specific value for Q is 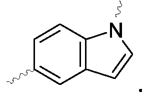 Another specific value for Q is 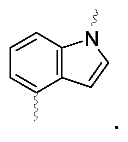 Another specific value for Q is 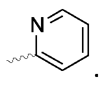 Another specific value for Q is 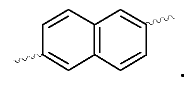 Another specific value for Q is 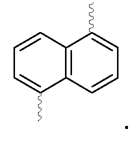 Still another specific value for Q is

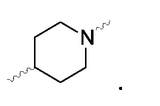

A specific value for Y is CH₂.

A specific value for Z is CH₂. Another specific value for Z is Z is absent.

A specific value for B is H. Another specific value for B is F. Another specific value for B is methyl. Another specific value for B is methoxy.

A specific value for D is H. Another specific value for D is Me. Another specific value for D is 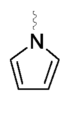 wherein " 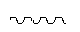 " indicates a point of attachment. Another specific value for D is 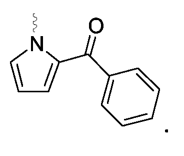 Another specific value for D is 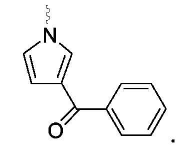 Another specific value for D is 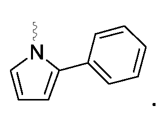 Another specific value for D is 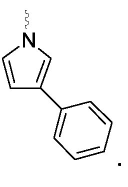 Another specific value for D is 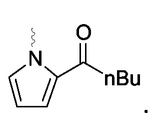 Another specific value for D is 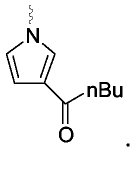 Another specific value for D is 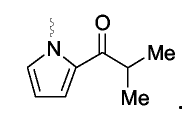 Another specific value for D is 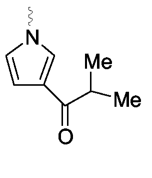 Another specific value for D is 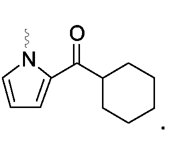 Another specific value for D is 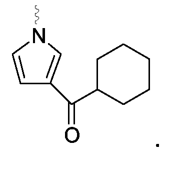 Another specific value for D is 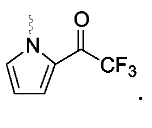 Another specific value for D is 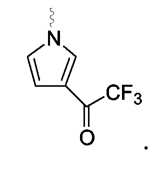 Another specific value for D is 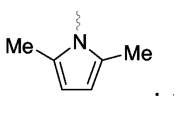 Another specific value for D is 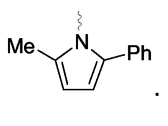 Another specific value for D is 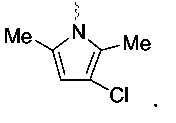 Another specific value for D is 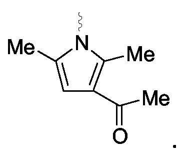 Another specific value for D is 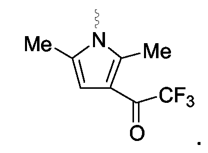 Another specific value for D is 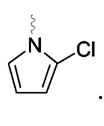 Another specific value for D is 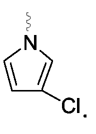 Another specific value for D is 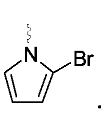 Another specific value for D is 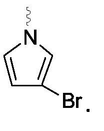 Another specific value for D is 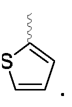 Another specific value for D is 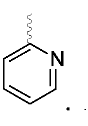 Another specific value for D is 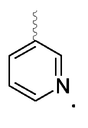 Another specific value for D is 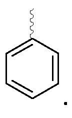 Another specific value for D is 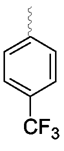 Another specific value for D is 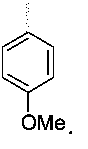 Another specific value for D is 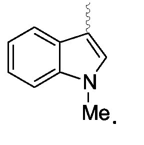 Another specific value for D is 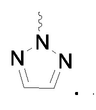 Another specific value for D is 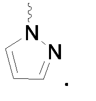 Another specific value for D is 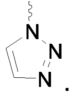 Still another specific value for D is 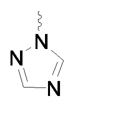 or

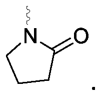

A group of compounds of the invention are compounds of formula I wherein A is aryl or substituted aryl, heteroaryl or substituted heteroaryl, fused heteroaryl or substituted fused heteroaryl, or heterocycloalkyl and substituted heterocycloalkyl; X is a tether 2 to 5 atoms in length selected from 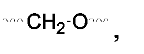 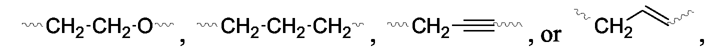 wherein " 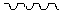 " indicates a point of attachment; Q is aryl or substituted aryl; heteroaryl or substituted heteroaryl; fused heteroaryl, or substituted fused heteroaryl; provided that when Q is a five-membered heteroaryl, the point of attachment from X to Q is not at the heteroatom of the heteroaryl; Y and Z are independently absent or are (CR₁R₂)ₙ and (CR₃R₄)ₘ, wherein R₁-R₄ are each independently H, halo, (C₁-C₆)alkyl, hydroxy, (C₁-C₆)alkoxy, and m and n are each independently 1, 2, or 3; B is H, halo, (C₁-C₆)alkyl, halo(C₁-C₆)alkyl, or (C₁-C₆)alkoxy; D is H, aryl and substituted aryl, or heteroaryl and substituted heteroaryl; E is CO₂H, CO₂Me, 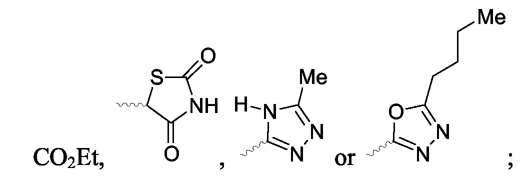 provided that when A is 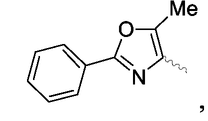 X is 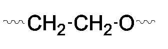 Q is phenyl, B is H, and E is CO₂H, D is not pyrrolyl.

Another group of compounds of the invention are compounds of formula I wherein A is aryl or substituted aryl, heteroaryl or substituted heteroaryl, fused heteroaryl or substituted fused heteroaryl, or heterocycloalkyl and substituted heterocycloalkyl; X is a tether 2 to 5 atoms in length selected from 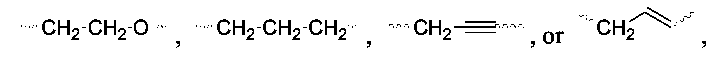 wherein " 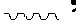 " indicates a point of attachment; Q is aryl or substituted aryl; heteroaryl or substituted heteroaryl; fused heteroaryl, or substituted fused heteroaryl; provided that when Q is a five-membered heteroaryl, the point of attachment from X to Q is not at the heteroatom of the heteroaryl; Y and Z are independently absent or are CH₂; B is H, halo, (C₁-C₆)alkyl, halo(C₁-C₆)alkyl, or (C₁-C₆)alkoxy; D is H,

heteroaryl or substituted heteroaryl; and E is CO₂H, CO₂Me, CO₂Et, 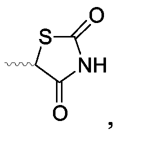 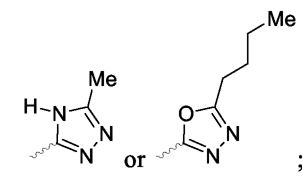 provided that when A is 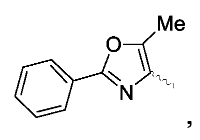 X is 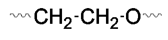 Q is phenyl, Y and Z are CH₂, B is H, and E is CO₂H, D is not pyrrolyl.

Another group of compounds of the invention are compounds of formula I wherein A is aryl or substituted aryl, heteroaryl or substituted heteroaryl, fused heteroaryl or substituted fused heteroaryl, or heterocycloalkyl and substituted heterocycloalkyl; X is a tether 2 to 5 atoms in length selected from 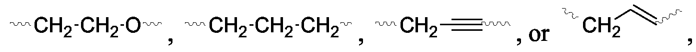 wherein " 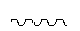 " indicates a point of attachment; Q is phenyl or substituted phenyl. naphthyl or substituted naphthyl, indolyl or substituted indolyl, pyridyl or substited pyridyl, or piperidinyl or substituted piperidinyl; Y and Z are independently absent or are CH₂; B is H, F, methyl, or methoxy. D is H, substituted pyrrolyl, pyridyl, substituted phenyl, diazoyl, or triazoyl; and E is CO₂H, CO₂Me, or CO₂Et.

Another group of compounds of the invention are compounds of formula I wherein A is aryl or substituted aryl, heteroaryl or substituted heteroaryl, fused heteroaryl or substituted fused heteroaryl, or heterocycloalkyl and substituted heterocycloalkyl; X is a tether 2 to 5 atoms in length selected from 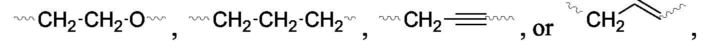 wherein " 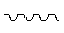 " indicates a point of attachment; Q is phenyl or substituted phenyl. naphthyl or substituted naphthyl, indolyl or substituted indolyl, pyridyl or substituted pyridyl, or piperidinyl or substituted piperidinyl; Y and Z are independently absent or are CH₂; B is H, F, methyl, or methoxy. D is H, substituted pyrrolyl, pyridyl, substituted phenyl, diazoyl, or triazoyl; and E is CO₂H, CO₂Me, or CO₂Et.

Another group of compounds of the invention are compounds of formula I wherein A is 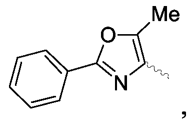 wherein " 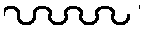 " indicates the point of attachment; X is 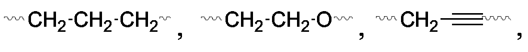 or 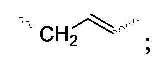 Q is aryl or substituted aryl, heteroaryl or substituted heteroaryl, or fused heteroaryl, or substituted fused heteroaryl; provided that when Q is a five-membered heteroaryl, the point of attachment from X to Q is not at the heteroatom of the heteroaryl; B is H, halo, (C₁-C₆)alkyl, halo(C₁-C₆)alkyl, or (C₁-C₆)alkoxy; D is heteroaryl or substituted heteroaryl; Y is CH₂; Z is CH₂ or is absent; E is CO₂H, CO₂Me, CO₂Et,

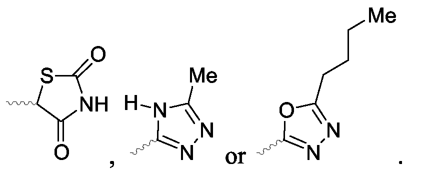

Another group of compounds of the invention are compounds of formula I wherein A is 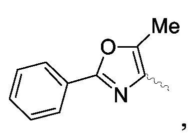 wherein " 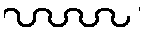 " indicates the point of attachment; X is 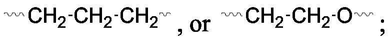 Q is aryl or substituted aryl, heteroaryl or substituted heteroaryl, or fused heteroaryl, or substituted fused heteroaryl; provided that when Q is a five-membered heteroaryl, the point of attachment from X to Q is not at the heteroatom of the heteroaryl; B is H; Y is CH₂; Z is CH₂ or is absent; D is phenyl or unsubstituted pyyrolyl or pyrrolyl substituted with 1, 2, 3, or 4 groups selected from methyl, bromo, chloro, acetyl, trifluoroacetyl, benzoyl, cyclohexanecarbonyl, and propionyl; or 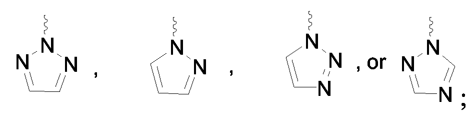 and E is CO₂H, CO₂Me, CO₂Et,

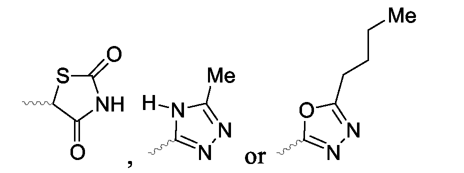

Another group of compounds of the invention are compounds of formula I wherein A is 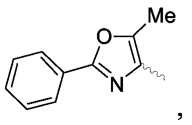 wherein " 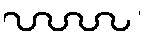 " indicates the point of attachment; X is 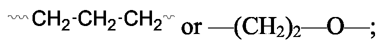 Q is phenyl, indolinyl, pyrrolyl or pyridinyl, any of which may be substituted or unsubstutited; Y is -(CH₂)₂-; Z is CH₂ or is absent; B is H; D is H, 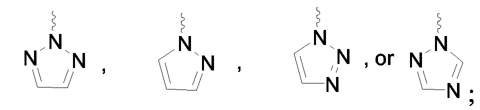 and E is CO₂H CO₂Me, CO₂Et.

Another group of compounds of the invention are compounds of formula I wherein A is 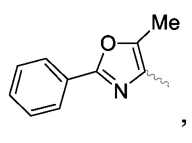 wherein " 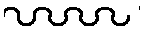 " indicates the point of attachment; X is 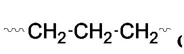 or -(CH₂)₂-_{O}; Q is 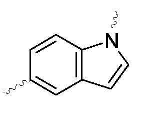 or 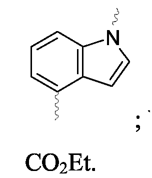 Y is -(CH₂)₂-; Z is CH₂; B is H; D is H; and E is CO₂H, CO₂Me,

Another group of compounds of the invention are compounds of formula I wherein A is 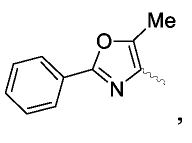 wherein " 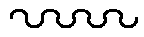 " indicates the point of attachment; X is 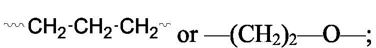 Q is 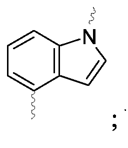 Y is -(CH₂)-; Z is absent; B is H; D is H; and E is CO₂H, CO₂Me, CO₂Et.

Another group of compounds of the invention are compounds of formula I wherein A is 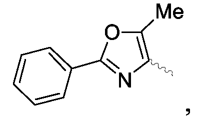 wherein " 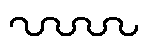 " indicates the point of attachment; X is 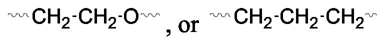 Q is 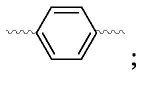 Y is CH₂; Z is absent; B is H; D is 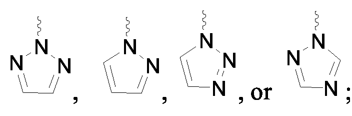 and E is CO₂H, CO₂Me, or CO₂Et.

Another group of compounds of the invention are compounds of formula I wherein A is 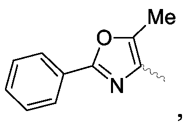 wherein " 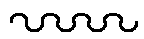 " indicates the point of attachment; X is 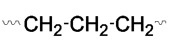 Q is 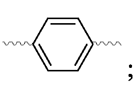 Y is CH₂; Z is absent; B is H; D is 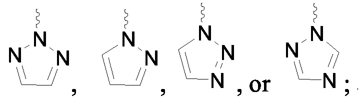 and E is CO₂H.

Another group of compounds of the invention are compounds of formula I wherein A is 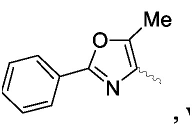 wherein " 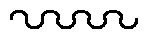 " indicates the point of attachment; X is 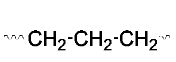 Q is 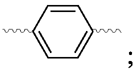 Y is CH₂; Z is absent; B is H; D is, 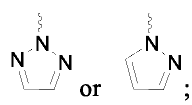 and E is CO₂H, CO₂Me, or CO₂Et.

Another group of compounds of the invention are compounds of formula I wherein A is 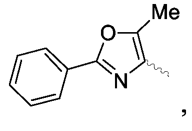 wherein " 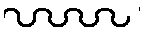 " indicates the point of attachment; X is 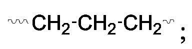 Q is 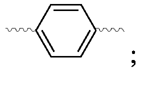 Y is CH₂; B is H; D is , 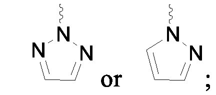 and E is CO₂H.

A group of compounds of the invention are compounds of formula II wherein X is a tether 2 to 5 atoms in length selected from 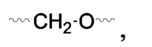 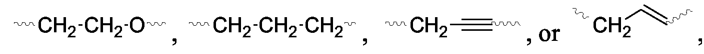 wherein " 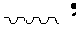 " indicates a point of attachment; Q is aryl or substituted aryl; heteroaryl or substituted heteroaryl; fused heteroaryl, or substituted fused heteroaryl; provided that when Q is a five-membered heteroaryl, the point of attachment from X to Q is not at the heteroatom of the heteroaryl; B is H, halo, (C₁-C₆)alkyl, halo(C₁-C₆)alkyl, or (C₁-C₆)alkoxy; and one or two of three of J, K and L, are N to form 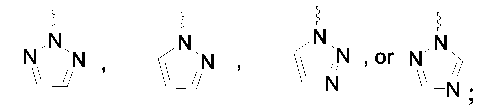 and E is COR₅, wherein R₅ is (C₁-C₆)alkyl, OH, (C₁-C₆)alkoxy, NR₆R₇, wherein R₆ and R₇ are each independently H or (C₁-C₆)alkyl, or one of R₆ and R₇ is H or (C₁-C₆)alkyl and the other is SO₂R₈, wherein R₈ is H or (C₁-C₆)alkyl, or E is substituted heteroaryl or

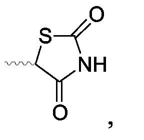

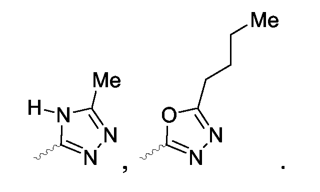

Another group of compounds of the invention are compounds of formula II wherein X is a tether 2 to 5 atoms in length selected from 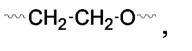 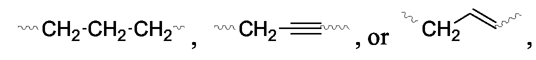 wherein " 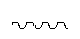 " indicates a point of attachment; Q is aryl or substituted aryl; heteroaryl or substituted heteroaryl; fused heteroaryl, or substituted fused heteroaryl; provided that when Q is a five-membered heteroaryl, the point of attachment from X to Q is not at the heteroatom of the heteroaryl; B is H, fluoro, methyl, trifluoromethyl, or methoxy; one or two of three of J, K and L, are N to form 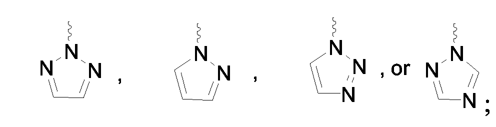 and E is CO₂H, CO₂Me, CO₂Et,

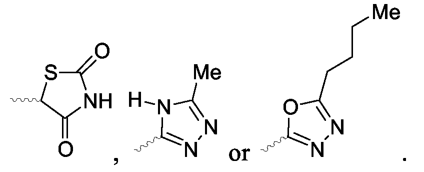

Another group of compounds of the invention are compounds of formula II wherein X is a tether 2 to 5 atoms in length selected from 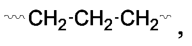 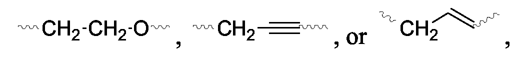 wherein " 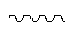 " indicates a point of attachment; Q is aryl or substituted aryl; heteroaryl or substituted heteroaryl; fused heteroaryl or substituted fused heteroaryl; provided that when Q is a five-membered heteroaryl, the point of attachment from X to Q is not at the heteroatom of the heteroaryl; B is H, fluoro, methyl, trifluoromethyl, or methoxy; one or two of three of J, K and L, are N to form 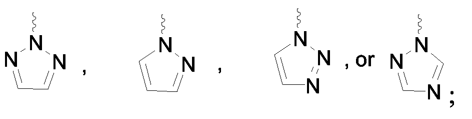 and E is CO₂H, CO₂Me, or CO₂Et.

Another group of compounds of the invention are compounds of formula II wherein X is a tether 2 to 5 atoms in length selected from 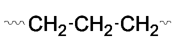 or 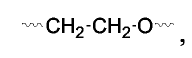 wherein " 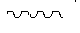 " indicates a point of attachment; Q is aryl or substituted aryl; B is H, fluoro, methyl, trifluoromethyl, or methoxy; one or two of three of J, K and L, are N to form 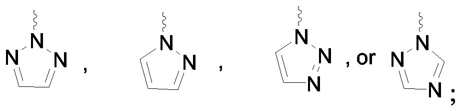 and E is CO₂H, CO₂Me, or CO₂Et.

Another group of compounds of the invention are compounds of formula II wherein X is a tether 2 to 5 atoms in length selected from 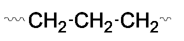 or 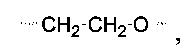 wherein " 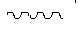 " indicates a point of attachment; Q is aryl or substituted aryl; B is H; one or two of three of J, K and L, are N to form 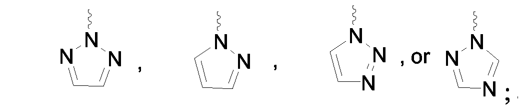 and E is CO₂H.

Another group of compounds of the invention are compounds of formula
II wherein X is a tether 2 to 5 atoms in length selected from 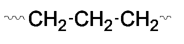 or 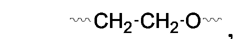 wherein " 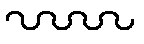 " indicates a point of attachment; Q is phenyl; B is H; one or two of three of J, K and L, are N to form 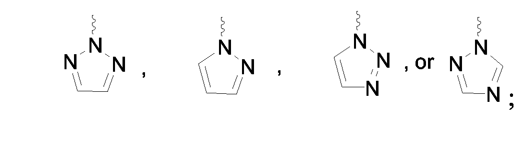 and E is CO₂H.

A group of compounds of the invention are compounds of formula III wherein A is aryl or substituted aryl; heteroaryl or substituted heteroaryl, fused heteroaryl or substituted fused heteroaryl, or heterocycloalkyl and substituted heterocycloalkyl; X is a tether 2 to 5 atoms in length selected from 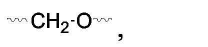 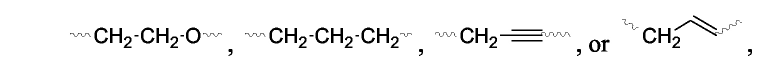 wherein " 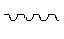 " indicates a point of attachment; and E is CO₂H, CO₂Me, CO₂Et,

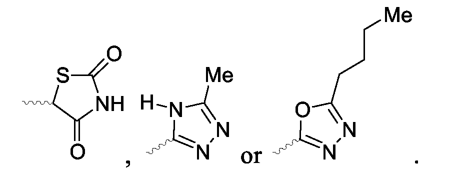

Another group of compounds of the invention are compounds of formula III wherein A is aryl or substituted aryl; heteroaryl or substituted heteroaryl, fused heteroaryl or substituted fused heteroaryl, or heterocycloalkyl and substituted heterocycloalkyl; X is a tether 2 to 5 atoms in length selected from 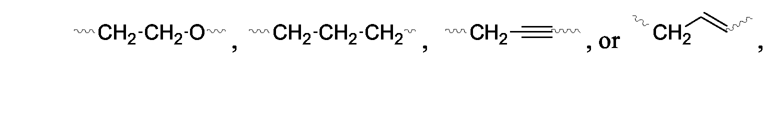 wherein " 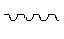 " indicates a point of attachment; and E is CO₂H, CO₂Me, CO₂Et,

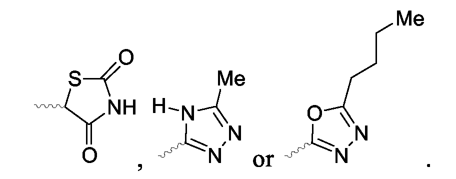

Another group of compounds of the invention are compounds of formula III wherein A is aryl or substituted aryl; heteroaryl or substituted heteroaryl, fused heteroaryl or substituted fused heteroaryl, or heterocycloalkyl and substituted heterocycloalkyl; X is a tether 2 to 5 atoms in length selected from 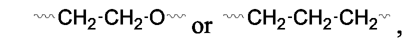 wherein " 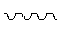 " indicates a point of attachment; and E is CO₂H CO₂Me, or CO₂Et.

Another group of compounds of the invention are compounds of formula III wherein A is heteroaryl or substituted heteroaryl, X is a tether 2 to 5 atoms in length selected from 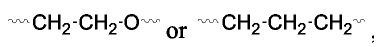 wherein " 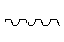 " indicates a point of attachment; and E is CO₂H, CO₂Me, or CO₂Et.

Another group of compounds of the invention are compounds of formula III wherein A is 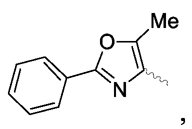 wherein " 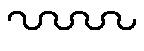 " indicates the point of attachment; X is 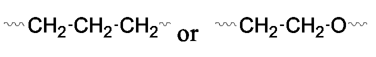 and E is CO₂H.

Processes and novel intermediates for preparing compounds of formula I are provided as further embodiments of the invention and are illustrated by the following procedures in which the meanings of the generic radicals are as given above unless otherwise qualified. Certain compounds of formula I are useful as intermediates for preparing other compounds of formula I.

It is also noted that compounds of formula I can be prepared using protecting groups. It is to be noted that the appropriate use and choice of protecting groups is well-known by one skilled in the art, and is not limited to the specific examples below. It is also to be understood that such groups not only serve to protect chemically reactive sites, but also to enhance solubility or otherwise change physical properties. A good general reference for protecting group preparation and deprotection is "Protecting Groups in Organic Synthesis" by T.W. Green and P.G. Wuts. A number of general reactions such as oxidations and reductions etc. are not shown in detail but can be done by methods understood by one skilled in the art. General transformations are well-reviewed in "Comprehensive Organic Transformation" by Richard Larock, and the series "Compendium of Organic Synthesis Methods" published by Wiler-Interscience. In general, the starting materials are obtained from commercial sources unless otherwise indicated.

It will be appreciated by those skilled in the art that compounds of the invention having one or more chiral centers may exist in and be isolated in optically active and racemic forms. Some compounds may exhibit polymorphism. It is to be understood that the present invention encompasses any racemic, optically-active, polymorphic, geometric, or stereoisometric form, or mixtures thereof, of a compound of the invention, which possess the useful properties described herein, it being well known in the art how to prepare optically active forms (for example, by resolution of the racemic form by recrystallization techniques, by synthesis from optically-active starting materials, by chiral synthesis, or by chromatographic separation using a chiral stationary phase) and how to determine acitvity or cytotoxicity using the standard tests described herein, or using other similar tests which are well known in the art.

It will be further appreciated by the skilled artisan that the following schemes depic the synthesis of compounds wherein A, X, Q, Y, B, D, Z, or E are defined. It is to be understood however, that compounds of the invention other than those specifically disclosed can be prepared using the strategies depicted in the schemes.

Scheme 1 depicts a general approach to the preparation of compounds of Formula I wherein the pyrrolyl group in unsubstituted.

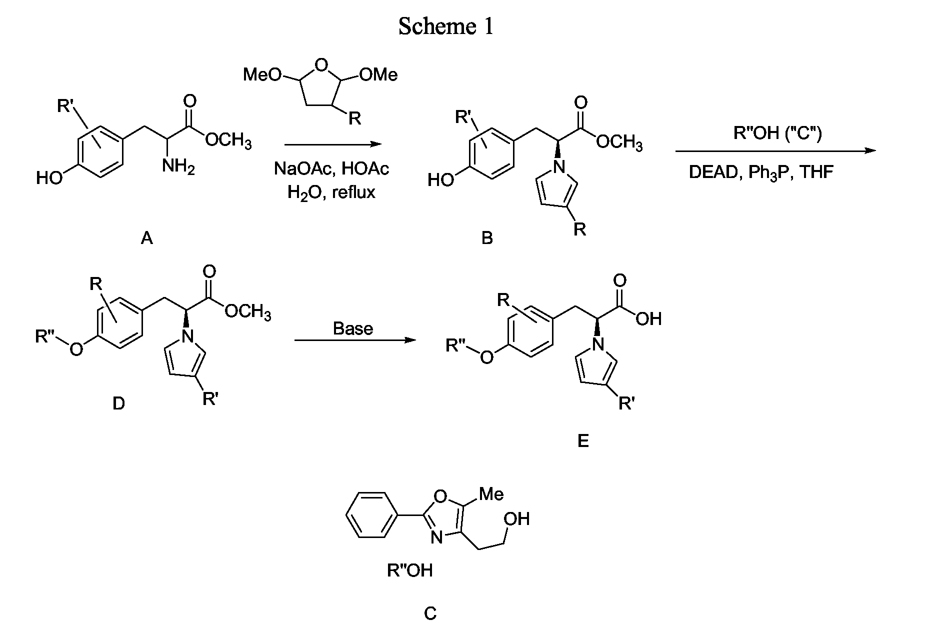

In Scheme 1, the pyrrolotyrosine ester B is first prepared by allowing tyrosine ester A to undergo reaction with 2,5 dimethoxy tetrahydrofuran in the presence of base. The methyl ester is depicted in Scheme 1, but it is possible that other esters may be used. Coupling of pyrrolotyrosine ester analogue with a compound bearing a primary or secondary alcohol under Mitsounobu-type or similar conditions provides pyrrolotyrosine methyl ester derivative D. Hydrolysis of pyrrolotyrosine methyl ester derivative D under basic conditions provides compound of the invention E.

Scheme 2 depicts the preparation of compounds of Formula I wherein the pyrrolyl group bears an alkyl or aryl carbonyl substitutent.

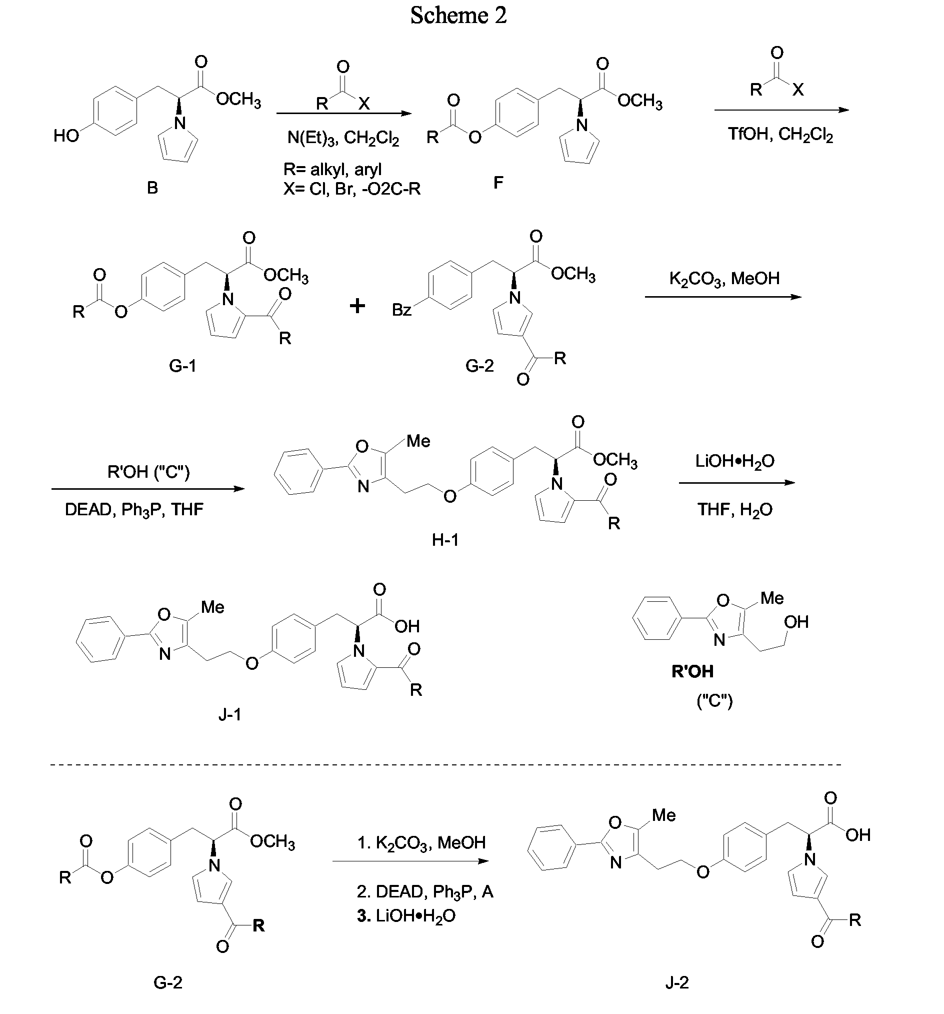

According to Scheme 2, the phenol group in pyrrolotyroisne ester B is first protected using an acid halide or anhydride using procedures known to the skilled artisan. Protected pyrrolotyrosine methyl ester E is then allowed to undergo reaction with an acid halide such as benzoyl chloride, although other acid halides or anhydrides may be used. Acylation occurs at the 2- or 3-postion of the pyrrole ring to provide a mixture of the 2- and 3- substituted benzoyl derivatives G-1 and G-2, which can be separated using conventional techniques. Deprotection of the benzoyl group of pyrrolotyrosine derivative G-1 or G-2, and subsequent Mitsunobu coupling provides penultimate methyl ester H-1 or H-2. Hydrolysis of the methyl ester provides compound of the invention J-1 or J-2.

An alternative approach to the preparation of substituted pyrrolotyrosine derivative G-1 is depicted in Scheme 3.

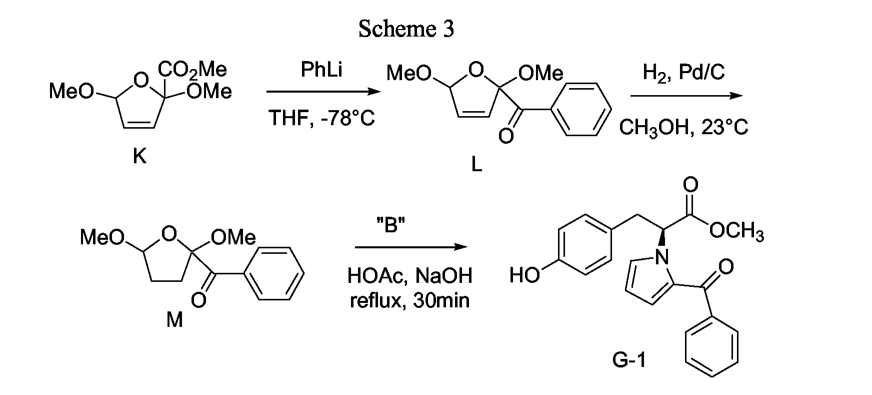

Accordingly, methyl 2,5 dihydro 2,5-dimethoxy-2-furan carboxylate K undergoes reaction with phenyl lithium to provide benzoyl derivative L. Benxoyl derivative L then undergoes reaction with tyrosine methly ester B to provide compound G-1. Compound G-1 can be converted to the invention compound according to the steps provided above.

Scheme 4 provides another route to compounds of the invention wherein the pyrrolyl group bears more than one substitutent.

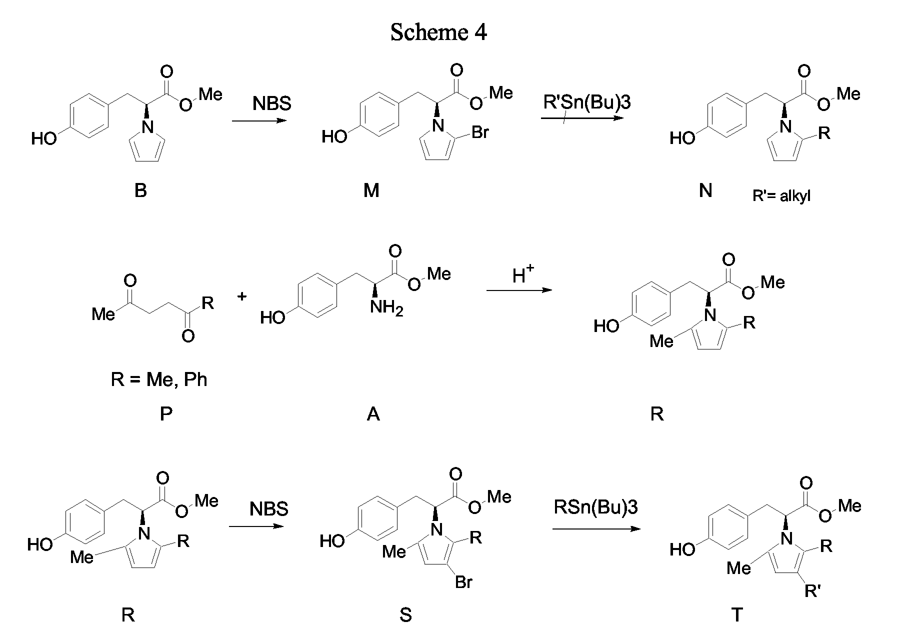

Thus, Row 1 of Scheme 4 depicts the synthesis of pyrrolotyrosine methyl ester derivatives bearing a substitutent at the pyrrolyl 2- postion. Pyrrolotyrosine methyl ester B undergoes reaction with N-bromosuccinimide or a similar agent to provide the 2-bromo pyrrolotyrosine methyl ester dervative M. The 2-bromo pyrrolotyrosine methyl ester dervative M may undergo coupling with alcohol C and hydrolysis to provide a compound of the invention. Alternatively, the 2-bromo pyrrolotyrosine methyl ester dervative M may undergo reaction with an alkyl tin reagent under conditions known in the art to provide 2-alkyl pyrrolotyrosine methyl ester derivative N, which may undergo coupling and hydrolysis to provide other compounds of the invention.

Row 2 of Scheme 4 depicts the synthesis of disubstituted pyrrolotyrosine methyl ester dervatives. Thus, diketone P undergoes reaction with tyrosine methyl ester A in the presence of acid to provide pyrrolotyrosine methyl ester derivative P, wherein the 2- and 5- positons of the pyrrolyl ring bear alky or aryl substitituents. Pyrrolotyrosine methyl ester derivative P can be coupled with alcohol C or an alternative alcohol under Mitsunobu-type conditions, followed by ester hydrolysis, to provide a compound of the invention R.

Alternatively, pyrrolotyrosine methyl ester derivative R can be used to provide other compounds of the invention wherein the pyrrolyl ring is trisubstituted. Thus, as depicted in Row 3 of Scheme 4, bromination of pyrrolotyrosine methyl ester derivative R using N-bromosuccinimde or a similar agent provides the brominated compound S. Brominated compound S may undergo coupling and hydrolysis as described above to provide a compound of the invention. Brominated compound S may also undergo reaction with an alkyl tin reagent to provide trisubstituted derviatives such as V.

Scheme 5 depicts an alternative synthesis of compounds of the invention wherein the pyrrolyl ring is substituted at the 2-position.

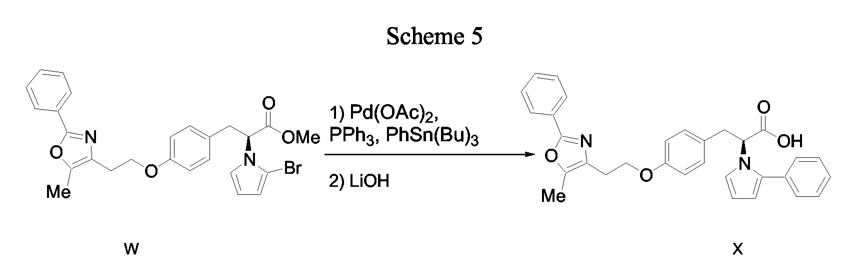

As Scheme 5 provides, methyl ester W, prepared by coupling 2-bromopyrrolotyrosine methyl ester derivative M with alcohol C, can undergo palladium catalyzed alkyl or aryl coupling to provide the 2-aryl substituted pyrrolotyrosine methyl derivative X. 2- alkyl or aryl substituted pyrrolotyrosine methyl derivative (aryl derivative X depicted). Upon hydrolysis, aryl derivative X gtives rise to a compound of the invention.

Scheme 6 provides compounds of the invention derived from disubstituted pyrrolotyroisne methyl ester derivative R.

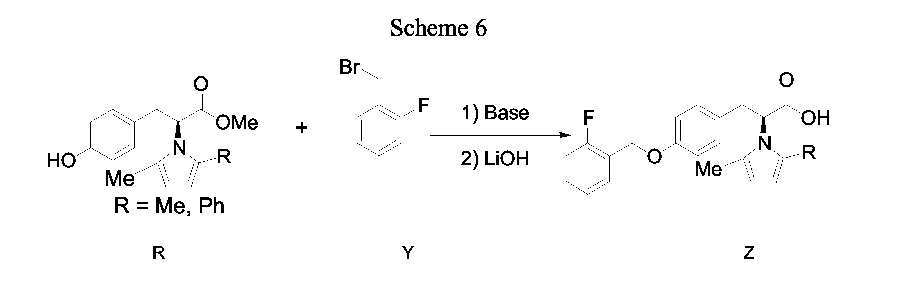

Upon treatment with base to generate phenoxide, pyrrolotyroisne methyl ester derivative R can undergo reaction with a wide variety of alkylating agents known in the art (See for instance, alylating agents listed in Aldrich Handbook of Fine Chemicals (2000-2001). In Scheme 6, the phenoxide of pyrrolotyroisne methyl ester derivative R undergoes reaction with 2-fluorobenzyl bromide Y, followed by hydrolysis to provide compound of the invention Z.

Scheme 7 depicts the synthesis of compounds of Formula I wherein X is -CH₂CH₂NH-.

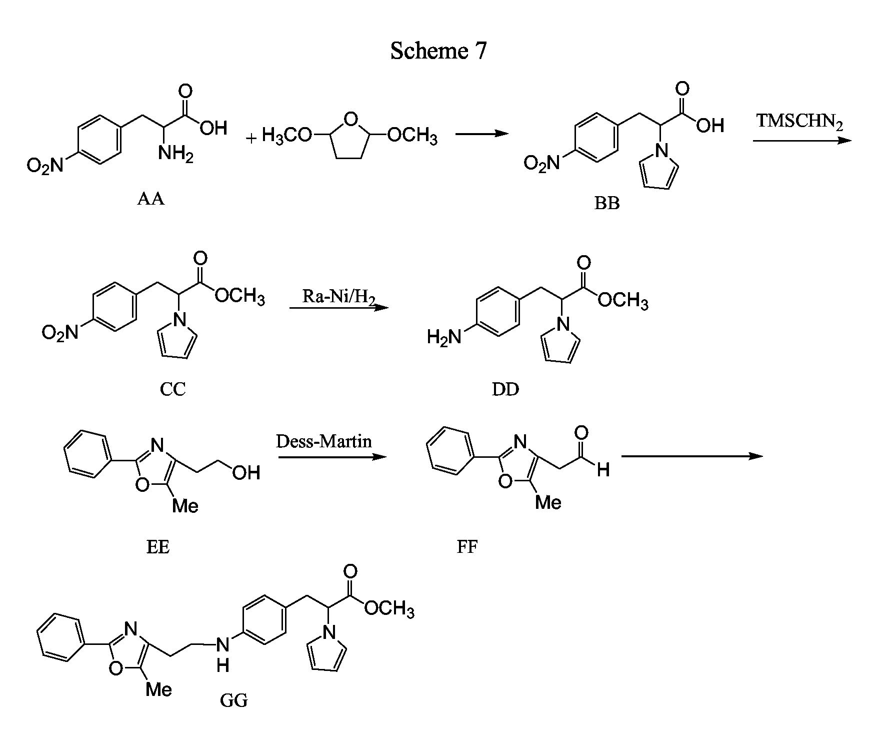

Thus, nitro compound AA undergoes reaction with 2,5 dimethoxy tetrahydrofuran to provide the pyrrolo derivative BB. Pyrrolo derivative BB is then esterified using trimethylsilyl azide to provide methyl ester CC. Reduction of the nitro group in ester CC using Raney nickel in the presence of Hydrogen provides the amine compound DD. Addition of amine compound DD to aldehyde FF (generated from alcohol EE oxidation) followed by reduction, provides the amine GG. Amine GG is hydrolyzed as described previously to provide a compound of the invention.

scheme 8 depicts the synthesis of compounds of Formula I wherein X is -CH₂CH₂S-.

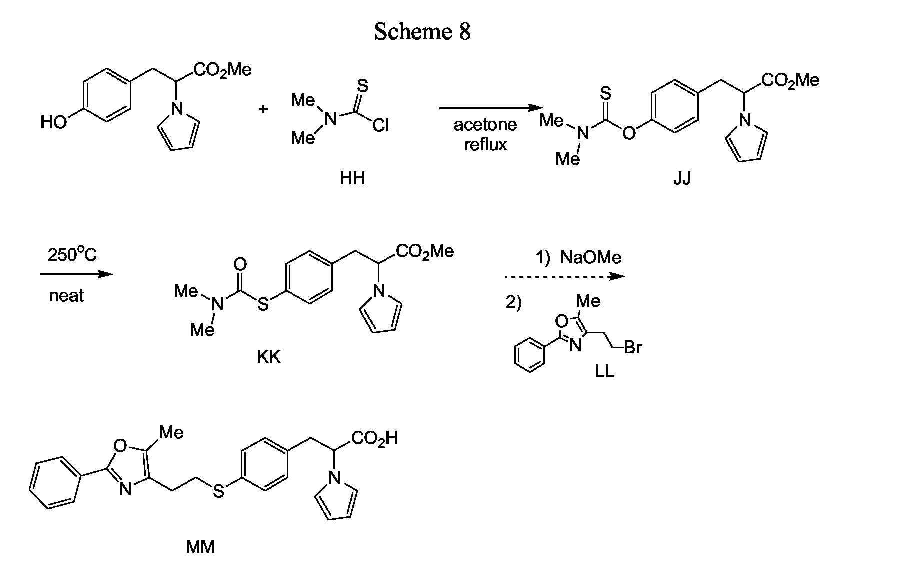

Thus, pyrrolotyrosine methyl ester B undergoes reation with dimethylthicarbamoyl chloride to provide compound JJ. When heated to 250 °C, compound JJ undergoes rearrangment to provide compound KK. Treatment of KK with base generated the thiophenoxide, which undergoes reaction with the depicted alkyl halide LL, to provide the thio-variant Formula I compound MM.

An alternative preparation of a thio-variant Formula I compound is depicted in Scheme 9.

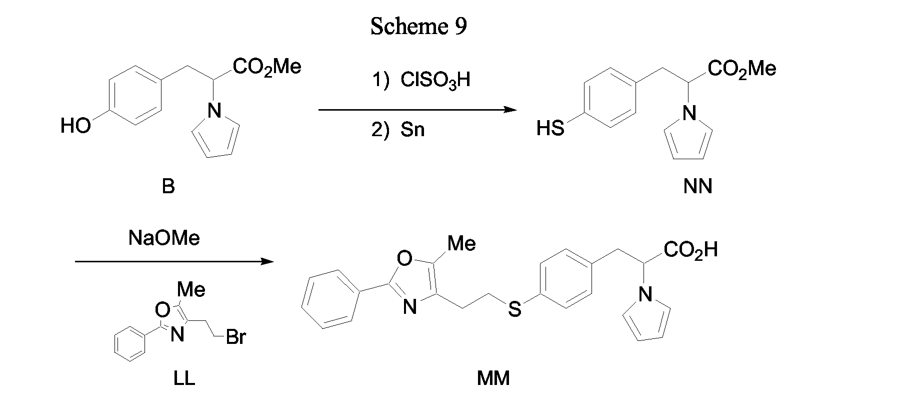

Thus, pyrrolotyrosine methyl ester B can undergo reaction with chlorosulfonic acid in the presence of elemental tin to provide thiophenol NN. Alkylation of NN and ester hydrolysis can provide the compound of the invetion MM.

Scheme 10 depicts the synthesis of additional compounds of the invention wherein the pyrrolyl group is replace by a thiopyrrolyl group.

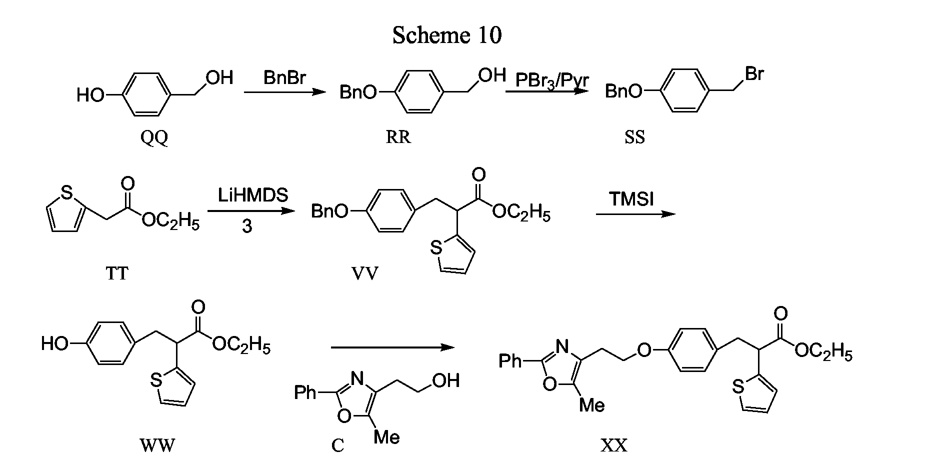

Thus, 4-hydroxy benzyl alcohol QQ undergoes reaction with benzyl bromide to provide alcohol RR. Alcohol RR is converted to the bromide SS by treatment with tribromo phosphine in the presence of pyridine. The bromide SS undergoes reaction with thiopyrrole VV under conventional alkylation conditions followed by deprotection to provide compound WW. Mitsunobu-type coupling of WW with alcohol C provided ester XX. Hydrolysis of ester XX pprovides a compound of the invention.

Scheme 11 depicts the synthesis of compounds of the invention wherein the pyrrolyl group is replaced by another aryl group, such as a phenyl-substituted phenyl group.

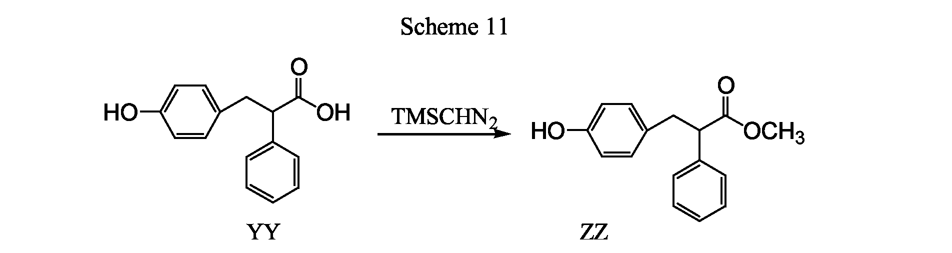

Thus, esterification of 4-hydroxy phenylalanine YY provides methyl ester ZZ. Mitsunobu type coupling and ester hydrolysi of compound ZZ provided a compound of the invention.

Scheme 12 depicts the synthesis of compounds of the invention wherein X is -CH-CH=CH- and D is heteroaryl.

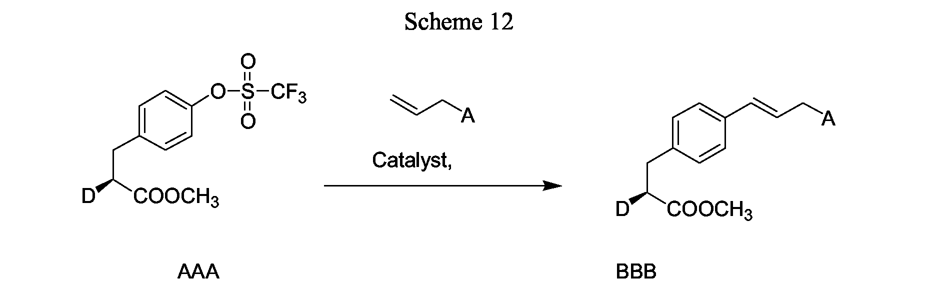

In Scheme 12, coupling of the triflate AAA with a vinyl compound in the presence of an organometallic catalyst provides vinyl ester BBB. Hydrolysis of the ester moiety in BBB as described above provides a compound of the invention wherein R is alkyl or substituted alkyl, aryl or substituted aryl, heteroaryl or substituted heteroaryl, heterocycloalkyl and substituted heterocycloalkyl, or heteroalkyl or substituted heteroalkyl.

Scheme 13 depicts the synthesis of additional compounds of the invention wherein X is -C≡C-CH₂- and D is heteroaryl.

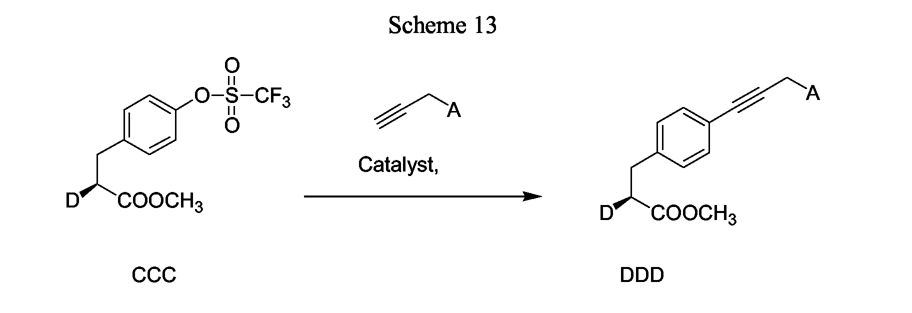

In Scheme 13, coupling of the triflate AAA with an alkynyl compound CCC in the presence of an organometallic catalyst provides alkynyl ester DDD. Hydrolysis of the ester moiety in DDD as described above provides a compound of the invention wherein R is alkyl or substituted alkyl, aryl or substituted aryl, heteroaryl or substituted heteroaryl, heterocycloalkyl and substituted heterocycloalkyl, or heteroalkyl or substituted heteroalkyl.

Scheme 14 depicts the synthesis of compounds of the invention wherein X is -(CH₂)₃- and D is heteroaryl.

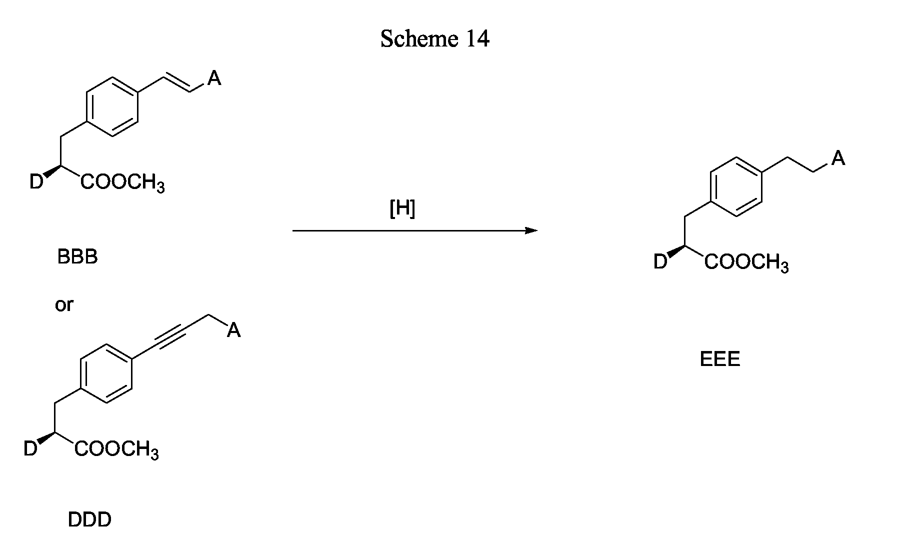

Scheme 14 indicates that either the vinyl compound BBB or the alkynyl compound DDD can be reduced under conditions known in the art to provide the saturated variant EEE.

Scheme 15 depicts one approach to preparing compounds of the invention wherein Q is heteroaryl or substituted heteroaryl..

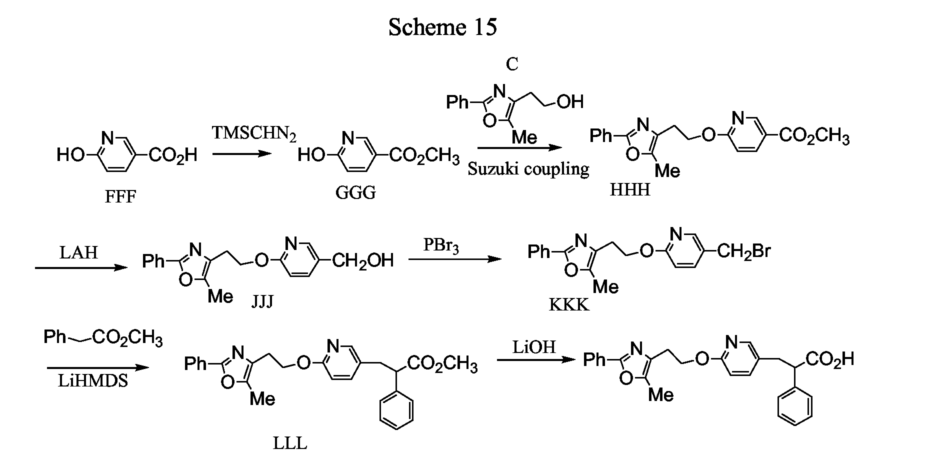

Thus, nicotinic acid FFF is esterified to provide GGG. GGG is coupled to C as discussed earlier to give HHH. The ester moiety in HHH is reduced to give alcohol JJJ, which is then converted to halide KKK using conventional techniques. Alkylation of the methyl estero f phenyl acetic acid using KKK, provides product LLL. LLL is hydrolyzed to a compound of the invention under conditions as discussed previously.

Scheme 16 depicts a compound of the invention wherein B is H.

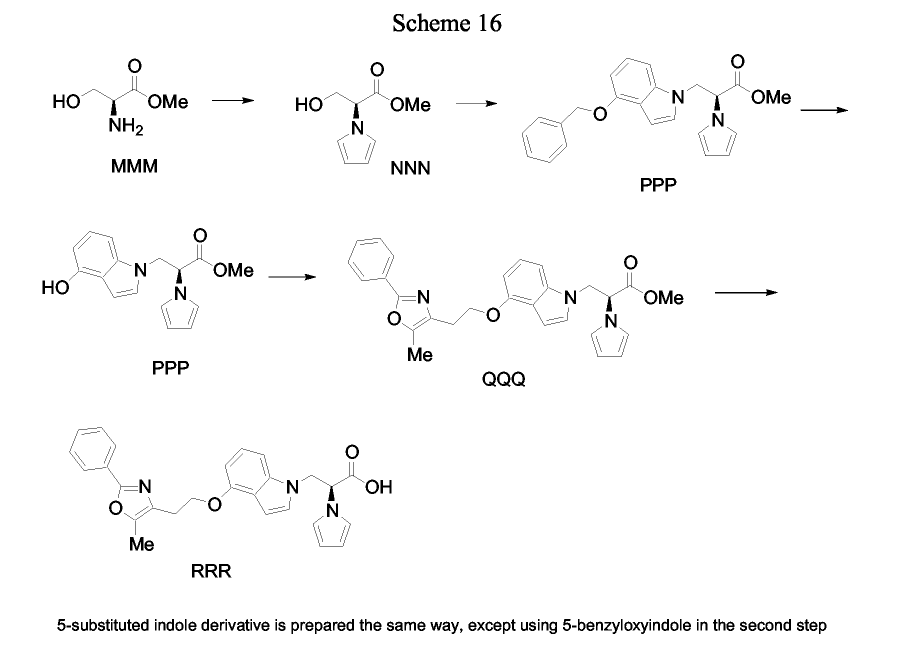

Thus, MMM is converted to the pyrrole derivative NNN as provided earlier. The hydroxzy group of compound NNN is converted to a leaving group, such as a bromide, mesylate, or tosylate or the like, and then undergoes addition with the benzyl-substituted hydroxyindole derivate at the N positon to give OOO. Removal of the benzyl protecting group in OOO, followed by coupling with C is provided earlier, gives rise to QQQ. Saponification of the ester moiety in QQQ gives the invention compound RRR.

Scheme 17 depicts the synthesis of a compound of the invention wherein B is haloalkyl.

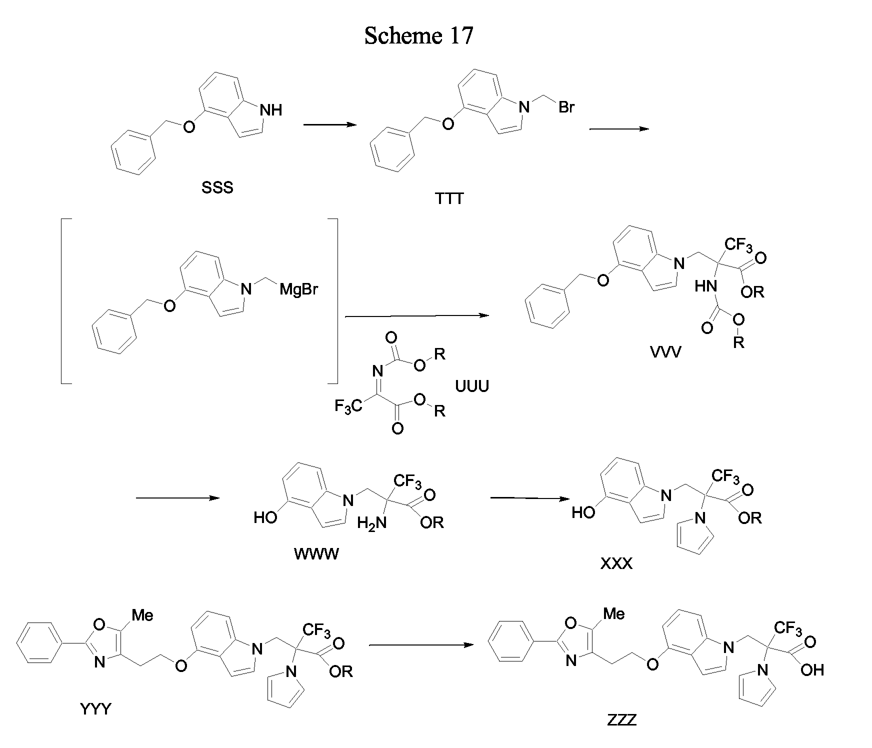

Thus the benzyloxy indole derivative SSS is converted to the bromomethyl compound TTT, by treatment with formaldehyde or its equivalent, followed by halogenation using conventional conditions. Compound TTT is then converted to the Grignard reagent, which then undergoes 1,2 addition with imine UUU to provide VVV. The amide moiety in VVV is hydrolyzed to provide WWW. Coupling of WWW with C as provided earlier gives rise to XXX. Construction of the pyrrolyl ring to provide XXX, saponification of the ester moiety in YYY as provided earlier gives rise to the invention compound ZZZ.

Scheme 18 depicts the synthesis of a compound of the invention wherein B is F.

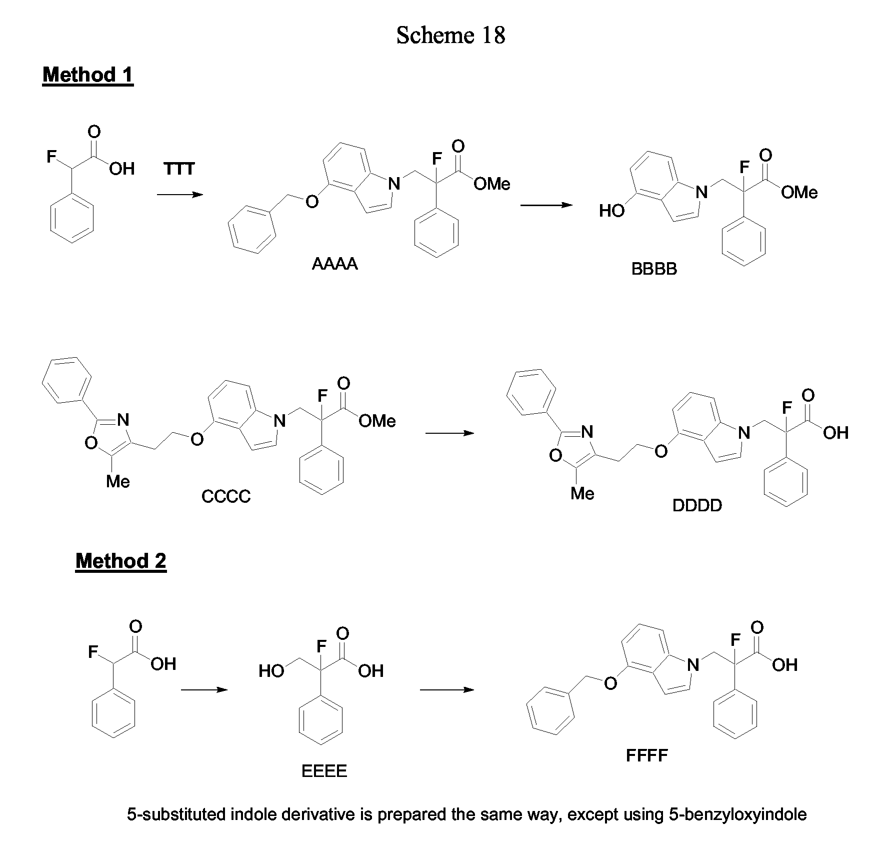

In Method 1 of Scheme 18, 1-fluoro-2-phenylacetic acid is deprotonated and allowed to treact with TTT to provide AAA. In Method 2 of Scheme 18, the Grignard reagent derived from TTT is combined with hydroxymethyl derivative EEE to provide FFFF.

Scheme 19 depicts alternative syntheses of a compound of the invention wherein B is F. The difference between Methods 1 and 2 in Scheme 19 is the route by which pyrrole (GGGG or QQQQ) is used.

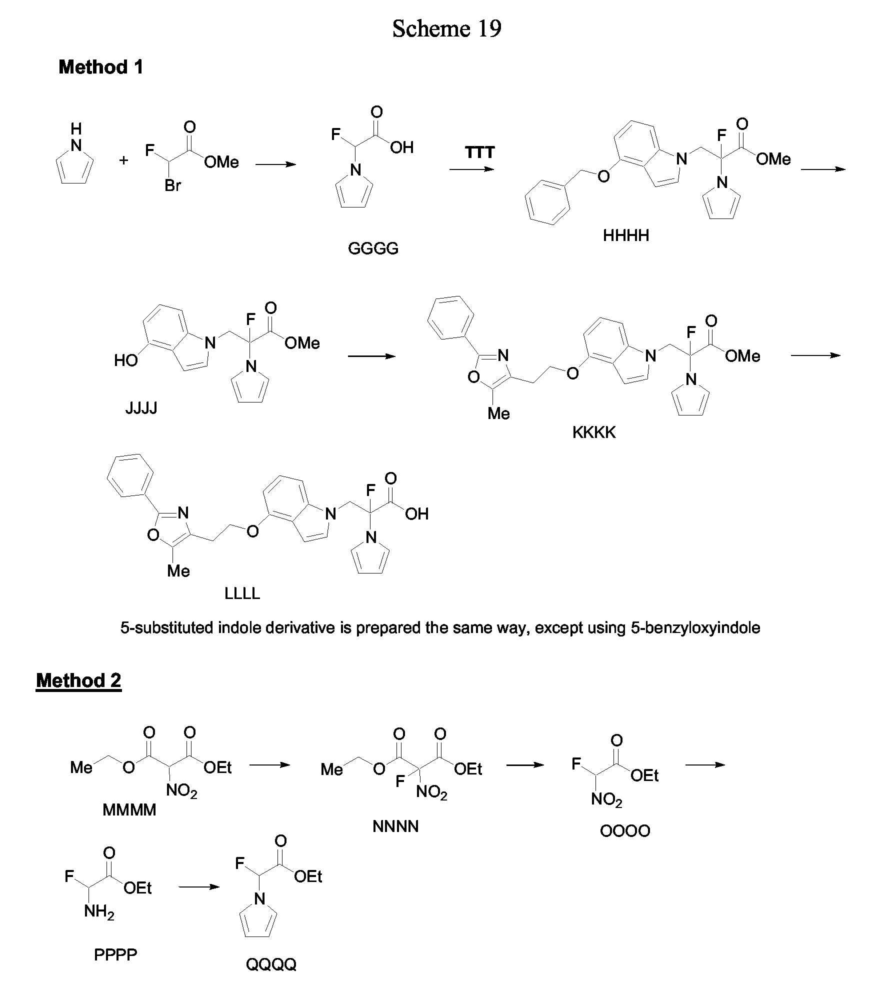

Thus in Method 1, the requisite pyrrole deriviative GGGG is coupled with TTT as provided in Scheme 17 to provide HHHH. Coupling of HHHH to C, followed by saponification, provides the invention compound LLLL. In Method 2, requisite pyrrole QQQQ is prepared from MMMM via fluorination, decarboxylation, and reduction. QQQQ is then used as in Method 1 to provide the invention compound.

Some of the compounds of Formula I are capable of further forming pharmaceutically acceptable acid-addition and/or base salts. All of these forms are within the scope of the present invention.

Pharmaceutically acceptable acid addition salts of the compounds of Formula I include salts derived from nontoxic inorganic acids such as hydrochloric, nitric, phosphoric, sulfuric, hydrobromic, hydriodic, hydrofluoric, phosphorous, and the like, as well as the salts derived from nontoxic organic acids, such as aliphatic mono- and dicarboxylic acids, phenyl-substituted alkanoic acids, hydroxy alkanoic acids, alkanedioic acids, aromatic acids, aliphatic and aromatic sulfonic acids, etc. Such salts thus include sulfate, pyrosulfate, bisulfate, sulfite, bisulfite, nitrate, phosphate, monohydrogenphosphate, dihydrogen phosphate, metaphosphate, pyrophosphate, acetate, trifluoroacetate, propionate, caprylate, isobutyrate, oxalate, malonate, succinates suberate, sebacate, fumarate, maleate, mandelate, benzoate, chlorobenzoate, methylbenzoate, dinitrobenzoate, phthalate, benzensoulfonate, toluenesulfonate, phenylacetate, citrate, lactate, maleate, tartrate, methanesulfonate, and the like. Also contemplated are salts of amino acids such as arginate and the like and gluconate, galacturonate (see, for example, Berge S.M. et al., "Pharmaceutical Salts," *Journal of Pharmaceutical Science,* 1977;66:1-19).

The acid addition salt of basic compounds are prepared by contacting the free base form with a sufficient amount of the desired acid to produce the salt in the conventional manner.

Pharmaceutically acceptable base addition salts are formed with metals or amines, such as alkali and alkaline earth metals or organic amines. Examples of metals used as cations are sodium, potassium, magnesium, calcium, and the like. Examples of suitable amines are N,N'-dibenzylethylenediamine, chloroprocaine, choline, diethanolamine, dicyclohexylamine, ethylenediamine, N-methylglucamine, and procaine (see, for example, Berge S.M., supra., 1977).

The base addition salts of acidic compounds are prepared by contacting the free acid form with a sufficient amount of the desired base to produce the salt in the conventional manner.

Certain of the compounds of the present invention can exist in unsolvated forms as well as solvated forms, including hydrated forms. In general, the solvated forms, including hydrated forms, are equivalent to unsolvated forms and are intended to be encompassed within the scope of the present invention.

Certain of the compounds of the present invention possess one or more chiral centers and each center may exist in the R(D) or S(L) configuration. The present invention includes all enantiomeric and epimeric forms, as well as the appropriate mixtures thereof

The compounds of formula I can be formulated as pharmaceutical compositions and administered to a mammalian host, such as a human patient in a variety of forms adapted to the chosen route of administration, i.e., orally or parenterally, by intravenous, intramuscular, topical or subcutaneous routes.

Thus, the present compounds may be systemically administered, e.g., orally, in combination with a pharmaceutically acceptable vehicle such as an inert diluent or an assimilable edible carrier. They may be enclosed in hard or soft shell gelatin capsules, may be compressed into tablets, or may be incorporated directly with the food of the patient's diet. For oral therapeutic administration, the active compound may be combined with one or more excipients and used in the form of ingestible tablets, buccal tablets, troches, capsules, elixirs, suspensions, syrups, wafers, and the like. Such compositions and preparations should contain at least 0.1 % of active compound. The percentage of the compositions and preparations may, of course, be varied and may conveniently be between about 2 to about 60% of the weight of a given unit dosage form. The amount of active compound in such therapeutically useful compositions is such that an effective dosage level will be obtained.

The tablets, troches, pills, capsules, and the like may also contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active compound may be incorporated into sustained-release preparations and devices.

The active compound may also be administered intravenously or intraperitoneally by infusion or injection. Solutions of the active compound or its salts can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations contain a preservative to prevent the growth of microorganisms.

The pharmaceutical dosage forms suitable for injection or infusion can include sterile aqueous solutions or dispersions or sterile powders comprising the active ingredient which are adapted for the extemporaneous preparation of sterile injectable or infusible solutions or dispersions, optionally encapsulated in liposomes. In all cases, the ultimate dosage form must be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filter sterilization. In the case of sterile powders for the preparation of sterile injectable solutions, the preferred methods of preparation are vacuum drying and the freeze drying techniques, which yield a powder of the active ingredient plus any additional desired ingredient present in the previously sterile-filtered solutions.

For topical administration, the present compounds may be applied in pure form, i.e., when they are liquids. However, it will generally be desirable to administer them to the skin as compositions or formulations, in combination with a dermatologically acceptable carrier, which may be a solid or a liquid.

Useful solid carriers include finely divided solids such as talc, clay, microcrystalline cellulose, silica, alumina and the like. Useful liquid carriers include water, alcohols or glycols or water-alcohol/glycol blends, in which the present compounds can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers.

Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Examples of useful dermatological compositions which can be used to deliver the compounds of formula I to the skin are known to the art; for example, see Jacquet et al. (U.S. Pat. No. 4,608,392), Geria (U.S. Pat. No. 4,992,478), Smith et al. (U.S. Pat. No. 4,559,157) and Wortzman (U.S. Pat. No. 4,820,508).

Useful dosages of the compounds of formula I can be determined by comparing their in vitro activity, and in vivo activity in animal models. Methods for the extrapolation of effective dosages in mice, and other animals, to humans are known to the art; for example, see U.S. Pat. No. 4,938,949.

Generally, the concentration of the compound(s) of formula I in a liquid composition, such as a lotion, will be from about 0.1-25 wt-%, preferably from about 0.5-10 wt-%. The concentration in a semi-solid or solid composition such as a gel or a powder will be about 0.1-5 wt-%, preferably about 0.5-2.5 wt-%.

The amount of the compound, or an active salt or derivative thereof, required for use in treatment will vary not only with the particular salt selected but also with the route of administration, the nature of the condition being treated and the age and condition of the patient and will be ultimately at the discretion of the attendant physician or clinician.

In general, however, a suitable dose will be in the range of from about 0.005 to about 100 mg/kg, e.g., from about 0.1 to about 75 mg/kg of body weight per day, such as 0.03 to about 50 mg per kilogram body weight of the recipient per day, preferably in the range of 0.06 to 90 mg/kg/day, most preferably in the range of 0.15 to 60 mg/kg/day.

The compound may conveniently be administered in unit dosage form; for example, containing 0.05 to 1000 mg, conveniently 0.1 to 750 mg, most conveniently, 0.5 to 500 mg of active ingredient per unit dosage form.

Ideally, the active ingredient should be administered to achieve peak plasma concentrations of the active compound of from about 0.005 to about 75 µM, preferably, about 0.01 to 50 µM, most preferably, about 0.02 to about 30 µM. This may be achieved, for example, by the intravenous injection of a 0.0005 to 5% solution of the active ingredient, optionally in saline, or orally administered as a bolus containing about 0.01-1 mg of the active ingredient. Desirable blood levels may be maintained by continuous infusion to provide about 0.0001-5 mg/kg/hr or by intermittent infusions containing about 0.004-15 mg/kg of the active ingredient(s).

The desired dose may conveniently be presented in a single dose or as divided doses administered at appropriate intervals, for example, as two, three, four or more sub-doses per day. The sub-dose itself may be further divided, e.g., into a number of discrete loosely spaced administrations; such as multiple inhalations from an insufflator or by application of a plurality of drops into the eye.

The antidiabetes ability of a compound of the invention is demonstrated using pharmacological models that are well known to the art, for example, using models such as the tests described below.

### TEST A―3T3-L1 Adipocyte Differentiation Assay (ADPDIFF)

This assay is used to determine the potential of putative PPAR-γ ligands to induce fat cell differentiation. A quantitative method was established for determining the ability of potential PPAR-γ ligands to promote adipogenesis of 3T3-L1 preadipocytes. 3T3-L1 preadipocytes are plated onto 96 well plates (20,000 cells per well). Upon confluency, the compounds which were initially scored as positives from the PPAR-γ ligand displacement and PPAR-γ chimeric receptor transcription assays were added for 4 days with the final concentrations of 2. 5, 5. 0 and 10 µM (n=3). Each plate contains positive controls (5 uM BRL 49653 and 5 uM Troglitazone) and a vehicle control (DMSO). Cells are replenished with FBS containing media on day 5 of post-drug treatment and incubated for additional 4-6 days. Cells are stained with BODIPY a fluorescent lipophilic stain to quantitate the lipid content of the cells. The assay is optimized for a 96-well plate format. Approximately after 10 days of post-drug treatment, cells are fixed in 3% formalin solution for 15 min followed by staining with BODIPY (80 µg/ml) for 20 min at room temperature. The plate is then put through the CYTOFLUOR instrument to measure the fluorescence of BODIPY (excitation=485/ 20; emission=530/ 25). A template is created in a spread sheet to calculate the average value of BODIPY measurements and reported as % of BRL 49653 at 5 µM.

### TEST B―ANTCV1 Antagonist Transcription Assay

The ANTCV1 transcription assay is an *in vitro* assay in which CV-1 cells, an African green monkey kidney cell line, are transfected with a luciferase reporter construct comprised of a fragment of the fatty acid binding protein (FATP) promoter located upstream of the TK TATA box. Transfection efficiency is controlled by co-transfectionof the reference plasmid CMV β-galactosidase. The DNAs are transiently transfected into the cells by electroporation and the cells are plated into 96-well dishes. Test compounds are diluted to final concentration of 25 µM in individual wells containing 300 nM BRL. Control wells include either the 0.5% DMSO vehicle control, the antagonist PD 0157724-0000 at 25 µM and 300nM BRL, or 300nM BRL alone. Cells are incubated with both the drugs for 48 hrs and then harvested. The lysates are measured for luciferase and β-galactosidase activities using the dual luciferase kit from Tropix on a EG&G Berthold MicroLumat LB96P luminometer. The fold activation of BRL 49653 in each assay must be above 4 in order for the assay to be considered valid.

Raw numbers are transferred to an Excel spreadsheet and luciferase/β-galactosidase ratios are determined for each compound. The percent BRL 49653 inhibition for each compound is calculated by dividing the luciferase/β-galactosidase ratio for each compound by the luciferase/β-galactosidase ratio of the DMSO vehicle control. This number is then plugged into the following equation: % BRL inhibition = (BRL fold activation ― test compound fold activation & BRL / BRL fold activation -1) x 100.

### TEST C―CV-1 NATIVE RECEPTORS TRANSCRIPTION ASSAY (MKNRCV1)

The purpose of this assay is to identify ligands that activate endogenous nuclear receptors in CV-1 cells. Protocol: CV-1 cells are co-transfected with a luciferase reporter containing a fragment of the FATP promoter upstream the TK TATA box and a CMV beta-galactosidase plasmid. Transfected cells are incubated with test ligands for 48 hours. Cell lysates are harvested and the luciferase and beta-galactosidase activities are determined. Description: Luciferase and beta-galactosidase activities in the cell lysates are measured using an EG&G Berthold luminometer. These values are entered into and Excel worksheet which calculates the luciferase to beta-galactosidase ratios and expresses the data as percent activity of the reference compound, BRL 49653.

### TEST D―BLOOD GLUCOSE MEASURMENT (GLUCOSE Δ)

Glucose is obtained 4 hours post dose via tail vein stick (5µl whole blood) in awake, 4 hour fasted animals. Blood is drawn by capillary action into a glucose cuvette and read in a HemoCue Glucose Analyzer (Ryan Diagnostics). Blood is diluted 1:2 with saline if glucose levels are greater than 400mg/dl (meter high range) and results multiplied by two.

### TEST E―3T3-L1 TRANSIENT REPORTER ASSAY

This assay is used to determine the potential of putative PPAR-γ ligands to activate the promoter/enhancer of the murine aP2 gene. 3T3-L1 preadipocytes are cultured on collagen coated plates in DMEM containing 10% Calf serum for 48 hours post-confluence. The cells are then cultured for approximately 3 days in DMEM containing 10% Fetal Bovine Serum (FBS), 0.5-mM methyl isobutylxanthine, 0.25 µM dexamethasone, and 1 µg/ml of insulin. Cells are detached from the plates with Trypsin-EDTA and resuspended in Phosphate Buffered Saline (PBS).

The reporter construct used in this assay is comprised of the -5.4 Kb 5' flanking region of the murine aP2 gene inserted into the cloning site of the TKpGL3 luciferase vector. Transfection efficiency is controlled by cotransfection of the reference plasmid CMV-β-galactosidase. The reporter construct and the reference plasmid are transiently co-transfected into the cells by electroporation. The transfected cells are plated into collagen coated 96-well plates and cultured overnight. Cells are then incubated for 48 hours with the test compounds and then harvested. The lysates are measured for luciferase and β-galactosidase activities using the Dual-Light® luciferase kit from Tropix on a EG&G Berthold MicroLumat LB96P luminometer.

Data are summarized in Tables 1-3.

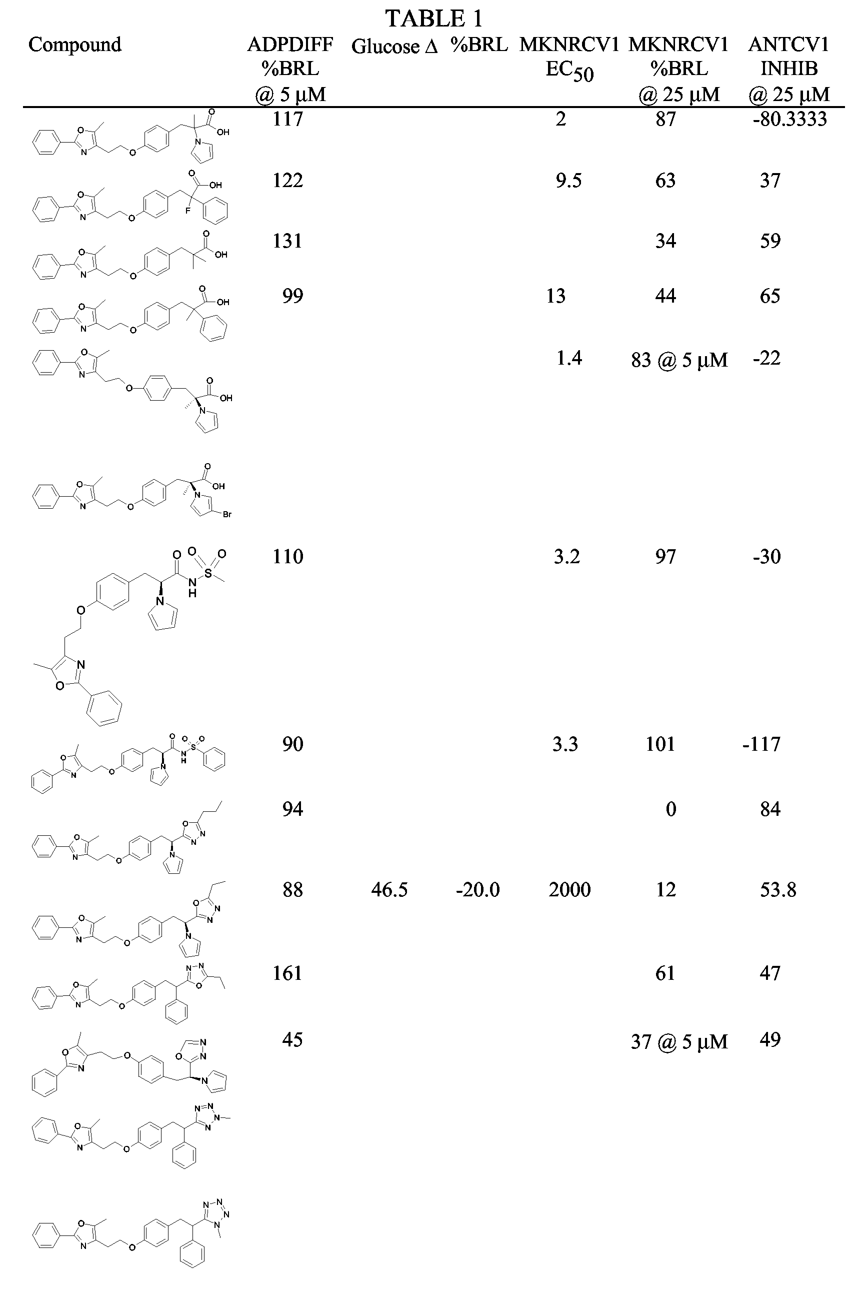

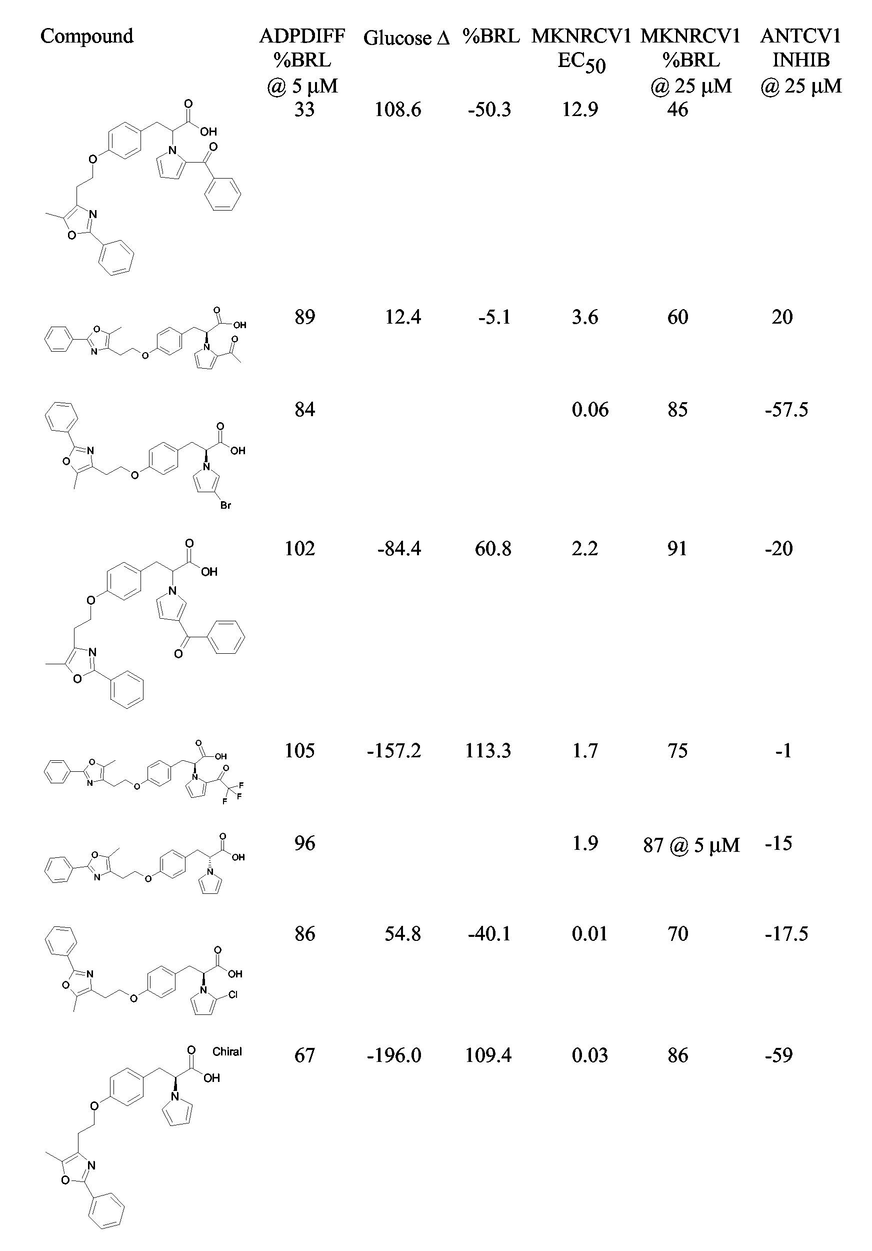

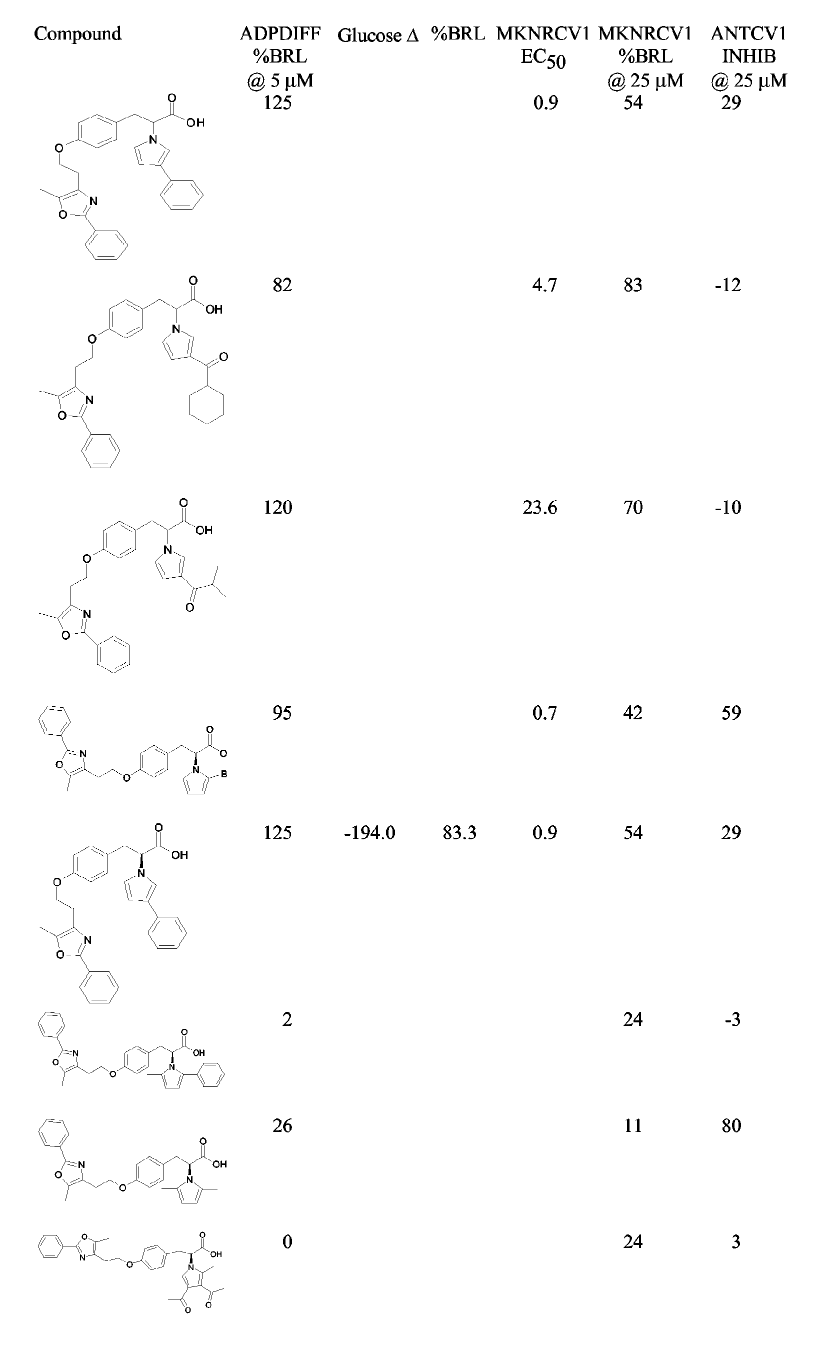

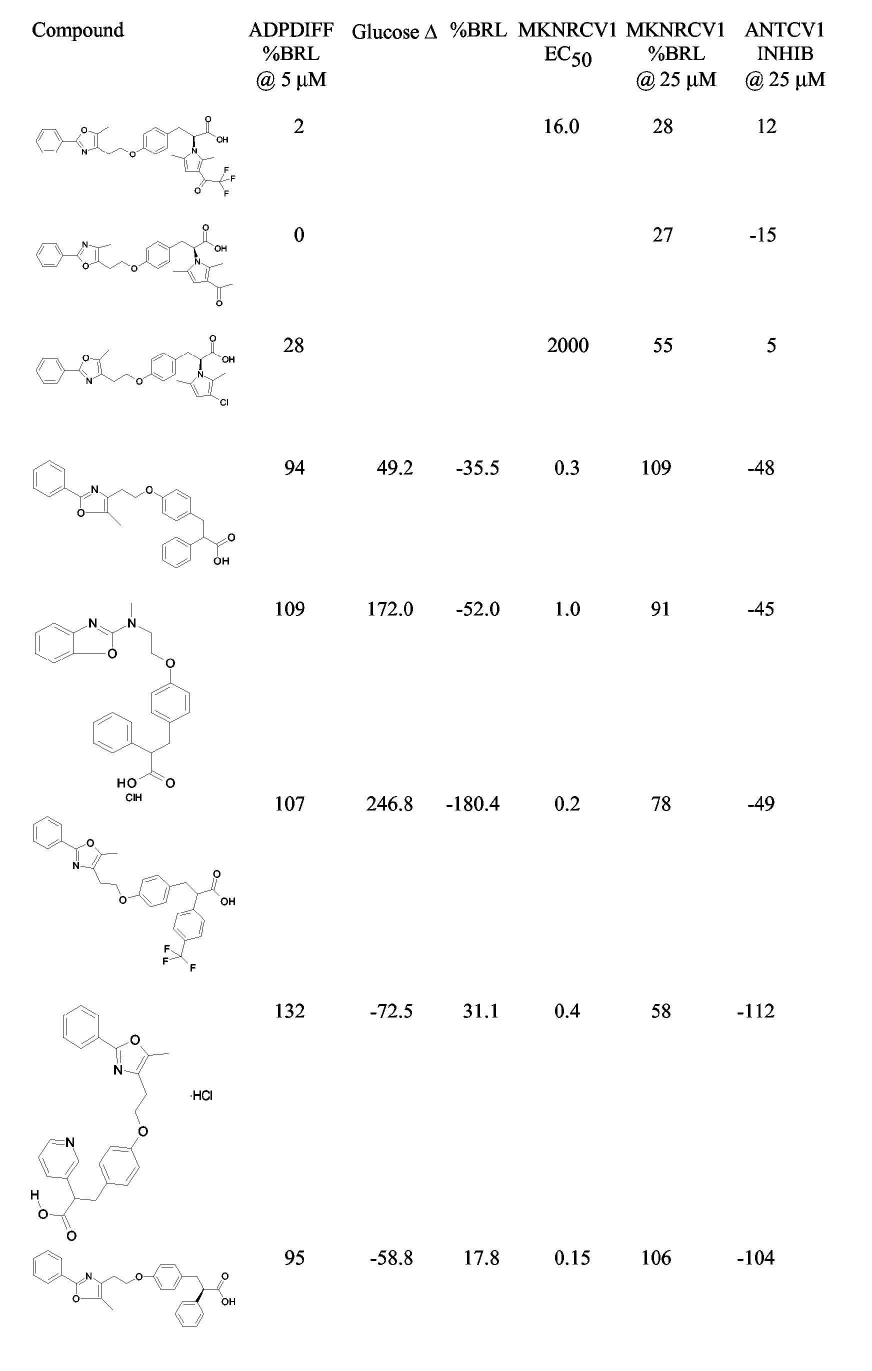

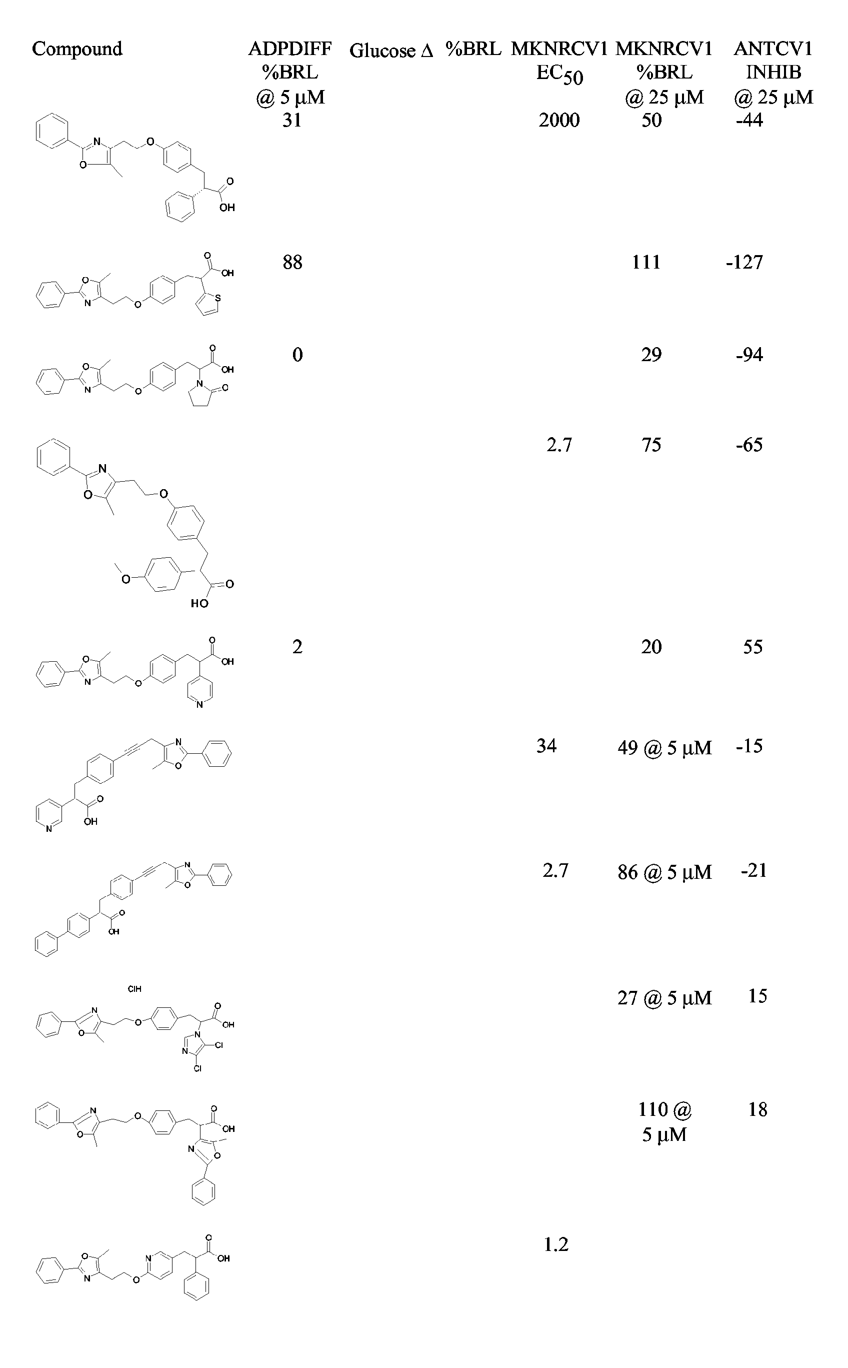

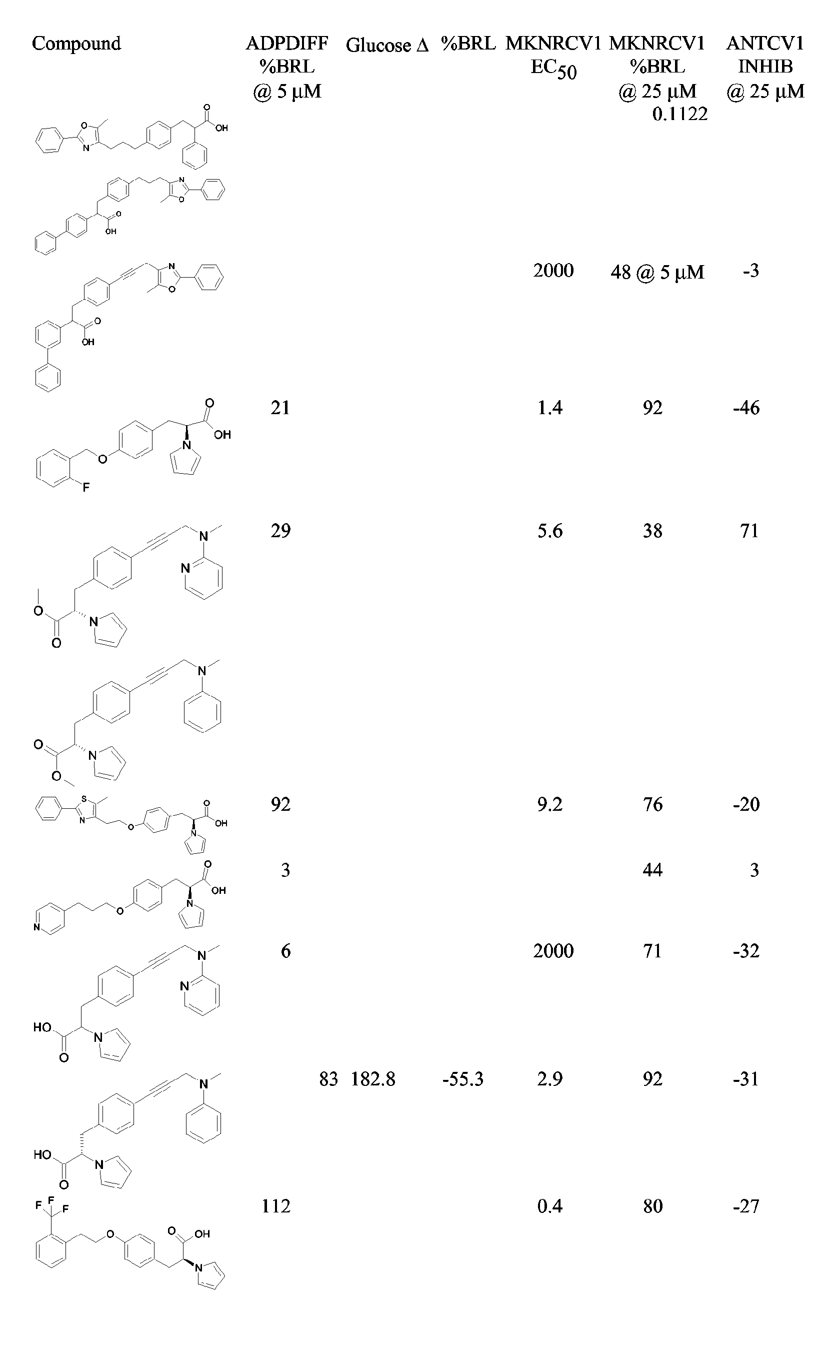

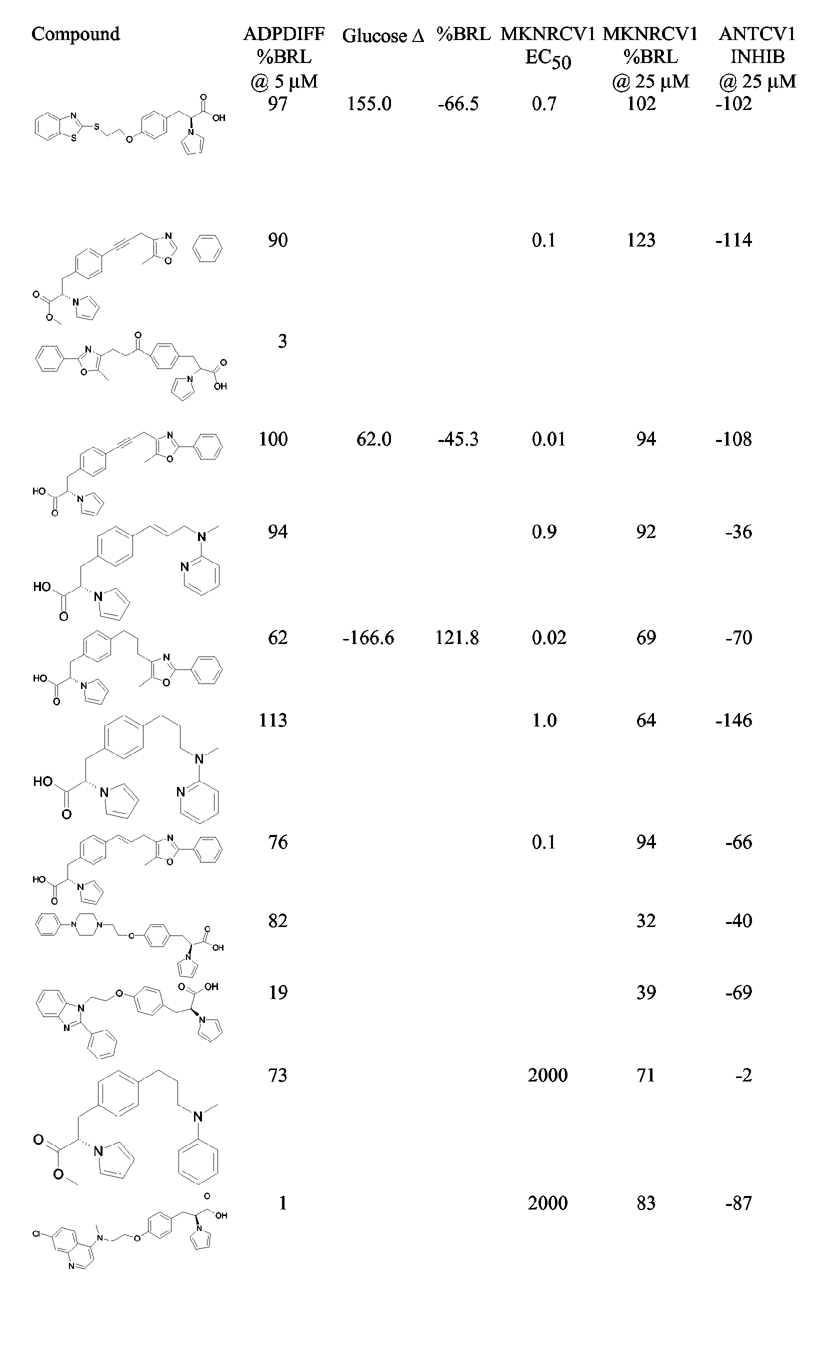

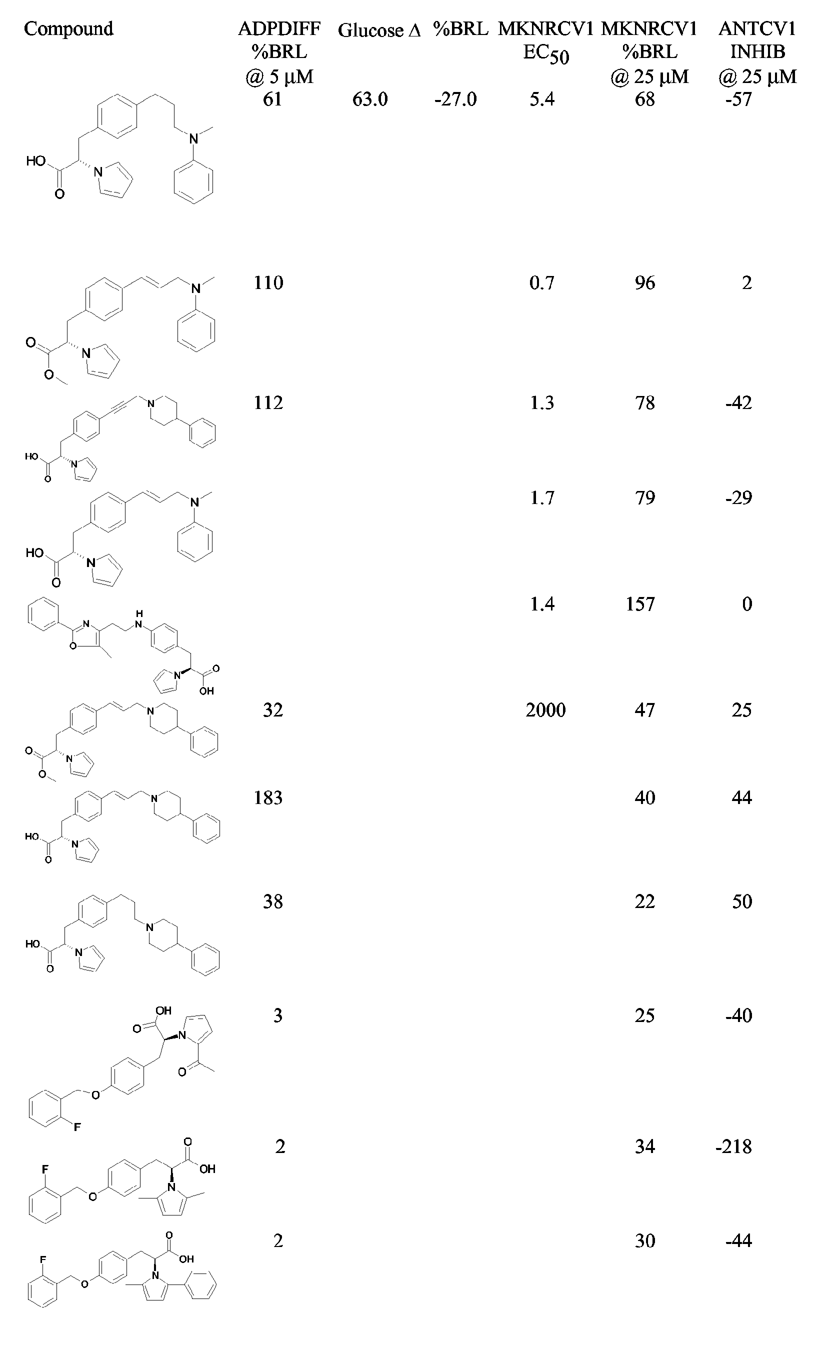

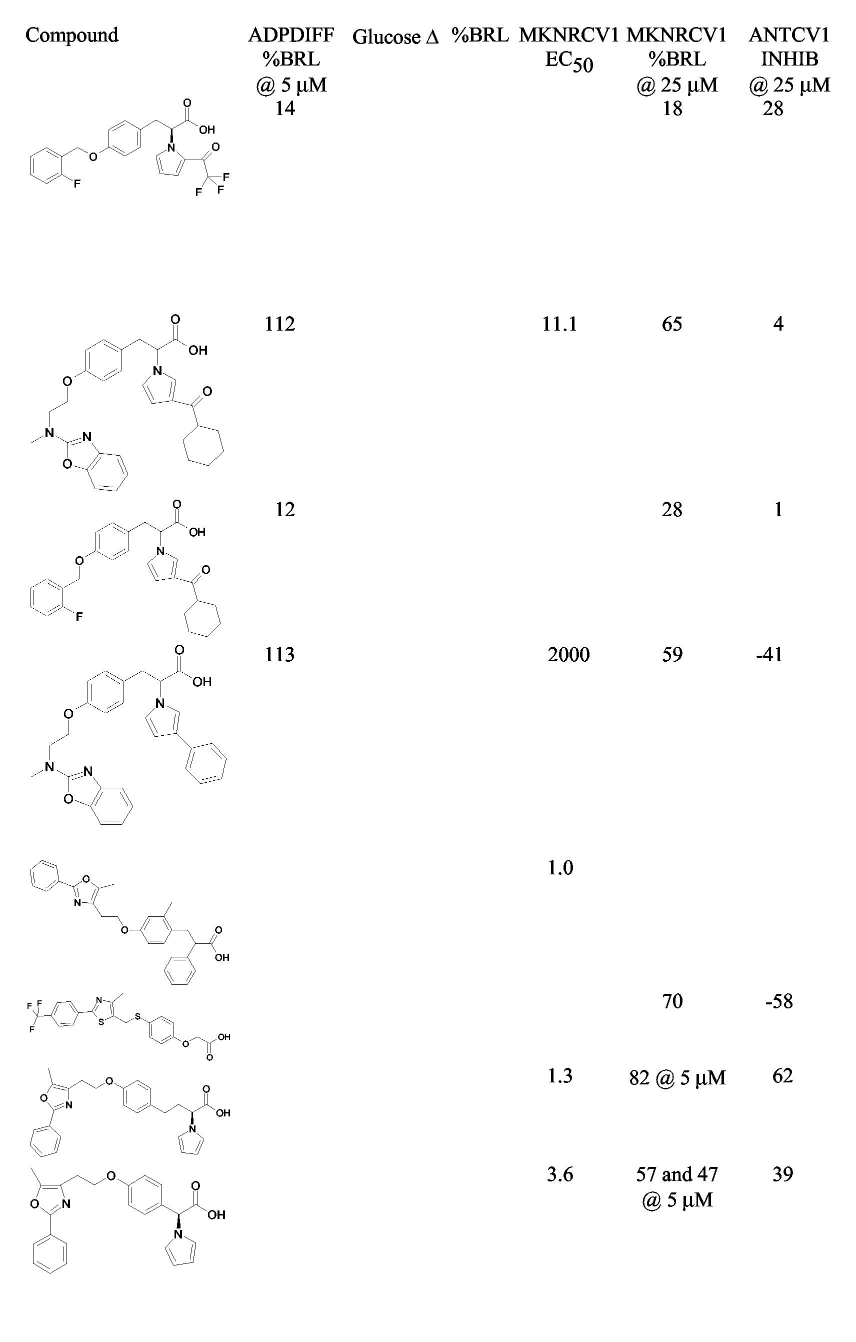

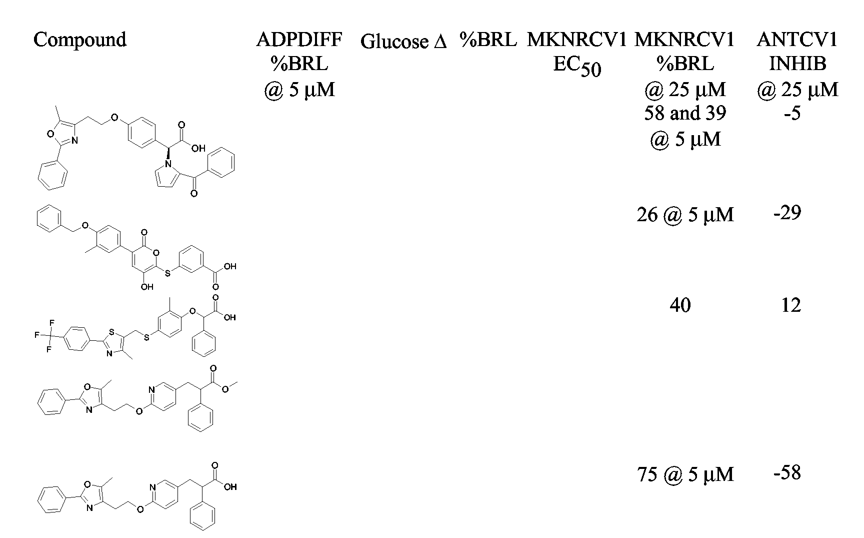

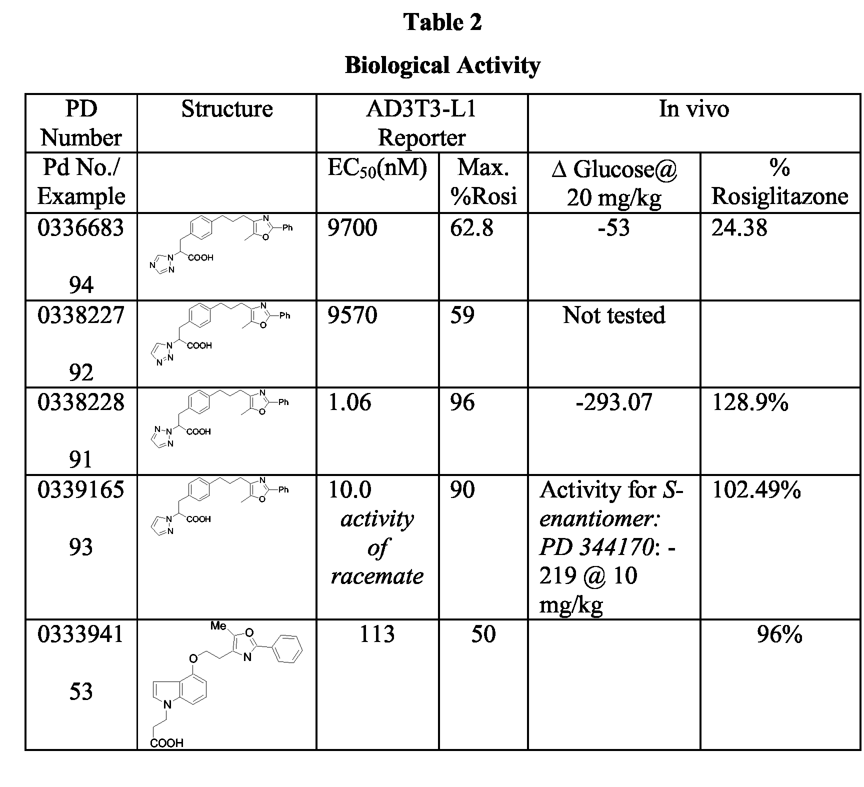

Referring to Table 2, PD 0333941 was shown to lower blood glucose in Ob/Ob diabetic mouse model at 3 mg/Kg. The compound shows a dose response with a maximal effect at 30 mg/Kg.

Referring again to Table 2, PD 0338228 was evaluated for its selective activity against the three types of PPARs using HepG2 cells. In these screens, PD 338228 showed an EC₅₀ = 71.8 nM in the HepG2-hALPHA assay (PPAR alpha) and an EC50 = 92.5 nM in the HepG2-mGAMMA assay (PPAR gamma). It was inactive against PPAR beta. In the PPAR alpha assay, PD 3328228 at max. effect gives only 61% of the max. activity of the reference agent (GW9578). The results obtained with Rosiglitazone and PD 326234 are included in the table shown below.

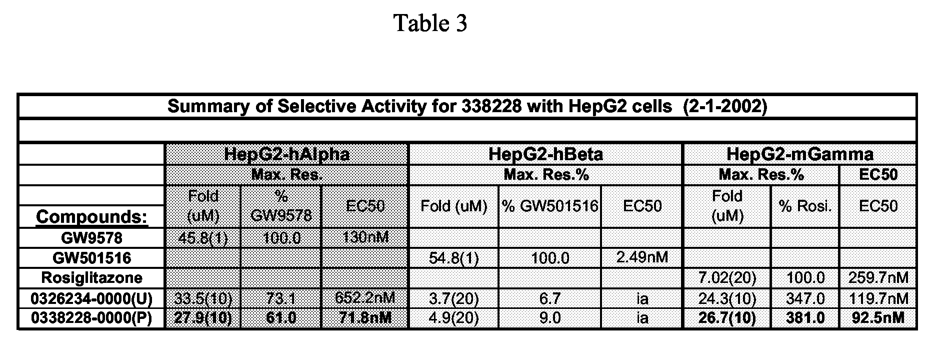

In the table, GW9578 refers to:

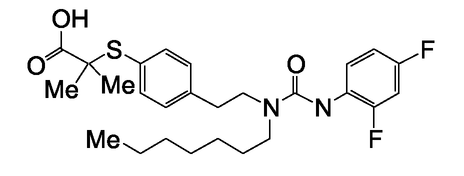

GW501516 refers to:

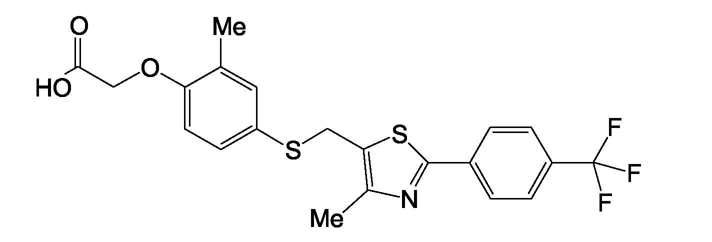

PD 338228 was identified as a potent, PPARgamma full agonist in the AD3T3-L1 reporter assay. In addition, this compound acts as a dual alpha/gamma agonist as demonstrated in the selectivity assays. It showed a partial agonist profile for PPARalpha. When evaluated in the Ob/Ob mice model for diabetes, PD 338228 was capable of completely normalizing plasma glucose levels at 20 mg/kg in 14 days. Its activity was comparable, or even better, than that of rosiglitazone. PD 338228 is a potential agent for the treatment of type II diabetes and dislipedemias, as that condition is described, for instance, in the Merck Manual (1992).

In general, compounds of the invention may induce fat cell differentiation as described in test A. Results from test E, in particular, demonstrate that the compounds of the invention may lower glucose levels in mice.

Because compounds of the invention induce adipocyte differentiation and lower blood glucose levels, they may be useful in treating disorders associated with insulin resistance. Such disorders include: NIDDM, diabetic angiopathy, atherosclerosis, diabetic nephropathy, diabetic neuropathy, and diabetic ocular complications such as retinopathy, cataract formation and glaucoma, as well as glucocortcoid induced insulin resistance, dyslipidemia, polycysitic ovarian syndrome, obesity, hyperglycemia, hyperlipidemia, hypercholerteremia, hypertriglyceridemia, hyperinsulinemia, and hypertension.

Accordingly, the invention also includes a method for treating a disease in a human or other mammal in need thereof in which insulin resistance has been implicated and glucose lowering is desired, comprising administering to said human or mammal an effective amount of a compound of formula I or a pharmaceutically acceptable salt thereof. The invention also includes a method for treating or preventing insulin resistance in a mammal comprising administering to said mammal an effective amount of a compound of formula I or a pharmaceutically acceptable salt thereof. Additionally, compounds of the invention can be used in vitro or in vivo as pharmacological and biochemical tools to assist in the discovery and evaluation of other glucose lowering agents and PPAR γ agonists.

Esters of the general formula VI are readily hydrolyzed to compounds of the invention. Accordingly, the invention also includes a method of preparing a compound of formula I by hydrolyzing a compound of formula VI.

Additionally, esters of the general formula VI may be determined to function as prodrugs for compounds of formula I. Accordingly, the invention also includes a method for treating a disease in a human or other mammal in need thereof in which insulin resistance has been implicated and glucose lowering is desired, comprising administering to said human or mammal an effective amount of a compound of formula VI; or a pharmaceutically acceptable salt thereof.

### EXAMPLES

### Example 1

### (S)-3-{4-[2-(5-Methyl-2-phenyl-oxazol-4-yl)-ethoxy]-phenyl}-2-pyrrol-1-yl-propionic acid (3).

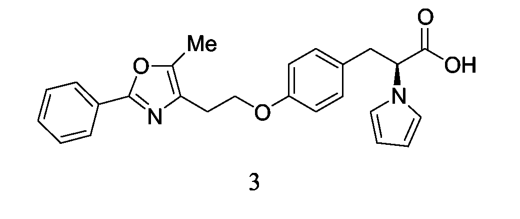

**General Procedure for Ester Hydrolysis (General Procedure A).** (S)-3-{4-[2-(5-Methyl-2-phenyl-oxazol-4-yl)-ethoxy]-phenyl}-2-pyrrol-1-yl-propionic acid methyl ester (400 mg, 0.930 mmol) was dissolved in THF (10 mL) and H₂O (10 mL). LiOH monohydrate (45 mg, 1.07 mmol) was added in one portion and the suspension stirred for 1 hour. The reaction was diluted with EtOAc (20 mL) and H₂O (20 mL) and organic layer separated. After acidification of the aqueous layer to pH of approximately 2 with aq.HCl and extraction with EtOAc (2x20 mL), the organic layers were combined and washed with H₂O (10 mL), brine (10 mL), and dried over MgSO₄. Removal of solvent *in vacuo* gave 3 as white crystals (320 mg, 0.769 mmol). Recrystalized from EtOAc/Hex as fine white needles in 51% yield. M.P. = 155-56 °C. C₂₅H₂₄N₂O₄ Mass Calc. = 416.475; M+1(obs) =417.2. ¹H NMR (400MHz) CDCl₃ δ: 7.91 (2H, m), 7.38 (3H, m), 6.90 (2H, d, *J*=8.5 Hz), 6.71 (2H, d, *J*=8.5 Hz), 6.69 (2H, t, *J*=2.0, 2.1 Hz), 6.12 (2H, t, *J=*1.9, 1.9 Hz), 4.69 (1H, dd, *J*=7.08, 8.03 Hz), 4.16 (2H, t, 6.5, 6.5 Hz), 3.34 (1H, dd, *J*=6.6, 6.6 Hz), 3.17 (1H, dd, *J*=8.3, 8.3 Hz), 2.93 (2H, t, *J*=6.6, 6.6 Hz), 2.32 (3H, s). CHN (theoretical=C=72.10, H=5.81, N=6.73, CHN obs.; C=72.09, H=5.58, N=6.63.

(S)-3-{4-[2-(5-Methyl-2-phenyl-oxazol-4-yl)-ethoxy]-phenyl}-2-pyrrol-1-yl-propionic acid methyl ester (2) was prepared in the following manner.

### (a) (S)-3-{4-[2-(5-Methyl-2-phenyl-oxazol-4-yl)-ethoxy]-phenyl}-2-pyrrol-1-yl-propionic acid methyl ester (2).

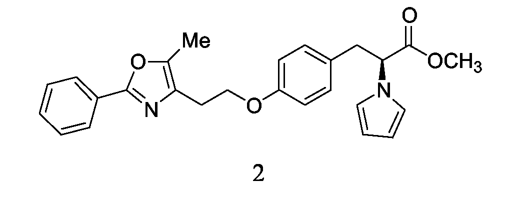

**General Coupling Procedure (General Procedure B).** Pyrrolotyrosine methyl ester (1) (2.68 g, 10.9 mmol), 2-(5-Methyl-2-phenyloxazol-4-yl) ethanol (2.22g, 10.9 mmol) and triphenylphosphine (3.15g, 12.0 mmol) were dissolved in anhydrous THF (100 mL) under N₂ at 0 °C. A solution of diethyl azodicarboxylate (DEAD) (1.89 mL, 12.0 mmol) in THF (50 mL) was added slowly over 30 min. and allowed to equilibrate to 23 °C over 18 hr. The solution was diluted with EtOAc (50mL) and washed with water (30 mL), brine (30 mL) and dried over MgSO₄. After removal of solvent *in vacuo,* SiO₂ gel chromatography with 10%EtOAc/Hex gave 2.4 g of **2** in 51 % as a clear oil. Low Resolution Mass Spectroscopy (LRMS) C₂₆H₂₆N₂O₄ MW=430.501 calc. M+1 = 431.2 obs. ¹H NMR (400MHz) CDCl₃ δ: 7.95 (2H, d, *J*=5.8Hz), 7.40 (3H, m), 6.85 (2H, d, *J*=8.3 Hz), 6.72 (2H, d, *J*=8.5 Hz), 6.66 (2H, s), 6.10 (2H, s), 4.63 (1H, dd, *J*=6.83, 8.30 Hz), 4.16 (2H, t, 6.6, 6.6 Hz), 3.65 (3H, s), 3.29 (1H, dd, *J*=6.3, 6.3 Hz), 3.13 (1H, dd, *J*=8.8, 8.8 Hz), 2.93 (2H, t, *J*=6.6, 6.6 Hz), 2.32 (3H, s).

### (b) Pyrrolotyrosine methyl ester (1).

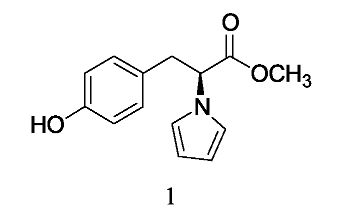

S-tyrosine methyl ester (25.6 g, 131 mmol), 2,5-dimethoxytetrahydrofuran (17 mL, 223 mmol) and NaOAc (21.5 g, 262 mmol) were dissolved in a 1:1 mixture of H₂O and acetic acid (150 mL:150 mL) and stirred until homogeneous (ca. 30 min.). The temperature was then raised to 100°C for 20 min. in an oil bath during which time the solution turned a dark brown color. After the indicated period of time, the solution was removed from the hot bath and allowed to cool to 23°C. The reaction mixture was diluted with H₂O (100 mL) and and extracted with EtOAc (3 x 75 mL). The organic layers were combined and washed consecutively with H₂O (2 x 75 mL), brine (50 mL) and dried over Mg₂SO₄. The solvent was removed *in vacuo* and the crude residue was chromatographed with 5% MeOH/CHCl₃ to give 15 g (35% yield) of cream colored crystals. ¹H NMR (400MHz) δ (CDCl₃) 6.83 (2H, d, 8 Hz), 6.67 (2H, d, 2Hz), 6.65 (2H, d, 8Hz), 6.11 (2H, d, 2Hz), 4.65 (1H, q, 6Hz), 4.66 (1H, s), 3.66 (3H, s), 3.31 (1H, dd, 6 Hz), 3.28 (1H, dd, 6 Hz).

The procedures described in Example 1 were used to prepare (R)-3-{4-[2-(5-Methyl-2-phenyl-oxazol-4-yl)-ethoxy]-phenyl}-2-pyrrol-1-yl-propionic acid and racemic (S)-3-{4-[2-(5-Methyl-2-phenyl-oxazol-4-yl)-ethoxy]-phenyl}-2-pyrrol-1-yl-propionic acid.

### Example 2

### (S)-2-(2-Benzoyl-pyrrol-1-yl)-3-{4-[2-(5-methyl-2-phenyl-oxazol-4-yl)-ethoxy]-phenyl]-propionic acid (9).

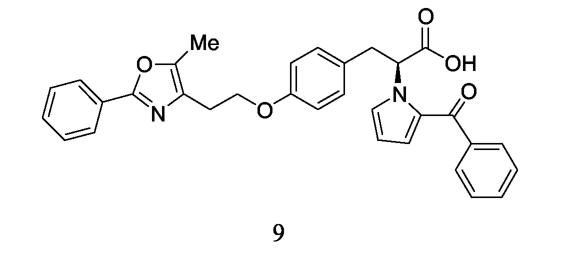

Ester hydrolysis of (S)-2-(2-Benzoyl-pyrrol-1-yl)-3-{4-[2-(5-methyl-2-phenyl-oxazol-4-yl)-ethoxy]-phenyl}-propionic acid methyl ester (8) was carried out in a manner similar to General Procedure A. After treatment with LiOH, (S)-2-(2-Benzoyl-pyrrol-1-yl)-3-{4-[2-(5-methyl-2-phenyl-oxazol-4-yl)-ethoxy]-phenyl}-propionic acid methyl ester (9) (498mg, 0.932 mmol) gave 430 mg of (S)-2-(2-Benzoyl-pyrrol-1-yl)-3-{4-[2-(5-methyl-2-phenyl-oxazol-4-yl)-ethoxy]-phenyl}-propionic acid (10) (0.827 mmol) in 89% yield. C₃₂H₂₈N₂O₅ Mol. Wt.: 520.5752; M+1(obs)= 521.2. ¹H NMR (400 MHz, CDCl₃) δ: 7.93 (2H, d, *J*=8.1Hz), 7.66 (2H, d, *J*=7.6 Hz), 7.48 (1H, m), 7.37 (6H, m), 7.16 (1H, s), 6.92 (2H, d, *J*=8.3Hz), 6.67 (2H, d, *J*=8.3Hz), 6.64 (1H, d, *J*=3.2Hz), 6.16 (1H, t, *J*=2.9, 2.9Hz), 5.78(1H, s), 4.09 (2H, m), 3.52 (1H, dd, *J*=6.1, 14.4 Hz), 3.21 (1H, dd, *J*=9.03, 14.1 Hz), 2.91 (2H, t, *J*=6.6, 6.6Hz), 2.31 (3H, s).

(S)-2-(2-Benzoyl-pyrrol-1-yl)-3-{4-[2-(5-methyl-2-phenyl-oxazol-4-yl)-ethoxy]-phenyl}-propionic acid methyl ester (8) was prepared in the following manner.

### (a) (S)-2-(2-Benzoyl-pyrrol-1-yl)-3-{4-[2-(5-methyl-2-phenyl-oxazol-4-yl)-ethoxy]-phenyl}-propionic acid methyl ester (8).

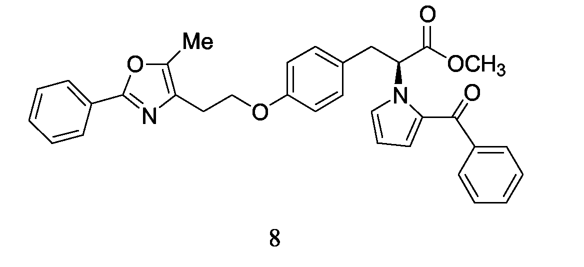

(S)-2-(2-Benzoyl-pyrrol-1-yl)-3-(4-hydroxy-phenyl)-propionic acid methyl ester (7) was coupled to 2-(5-Methyl-2-phenyloxazol-4-yl) ethanol in a manner similar to that described in General Procedure B. Phenol 7 (3.1 g, 8.88 mmol), was treated with 1 mole equivalent each of alcohol (1.8 g, 8.88 mmol), triphenylphosphine (2.33 g, 8.88 mmol), and diethyl azodicarboxylate (1.40 mL, 8.88 mmol) and gave 2.3g (4.31 mmol) of 2-substituted product in 49% yield as a pale yellow oil. C₃₃H₃₀N₂O₅ Mol. Wt.: 534.602; M+1(obs)=535.2. ¹H NMR (CDCl₃) δ: 7.96 (2H, dd, *J*=1.9, 7.8 Hz), 7.54 (2H, m), 7.40 (6H, m), 7.09 (1H, s), 6.88 (2H, d, *J*=8.5Hz), 6.66 (2H, d, *J*=8.5 Hz), 6.56 (1H, dd, *J*=1.5, 3.9Hz), 6.12 (1H, dd, *J*=1.5, 2.7Hz), 6.10 (1H, bs), 4.10 (2H, m), 3.74 (3H, s), 3.44 (1H, dd, *J*=5.4, 13.9Hz), 3.18 (1H, dd, J= 10, 14 Hz), 2.91 (2H, t, *J*=6.6, 6.6 Hz), 2.32 (3H, s).

### (b) (S)-2-(2-Benzoyl-pyrrol-1-yl)-3-(4-hydroxy-phenyl)-propionic acid methyl ester (7).

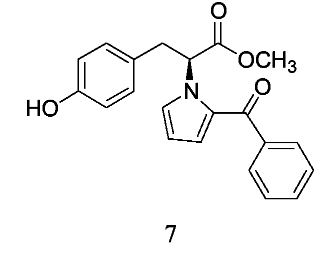

**General Procedure C.** Benzoic acid (S)-4-[2-(2-benzoyl-pyrrol-1-yl)-2-methoxycarbonyl-ethyl]-phenyl ester (5) (300mg, 0.661 mmol) was dissolved in anhydrous methyl alcohol (10 mL) and placed under N₂ at 23°C. After addition of K₂CO₃ (136 mg, 0.991 mmol) the suspension was vigorously stirred for 3 hours and periodically monitored by TLC. Once complete, the methanol was removed *in vacuo* and the residue chromatographed with 10% EtOAc/Hexanes to give free phenol 7 in 90% yield (200 mg, 0.60 mmol) as white powder. (S)-2-(2-Benzoylpyrrol-1-yl)-3-(4-hydroxy-phenyl)-propionic acid methyl ester (7). C₂₁H₁₉NO₄ Mol. Wt.: 349.380; M+1 (obs) = 350.3. ¹H NMR (400 MHz, CDCl₃) δ: 7.57 (2H, dd, *J*=1.5, 7.5 Hz) 7.46 (1H, m), 7.36 (2H, m), 7.09 (1H, s), 6.86 (2H, d, *J*=8.5 Hz), 6.60 (2H, d, *J*=8.5 Hz), 6.60 (1H, s), 6.13 (1H, dd, *J*=2.7, 4.1 Hz), 6.09 (1H, s), 4.62 (1H, s), 3.73 (3H, s), 3.44 (1H, dd, *J*=5.6, 14.1 Hz), 3.17 (1H, dd, *J*=9.8, 14.1 Hz).

### (c) Benzoic acid (S)-4-[2-(2-benzoyl-pyrrol-1-yl)-2-methoxycarbonylethyl]-phenyl ester (5) and Benzoic acid (S)-4-[2-(3-benzoylpyrrol-1-yl)-2-methoxycarbonyl-ethyl]-phenyl ester (6).

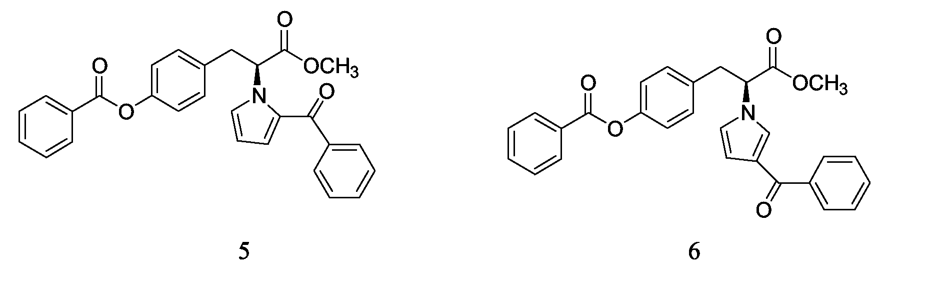

**General Procedure D.** Trifluoromethane sulfonic acid (45µL, 0.516 mmol) was added to a stirring solution of Benzoic acid 4-(2-methoxycarbonyl-2-pyrrol-1-yl-ethyl)-phenyl ester (4) (150 mg, 0.430 mmol) and benzoyl chloride (60 µL, 0.516 mmol) in dichloromethane (20 mL) at 28°C under N₂. After stirring for 18 hours, solvent was removed *in vacuo.* SiO₂ gel chromatography of the resulting residue with 10%EtOAc/Hexanes gave 80 mg of 2 substituted pyrrole (5) (0.177mmol, 40%) and 37 mg of 3-substituted (6) (0.082 mmol, 20% yield).

Benzoic acid (S)-4-[2-(2-benzoyl-pyrrol-1-yl)-2-methoxycarbonyl-ethyl]-phenyl ester (5) C₂₈H₂₃NO₅: Mass (calc) 453.4860; M+1 (obs)=454.2. ¹H NMR (400 MHz, CDCl₃) δ: 8.13 (2H, dd, *J*=1.2, 8.5 Hz), 7.61 (3H, m), 7.47 (3H, m), 7.40 (2H, m), 7.07 (2H, d, *J*=8.8 Hz),7.04 (1H, s), 7.00 (2H, d, *J*=8.8 Hz), 6.62 (1H, t, *J*=1.7, 2.4 Hz), 6.15 (1H, dd, *J*=2.7, 3.9 Hz), 6.15 (1H, s), 3.76 (3H, s), 3.55 (1H, dd, *J*=5.6, 14.1Hz), 3.29 (1H, dd, *J*=10, 14.1 Hz).

Benzoic acid (S)-4-[2-(3-benzoyl-pyrrol-1-yl)-2-methoxycarbonyl-ethyl]-phenyl ester (6). C₂₈H₂₃NO₅: Mass (calc) 453.4860; M+1 (obs)=454.2. ¹H NMR (400 MHz, CDCl₃) δ: 8.14 (2H, d, *J*=7.1 Hz), 7.70 (2H, d, *J*=7.1 Hz), 7.60 (1H, m), 7.43 (5H, m), 7.16 (1H, t, *J*=1.7, 1.9 Hz), 7.09 (2H, d, *J*=8.8), 7.04 (2H, d, *J*=8.5Hz), 6.76 (1H, q, *J*=1.0, 1.9, 2.2 Hz), 6.69 (1H, q, *J*=1.2, 1.7, 1.7 Hz), 4.73 (1H, dd, *J*=5.9, 9.5 Hz), 3.74 (3H, S), 3.43 (1H, dd, 5.8, 14.1 Hz), 3.25 (1H, dd, *J*=9.2, 13.9 Hz).

### (d) Benzoic acid 4-(2-methoxycarbonyl-2-pyrrol-1-yl-ethyl)-phenyl ester (4).

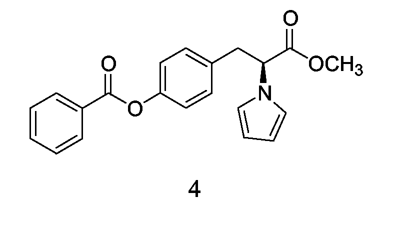

**General Procedure E.** Triethylamine (290 µL, 2.06 mmol) was added to a stirring solution of Pyrrolotyrosine methyl ester (252 mg, 1.03 mmol) in dichloromethane (20 mL) under N₂ at 23°C. Benzoyl chloride (143 µL, 1.23 mmol) was added dropwise over a 5 minute period and the reaction was stirred for 2 hours. Solvent was removed *in vacuo* and chromatographed via SiO₂ with 10% EtOAc/hexanes to give the benzoyl ester 4 in 86% yield (310 mg, 0.89 mmol) as a white foam. C₂₁H₁₉NO₄ Mass Calc. = 349.380; M+1 (obs)= 350.2. ¹H NMR (400MHz, CDCl₃) δ: 8.17 (2H, d, *J*=6.35 Hz), 7.62 (1H, m), 7.47 (2H, m), 7.09 (2H, d, *J*=8.8Hz), 7.04 (2H, d, *J*=8.6Hz), 6.71 (2H, t, *J*=2.2, 2.1 Hz), 6.16 (2H, t, *J*=2.2, 2.2 Hz), 4.72 (1H, dd, *J*=2.4, 2.4 Hz), 3.70 (3H, s), 3.43 (1H, dd, *J*=6.3, 6.3 Hz), 3.25 (1H, dd, *J*=9.0, 9.0 Hz).

### Example 3

### (S)-2-(3-Benzoyl-pyrrol-1-yl)-3-{4-[2-(5-methyl-2-phenyl-oxazol-4-yl)-ethoxy]-phenyl]-propionic acid (12).

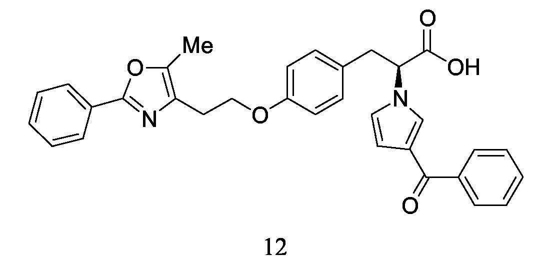

Ester hydrolysis was carried out in a manner similar to General Procedure A. After treatment with LiOH, (S)-2-(3-Benzoyl-pyrrol-1-yl)-3-{4-[2-(5-methyl-2-phenyl-oxazol-4-yl)-ethoxy]-phenyl}-propionic acid methyl ester (11) (400mg, 0.749 mmol) gave 260mg of carboxylic acid (S)-2-(3-Benzoyl-pyrrol-1-yl)-3-{4-[2-(5-methyl-2-phenyl-oxazol-4-yl)-ethoxy]-phenyl}-propionic acid (12). (0.500 mmol) in 67% yield. C₃₂H₂₈N₂O₅ Mol. Wt.: 520.5752; M+1(obs)= 521.2. ¹H NMR (400 MHz, CDCl₃) δ: 7.92 (2H, m), 7.62 (2H, d, *J*=8.0Hz), 7.26 (6H, m), 7.06 (1H, s), 6.85 (2H, d, *J*=8.3 Hz), 6.69 (2H, d, *J*=7.8Hz), 6.68 (1H, s), 6.60 (1H, t, *J*=1.5, 1.5 Hz), 4.65 (1H, t, *J*=6.8, 8.1Hz), 4.09 (3H,m), 3.31 (1H, dd, *J*=6.6, 14.0 Hz), 3.10 (1H, dd, *J*=9.0, 14.1Hz), 2.95 (2H, *J*=6.6, 6.1Hz), 2.31 (3H, s).

(S)-2-(3-Benzoyl-pyrrol-1-yl)-3-{4-[2-(5-methyl-2-phenyl-oxazol-4-yl)-ethoxy]-phenyl}-propionic acid methyl ester (11) was prepared in the following manner.

### (a) (S)-2-(3-Benzoyl-pyrrol-1-yl)-3-{4-[2-(5-methyl-2-phenyl-oxazol-4-yl)-ethoxy]-phenyl}-propionic acid methyl ester (11).

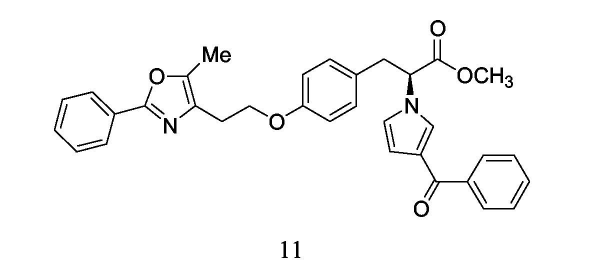

Prepared in a manner similar to that described in General Procedure B. (S)-2-(3-Benzoyl-pyrrol-1-yl)-3-(4-hydroxy-phenyl)-propionic acid methyl ester (8) (800 mg, 2.29 mmol), was treated with 1 mole equivalent each of 2-(5-Methyl-2-phenyloxazol-4-yl) ethanol (465 mg, 2.29 mmol), triphenylphosphine (600 mg, 2.29 mmol), and diethyl azodicarboxylate (360 µL, 2.29 mmol) and gave 400 mg (0.749 mmol) of 3-substituted product (11) in 32% yield as a pale yellow oil. C₃₃H₃₀N₂O₅ Mol. Wt.: 534.602; M+1(obs)=535.2. ¹H NMR (400 MHz, CDCl₃) δ: 8.00 (2H, m), 7.67 (2H, d, *J*=6.8Hz), 7.40 (6H, m), 7.08 (1H, t, *J*=1.9, 1.9Hz), 6.84 (2H, d, *J*=8.5Hz), 6.72 (2H, d, *J*=8.5Hz), 6.71 (1H, t, *J*=2.2, 2.2Hz), 6.66 (1H, t, *J*=1.7Hz), 4.65 (1H, dd, *J*=5.6Hz), 4.16 (2H, m), 3.71 (3H, s), 3.31 (1H, dd, *J*=5.8, 14.1Hz), 3.13 (1H, dd, *J*=9.3, 14.0Hz), 2.98 (2H, m), 2.01 (3H, s).

### (b) (S)-2-(3-Benzoyl-pyrrol-1-yl)-3-(4-hydroxy-phenyl)-propionic acid methyl ester (10).

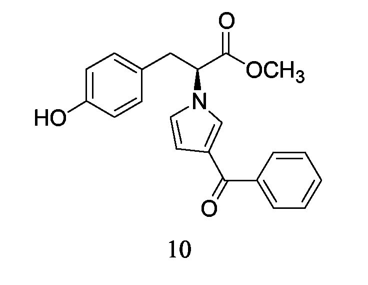

Prepared from benzoic acid (S)-4-[2-(3-benzoyl-pyrrol-1-yl)-2-methoxycarbonyl-ethyl]-phenyl ester (6) according to General Procedures C-E. C₂₁H₁₉NO₄ Mol. Wt.: 349.380; M+1 (obs) = 350.3. ¹H NMR (400 MHz, CDCl₃) δ: 7.68 (2H, d, *J*=6.8 Hz), 7.48 (1H, m), 7.40 (3H, m), 7.10 (1H, t, *J*=1.7, 1.9Hz), 6.81 (2H, d, *J*=8.5Hz), 6.73 (1H, t, *J*=2.9 Hz, 2.2 Hz), 6.67 (2H, d, *J*=8.5Hz), 6.65 (1H, s), 4.66 (1H, dd, *J*=5.6, 9.2 Hz), 3.72 (3H, s), 3.31 (1H, dd, *J*=5.6, 13.9 Hz), 3.13 (1H, dd, *J*=9.5, 14.1 Hz).

### Example 4

(S)-3-{4-[2-(5-Methyl-2-phenyl-oxazol-4-yl)-ethoxy]-phenyl}-2-(3-phenyl-pyrrol-1-yl)-propionic acid (15).

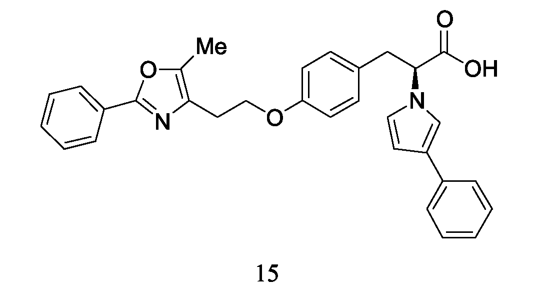

The compound was prepared as described in General Procedure A. (S)-3-{4-[2-(5-Methyl-2-phenyl-oxazol-4-yl)-ethoxy]-phenyl}-2-(3-phenyl-pyrrol-1-yl)-propionic acid methyl ester (14) (1.6g, 3.14 mmol) was hydrolyzed to give (S)-3-{4-[2-(5-Methyl-2-phenyl-oxazol-4-yl)-ethoxy]-phenyl}-2-(3-phenyl-pyrrol-1-yl)-propionic acid (15) (1.2 g, 2.43 mmol) in 77% yield as cream colored crystals, which were recrystalized from EtOAc/Hexanes. Mp. 146°C-147°C. C₃₁H₂₈N₂O₄ Mol. Wt.: 492.565; M+1 = 493.2. ¹H NMR (400 MHz, CDCl₃) δ: 7.91 (2H, dd, *J*=3.7, 7.3Hz), 7.44 (2H, d, *J*=7.3Hz), 7.37 (3H, m), 7.27 (2H, t, *J*=7.5, 7.8Hz), 7.11 (1H, t, *J*=7.3, 7.3Hz), 7.01 (1H, s), 6.93 (2H, d, *J*=8.5Hz), 6.71 (2H, d, *J*=8.5Hz), 6.69 (1H, t, *J*=2.4, 2.4 Hz), 6.42 (1H, t, *J*=2.0, 2.7Hz), 4.67 (1H, t, *J*=7.1, 7.8Hz), 4.10 (2H, t, *J*=6.3, 6.6Hz), 3.36 (1H, dd, *J*=6.8, 14.2Hz), 3.18 (1H, dd, *J*=8.0, 13.9Hz), 2.93 (2H, m), 2.32 (3H, s). CHN(theoretical) C= 75.59; H= 5.73; N= 5.69; (Obs) C= 75.42; H= 5.80; N= 5.50.

(S)-3-{4-[2-(5-Methyl-2-phenyl-oxazol-4-yl)-ethoxy]-phenyl}-2-(3-phenyl-pyrrol-1-yl)-propionic acid methyl ester (14) was prepared in the following manner.

### (a) (S)-3-{4-[2-(5-Methyl-2-phenyl-oxazol-4-yl)-ethoxy]-phenyl}-2-(3-phenyl-pyrrol-1-yl)-propionic acid methyl ester (14).

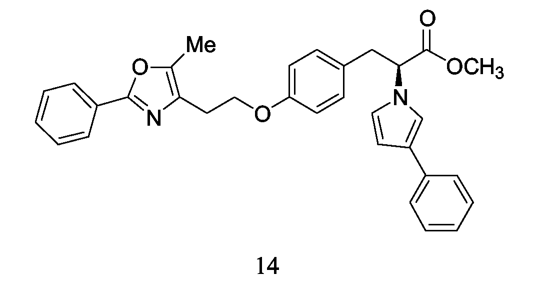

The compound was prepared via Mitsunobu coupling as described in General Procedure B. (S)-3-(4-Hydroxy-phenyl)-2-(3-phenyl-pyrrol-1-yl)-propionic acid methyl ester (13) (2.0 g, 6.23 mmol) gave coupled product (S)-3-{4-[2-(5-Methyl-2-phenyl-oxazol-4-yl)-ethoxy]-phenyl}-2-(3-phenyl-pyrrol-1-yl)-propionic acid methyl ester (14) (1.6g, 3.16 mmol) in 51% yield as a pale yellow oil. C₃₂H₃₀N₂O₄ Mol. Wt.: 506.592; M+1(obs)=507.1. ¹H NMR (400 MHz, CDCl₃) δ: 7.94 (2H, d, *J*=8.1Hz), 7.46 (2H, d, *J*=8.1Hz), 7.40 (3H, m), 7.29 (2H, t, *J*=7.3, 7.6Hz), 7.12 (1H, t, *J*=7.3, 7.2Hz), 7.00 (1H, s), 6.90 (2H, d, *J*=8.1Hz), 6.74 (2H, d, *J*=8.1Hz), 6.68 (1H, s), 6.42 (1H, s), 4.65 (1H, t, *J*=7.8, 7.3 Hz), 4.17 (2H, t, *J*=6.6, 6.8Hz), 3.69 (3H, s), 3.33 (1H, dd, *J*=6.6, 13.9Hz), 3.19 (1H, dd, *J*=9.3, 13.7Hz), 2.92 (2H, t, *J*=6.6, 7.1 Hz), 2.35 (3H, s).

### (b) (S)-3-(4-Hydroxy-phenyl)-2-(3-phenyl-pyrrol-1-yl)-propionic acid methyl ester (13).

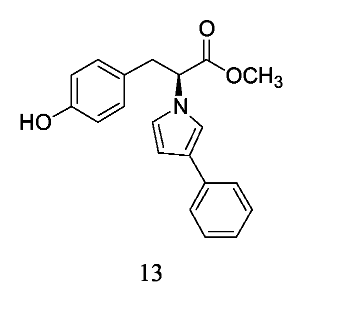

Tyrosine methyl ester (390 mg, 2.0 mmol) was added to a suspension of 3-phenyl-2,5-dimethoxy-tetrahydrofuran (500 mg, 2.4 mmol) and NaOAc (328 mg, 2.4 mmol) in H₂O (10 mL) and HOAc (10 mL). After heating at reflux for 1 hour, 562 mg (1.75 mmol) of (S)-3-(4-Hydroxy-phenyl)-2-(3-phenyl-pyrrol-1-yl)-propionic acid methyl ester (13) was obtained in 87% yield as a pale yellow oil. C₂₀H₁₉NO₃ Mol. Wt.: 321.370; M+1(obs)=322.1. ¹H NMR (400 MHz, CDCl₃) δ: 7.47 (2H, d, *J*=7.3Hz), 7.30 (2H, t, *J*=7.6, 7.8Hz), 7.14 (1H, t, *J*=7.3, 7.3Hz), 7.01 (1H, s), 6.88 (2H, d, *J*=8.3Hz), 6.67 (1H, s), 6.67 (2H, d, *J*=8.5Hz), 6.43 (1H, s), 4.65 (1H, dd, *J*=6.3, 8.5Hz), 4.60 (1H, s), 3.71 (3H, s), 3.34 (1H, dd, *J*=6.3, 13.9Hz), 3.20 (1H, dd, *J*=8.8, 13.9 Hz).

### Example 5

### (S)-3-{4-[2-(Benzooxazol-2-yl-methyl-amino)-ethoxy]-phenyl}-2-(3-phenyl-pyrrol-1-yl)-propionic acid (16).

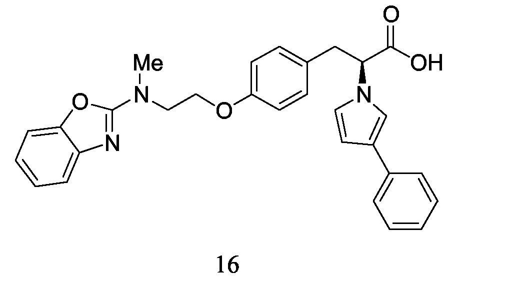

(S)-3-(4-Hydroxy-phenyl)-2-(3-phenyl-pyrrol-1-yl)-propionic acid methyl ester (13) was coupled with 2-(Benzooxazol-2-yl-methyl-amino)-ethanol as in General Procedure B (24% yield). Hydrolysis of the ester as in General Procedure A gave acid (S)-3-{4-[2-(Benzooxazol-2-yl-methyl-amino)-ethoxy]-phenyl]-2-(3-phenyl-pyrrol-1-yl)-propionic acid (16) in 34% yield. C₂₉H₂₇N₃O₄, Mol. Wt.: 481.543; M+1 = 482.2; ¹H NMR (400 MHz, CDCl₃) δ: 7.49 (2H, d, *J*=7.1Hz), 7.30 (5H, m), 7.13 (2H, dt, *J*=1.5, 7.3 Hz), 7.09 (1H, t, *J*=2.0, 2.0Hz), 7.00 (2H, d, *J*=8.3Hz), 6.77 (1H, t, *J*=2.4, 2.7Hz), 6.73 (2H, d, *J*=8.5Hz), 6.47 (1H, t, *J*=2.0, 2.7Hz), 4.73 (1H, t, *J*=7.6, 7.8Hz), 4.15 (1H, m), 4.02 (1H, m), 3.92 (1H, m), 3.80 (1H, m), 3.40 (1H, dd, *J*=7.6, 13.9 Hz), 3.26 (3H, s), 3.25 (1H, m).

### Example 6

### N-(S)-(3-{4-[2-(5-Methyl-2-phenyl-oxazol-4-yl)-ethoxy]-phenyl}-2-pyrrol-1-yl-propionyl)-Benzenesulfonamide (17).

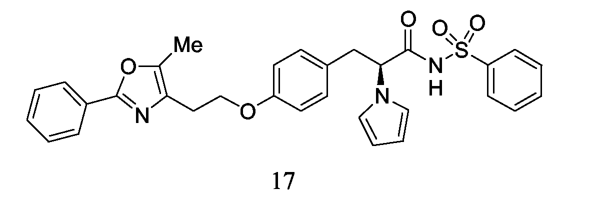

(S)-3-{4-[2-(5-Methyl-2-phenyl-oxazol-4-yl)-ethoxy]-phenyl}-2-pyrrol-1-yl-propionic acid (3) (275 mg, 0.661 mmol) was dissolved in CH₂Cl₂ (10 mL) under N₂ and at 23 °C. 1,1-carbonyldiimidazole (128 mg, 0.793 mmol) was then added and the solution stirred for 16 hours. In a separate flask, a 60% suspension of sodium hydride (26 mg, 0.661 mmol) was added to a stirring solution of benzenesulfonamide (124 mg, 0793 mmol) in dry DMF (5 mL) at -78°C under N₂. After 30 minutes, the solution was allowed to equilibrate to 23°C over 1 hour, then transferred drop-wise via syringe to the aforementioned solution of mixed anhydride. After two hours, the reaction was diluted with CH₂Cl₂ (50 mL) and washed with H₂O (20 mL), brine (20 mL), and dried over MgSO₄. SiO₂ gel chromatography with 30% EtOAc/Hex gave acylsulfonamide 17 (37 mg, 0.067 mmol) in 10% yield as a clear oil. C₃₁H₂₉N₃O₅S; Mol. Wt.: 555.645; M+1= 556.0. ¹H NMR (400 MHz, CDCl₃) δ: 8.01 (4H, m), 7.70 (1H, m), 7.55 (3H, m), 7.44 (2H, d, *J*=1.9Hz), 6.74 (2H, d, *J*=8.5Hz), 6.67 (2H, d, *J*=8.3Hz), 6.48 (2H, s), 6.21 (2H, s), 4.73 (1H, s), 4.55 (1H, dd, *J*=4.4, 10.5 Hz), 4.19 (2H, t, *J*=6.3, 6.1Hz), 3.41 (1H, dd, *J*=3.9, 14.6Hz), 3.02 (3H, m), 2.37 (3H, s).

### Example 7

### N-(S)-(3-{4-[2-(5-Methyl-2-phenyl-oxazol-4-yl)-ethoxy]-phenyl}-2-pyrrol-1-yl-propionyl)-Methanesulfonamide (18).

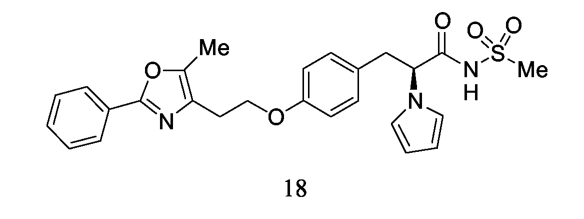

Using the procedure described in Example 6, (S)-3-{4-[2-(5-Methyl-2-phenyl-oxazol-4-yl)-ethoxy]-phenyl}-2-pyrrol-1-yl-propionic acid (3) (200 mg, 0.481mmol) gave N-(S)-(3-{4-[2-(5-Methyl-2-phenyl-oxazol-4-yl)-ethoxy]-phenyl]-2-pyrrol-1-yl-propionyl)-methanesulfonamide (18) (130 mg, 0.263) in 55% yield as a clear oil in a manner similar to that used to obtain N-(S)-(3-{4-[2-(5-Methyl-2-phenyl-oxazol-4-yl)-ethoxy]-phenyl}-2-pyrrol-1-yl-propionyl)-Benzenesulfonamide (17). C₂₆H₂₇N₃O₅S; Mol. Wt.: 493.576; M+1=494.2. ¹H NMR (400 MHz, CDCl₃) δ: 7.93 (2H, dd, *J*=2.4, 7.8Hz), 7.4 (3H, m), 6.99 (1H, s), 6.85 (2H, d, *J*=8.3Hz), 6.68 (2H, d, *J*=8.5Hz), 6.66 (2H, s), 6.03 (2H ,s), 4.62 (1H, bs), 4.57 (1H, m), 4.13 (2H, t, *J*=6.5, 6.8Hz), 3.35 (1H ,dd, *J*=5.1, 14.2 Hz), 3.09 (1H, m), 3.09 (3H, s), 2.91 (2H, t, *J*=6.6, 6.6Hz), 2.33 (3H, s).

### Example 8

### (S)-2-(3-Isobutyryl-pyrrol-1-yl)-3-{4-[2-(5-methyl-2-phenyl-oxazol-4-yl)-ethoxy]-phenyl}-propionic acid (21).

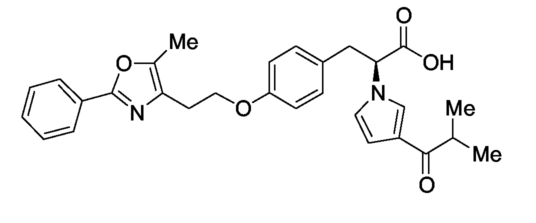

Hydrolysis of 107 mg of (S)-2-(3-Isobutyryl-pyrrol-1-yl)-3-{4-[2-(5-methyl-2-phenyl-oxazol-4-yl)-ethoxy]-phenyl}-propionic acid methyl ester (20) according to General Procedure A gave (S)-2-(3-Isobutyryl-pyrrol-1-yl)-3-{4-[2-(5-methyl-2-phenyl-oxazol-4-yl)-ethoxy]-phenyl}-propionic acid (21) (23 mg, 0.047 mmol) in 22% yield as a yellow oil. C₂₉H₃₀N₂O₅ Mol. Wt. for (S)-2-(3-Isobutyryl-pyrrol-1-yl)-3-{4-[2-(5-methyl-2-phenyl-oxazol-4-yl)-ethoxy]-phenyl}-propionic acid (21): 486.559; M-1=485.2. ¹H NMR (400 MHz, CDCl₃) δ: 7.96 (2H, dd, *J*=2.2, 2.2Hz), 7.41(3H, m), 7.31 (1H, t, *J*=2.0, 2.0Hz), 6.92 (2H, d, *J*=8.8Hz), 6.73 (2H, d, *J*=8.8Hz), 6.69 (1H, d, *J*=2.2Hz), 6.53 (1H, dd, *J*=1.4, 2.9Hz), 4.72 (1H, dd, *J*=7.1, 8.1Hz), 4.12 (2H, t, *J*=6.6, 6.8Hz), 3.38(1H, dd, *J*=6.8, 13.9Hz), 3.17 (1H, dd, *J*=8.1, 13.9Hz), 3.12 (1H, m), 2.99 (2H, t, *J*=6.6, 6.6Hz), 2.37 (3H, s), 1.11 (6H, dd, 6.8, 8.8Hz).

### (a) (S)-2-(3-Isobutyryl-pyrrol-1-yl)-3-{4-[2-(5-methyl-2-phenyloxazol-4-yl)-ethoxy]-phenyl}-propionic acid methyl ester (20) was prepared in the following manner.

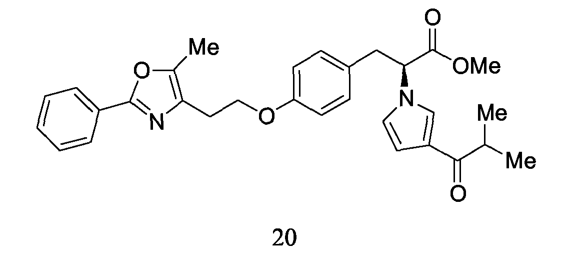

(S)-2-(3-Isobutyryl-pyrrol-1-yl)-propionic acid methyl ester (19) (300 mg, 0.952 mmol) was coupled with 2-(5-Methyl-2-phenyloxazol-4-yl) ethanol as in General Procedure B to give (S)-2-(3-Isobutyryl-pyrrol-1-yl)-3-{4-[2-(5-methyl-2-phenyl-oxazol-4-yl)-ethoxy]-phenyl]-propionic acid methyl ester (20) (120 mg, 0.240 mmol) in 25% yield.

### (b) (S)-2-(3-Isobutyryl-pyrrol-1-yl)-propionic acid methyl ester (19).

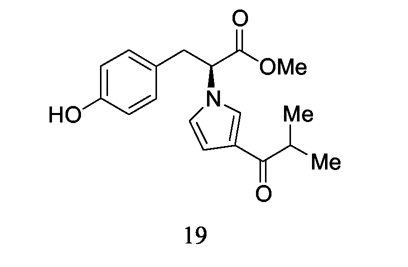

Prepared according to General Procedures C-E. The title compound was obtained in a manner similar to that used to obtain compound #10 utilizing isobutyryl chloride. NMR (δ;CHCl₃); 7.22 (1H, s); 6.79 (2H, d, J=8.5Hz); 6.69 (2H, d, J=8.4Hz); 6.67 (1H, m); 6.57 (1H, m);4.68 (1H, m); 3.74 (3H, s); 3.34 (1H, m); 3. 11 (2H, m); 1.13 (6H, t, J=7.0, 7.0 Hz). CIMS *m*/*z* 316.1 (M)⁺

### Example 9

### (S)-2-(3-Cyclohexanecarbonyl-pyrrol-1-yl)-3-{4-[2-(5-methyl-2-phenyloxazol-4-yl)-ethoxy]-phenyl}-propionic acid (24).

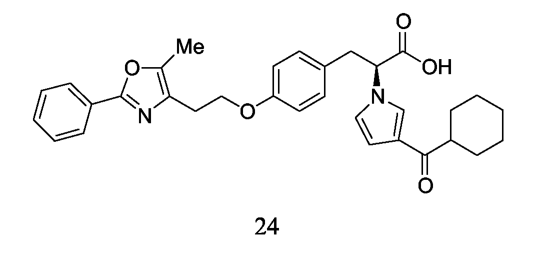

Prepared from (S)-2-(3-Cyclohexanecarbonyl-pyrrol-1-yl)-3-{4-[2-(5-methyl-2-phenyl-oxazol-4-yl)-ethoxy]-phenyl]-propionic acid methyl ester (23) as in General Procedure A. C₃₂H₃₄N₂O₅ Mol. Wt.: 526.623; M+1= 527.2. ¹H NMR (400 MHz, CDCl₃) δ: 7.91 (2H, dd, *J*=3.7, 7.1Hz), 7.39 (3H, t, *J*=3.2, 2.7Hz), 7.30 (1H, s), 6.89 (2H, m), 6.75 (3H, m), 6.45 (1H, m), 4.68 (1H, m), 4.09 (2H, m), 3.34 (1H, m), 3.13 (1H, m), 2.95 (2H, m), 2.79 (1H, m), 2.34 (3H, s), 1.74 (5H, m), 1.66 (1H, m), 1.45 (2H, m), 1.26 (1H, m).

(S)-2-(3-Cyclohexanecarbonyl-pyrrol-1-yl)-3-{4-[2-(5-methyl-2-phenyloxazol-4-yl)-ethoxy]-phenyl}-propionic acid methyl ester (23) was prepared in the following manner.

### (a) (S)-2-(3-Cyclohexanecarbonyl-pyrrol-1-yl)-3-{4-[2-(5-methyl-2-phenyl-oxazol-4-yl)-ethoxy]-phenyl]-propionic acid methyl ester (23).

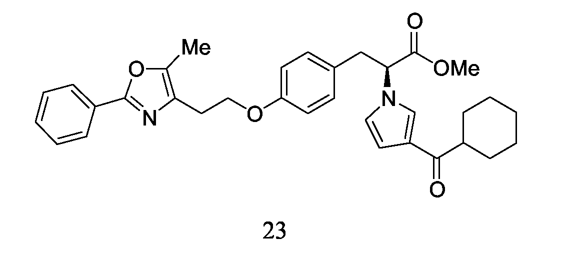

(S)-2-(3-Cyclohexanecarbonyl-pyrrol-1-yl)-propionic acid methyl ester (22) was coupled with 2-(5-Methyl-2-phenyloxazol-4-yl) ethanol according to General Procedure B to give the title compound.

### (b) (S)-2-(3-Cyclohexanecarbonyl-pyrrol-1-yl)-propionic acid methyl ester (22).

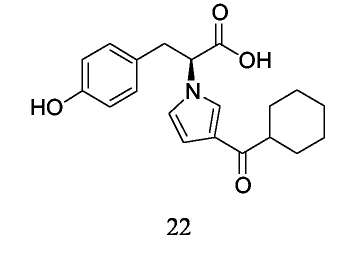

The title compound was obtained in 43% yield in a manner similar to that used to obtain compound #10 utilizing cyclohexane carbonylchloride. NMR (δ;CHCl₃);6.80 (2H, d, J=8.5Hz); 6.65 (2H, d, J=8.5Hz); 6.63 (1H, s); 6.54 (1H, s); 4.90 (1H, s); 4.65 (1H, m); 3.71 (3H, s); 3.30 (1H, m); 3.13 (1H, m); 2.81 (1H, m); 1.79 (4H, m); 1.67 (1H, m); 1.45 (2H, m); 1.29 (3H, m).

### Example 10

### (S)-3-{4-[2-(Benzooxazol-2-yl-methyl-ammo)-ethoxy]-phenyl}-2-(3-cyclohexanecarbonyl-pyrrol-1-yl)-propionic acid (26).

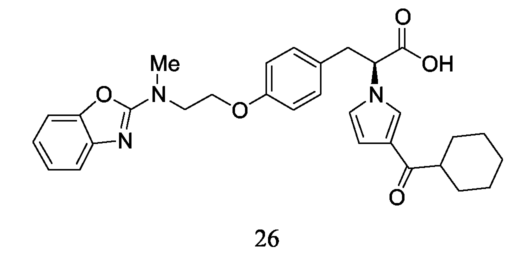

Hydrolysis of (S)-3-{4-[2-(Benzooxazol-2-yl-methyl-amino)-ethoxy]-phenyl}-2-(3-cyclohexanecarbonyl-pyrrol-1-yl)-propionic acid methyl ester (25) as in General Procedure A gave the title compound. C₃₀H₃₃N₃O₅ Mol. Wt.: 515.600; M+1=516.2. ¹H NMR (400 MHz, CDCl₃) δ: 7.35 (2H, m), 7.34 (1H, s), 7.23 (1H, s), 7.15 (1H, dt= *J*=1.0, 7.5 Hz), 7.02 (1H, dt, *J*=1.2, 7.8 Hz), 6.97 (2H, d, *J*=8.5Hz), 6.74 (1H, s), 6.72 (2H, d, *J*=8.5 Hz), 6.57 (1H, dd, *J*=1.5, 2.9 Hz), 4.73 (1H, t, *J*=7.6, 7.6 Hz), 4.07 (2H, m), 3.88 (2H, m), 3.37 (1H, dd, *J*=7.6, 13.9Hz), 3.28 (3H, s), 3.18 (1H, dd, *J*=7.6, 13.9 Hz), 2.83 (1H, t, *J*=8.8, 8.8 Hz), 1.77 (4H, m), 1.66 (1H, m), 1.46 (2H, dd, *J*=2.4, 3.9 Hz), 1.27 (3H, m).

(S)-3-{4-[2-(Benzooxazol-2-yl-methyl-amino)-ethoxy]-phenyl}-2-(3-cyclohexanecarbonyl-pyrrol-1-yl)-propionic acid methyl ester (25) was prepared in the following manner.

### (a) (S)-3-{4-[2-(Benzooxazol-2-yl-methyl-amino)-ethoxy]-phenyl}-2-(3-cyclohexanecarbonyl-pyrrol-1-yl)-propionic acid methyl ester (25).

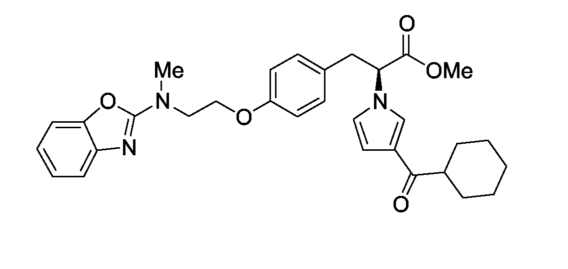

Prepared according to General Procedure B using 2-(Benzooxazol-2-yl-methyl-amino)-ethanol as the alcohol. NMR (δ;CHCl₃) 7.34 (1H, d, J=7.5Hz); 7.25 (1H, s); 7.15 (1H, t, J=7.5, 7.8Hz); 7.00 (1H, t, J=7.5, 7.8Hz); 6.86 (2H, d, J=8.3Hz); 6.73 (2H, d, J=8.3Hz); 6.65 (1H, s); 6.55 (1H, s); 4.66 (1H, m); 4.20 (2H, t, J=5.1, 5.1Hz); 3.92 (2H, t, J=5.1, 5.1Hz); 3.72 (3H, s); 3.32 (1H, m); 3.32 (3H, s); 3.18 (1H, m); 2.81 (1H, m); 1.79 (4H, m); 1.65 (1H, m); 1.42 (2H, m); 1.22 (3H, m).

### Example 11

### (S)-2-(3-Cyclohexanecarbonyl-pyrrol-1-yl)-3-[4-(2-fluoro-benzyloxy)-phenyl]-propionic acid (28).

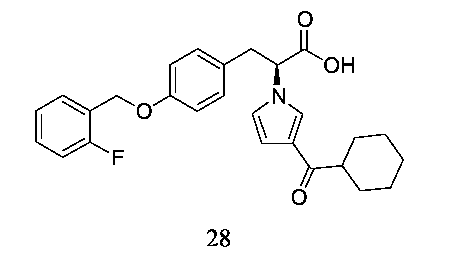

Hydrolysis of (S)-2-(3-Cyclohexanecarbonyl-pyrrol-1-yl)-3-[4-(2-fluorobenzyloxy)-phenyl]-propionic acid methyl ester (27) according to General Procedure A gave (S)-2-(3-Cyclohexanecarbonyl-pyrrol-1-yl)-3-[4-(2-fluorobenzyloxy)-phenyl]-propionic acid (28) (20mg, 0.044mmol) in 16% yield as a pale yellow oil. C₂₇H₂₈FNO₄ Mol. Wt.: 449.514; M+1 = 450.1 ¹H NMR (400 MHz, CDCl₃) δ: 7.46 (1H, t, *J=* 7.5, 7.5Hz), 7.20 (1H, m), 7.14 (1H, t, *J*=7.3, 7.3Hz), 7.07 (1H, t, *J*=9.0, 9.5Hz), 6.91 (2H, d, *J*=8.5, 8.5Hz), 6.90 (1H, s), 6.84 (2H, d, *J*=8.5, 8.5 Hz), 6.66 (1H, s), 6.58 (1H, s), 5.06 (2H, s), 4.74 (1H, m), 3.40 (1H, m), 3.22 (1H, m), 2.83 (1H, t, d=12.2, 12.2Hz).1.80 (2H, m), 1.68 (2H, m), 1.47 (3H, m), 1.29 (3H, m).

(S)-2-(3-Cyclohexanecarbonyl-pyrrol-1-yl)-3-[4-(2-fluoro-benzyloxy)-phenyl]-propionic acid methyl ester (27) was prepared in the following manner.

### (a) (S)-2-(3-Cyclohexanecarbonyl-pyrrol-1-yl)-3-[4-(2-fluorobenzyloxy)-phenyl]-propionic acid methyl ester (27).

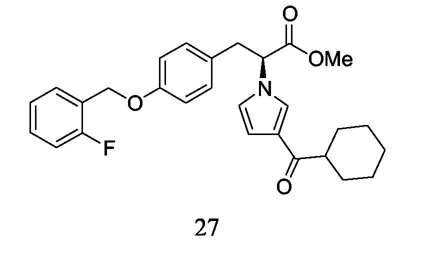

According to General Procedure B by coupling of (S)-2-(3-Cyclohexanecarbonyl-pyrrol-1-yl)-propionic acid methyl ester (27) with 2-fluorobenzyl alcohol. The title compounds was isolated in 70% yield.

### Example 12

### (S)-3-{4-[2-(5-Methyl-2-phenyl-oxazol-4-yl)-ethoxy]-phenyl}-2-[2-(2,2,2-trifluoro-acetyl)-pyrrol-1-yl]-propionic acid (33).

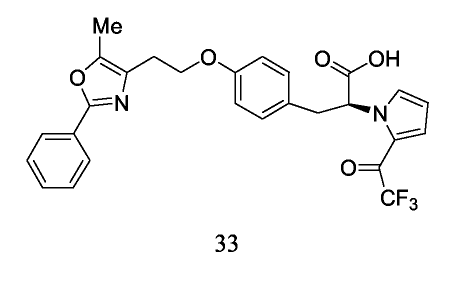

According to General Procedure A. To a stirred solution of (S)-3-{4-[2-(5-Methyl-2-phenyl-oxazol-4-yl)-ethoxy]-phenyl}-2-[2-(2,2,2-trifluoro-acetyl)-pyrrol-1-yl]-propionic acid methyl ester (32) (2.608 g, 4.7 mmol) in 2-methoxy ethanol, (8 mL) and water (2 mL) was added lithium hydroxide (113 mg). The reaction mixture stirred at room temperature for 20 minutes, poured into water, acidified with aqueous HCl, extracted with EtOAc and dried over MgSO₄. The solvent was removed under reduced pressure. The remaining residue was purified by chromatography on silica gel eluting with 1:1 EtOAc:Hexane. The resulting white foam was recrystallized using ethyl acetate hexane to give white needle crystals. Yield = 1.232g, 51%. Mp 187-188°C. 500 MHz ¹H NMR (CDCl₃) δ 7.87 (m, 2H), 7.35 (m, 3H), 7.17 (m, 1H), 7.0 (s, 1H, br) 6.80 (d, 2H *J*=8Hz), 6.66 (d, 2H, *J*=8Hz), 6.15 (m, 1H), 4.07 (t, 2H, *J=* 6.7 Hz), 3.41 (d,d 1H *J*=14.4, 5.0) 3.11 (dd, 1H *J*=14.4, 9.28) 2.89 (m, 2H) , 2.29 (s, 3H); MS *m*/*z* 513 (M+1), 514 Anal. Calc'd for C₂₇H₂₃F₃N₂O₅ C, 63.28: H 4.52; N 5.47 found: C, 63.19; H, 4.48; N 5.27.

(S)-3-{4-[2-(5-Methyl-2-phenyl-oxazol-4-yl)-ethoxy]-phenyl}-2-[2-(2,2,2-trifluoro-acetyl)-pyrrol-1-yl]-propionic acid methyl ester (32) was prepared in the following manner.

### (a) (S)-3-{4-[2-(5-Methyl-2-phenyl-oxazol-4-yl)-ethoxy]-phenyl}-2-[2-(2,2,2-trifluoro-acetyl)-pyrrol-1-yl]-propionic acid methyl ester (32).

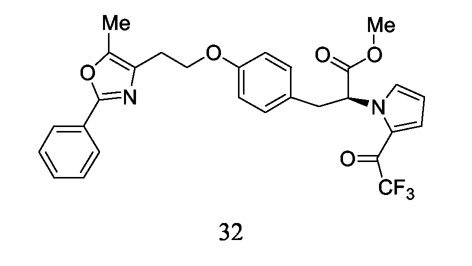

By Procedure 32-A: To a stirred solution of (S)-3-(4-Hydroxy-phenyl)-2-[2-(2,2,2-trifluoro-acetyl)-pyrrol-1-yl]-propionic acid methyl ester (31) (1.0g, 2.93 mmol) in anhydrous THF (10 mL) was added triphenyl phosphine (768 mg, 2.93 mmol) and 2-(5-methyl-2-phenyloxazol-4-yl) ethanol (595 mg, 2.93 mmol). A solution of diethyl azodicarboxylate (DEAD) (461 µL, 2.93 mmol) in anhydrous THF (5 mL) was added dropwise and the reaction mixture stirred at room temperature for 48 hr. The solvent was then removed under reduced pressure to give a yellow oil.

By Procedure 32-B: To a stirred solution of of (S)-3-(4-Hydroxy-phenyl)-2-[2-(2,2,2-trifluoro-acetyl)-pyrrol-1-yl]-propionic acid methyl ester (32) (800 mg, 2.34 mmol) in anhydrous THF (10 mL) was added triphenyl phosphine (615 mg, 2.34 mmol) and 2-(5-methyl-2-phenyloxazol-4-yl) ethanol (476 mg, 2.34 mmol). A solution of diisopropylazodicarboxylate (DIAD) (369 µL, 2.34 mmol) in anhydrous THF (5 mL) was added dropwise and the reaction mixture stirred at room temperature for 48hr. The solvent was then removed under reduced pressure to give a yellow oil.

The combined reaction mixtures from Procedures 32-A and 32-B were combined and purified by chromatography eluting with 1:10 to 1:1 EtOAc: Hexane. The title compound was isolated as an opaque oil (2.608g, 89% yield). 500 MHz ¹H NMR (CDCl₃) δ 7.96 (m, 2H), 7.39 (m, 3H), 7.18 (m, 1H), 7.0 (s, 1H, br) 6.79 (d, 2H *J*=8.3 Hz), 6.68 (d, 2H, *J*=8.3Hz), 6.20 (m, 1H), 4.15 (t, 2H, *J* = 6.6 Hz), 3.70 (s, 3H) 3.43 (d,d 1H *J*=14.4, 5.12) 3.12 (dd, 1H *J*=14.4, 9.5) 2.92 (t, 2H *J*= 6.6) , 2.32 (s, 3H); MS *m*/*z* 526 (M+1), 527.

### (b) (S)-3-(4-Hydroxy-phenyl)-2-[2-(2,2,2-trifluoro-acetyl)-pyrrol-1-yl]-propionic acid methyl ester (31).

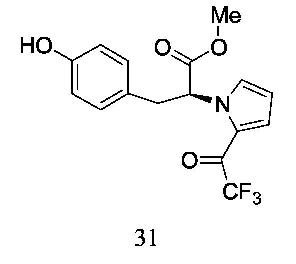

To a stirred solution of (S)-3-(4-Acetoxy-phenyl)-2-[2-(2,2,2-trifluoroacetyl)-pyrrol-1-yl]-propionic acid methyl ester (30) (2.13g, 5.57 mmol) in anhydrous methanol (20 mL) was added K₂CO₃ (770 mg, 5.57 mmol). The reaction mixture was stirred at room temperature for 20 min. and poured into water, acidified with aqueous HCl, extracted with EtOAc, dried MgSO4 and the solvent removed under reduced pressure to give a pale yellow oil. (1.826 g, 96% yield). 500 MHz ¹H NMR (CDCl₃) δ 7.19 (m, 1H), 7.1 (s, 1H, br), 6.79 (d, 2H *J*=8.6 Hz), 6.62 (d, 2H, *J*=8.6Hz), 6.22 (m, 1H), 4.67 (s, 1H) 3.72 (s, 3H), 3.44 (d,d 1H *J*=14.4, 5.4), 3.12 (dd, 1H *J*=14.4, 9.8), 2.01 (s, 3H); MS *m*/*z* 342 (M+1), 343.

### (c) (S)-3-(4-Acetoxy-phenyl)-2-[2-(2,2,2-trifluoro-acetyl)-pyrrol-1-yl]-propionic acid methyl ester (30).

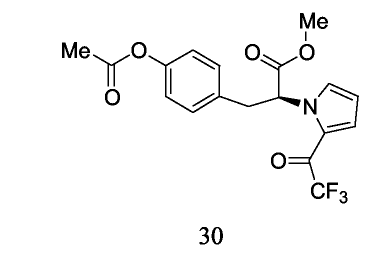

To a stirred solution of (S)-3-(4-Acetoxy-phenyl)-2-[pyrrol-1-yl]-propionic acid methyl ester (29) (2.9 g, 10.1 mmol) in CH₂Cl₂ (20 mL) was added trifluoroacetic anhydride (1.71 mL, 12.1 mmol) and. trifluoromethansulfonic acid (1.07 µL, 12.1mmol). The reaction mixture turned orange in color. The reaction mixture was stirred for 5 minutes and poured into NH₄Cl solution, extracted with EtOAc, dried (MgSO₄). The solvent removed under reduced pressure. The resulting orange oil was purified by chromatography 1:10 to 1:1 EtOAc: Hexane to give the desired product as a clear oil (2.13 g, 55% yield) 500 MHz ¹H NMR (CDCl₃) δ 7.21 (m, 1H), 7.19 (s, 1H, br), 6.94 (d, 2H *J*=8.6 Hz), 6.90 (d, 2H, *J*=8.6Hz), 6.23 (m, 1H), 3.72 (s, 3H), 3.51 (dd, 1H, *J*=14.4, 5.4), 3.18 (dd, 1H *J*=14.4, 9.5), 2.23 (s, 3H); MS *m*/*z* 384 (M+1), 385.

### (d) (S)-3-(4-Acetoxy-phenyl)-2-[pyrrol-1-yl]-propionic acid methyl ester (29).

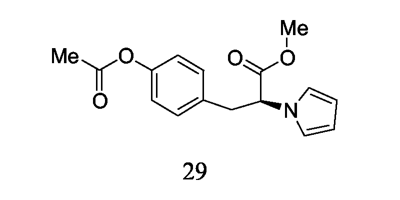

To a stirred solution of (S)-3-(4-hydroxy-phenyl)-2-[pyrrol-1-yl]-propionic acid methyl ester 1 (10 g, 40 mmol) in CH₂CL₂ (100 mL), was added 2.,6-lutidine (9.5 mL, 81.6 mmol) and acetyl chloride (5.8 mL, 8.16 mmol). The reaction mixture was stirred at room temperature overnight. The reaction mixture was poured into water, extracted with EtOAc, dried (MgSO₄) and the solvent removed under reduced pressure. The residual oil was purified by chromatography, 1:5 EtOAc:Hexane and the product obtained as a clear oil (8.25 g, 71%). 500 MHz ¹H NMR (CDCl₃) 6.96 (d, 2H *J*=8.8 Hz), 6.91 (d, 2H, *J*=8.8Hz), 6.67 (t, 2H, *J*=2.0Hz), 6.11 (t, 2H, *J*=2.0Hz), 4.68 (dd, 1H, *J*=6.3, 8.8), 3.67 (s, 3H), 3.37 (dd, 1H, *J*=14.4, 6.3), 3.25 (dd, 1H *J*=14.4, 8.8), 2.24 (s, 3H); MS *m*/*z* 288 (M+1), 289.

### Example 13

### 3-{4-[2-(5-Methyl-2-phenyl-oxazol-4-yl)-ethoxy]-phenyl}-2-pyridin-4-yl-propionic acid (34, PD 0330732).

This compound was synthesized as described for Compound 33 using ethyl 4-pyridyl acetate as the starting material. ¹H NMR d6-DMSO δ 2.30 (s, 3H) 2.85 (m, 3H) 3.02 (m, 2H) 4.11 (t, 2H) 6.80 (d, 2H) 7.06 (d, 2H) 7.44 (m, 3H) 7.65 (d, 2H) 7.84 (d, 2H)8.63 (s, 2H). MS(M-44) (minus carboxylate): 385.1. Anal. Calcd for C₂₆H₂₄O₄N₂ · 1.67 H₂O: C, 68.05; H, 5.82; N, 6.11. Found: C, 68.03; H, 6.00; N, 6.11.

### Example 14

### (S)-2-(2-Acetyl-pyrrol-1-yl)-3-{4-[2-(5-methyl-2-phenyl-oxazol-4-yl)-ethoxy]-phenyl}-propionic acid (37).

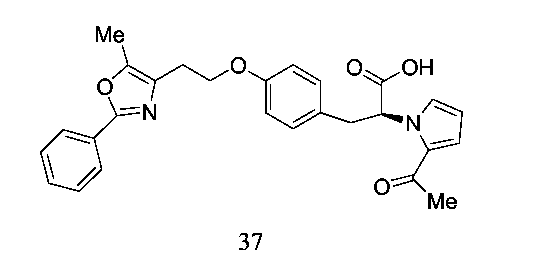

(S)-2-(2-Acetyl-pyrrol-1-yl)-3-{4-[2-(5-methyl-2-phenyl-oxazol-4-yl)-ethoxy]-phenyl}-propionic acid methyl ester (36) (601 mg, 1.27 mmol) was hydrolyzed as described in General Procedure A to give the title compound as a white foam (129 mg, 22% yield). Mp 110°C-112°C. 500 MHz ¹H NMR (CDCl₃) δ 7.91 (m, 2H), 7.37 (m, 3H), 6.95 (m, 1H), 6.87 (m, 3H, br) 6.70 (d, 2H, *J*=8.6Hz), 6.09 (m, 1H), 4.12 (t, 2H, *J=* 6.8 Hz), 3.47 (d,d 1H *J*=14.4, 4.0) 3.15 (dd, 1H *J*=14.4, 9.0) 2.90 (t, 2H, *J=* 6.8Hz) , 2.37 (s, 3H), 2.31 (s, 3H); MS *m*/*z* 459 (M+1), 460.

(S)-2-(2-Acetyl-pyrrol-1-yl)-3-{4-[2-(5-methyl-2-phenyl-oxazol-4-yl)-ethoxy]-phenyl}-propionic acid methyl ester (36) was prepared in the following manner.

### (a) (S)-2-(2-Acetyl-pyrrol-1-yl)-3-{4-[2-(5-methyl-2-phenyl-oxazol-4-yl)-ethoxy]-phenyl}-propionic acid methyl ester (36).

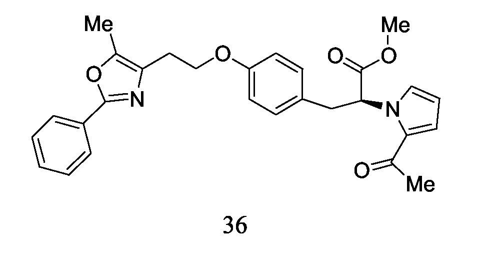

By Procedure 36-A: To a stirred solution of (S)-2-(2-Acetyl-pyrrol-1-yl)-3-{4-[hydroxy]-phenyl}-propionic acid methyl ester (35) (500 mg, 1.74 mmol) in anhydrous THF (15 mL) was added triphenyl phosphine (685 mg, 2.61 mmol) and 2-(5-methyl-2-phenyloxazol-4-yl) ethanol (388 mg, 1.91 mmol). A solution of DEAD (411 µL, 2.61 mmol) in anhydrous THF (5 mL) was added dropwise and the reaction mixture stirred at room temperature for 48 hours. The solvent was removed under reduced pressure and the residue obtained as a yellow oil.

By Procedure 36-B: To a stirred solution of (S)-2-(2-Acetyl-pyrrol-1-yl)-3-{4-[hydroxy]-phenyl}-propionic acid methyl ester (35) (500 mg, 1.74 mmol) in anhydrous THF (15 mL) was added triphenyl phosphine (685 mg, 2.61 mmol) and 2-(5-methyl-2-phenyloxazol-4-yl) ethanol (388 mg, 1.91 mmol). A solution of DIAD (514 µL, 2.61 mmol) in anhydrous THF (5 mL) was added dropwise and the reaction mixture stirred at room temperature for 48 hours. The solvent was removed under reduced pressure and the residue obtained as a yellow oil.

The crude products from reactions 36-A and 36-B were combined and purified by chromatography 1:10 to 1:1 Ethyl Acetate: Hexane to give the title compound as an opaque oil. (601 mg, 73% yield). 500 MHz ¹H NMR (CDCl₃) δ 7.94 (m, 2H), 7.40 (m, 3H), 6.92 (m, 1H), 6.88 (s, 1H, br) 6.82 (m, 2H) 6.61 (m, 2H), 6.09 (m, 1H), 4.12 (t, 2H, *J=* 6.8 Hz), 3.69 (s, 3H) 3.42 (dd 1H *J*=14.2, 5.37) 3.12 (dd, 1H *J*=14.4, 9.76) 2.91 (t, 2H, *J*= 6.8Hz) , 2.35 (s, 3H), 2.31 (s, 3H); MS *m*/*z* 473 (M+1), 474.

### (b) (S)-2-(2-Acetyl-pyrrol-1-yl)-3-{4-[hydroxy]-phenyl}-propionic acid methyl ester (35).

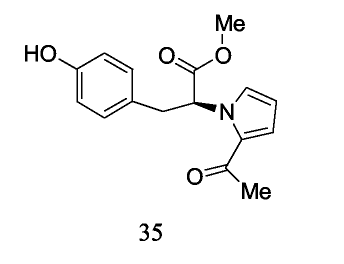

The intermediate (S)-2-(2-Acetyl-pyrrol-1-yl)-3-{4-[Acetoxy]-phenyl}-propionic acid methyl ester (34) was deacetylated according to the General Procedure C. The product was purified by chromatography EtOAc: Hexane 1:1 to give a clear oil which solidified on standing (1.756 g, 88% yield). 500 MHz ¹H NMR (CDCl₃) δ 6.93 (m, 1H), 6.83 (d, 2H *J*=8.3Hz) 6.63 (d, 2H, *J*=8.3Hz), 6.10 (m, 1H), 5.37 (s, 1H, br), 3.68 (s, 3H) 3.40 (d,d 1H *J*=14.2, 5.61Hz) 3.12 (dd, 1H *J*=14.2, 9.52) 2.35 (s, 3H), MS *m*/*z* 288 (M+1), 289.

### (c) (S)-2-(2-Acetyl-pyrrol-1-yl)-3-14-[Acetoxy]-phenyl}-propionic acid methyl ester (34).

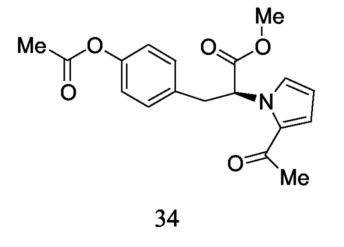

According to General Procedures D and E. To a stirred solution of (S)-3-(4-Acetoxy-phenyl)-2-[pyrrol-1-yl]-propionic acid methyl ester (30) (4.40 g, 15.3 mmol) in CH₂Cl₂ (50 mL) was added acetyl chloride (1.31 mL, 18.4 mmol) and. trifluoromethansulfonic acid (1.63 mL, 18.4 mmol). The reaction mixture turned orange in color. The reaction mixture was stirred for 5 minutes and poured into NH4Cl solution, extracted with EtOAc, and dried (MgSO₄). The solvent was removed *in vacuo.* The resulting orange oil was purified by chromaography 1:2 EtOAc: Hexane to give the desired product as a clear oil (2.27g, 45%). 500 MHz ¹H NMR (CDCl₃) δ 500 MHz ¹H NMR (CDCl₃) δ 6.96 (d, 2H *J*=8.3Hz) 6.92 (m, 2H) 6.89 (d, 2H, *J*=8.3Hz), 6.10 (m, 1H), 3.70 (s, 3H) 3.47 (d,d 1H *J*=14.4, 5.62Hz) 3.12 (dd, 1H *J*=14.4, 9.52) 2.35 (s, 3H), 2.24 (s, 3H), MS *m*/*z* 330 (M+1), 331.

### Example 15

### 3- {4-[2-(Benzothiazol-2-ylsulfanyl)-ethoxy]-phenyl}-2-pyrrol-1-yl-propionic acid (39).

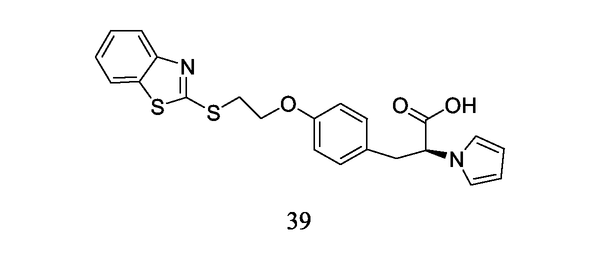

According to General Procedure A. The intermediate 3-{4-[2-(Benzothiazol-2-ylsulfanyl)-ethoxy]-phenyl]-2-pyrrol-1-yl-propionic acid methyl ester (38) was hydrolyzed to give a white solid which was recrystallised using EtOAC and Hexane to give a white crystalline powder (390mg, 79%) Mp 165°C-166°C; 500 MHz ¹H NMR (CDCl₃) δ 7.85 (d, 1H, *J=* 8.05Hz), 7.75 (d, 8.05Hz), 7.42 (m, 1H), 7.30 (m, 1H), 6.91 (d, 2H *J*=8.54Hz), 6.82 (d, 2H, *J*=8.54Hz), 6.69 (m, 2H), 6.14 (m, 2H), 4.72 (dd, 1H, *J*=5.86, 8.79Hz), 4.31 (t, 2H, *J*=6.59Hz), 3.70 (t, 2H, *J*=6.59Hz), 3.38 (d,d 1H *J*=14.16, 5.86Hz), 3.22 (dd, 1H *J*=14.16, 8.79); MS *m*/*z* 404 (M+1).

(S)-3-{4-[2-(Benzothiazol-2-ylsulfanyl)-ethoxy]-phenyl}-2-pyrrol-1-yl-propionic acid methyl ester (38) was prepared in the following manner.

### (a) (S)-3-{4-[2-(Benzothiazol-2-ylsulfanyl)-ethoxy]-phenyl}-2-pyrrol-1-yl-propionic acid methyl ester (38).

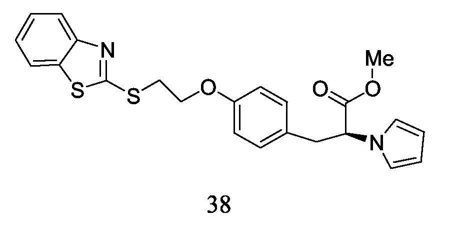

According to General Procdure B. To a stirred solution of (S)-3-(4-hydroxy-phenyl)-2-[pyrrol-1-yl]-propionic acid methyl ester (1) (828 mg, 3.38 mmol) in anhydrous THF (15 mL) was added triphenyl phosphine (886 mg, 3.38 mmol) and 2-(Benzothiazol-2-ylsulfanyl)-ethanol (800 mg, 3.38 mmol). A solution of DIAD (665 µL, 3.38 mmol) in anhydrous THF (5 mL) was added dropwise and the reaction mixture stirred at room temperature for 48 hours. The solvent was removed under reduced pressure and the residue purified by chromatography 1:10 to 1:1 EtOAc: Hexane. The product was obtained as an opaque oil. Yield = 510 mg, 29%. 500 MHz ¹H NMR (CDCl₃) δ 7.85 (d, 1H, *J*= 8.06Hz), 7.75 (d, 8.06Hz), 7.40 (m, 1H), 7.30 (m,1H), 6.90 (d, 2H *J*=8.8Hz), 6.83 (d, 2H, *J*=8.8Hz), 6.70 (m, 2H), 6.14 (m, 2H), 4.68 (dd, 1H, *J*=6.34, 8.79Hz), 4.32 (t, 2H, *J*=6.59Hz), 3.71 (t, 2H, *J*=6.59Hz), 3.693 (s, 3H), 3.33 (d,d 1H *J*=13.9, 6.59Hz), 3.19 (dd, 1H *J*=13.9, 8.79); MS *m*/*z* 439 (M+1).

### Example 16

### (S)-2-Pyrrol-1-yl-3-{4-[2-(2-trifluoromethyl-phenyl)-ethoxy]-phenyl}-propionic acid (41).

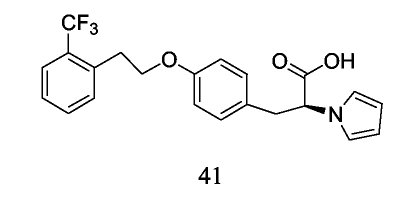

Prepared from (S)-2-Pyrrol-1-yl-3-{4-[2-(2-trifluoromethyl-phenyl)-ethoxy]-phenyl}-propionic acid methyl ester (40) using the hydrolysis method described in General Procedure A. (S)-2-Pyrrol-1-yl-3-{4-[2-(2-trifluoromethylphenyl)-ethoxy]-phenyl}-propionic acid (42) was obtained as clear oil. (454 mg, 45% yield) δ 7.85 (d, 1H, *J*= 8.05Hz), 7.45 (m, 2H), 7.33 (t, 1H, *J*=8.05Hz) 6.90 (d,1H, *J*=8.54), 6.75 (d, 2H *J*=8.54) 6.69 (m, 2H), 6.15 (m, 2H) 4.73 (dd, 1H, *J*=5.86, 9.3), 4.12 (t, 2H, *J*=6.84) 3.37 (dd, 1H, *J*=13.9, 5.86) 3.25 (m, 5H) MS *m*/*z* 425 (M+1) Anal. Calc'd for C₂₂H₂₀F₃NO₃ C, 65.50: H 5.00; N 3.47 found: C, 64.92; H, 5.15; N 3.28.

(S)-2-Pyrrol-1-yl-3-{4-[2-(2-trifluoromethyl-phenyl)-ethoxy]-phenyl}-propionic acid methyl ester (40) was prepared in the following manner.

### (a) (S)-2-Pyrrol-1-yl-3-{4-[2-(2-trifluoromethyl-phenyl)-ethoxy]-phenyl]-propionic acid methyl ester (40).

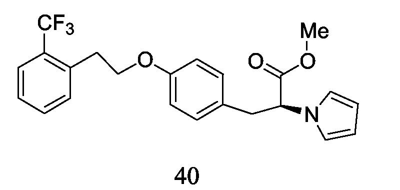

According to General Procedure B. Prepared from the intermediate (S)-3-(4-hydroxy-phenyl)-2-[pyrrol-1-yl]-propionic acid methyl ester (1) and 2-(2-trifluoromethyl-phenyl)-ethanol. (S)-2-Pyrrol-1-yl-3-{4-[2-(2-trifluoromethylphenyl)-ethoxy]-phenyl}-propionic acid methyl ester (40) was obtained as an opaque oil (1.04 g, 61% yield). δ 7.64 (d, 1H, *J*= 7.8Hz), 7.46 (m, 2H), 7.33 (t, 1H, *J*=7.8) 6.89 (d, 1H, *J*=6.59), 6.75 (d, 2H *J*=6.59), 6.69 (m, 2H), 6.14 (m, 2H), 4.68 (dd, 1H, *J*=6.59, 8.78), 4.12 (t, 2H, *J*=6.8), 3.69 (s, 3H), 3.33 (dd, 1H, *J*=13.9, 6.59), 3.25 (t, 2H, *J*=6.8), 3.16 (dd, 1H, *J*= 13.9, 8.78); MS *m*/*z* 418 (M+1).

### Example 17

### (S)-3-{4-[2-(4-Phenyl-piperazin-1-yl)-ethoxy]-phenyl}-2-pyrrol-1-yl-propionic acid (43).

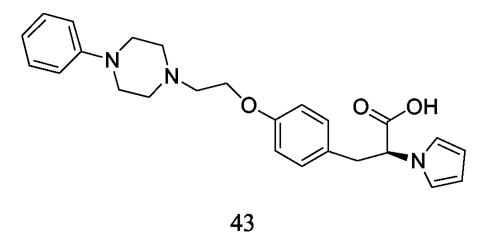

Prepared from (S)-3-{4-[2-(4-Phenyl-piperazin-1-yl)-ethoxy]-phenyl}-2-pyrrol-1-yl-propionic acid methyl ester (42) using the hydrolysis method described in General Procedure A to give the desired product as a pink foam. Yield = 270 mg, 38%. 500 MHz ¹H NMR (CDCl₃) δ 7.28 (m, 2H), 7.09 (d, 2H, *J*=9Hz), 7.18 (m, 1H), 6.90(m, 3H) 6.73 (m, 4H), 6.10 (m, 2H), 4.65 (d,d 1H, *J*=5.85, 8.79Hz), 4.125 (t, 2H, J= 5.15 Hz), 3.40 (dd, 1H *J*=13.7, 8.79), 3.27-3.08 (m, 11H); MS m/z 420 ( M+1), 419 (M-1).

(S)-3-{4-[2-(4-Phenyl-piperazin-1-yl)-ethoxy]-phenyl}-2-pyrrol-1-yl-propionic acid methyl ester (42) was prepared in the following manner.

### (a) (S)-3-{4-[2-(4-Phenyl-piperazin-1-yl)-ethoxy]-phenyl}-2-pyrrol-1-yl-propionic acid methyl ester (42).

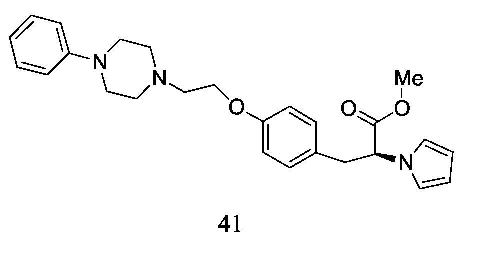

According to General Procedure B. Prepared from the intermediate (S)-3-(4-hydroxy-phenyl)-2-[pyrrol-1-yl]-propionic acid methyl ester (1) and 2-(4-Phenyl-piperazin-1-yl)-ethanol. (S)-3-{4-[2-(4-Phenyl-piperazin-1-yl)-ethoxy]-phenyl}-2-pyrrol-1-yl-propionic acid methyl ester (41) was obtained as a clear oil (740 mg, 70% yield). 500 MHz ¹H NMR (CDCl₃) δ 7.26 (m, 2H), 6.91 (d, 2H, *J*=9Hz), 6.98-6.76 (m, 5H), 6.70(m, 2H), 6.14 (m, 2H), 4.69 (d,d 1H, *J*=6.95, 8.79Hz), 4.12 (t, 2H, *J*= 7.08 Hz), 3.69 (s, 3H), 3.43 (dd, 1H *J*=6.35, 13.9), 3.21-3.16 (m, 5H), 2.85 (br s, 2H), 2.73 (br s, 4H); MS *m*/*z* 434 (M+1).

### Example 18

### (S)-3-{4-[2-(5-Methyl-2-phenyl-thiazol-4-yl)-ethoxy]-phenyl}-2-pyrrol-1-yl-propionic acid (45).

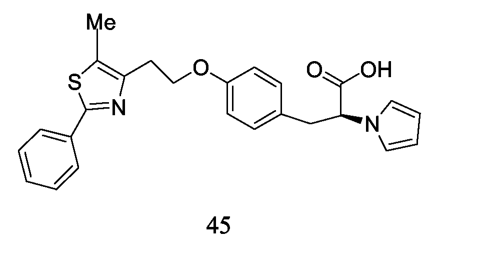

Prepared from (S)-3-{4-[2-(5-Methyl-2-phenyl-thiazol-4-yl)-ethoxy]-phenyl}-2-pyrrol-1-yl-propionic acid methyl ester (44) using the hydrolysis method described in General Procdure A. The product was recrystallized from ethyl acetate and hexane to give the desired product as white needles. Yield = 1.20g, 58% M.P. = 157-158°C. ¹H NMR (500MHz) CDCl₃ δ: 7.80 (2H, m), 7.34 (3H, m), 6.87 (d, 2H *J*=8.5 Hz), 6.73 (2H, d, *J*=8.5 Hz), 6.66 (2H, m), 6.11 (2H, m), 4.68 (1H, dd, *J*=6.35, 8.79 Hz), 4.20 (2H, t, 6.83 Hz), 3.34 (1H, dd, *J*=6.1, 13.9 Hz), 3.17 (1H, dd, *J*=, 8.79, 13.9 Hz), 3.12 (2H, t, *J*=6.59 Hz), 2.40 (3H, s). Anal. Calc'd for C₂₅H₂₄N₂O₃S: C 69.42, H 5.59, N 6.48 found: C 69.41, H 5.53, N 6.29 MS *m*/*z* 433 (M+1), 431 (M-1).

(S)-3-{4-[2-(5-Methyl-2-phenyl-thiazol-4-yl)-ethoxy]-phenyl}-2-pyrrol-1-yl-propionic acid methyl ester (44) was prepared in the following manner.

### (a) (S)-3-{4-[2-(5-Methyl-2-phenyl-thiazol-4-yl)-ethoxy]-phenyl}-2-pyrrol-1-yl-propionic acid (44).

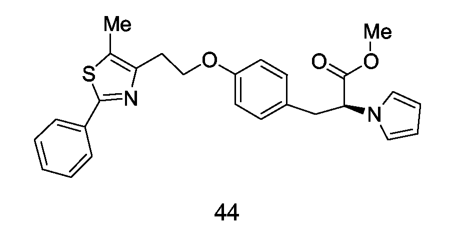

According to General Procedure B. Prepared from the intermediate (S)-3-(4-hydroxy-phenyl)-2-[pyrrol-1-yl]-propionic acid methyl ester (1) and 2-(5-Methyl-2-phenyl-thiazol-4-yl)-ethanol. (S)-3-{4-[2-(5-Methyl-2-phenyl-thiazol-4-yl)-ethoxy]-phenyl}-2-pyrrol-1-yl-propionic acid (44) was obtained as a clear oil. Yield = 2.15g, 59%. ¹H NMR (500MHz) CDCl₃ δ: 7.82 (2H, m), 7.35 (3H, m), 6.84 (m, 2H), 6.73 (2H, m), 6.66 (2H, m), 6.10(2H, m), 4.65 (1H, dd, *J=* 6.59, 8.78 Hz), 4.23 (2H, t, 6.83 Hz), 3.65 (3H, m), 3.30 (1H, dd, *J*=6.59, 13.9 Hz), 3.17-3.11 (3H, m), 2.41 (3H, s). MS *m*/*z* 447 (M+1).

### Example 19

### (S)-2-(3-Chloro-2,5-dimethyl-pyrrol-1-yl)-3-{4-[2-(5-methyl-2-phenyl-oxazol-4-yl)-ethoxy]-phenyl}-propionic acid (49).

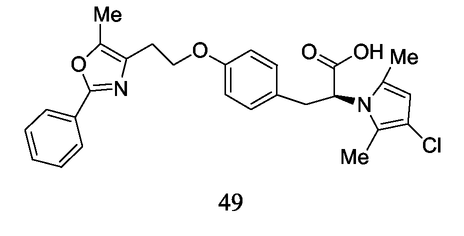

Prepared from the intermediate 2-(3-Chloro-2,5-dimethyl-pyrrol-1-yl)-3-{4-[2-(5-methyl-2-phenyl-oxazol-4-yl)-ethoxy]-phenyl}-propionic acid methyl ester (48) using the hydrolysis method described in General Procedure A. The product was purified by chromatography and recrystallized from EtOAc/hexane to give the desired product as white crystalline powder. Yield =158mg, 25%. ¹H NMR (500MHz) CDCl₃ δ: 7.98 (2H, m), 7.43 (3H, m), 6.75 (dd, 4H, *J*=9.0Hz), 5.75 (1H, s), 4.70 (1H, dd, *J*= 4.64, 10.25 Hz), 4.19 (2H, t, 6.59 Hz), 3.30 (1H, dd, *J*=4.64, 14.16 Hz), 3.08 (1H, dd, *J*=14.16, 10.25 Hz), 2.98 (2H, t, 6.59), 2.37 (3H, s), 1.99 (3H, br,s), 1.88 (3H br,s) MS *m*/*z* 479 (M+1) 477 (M-1).

(S)-2-(3-Chloro-2,5-dimethyl-pyrrol-1-yl)-3-{4-[Acetoxy]-phenyl}-propionic acid methyl ester (48) was prepared in the following manner.

### (a) (S)-2-(3-Chloro-2,5-dimethyl-pyrrol-1-yl)-3-{4-[2-(5-methyl-2-phenyl-oxazol-4-yl)-ethoxy]-phenyl}-propionic acid methyl ester (48).

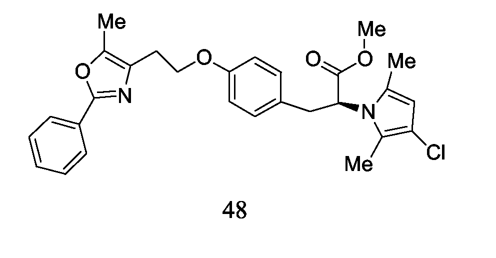

To a stirred solution of (S)-2-(3-Chloro-2,5-dimethyl-pyrrol-1-yl)-3-{4-[acetoxy]-phenyl}-propionic acid methyl ester (674 mg, 1.93 mmol) described in Example 47 in anhydrous methanol (10 mL) was added K₂CO₃ (266 mg, 1.93 mmol). The reaction was stirred at room temperature for 40 minutes, poured into EtOAc, washed with water, dried (MgSO₄) and the solvent removed *in vacuo* to give 2-(3-Chloro-2,5-dimethyl-pyrrol-1-yl)-3-{4-[hydoxy]-phenyl}-propionic acid methyl ester as an oil. This material was used in the General Procedure B coupling reaction.

To a stirred solution of (S)-2-(3-Chloro-2,5-dimethyl-pyrrol-1-yl)-3-{4-[hydoxy]-phenyl]-propionic acid methyl ester (47) in THF (10mL) at room temperature was added triphenyl phosphine (541 mg, 2.06 mmol) and 2-(5-methyl-2-phenyloxazol-4-yl) ethanol (541 mg 2.06 mmol). A solution of DIAD (406 µL, 2.06 mmol) in anhydrous THF (5 mL) was added dropwise and the reaction mixture stirred at room temperature for 72 hours. The solvent was then removed *in vacuo* and the residue purified by chromatography eluting with 1:10 EtOAc: hexane to 1:1 EtOAc: Hexane. This gave the desired product as a clear oil. Yield = 651 mg, 68%. ¹H NMR (500MHz) CDCl₃ δ: 7.95 (2H, m), 7.40 (3H, m), 6.76 (m, 4H), 5.75 (1H, s), 4.65 (1H, dd, *J =* 4.64, 10.25 Hz), 4.20 (2H, t, 6.59 Hz), 3.78 (3H,s), 3.42 (1H, dd, *J*=4.64, 14.16 Hz), 3.06 (1H, dd, *J*=14.16, 10.25 Hz), 2.91 (2H, t, 6.59), 2.30 (3H, s), 1.90 (3H, br,s), 1.82 ( 3H br,s) MS *m*/*z* 493 (M+1), 492 (M-1).

### (b) (S)-2-(3-Chloro-2,5-dimethyl-pyrrol-1-yl)-3-{4-[Acetoxy]-phenyl}-propionic acid methyl ester (47).

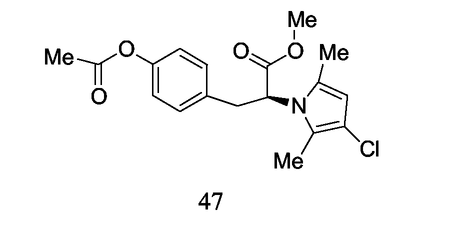

To a stirred solution of (S)-2-(2,5-dimethyl-pyrrol-1-yl)-3-{4-[Acetoxy]-phenyl}-propionic acid methyl ester (46) (2.45 g, 7.77 mmol) in THF (20 mL) at 0 °C was added N-chlorosuccinamide (1.04 g, 7.77 mmol). After 30 minutes, the solvent was removed *in vacuo* and the residue purified by chromatography 1:10 to 1;1 EtOAc: Hexane to give a yellow oil. Yield = 674mg, 25%. ¹H NMR (500MHz) CDC1₃ δ: 6.92-6.83 (m, 4H), 5.71 (s, 1H), 4.66 (1H, dd, *J=* 4.64, 10.01 Hz), 3.49 (1H, dd, *J*=4.64, 14.16 Hz), 3.08 (1H, dd, *J*=14.16, 10.01 Hz), 2.23 (3H, s), 2.30 (3H, s), 1.92 (3H, br,s), 1.82 ( 3H br,s) MS *m*/*z* 350 (M+1) 348 (M-1).

### (c) (S)-2-(2,5-dimethyl-pyrrol-1-yl)-3-{4-[Acetoxy]-phenyl}-propionic acid methyl ester (46).

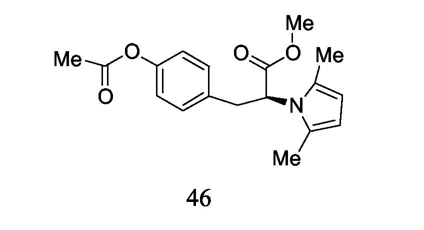

To a stirred solution of L-tyrosine methyl ester (25 g, 0.128 mol) in toluene (300 mL) was added 2,5-hexandione (15 mL, 0.128 mol) and p-toluenesulfonic acid (1.22 g, 6.4 mmol). The mixture was heated at reflux with a Dean-Starke trap for 12 hours. The solvent was removed *in vacuo* and the residue redissolved in ethyl acetate and filtered through a bed of silica gel. The solvent was removed in vacuo and the product, a yellow oil used in the next step without further purification. 500 MHz ¹H NMR (CDCl₃) δ 6.60 (d, 2H, *J=* 6.6 Hz), 6.47 (d, 2H, *J=* 8.5 Hz), 5.47 (s, 2H), 4.90 (dd, 1H, *J=* 11.2, 4.3 Hz), 3.63 (s, 3H), 3.24 (dd, 1H, *J=* 13.9, 4.3 Hz), 2.92 (dd, 1H, *J=* 13.9, 11.2 Hz), 1.85 (s, 6H); MS *m*/*z* 274 (M+1).

The (S)-2-(2,5-Dimethyl-pyrrol-1-yl)-3-(4-hydroxy-phenyl)-propionic acid methyl ester was then dissolved in dichloromethane (300 mL), and acetyl chloride (9.1 mL, 0.128 mol) and 2,6 lutidine (14.9 mL, 0.128 mol) were added. The reaction mixture was stirred overnight and was then poured into ethyl acetate and extracted with brine. The organic layer was dried over MgSO₄ and the solvent removed in vacuo to give a brown oil. This was purified by chromatography ethyl acetate: hexane 1:5 to 1:1 to give a clear oil. Yield= 22.14g 55% ¹H NMR 500 MHz (CDCl₃) δ 6.89-6.83(m, 4H) 5.70 (s, 2H), 4.70 (dd, 1H, *J=* 9.77, 4.88 Hz), 3.72 (s, 3H), 3.50 (dd, 1H, *J=* 13.9,4.88 Hz), 3.11 (dd, 1H, *J=* 13.9, 9.77 Hz), 2.23 (3H, s), 1.93 (s, 6H); MS *m*/*z* 316 (M+1), 314 (M-1)

### Example 20

### (S)-3-(4-{2-[(7-Chloro-quinolin-4-yl)-methyl-amino]-ethoxy}-phenyl)-2-pyrrol-1-yl-propionic acid (51).

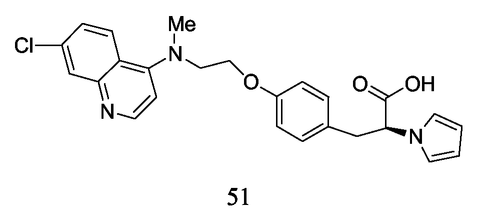

3-(4-{2-[(7-Chloro-quinolin-4-yl)-methyl-amino]-ethoxy}-phenyl)-2-pyrrol-1-yl-propionic acid methyl ester (50) was hydrolyzed using the method described in General Procedure A to provide 3-(4-{2-[(7-Chloro-quinolin-4-yl)-methyl-amino]-ethoxy}-phenyl)-2-pyrrol-1-yl-propionic acid 253 mg, 19%. ¹H NMR 500 MHz (D₆DMSO) δ 8.59 (1H, d, *J*=5.13Hz), 8.22 (1H, d, *J*=9.03Hz), 7.90 (1H, d, *J*= 2.13Hz) 7.46 (1H, d, *J*=9.03Hz), 6.95 (1H, d, *J*=5.13Hz), 6.85 (1H, d, *J*=8.05) 6.66-6.64 (4H, m), 5.80 (2H, s), 4.20 (1H br s), 4.17 (2H, br s), 3.60 ( 2H, br s) 3.26-3.19 (1H, m), 2.96, (3H, s), 2.91-2.88 (1H, m) MS *m*/*z* 448 (M-1).

(S)-3-(4-{2-[(7-Chloro-quinolin-4-yl)-methyl-amino]-ethoxy]-phenyl)-2-pyrrol-1-yl-propionic acid methyl ester (50) was prepared in the following manner.

### (a) (S)-3-(4-{2-[(7-Chloro-quinolin-4-yl)-methyl-amino]-ethoxy}-phenyl)-2-pyrrol-1-yl-propionic acid methyl ester (50).

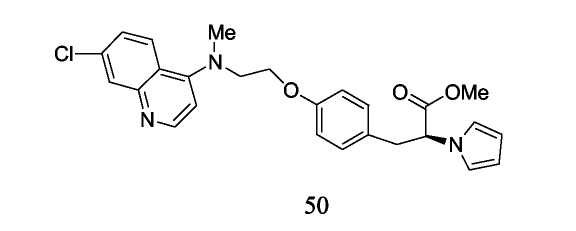

According to General Procedure B. To a stirred solution of 3-(4-hydroxyphenyl)-2-[pyrrol-1-yl]-propionic acid methyl ester described in example 1 (828 mg, 3.38 mmol) in anhydrous THF (15 mL) was added triphenyl phosphine (886 mg, 3.38 mmol and 2-[(7-chloro-4-quinolyl)methylamino] ethanol (800 mg, 3.38 mmol). A solution of DIAD (665 µL, 3.38 mmol) in anhydrous THF (5 mL) was added dropwise and the reaction mixture stirred at room temperature for 48 hours. The solvent was removed *in vacuo* and the residue purified by chromatography 1:10 EtOAc/Hexane to 1:1 EtOAc/Hexane. The product was obtained as an oil. Yield = 1.36g 87%. MS *m*/*z* 464 (M+1), 462 (M-1).

### Example 21

### (S)-2-(3-Acetyl-2,5-dimethyl-pyrrol-1-yl)-3-{4-[2-(4-methyl-2-phenyl-oxazol-5-yl)-ethoxy]-phenyl}-propionic acid (55).

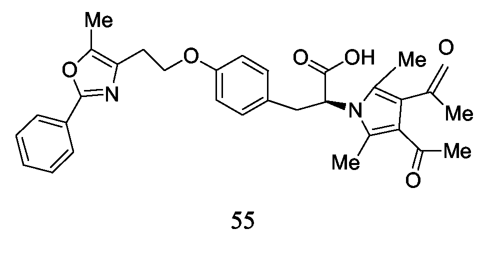

Prepared from (S)-2-(3-Acetyl-2,5-dimethyl-pyrrol-1-yl)-3-{4-[2-(4-methyl-2-phenyl-oxazol-5-yl)-ethoxy]-phenyl}-propionic acid methyl ester (54) described in General Procedure A. The desired product was obtained as cream crystalline powder. Yield = 148mg.

(S)-2-(3-Acetyl-2,5-dimethyl-pyrrol-1-yl)-3-{4-[2-(4-methyl-2-phenyl-oxazol-5-yl)-ethoxy]-phenyl]-propionic acid methyl ester (54) was prepared in the following manner.

### (a) (S)-2-(3-Acetyl-2,5-dimethyl-pyrrol-1-yl)-3-{4-[2-(4-methyl-2-phenyl-oxazol-5-yl)-ethoxy]-phenyl}-propionic acid methyl ester (54).

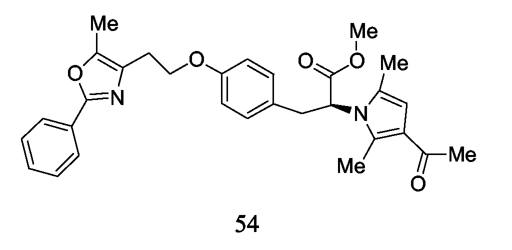

Prepared from (S)-2-(3-Acetyl-2,5-dimethyl-pyrrol-1-yl)-3-{4-[Hydroxy]-phenyl}-propionic acid methyl ester (53) and 2-(5-methyl-2-phenyloxazol-4-yl) ethanol using the method described in General Procedure B. The desired product was obtained as a clear oil. Yield = 474mg.

### (b) (S)-2-(3-Acetyl-2,5-dimethyl-pyrrol-1-yl)-3-{4-[Hydroxy]-phenyl]-propionic acid methyl ester (53).

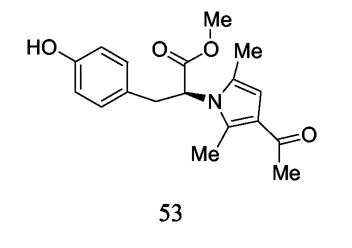

2-(3-Acetyl-2,5-dimethyl-pyrrol-1-yl)-3-{4-[Acetoxy]-phenyl}-propionic acid methyl ester (52) was deacetylated using the method described in Example 32. The product was purified using chromatography 1:1 EtOAc : Hexane and the product obtained as an oil which solidified on standing to give a white crystalline solid. Yield= 487mg, 72%.

### (c) (S)-2-(3-Acetyl-2,5-dimethyl-pyrrol-1-yl)-3{4-[Acetoxy]-phenyl}-propionic acid methyl ester (52).

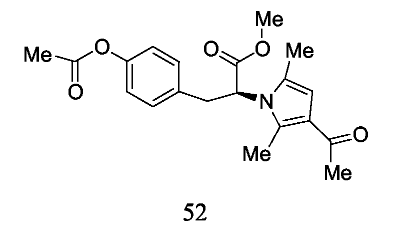

To a stirred solution of 2-(2,5-dimethyl-pyrrol-1-yl)-3-{4-[Acetoxy]-phenyl}-propionic acid methyl ester (2.45 g, 7.77 mmol) in CH₂Cl₂ was added acetyl chloride and trifluoromethane sulfonic acid. The solvent was removed *in vacuo.* The residue was purified by chromatography 1:10 to 1:1 EtOAc: Hexane to give the title compound as a yellow oil. Yield = 674mg, 25%.

### Example 22

### (S)-2-[2,5-Dimethyl-3-(2,2,2-trifluoro-acetyl)-pyrrol-1-yl]-3-{4-[2-(5-methyl-2-phenyl-oxazol-4-yl)-ethoxy]-phenyl}-propionic acid (59).

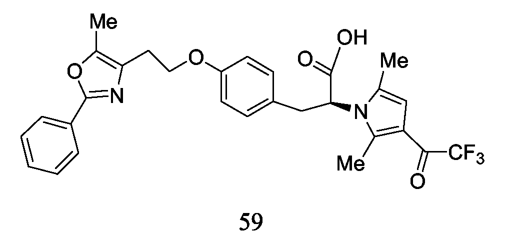

According to General Procedure A. Prepared from the ester (58) (1.48g, 2.62mmol) to give the desired product as a white powder 0.50g, 34%. Mass spectra (M+1), 541 (M-1) 539

(S)-2-[2,5-Dimethyl-3-(2,2,2-trifluoro-acetyl)-pyrrol-1-yl]-3-{4-[2-(5-methyl-2-phenyl-oxazol-4-yl)-ethoxy]-phenyl}-propionic acid methyl ester (58) was prepared in the following manner.

### (a) (S)-2-[2,5-Dimethyl-3-(2,2,2-trifluoro-acetyl)-pyrrol-1-yl]-3-{4-[2-(5-methyl-2-phenyl-oxazol-4-yl)-ethoxy]-phenyl}-propionic acid methyl ester (58)

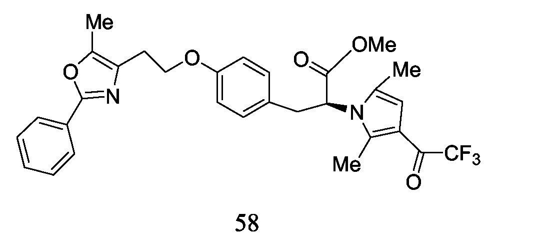

Prepared according to General Procedure B using (S)-2-[2,5-Dimethyl-3-(2,2,2-trifluoro-acetyl)-pyrrol-1-yl]-3-{4-[hydroxy]-phenyl}-propionic acid methyl ester (57) (1.08g, 2.9mmol) to give the desired product isolated by chromatography (1:10: 1:1 ethyl acetate: hexane as a white foam. Yield=1.48g, 92% Mass spectra (M+1), 555 (M-1) 553

### (b) (S)-2-[2,5-Dimethyl-3-(2,2,2-trifluoro-acetyl)-pyrrol-1-yl]-3-{4-[hydroxy]-phenyl}-propionic acid methyl ester (57).

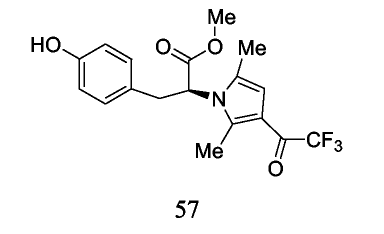

Prepared from the intermediate described in example (S)-2-[2,5-Dimethyl-3-(2,2,2-trifluoro-acetyl)-pyrrol-1-yl]-3-{4-[Acetoxy]-phenyl}-propionic acid methyl ester (56) deacetylated using the method described in General Procedure C. The product was purified using chromatography 1:1 EtOAc : Hexane and the product obtained as an oil which solidified on standing to give a white crystalline solid. Yield= 4.5g, 81% (M+1) 370 (M-1) 368.

### (c) (S)-2-[2,5-Dimethyl-3-(2,2,2-trifluoro-acetyl)-pyrrol-1-yl]-3-{4-[Acetoxy]-phenyl}-propionic acid methyl ester (57)

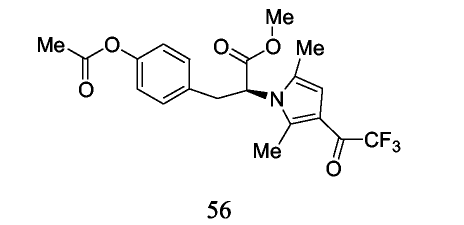

To a stirred solution of 2-(2,5-dimethyl-pyrrol-1-yl)-3-{4-[Acetoxy]-phenyl}-propionic acid methyl ester (6.0g, 19 mmol) in CH₂Cl₂ ( 50mL) was added trifluoroacetic anhydride (4.0mL, 28.5 mmol) and trifluoromethane sulfonic acid (4.0mL, 28.5 mmol). The reaction was stirred at room temperature for 20 minutes. The reaction mixture was poured into ammonium chloride solution and extracted with ethyl acetate. The organic layer was dried (MgSO4) and the solvent removed *in vacuo.* The residue was purified by chromatography 1:10 to 1:1 EtOAc: Hexane to give a yellow oil. Yield = 6.22g, 80% (M+1) 412 (M-1) 410.

### Example 23

### 3-{4-[2-(2-Phenyl-benzimidazol-1-yl)-ethoxy]-phenyl}-2-pyrrol-1-yl-propionic acid (61).

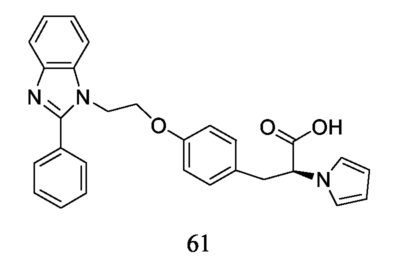

Prepared from 3-{4-[2-(2-Phenyl-benzimidazol-1-yl)-ethoxy]-phenyl}-2-pyrrol-1-yl-propionic acid methyl ester (60) using the hydrolysis method described in General Procedure A to give the desired product as a white foam. Yield = 697mg, 65%. Mp 210-212 °C.. (M+1) 452, (M-1) 450.

3-{4-[2-(2-Phenyl-benzimidazol-1-yl)-ethoxy]-phenyl}-2-pyrrol-1-yl-propionic acid methyl ester (60) was prepared in the following manner.

### (a) 3-{4-[2-(2-Phenyl-benzimidazol-1-yl)-ethoxy]-phenyl}-2-pyrrol-1-yl-propionic acid methyl ester (60).

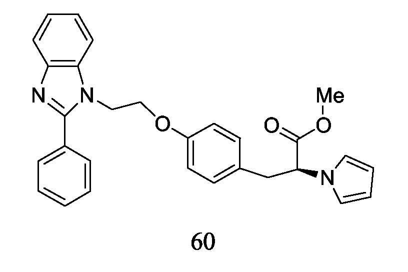

Prepared from the intermediate 3-(4-hydroxy-phenyl)-2-[pyrrol-1-yl]-propionic acid methyl ester (1) described in Example 1 and 2-(2-Phenyl-benzimidazol-1-yl)-ethanol using the method described in General Procedure B. The desired product was obtained as a white foam. 1.125g, 71% yield. M+1(obs)=466

### Example 24

### 2-(3-Bromo-pyrrol-1-yl)-3-{4-[2-(5-methyl-2-phenyl-oxazol-4-yl)-ethoxy]-phenyl}-propionic acid (64).

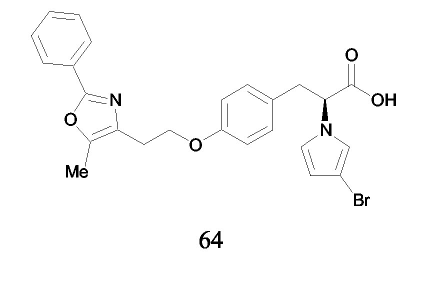

A mixture of (S)-2-(3-Bromo-pyrrol-1-yl)-3-14-[2-(5-methyl-2-phenyl-oxazol-4-yl)-ethoxy]-phenyl}-propionic acid methyl ester (64) (150 mg, 0.29 mmol) and lithium hydroxide (35 mg, 1.47 mmol) in 2-methoxyethanol (4 mL) and water (1 mL) and stirred at room temperature for 1 hour. The mixture was poured into water, acidified with aqueous 2N HCl, extracted with EtOAc and dried over MgSO₄. The solvent was removed under reduced pressure to yield the desired propionic acid (125 mg, 87%) as a white solid. 500 MHz ¹H NMR (CDCl₃) δ 7.96 (m, 2H), 7.43 (m, 3H), 6.92 (d, 2H, *J*= 8.5 Hz), 6.74 (m, 3H), 6.57 (m, 1H), 6.12 (m, 1H), 4.63 (dd, 1H, *J*= 8.3, 6.8 Hz), 4.16 (t, 2H, *J*= 6.6 Hz), 3.33 (dd, 1H, *J*= 13.9, 6.8 Hz), 3.14 (dd, 1H, *J*= 13.9, 8.3 Hz), 2.98 (t, 2H, *J* = 6.6 Hz), 2.37 (s, 3H); MS *m*/*z* 495, 497 (M+1).

(S)-2-(3-Bromo-pyrrol-1-yl)-3-{4-[2-(5-methyl-2-phenyl-oxazol-4-yl)-ethoxy]-phenyl}-propionic acid methyl ester (63) was prepared in the following manner.

### (a) (S)-2-(3-Bromo-pyrrol-1-yl)-3-{4-[2-(5-methyl-2-phenyl-oxazol-4-yl)-ethoxy]-phenyl}-propionic acid methyl ester (63).

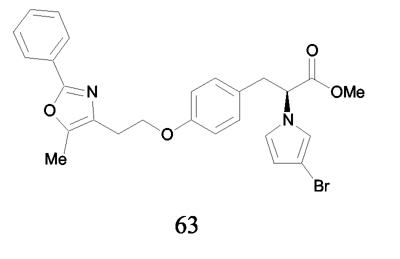

According to General Procedure B. To a stirred solution of (S)-2-(2-Bromo-pyrrol-1-yl)-3-(4-hydroxy-phenyl)-propionic acid methyl ester (63) (1.00 g, 3.08 mmol), triphenylphosphine (809 mg, 3.08 mmol) and 2-(5-methyl-2-phenyl-oxazol-4-yl)-ethanol (626 mg, 3.08 mmol) in THF (15 mL) was added dropwise a solution of diethylazodicarboxylate (500 µL, 3.08 mmol) in THF (4 mL). The mixture was stirred overnight, then concentrated. The residue was chromatographed on silica gel eluting with 30%-40% EtOAc in hexanes to yield the coupled product (590 mg, 38%) as a yellow oil. 500 MHz ¹H NMR (CDCl₃) δ 7.99 (m, 2H), 7.43 (m, 3H), 6.87 (m, 2H), 6.73 (m, 3H), 6.55 (m, 1H), 6.11 (m, 1H), 4.59 (dd, 1H, *J*= 9.0, 6.1 Hz), 4.20 (t, 2H, *J*= 6.6 Hz), 3.71 (s, 3H), 3.29 (dd, 1H, *J*= 13.9, 6.1 Hz), 3.14 (dd, 1H, *J*= 13.9, 9.0 Hz), 2.97 (t, 2H, *J*= 6.6 Hz), 2.36 (s, 3H); MS *m*/*z* 509, 511 (M+1).

### (b) (S)-2-(3-Bromo-pyrrol-1-yl)-3-(4-hydroxy-phenyl)-propionic acid methyl ester (62).

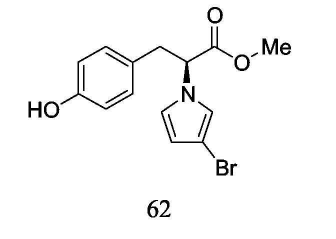

To a stirred, cooled (-78°C) solution of (S)-3-(4-Hydroxy-phenyl)-2-pyrrol-1-yl-propionic acid methyl ester (1.00 g, 4.08 mmol) in THF (20 mL) was added *N*-bromosuccinimide (724 mg, 4.08 mmol). The resulting mixture was warmed to 0°C and stirred for 3 hours. The solvent was removed under reduced pressure and the residue chromatographed on silica gel eluting 17%-27% EtOAc in hexanes to provide the desired product (1.08 g, 82%) as a light brown oil. 500 MHz ¹H NMR (CDCl₃) δ 6.86 (d, 2H, *J* = 8.5 Hz), 6.71 (m, 3H), 6.55 (m, 1H), 6.11 (m, 1H), 4.59 (dd, 1H, *J*= 9.0, 6.3), 3.71 (s, 3H), 3.29 (dd, 1H, *J*= 13.9, 6.3), 3.12 (dd, 1H, *J*= 13.9, 9.0); MS *m*/*z* 325 (M+1).

### Example 25

### 2-(2-Bromo-pyrrol-1-yl)-3-{4-[2-(5-methyl-2-phenyl-oxazol-4-yl)-ethoxy]-phenyl}-propionic acid (67).

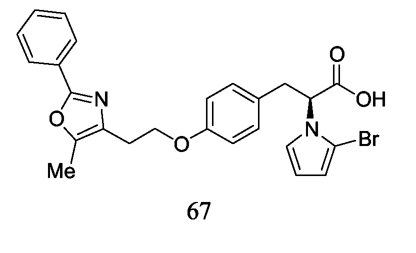

Prepared by the method described in General Procedure A from (S)-2-(2-Bromo-pyrrol-1-yl)-3-{4-[2-(5-methyl-2-phenyl-oxazol-4-yl)-ethoxy]-phenyl}-propionic acid methyl ester (67) to provide the desired propionic acid (93%) as an off-white solid. 500 MHz ¹H NMR (CDCl₃) δ 7.95 (m, 2H), 7.41 (m, 3H), 6.94 (m, 2H), 6.88 (m, 1H), 6.75 (m, 2H), 6.18 (t, 1H, *J=* 3.4 Hz), 6.10 (m, 1H), 5.10 (dd, 1H, *J=* 8.8, 6.1 Hz), 4.15 (m, 2H), 3.41 (dd, 1H, *J=* 14.2, 6.1 Hz), 3.18 (dd, 1H, *J=* 14.2, 8.8 Hz), 2.96 (m, 2H), 2.35 (s, 3H); MS *m*/*z* 495, 497 (M+1).

(S)-2-(2-Bromo-pyrrol-1-yl)-3-{4-[2-(5-methyl-2-phenyl-oxazol-4-yl)-ethoxy]-phenyl}-propionic acid methyl ester (66) was prepared in the following manner.

### (a) (S)-2-(2-Bromo-pyrrol-1-yl)-3-{4-[2-(5-methyl-2-phenyl-oxazol-4-yl)-ethoxy]-phenyl}-propionic acid methyl ester (66).

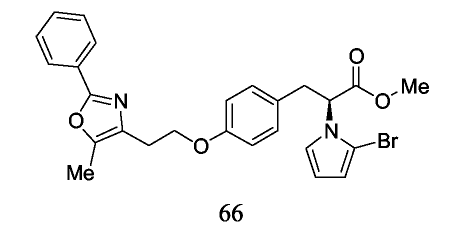

Prepared by the method described in General Procedure B from (S)-2-(2-Bromo-pyrrol-1-yl)-3-(4-hydroxy-phenyl)-propionic acid methyl ester (65) yielding a mixture of starting material and desired product. To a solution of the residue (0.61 mmol) in acetonitrile (10 mL) was added triethylamine (110 µL, 0.93 mmol) and benzoyl chloride (130 µL, 0.93 mmol). The mixture stirred at room temperature for 30 minutes and the solvent was removed under reduced pressure. The residue was chromatographed on silica gel eluting with 20% to 30% EtOAc in hexanes to afford the desired ester (190 mg, 12% overall) as a yellow oil. 500 MHz ¹H NMR (CDCl₃) δ 7.97 (m, 2H), 7.41 (m, 3H), 6.89 (m, 3H), 6.76 (d, 2H, *J=* 8.5 Hz), 6.17 (m, 1H), 6.09 (m, 1H), 5.05 (dd, 1H, *J=* 9.0, 6.1 Hz), 4.19 (t, 2H, *J=* 6.6 Hz), 3.71 (s, 3H), 3.35 (dd, 1H, *J=* 14.2, 6.1 Hz), 3.15 (dd, 1H, *J=* 14.2, 9.0 Hz), 2.95 (t, 2H, *J=* 6.6 Hz), 2.35 (s, 3H); MS *m*/*z* 509, 511 (M+1).

### (b) (S)-2-(2-Bromo-pyrrol-1-yl)-3-(4-hydroxy-phenyl)-propionic acid methyl ester (65).

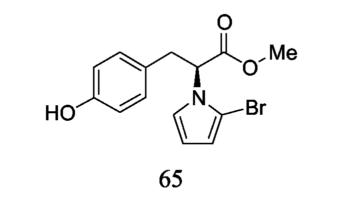

To a stirred, cooled (-78°C) solution of (S)-3-(4-Hydroxy-phenyl)-2-pyrrol-1-yl-propionic acid methyl ester (1.00 g, 4.08 mmol) in THF (20 mL) was added *N*-bromosuccinimide (724 mg, 4.08 mmol). The resulting mixture was stirred at -78°C for 15 minutes, then warmed to 0°C and stirred for 3 hours. The solvent was removed under reduced pressure and the residue chromatographed on silica gel eluting 17% to 27% EtOAc in hexanes to yield the desired pyrrole (1.01 g, 77%) as a light brown oil. 500 MHz ¹H NMR (CDCl₃) δ 6.89 (m, 3H), 6.69 (d, 2H, *J* = 8.5 Hz), 6.18 (m, 1H), 6.09 (m, 1H), 5.05 (dd, 1H, *J* = 9.0, 6.3 Hz), 3.72 (s, 3H), 3.35 (dd, 1H, *J*= 14.2, 6.3 Hz), 3.15 (dd, 1H, *J*= 14.2, 9.0 Hz); MS *m*/*z* 324, 326 (M+1).

### Example 26

### 2-(2-Chloro-pyrrol-1-yl)-3-{4-[2-(5-methyl-2-phenyl-oxazol-4-yl)-ethoxy]-phenyl}-propionic acid (70).

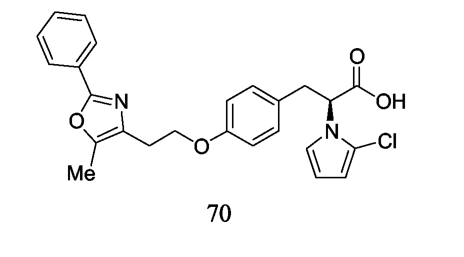

Prepared by the method described in General Procedure A. Hydrolysis of the methyl ester (70) afforded the desired acid (59%) as a white solid. 500 MHz ¹H NMR (CDCl₃) δ 8.03 (m, 2H), 7.44 (m, 3H), 6.93 (m, 2H), 6.75 (m, 3H), 6.13 (m, 1H), 6.00 (m, 1H), 5.08 (dd, 1H, J = 9.0, 6.1 Hz), 4.20 (t, 2H, *J* = 6.4 Hz), 3.40 (dd, 1H, *J=* 14.2, 6.1 Hz), 3.19 (dd, 1H, *J=* 14.2, 9.0 Hz), 3.00 (t, 2H, *J=* 6.4 Hz), 2.37 (s, 3H); MS *m*/*z* 451, 453 (M+1).

(S)-2-(2-Chloro-pyrrol-1-yl)-3-{4-[2-(5-methyl-2-phenyl-oxazol-4-yl)-ethoxy]-phenyl}-propionic acid methyl ester (69) was prepared in the following manner.

### (a) (S)-2-(2-Chloro-pyrrol-1-yl)-3-{4-[2-(5-methyl-2-phenyl-oxazol-4-yl)-ethoxy]-phenyl}-propionic acid methyl ester (69).

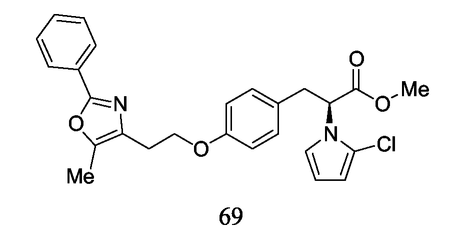

Prepared by the method described in General Procedure B from (S)-2-(2-Chloro-pyrrol-1-yl)-3-(4-hydroxy-phenyl)-propionic acid methyl ester (70) to afford the desired product (45%) as a yellow oil. 500 MHz ¹H NMR (CDCl₃) δ 7.97 (m, 2H), 7.40 (m, 3H), 6.90 (d, 2H, *J*= 8.5 Hz), 6.75 (m, 3H), 6.12 (m, 1H), 5.98 (m, 1H), 5.02 (dd, 1H, *J*= 9.3, 6.1 Hz), 4.19 (t, 2H, *J*= 6.7 Hz), 3.71 (s, 3H), 3.36 (dd, 1H, *J*= 14.2, 6.1 Hz), 3.15 (dd, 1H, *J*= 14.2, 9.3 Hz), 2.95 (t, 2H, *J*= 6.7 Hz), 2.35 (s, 3H); MS *m*/*z* 465, 467 (M+1).

### (b) (S)-2-(2-Chloro-pyrrol-1-yl)-3-(4-hydroxy-phenyl)-propionic acid methyl ester (68).

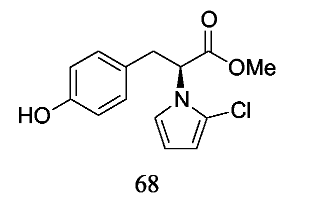

Prepared from (S)-3-(4-Hydroxy-phenyl)-2-pyrrol-1-yl-propionic acid methyl ester (1) and *N*-chlorosuccinimide to provide the desired pyrrole (41 %) as a yellow oil. 500 MHz ¹H NMR (CDCl₃) δ 6.88 (d, 2H, *J=* 8.5 Hz), 6.77 (m, 1H), 6.6 (d, 2H, *J=* 8.5 Hz), 6.13 (m, 1H), 5.98 (m, 1H), 5.02 (dd, 1H, *J=* 9.3, 6.1 Hz), 3.72 (s, 3H), 3.36 (dd, 1H, *J=* 14.2, 6.1 Hz), 3.15 (dd, 1H, *J=* 14.2, 9.3 Hz); MS *m*/*z* 314, 316 (M+1).

### Example 27

### (S)-2-(2-Butyryl-pyrrol-1-yl)-3-{4-[2-(5-methyl-2-phenyl-oxazol-4-yl)-ethoxy]-phenyl]-propionic acid (74).

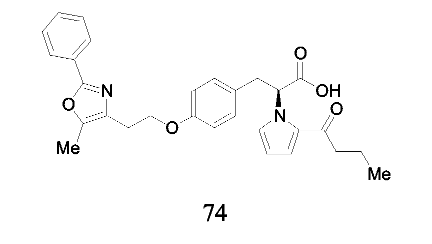

Prepared by the method described in General Procedure A from (S)-2-(2-Butyryl-pyrrol-1-yl)-3-{4-[2-(5-methyl-2-phenyl-oxazol-4-yl)-ethoxy]-phenyl}-propionic acid methyl ester (75) to afford the title acid. 500 MHz ¹H NMR (CDCl₃) δ 7.97 (m, 2H), 7.42 (m, 3H), 6.98 (m, 1H), 6.89 (m, 3H), 6.71 (d, 2H, *J* = 8.8 Hz), 6.12 (m, 1H), 4.16 (t, 2H, *J*= 6.6 Hz), 3.51 (m, 1H), 3.19 (dd, 1H, *J*= 14.5, 9.4 Hz), 2.95 (t, 2H, *J*= 6.6 Hz), 2.71 (m, 2H), 2.35 (s, 3H), 1.67 (q, 2H, *J*= 7.3 Hz), 0.92 (t, 3H, *J*= 7.3 Hz). MS *m*/*z* 487 (M+1).

(S)-2-(2-Butyryl-pyrrol-1-yl)-3-{4-[2-(5-methyl-2-phenyl-oxazol-4-yl)-ethoxy]-phenyl}-propionic acid methyl ester (73) was prepared in the following manner.

### (a) (S)-2-(2-Butyryl-pyrrol-1-yl)-3-{4-[2-(5-methyl-2-phenyl-oxazol-4-yl)-ethoxy]-phenyl}-propionic acid methyl ester (73).

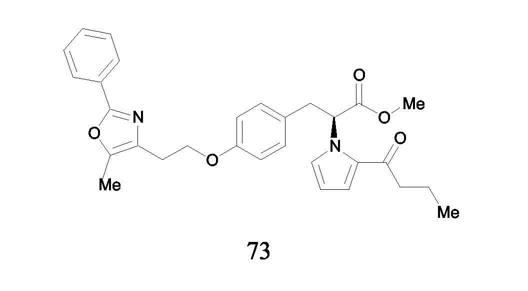

Prepared by the method described in General Procedure B from (S)-2-(2-Butyryl-pyrrol-1-yl)-3-(4-hydroxy-phenyl)-propionic acid methyl ester (74) to afford the desired product (27%) as a colorless oil. 500 MHz ¹H NMR (CDCl₃) δ 7.96 (m, 2H), 7.42 (m, 3H), 6.94 (m, 1H), 6.84 (d, 2H, *J=* 8.3 Hz), 6.08 (m, 1H), 4.16 (t, 2H, *J=* 6.6 Hz), 3.70 (s, 3H), 3.44 (dd, 1H, *J=* 14.2, 5.4 Hz), 3.15 (dd, 1H, *J=* 14.2, 9.8 Hz), 2.93 (t, 2H, *J=* 6.6 Hz), 2.66 (m, 2H), 2.34 (s, 3H), 1.64 (m, 2H), 0.90 (t, 3H, *J=* 7.3 Hz); MS *m*/*z* 501 (M+1).

The following compounds were prepared according to General Procedures C-E.

### (b) (S)-2-(2-Butyryl-pyrrol-1-yl)-3-(4-hydroxy-phenyl)-propionic acid methyl ester (72).

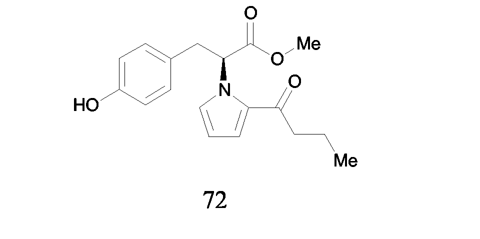

To a stirred solution of (S)-3-(4-Acetoxy-phenyl)-2-(2-butyryl-pyrrol-1-yl)-propionic acid methyl ester (73) (790 mg, 2.21 mmol) in anhydrous methanol (20 mL) was added potassium carbonate (306 mg, 2.21 mmol). The mixture was stirred at room temperature for 30 minutes. Solvent was removed under reduced pressure. The residue was taken up in EtOAc, washed with 2N aqueous HCl, and dried over MgSO₄. The solvent was removed under reduced pressure to provide the desired phenol (580 mg, 83%) as a colorless oil. 500 MHz ¹H NMR (CDCl₃) δ 6.90 (m, 4H), 6.65 (d, 2H, J= 8.5 Hz), 6.12 (m, 1H), 3.70 (s, 3H), 3.44 (dd, 1H, *J* = 14.3, 5.6 Hz), 3.15 (dd, 1H, *J*= 14.3, 9.5 Hz), 2.68 (m, 2H), 1.66 (m, 2H), 0.92 (t, 3H, *J*= 7.4 Hz); MS *m*/*z* 316 (M+1).

### (c) (S)-3-(4-Acetoxy-phenyl)-2-(2-butyryl-pyrrol-1-yl)-propionic acid methyl ester (71).

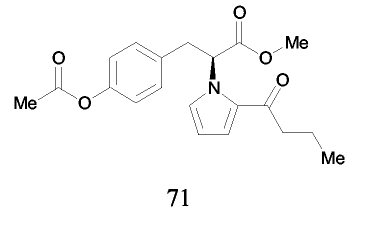

To a stirred solution of (S)-3-(4-Hydroxy-phenyl)-2-pyrrol-1-yl-propionic acid methyl ester (1) (1.0 g, 4.04 mmol) in acetonitrile (40 mL) was added triethylamine (735 µL, 5.26 mmol) and acetyl chloride (375 µL, 5.26). After stirring at room temperature overnight, the reaction was concentrated. The residue was taken up in water, extracted with EtOAc, dried over MgSO₄, and concentrated. The crude material was then dissolved in dichloromethane (15 mL). Butyryl chloride (505 µL, 4.85 mmol) was added, followed by triflic acid (430 µL, 4.85 mmol). The reaction stirred for 10 seconds, poured into saturated aqueous NH₄Cl solution, and extracted with EtOAc. The organic layer was dried and the solvent removed under reduced pressure. The residue was chromatographed on silica gel eluting with 25% to 30% EtOAc in hexanes to afford the desired pyrrole (790 mg, 55%) as a clear oil. 500 MHz ¹H NMR (CDCl₃) δ 6.91 (m, 6H), 6.14 (m, 1H), 4.86 (m, 1H), 3.72 (s, 3H), 3.52 (m, 1H), 3.23 (m, 1H), 2.70 (m, 2H), 2.25 (s, 3H), 1.66 (m, 2H), 0.93 (t, 3H, *J*= 8.5 Hz); MS *m*/*z* 358 (M+1).

### Example 28

### (S)-2-(2,5-Dimethyl-pyrrol-1-yl)-3-[4-(2-fluoro-benzyloxy)-phenyl]-propionic acid (76).

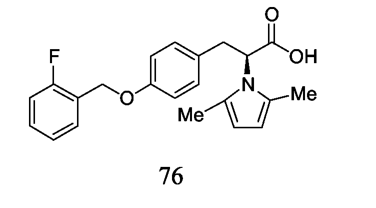

To a stirred solution of (S)-2-(2,5-Dimethyl-pyrrol-1-yl)-3-(4-hydroxyphenyl)-propionic acid methyl ester (1) (500 mg, 1.82 mmol) in dimethylformamide (20 mL) was added sodium hydride (218 mg, 5.46 mmol). The mixture was stirred at room temperature for 15 minutes and 2-fluorobenzyl bromide (440 µL, 3.64 mmol) was added. The mixture was stirred at room temperature overnight. The reaction was diluted with water, acidified with aqueous 2N HCl, extracted with EtOAc. The solvent was dried over MgSO₄ and concentrated under reduced pressure. The residue was taken up in methanol (20 mL) and lithium hydroxide (437 mg, 18.2 mmol) was added. The reaction was stirred at room temperature for 3 hours. The reaction was diluted with water, acidified with aqueous 2N HCl, extracted with EtOAc. The solvent was dried over MgSO₄, concentrated under reduced pressure, and chromatographed eluting with 10% to 25% 6:1 EtOAc:HOAc in hexanes to yield the desired propionic acid (300 mg, 45%). 500 MHz ¹H NMR (DMSO) δ 7.46 (m, 1H), 7.35 (m, 1H), 7.16 (m, 2H), 6.76 (m, 4H), 5.47 (s, 2H), 5.00 (s, 2H), 4.82 (dd, 1H, *J=* 11.2, 4.4 Hz), 3.29 (m, 1H), 2.96 (dd, 1H, *J=* 13.8, 11.2 Hz), 2.45 (s, 6H); MS *m*/*z* 368 (M+1).

(S)-2-(2,5-Dimethyl-pyrrol-1-yl)-3-(4-hydroxy-phenyl)-propionic acid methyl ester (75) was prepared in the following manner.

(S)-2-(2,5-Dimethyl-pyrrol-1-yl)-3-(4-hydroxy-phenyl)-propionic acid methyl ester (75).

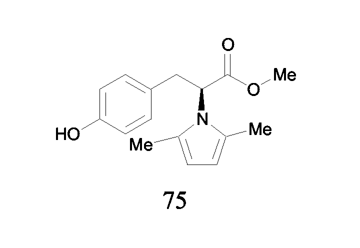

To a stirred solution of L-tyrosine methyl ester (3.90 g, 20.0 mmol) in toluene (200 mL) was added 2,5-hexandione (2.34 mL, 20.0 mmol) and p-toluenesulfonic acid (100 mg, 0.5 mmol). The mixture was heated to reflux 96 hours, then concentrated. The insoluble solid was filtered and washed with EtOAc. The filtrate was concentrated and chromatographed eluting with 10% to 25% 6:1 EtOAc:HOAc in hexanes to yield the desired pyrrole (3.14 g, 57%) as a yellow oil. 500 MHz ¹H NMR (CDCl₃) δ 6.60 (d, 2H, *J=* 6.6 Hz), 6.47 (d, 2H, *J*= 8.5 Hz), 5.47 (s, 2H), 4.90 (dd, 1H, *J =* 11.2, 4.3 Hz), 3.63 (s, 3H), 3.24 (dd, 1H, *J* = 13.9, 4.3 Hz), 2.92 (dd, 1H, *J=* 13.9, 11.2 Hz), 1.85 (s, 6H); MS *m*/*z* 274 (M+1).

### Example 29

### (S)-3-{4-[2-(5-Methyl-2-phenyl-oxazol-4-yl)-ethoxy]-phenyl}-2-(2-methyl-5-phenyl-pyrrol-1-yl)-propionic acid (80).

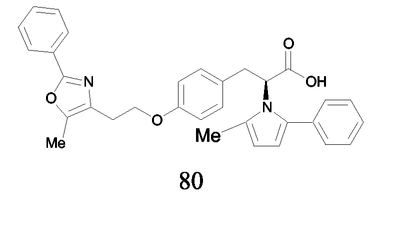

Prepared according to General Procedure A from (S)-3-{4-[2-(5-Methyl-2-phenyl-oxazol-4-yl)-ethoxy]-phenyl}-2-(2-methyl-5-phenyl-pyrrol-1-yl)-propionic acid methyl ester (79) which was hydrolyzed to afford the desired propionic acid (98%) as a brown solid. 500 MHz ¹H NMR (CDCl₃) δ 81.3 (m, 2H), 7.46 (m, 3H), 7.16 (m, 3H), 6.83 (m, 2H), 6.60 (d, 2H, *J=* 9.0 Hz), 6.55 (d, 2H, *J=* 8.8 Hz), 5.96 (m, 1H), 5.89 (d, 1H, *J=* 3.4 Hz), 4.95 (dd, 1H, *J=* 10.0, 5.1 Hz), 4.22 (t, 2H, *J*= 6.1 Hz), 3.25 (m, 2H), 3.07 (m, 3H), 2.86 (m, 1H), 2.40 (s, 3H), 2.33 (s, 3H); MS *m*/*z* 507 (M+1).

(S)-3-{4-[2-(5-Methyl-2-phenyl-oxazol-4-yl)-ethoxy]-phenyl}-2-(2-methyl-5-phenyl-pyrrol-1-yl)-propionic acid methyl ester (79) was prepared in the following manner.

### (a) (S)-3-{4-[2-(5-Methyl-2-phenyl-oxazol-4-yl)-ethoxy]-phenyl}-2-(2-methyl-5-phenyl-pyrrol-1-yl)-propionic acid methyl ester (79).

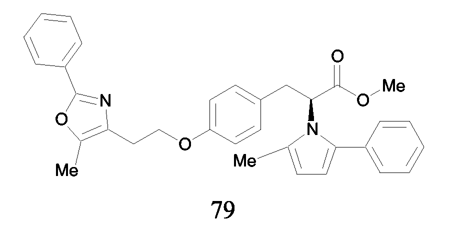

Prepared according to General Procedure B from 3-(4-Hydroxy-phenyl)-2-(2-methyl-5-phenyl-pyrrol-1-yl)-propionic acid methyl ester (1) and chromatographed on silica gel eluting with 15% to 50% EtOAc in hexanes to afford the desired pyrrole (300 mg, 39%) as a yellow oil. 500 MHz ¹H NMR (CDCl₃) δ 8.03 (m, 2H), 7.43 (m, 3H), 7.17 (m, 3H), 6.84 (m, 2H), 6.59 (d, 2H, *J* = 8.8 Hz), 6.53 (d, 2H, *J*= 8.8 Hz), 5.94 (d, 1H, *J*= 3.4 Hz), 5.87 (d, 1H, *J*= 3.4 Hz), 4.92 (dd, 1H, *J*= 9.9, 5.0 Hz), 4.18 (m, 2H), 3.76 (s, 3H), 3.25 (m, 2H), 2.99 (m, 3H), 2.86 (t, 1H, *J*= 6.3 Hz), 2.37 (s, 3H), 2.25 (s, 3H); MS *m*/*z* 521 (M+1).

### (b) 3-[4-(2-Fluoro-benzyloxy)-phenyl]-2-(2-methyl-5-phenyl-pyrrol-1-yl)-propionic acid (78).

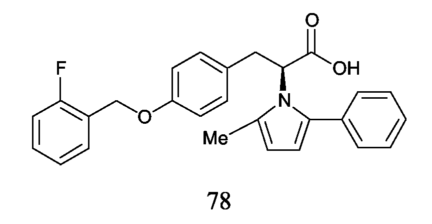

Prepared according to General Procedure A from 3-(4-Hydroxy-phenyl)-2-(2-methyl-5-phenyl-pyrrol-1-yl)-propionic acid methyl ester (77) to afford the desired propionic acid (23%) as a yellow solid. 500 MHz ¹H NMR (DMSO) δ 7.49 (t, 1H, J= 7.3 Hz), 7.38 (m, 1H), 7.19 (m, 5H), 6.78 (m, 2H), 6.67 (d, 2H, *J*= 8.8 Hz), 6.53 (d, 2H, *J*= 8.5 Hz), 5.81 (d, 2H, *J*= 3.2 Hz), 5.70 (d, 2H, *J*= 3.4 Hz), 5.02 (s, 2H), 4.85 (dd, 1H, *J=* 10.7, 4.6 Hz), 3.17 (m, 1H), 2.92 (m, 1H), 2.23 (s, 3H); MS *m*/*z* 430 (M+1).

### (c) 3-(4-Hydroxy-phenyl)-2-(2-methyl-5-phenyl-pyrrol-1-yl)-propionic acid methyl ester (77).

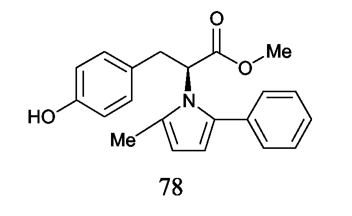

Prepared by the method described in Example 27(a) from 1-phenyl-1,4-pentanedione to afford the crude pyrrole which was used in subsequent steps. MS *m*/*z* 336 (M+1).

### Example 30

### (S)-2-(2,5-Dimethyl-pyrrol-1-yl)-3-{4-[2-(5-methyl-2-phenyl-oxazol-4-yl)-ethoxy]-phenyl}-propionic acid (82).

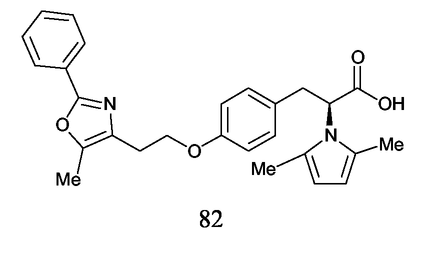

Prepared by the method described General Procedure A by in situ hydrolysis of the methyl ester (81). The product was chromatographed on silica gel eluting with 30% to 60% EtOAc in hexanes to isolate the desired propionic acid (5%) as red crystals. 500 MHz ¹H NMR (CDCl₃) δ 7.96 (m, 2H), 7.40 (m, 3H), 6.63 (m, 4H), 5.61 (s, 2H), 4.63 (dd, 1H, *J*= 10.0, 4.9 Hz), 4.38 (t, 2H, *J*= 6.5 Hz), 3.38 (dd, 1H, *J* = 13.9, 4.9 Hz), 3.00 (dd, 1H, *J*= 13.9, 10.0 Hz), 2.77 (m, 2H), 2.09 (s, 3H), 1.84 (s, 6H); MS *m*/*z* 445 (M+1).

### Example 31

### 3- {4-[3-(5-Methyl-2-phenyl-oxazol-4-yl)-propionyl]-phenyl}-2-pyrrol-1-yl-propionic acid (87).

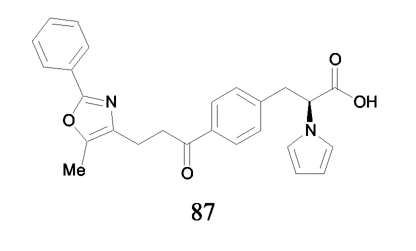

According to General Proceudure A. LiOH (0.24 g, 10 mmole) in 10 mL H₂O was added to a solution of 3-{4-[3-(5-Methyl-2-phenyl-oxazol-4-yl)-propionyl]-phenyl}-2-pyrrol-1-yl-propionic acid ethyl ester (86) in 20 mL of THF/Methanol (1/1) and stirred for 17 hours. The solvent was removed and the residue was redissolved in H₂O. This mixture was acidified with 1N HCl, and the product was collected by filtration. 0.36g (56% yield). Mp: 90°C to 92°C, MS (M+1) 429.

3- {4-[3-(5-Methyl-2-phenyl-oxazol-4-yl)-propionyl]-phenyl}-2-pyrrol-1-yl-propionic acid ethyl ester (86) was prepared in the following manner.

### (a) 3-{4-[3-(5-Methyl-2-phenyl-oxazol-4-yl)-propionyl]-phenyl}-2-pyrrol-1-yl-propionic acid ethyl ester (86).

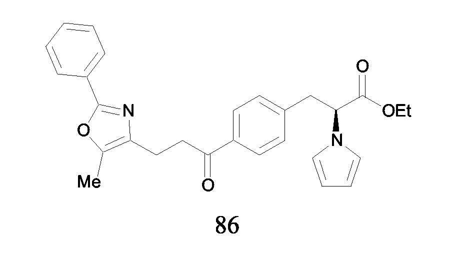

Oxalyl chloride (0.5 mL) was added to a solution of 2-(5-methyl-2-phenyloxazol-4-yl)acetic acid (84) (0.7 g, 2.88 mmole) in 10 mL of CH₂Cl₂ with a drop of DMF, and stirred at room temperature for one hour. The solvent was then evaporated, and the residue was redissolved in CH₂Cl₂ (20 mL). To this mixture was added ethyl 3-phenyl-2-pyrrolopropionate (85), followed by AlCl₃ (0.77 g, 5.75 mmole). The mixture was stirred for 17 hours. Water and EtOAc were then added. The organic layer was washed with brine and saturated aqueous NaHCO₃. The pure product was isolated by column chromatography (2/1 = hexane/EtOAc) (0.62 g, 45%). MS: M+1: 457.2.

### (b) Ethyl 3-phenyl-2-pyrrolopropionate (85).

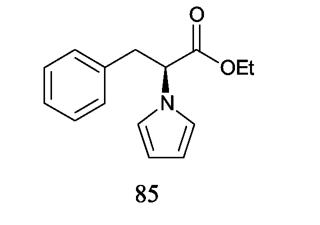

Triethylamine (3.3 mL) was added to a solution of ethyl phenylalanine in 50 mL CH₂Cl₂. The mixture was stirred at room temperature for 20 minutes. The solvent was then removed and the residue was redisolved in diethyl ether. The triethylamine HCl salt was removed by filtration, and the filtrate was concentrated to obtained an oil. To this oil was added H₂O (25 mL), acetic acid (25 mL), and NaOAc (3.87 g, 47.2 mmole), and 2,5-dimethoxytetrahydrofurane (5.2 mL, 40.1 mmole). The mixture was heated at 100°C for 15 minutes then cooled to room temperature and diluted with EtOAc/H₂O. The organic layer was washed with brine and dried over MgSO₄, and the solvent was removed at reduced pressure. Purification of the residue by column chromatography (10% EtOAc/hexanes) afforded 2.54 g of the pure product (47%).

### (c) 2-(5-methyl-2-phenyloxazol-4-yl)acetic acid (84).

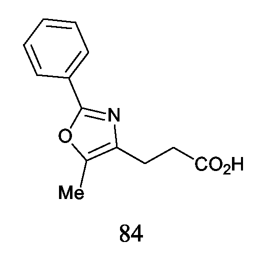

KOH (1.79 g, 31.8 mmole) was added to a solution of 4-(2-Cyanoethyl)-5-methyl-2-phenyloxazole (83) (1.5 g, 7.07 mmole) in ethanol/H₂O at room temperature. The mixture was then heated at reflux for 20 hours. The solvent was removed at reduced pressure. The residue was dissolved in H₂O, washed with ether, and the aqueous layer was acidified with 1 N HCl. The product was collected by filtration and dried to give a white powder. 0.7 g (44% yield).

### (d) 4-(2-Cyanoethyl)-5-methyl-2-phenyloxazole (83).

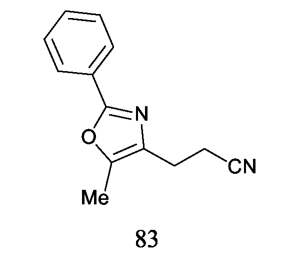

4-(2-bromoethyl)-5-methyl-2-phenyloxazole (84-1) (1.33 g, 5 mmole) was add to a suspension of KCN (0.65 g, 10 mmole) in 10 mL of DMSO at room temperature. The mixture was stirred for 17 hours, then diluted with water (100 mL), extracted with EtOAc, dried and evaporated, to give 1.1 g of the product which was used in the next step without further purification.

### (e) 4-(2-bromoethyl)-5-methyl-2-phenyloxazole (82).

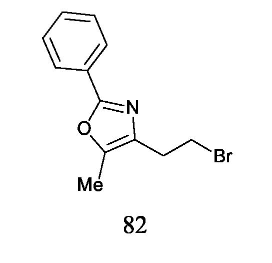

Triphenylphosphine (18 g, 68.9 mmole) and N-bromosuccinimide (12 g, 68.9 mmole) were added to a solution of 2-(5-methyl-2-phenyloxazol-4-yl)ethanol (11.16 g, 54.9 mmole) in 100 mL of THF. The mixture was stirred at room temperature for 2 hours. TLC and MS showed the reaction was completed. The reaction mixture was then distributed between EtOAc and saturated NaHCO₃. The organic layer was washed with brine, dried and purified by column chromatography (3:1 = Hexanes:EtOAc). The product was isolated as a pale yellow powder (13.5 g, 92.5%).

### Example 32

### 3-{4-[2-(5-Methyl-2-phenyl-oxazol-4-yl)-ethoxy]-phenyl}-2-thiophen-2-yl-propionic acid (91).

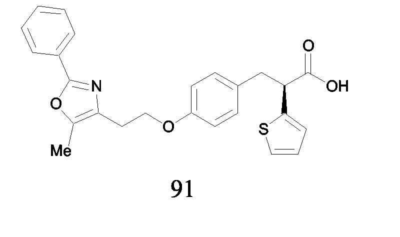

According to General Procedure A. The target compound was obtained by base (LiOH) hydrolysis of 3-{4-[2-(5-Methyl-2-phenyl-oxazol-4-yl)-ethoxy]-phenyl}-2-thiophen-2-yl-propionic acid (90) in THF/H₂O. Mp: 146°C to 147°C, MS(M+1):434, Anal. Calcd for C₂₅H₂₃O₄NS: C, 69.26; H, 5.35, N, 3.23. Found: C, 68.90; H, 5.43, N, 3.06. ¹H NMR (DMSO-D₆) δ 7.95-6.75 (m, 14H), 4.1 (t, 2H), 4.05 (t, 2H), 3.1 (m, 1H), 2.85 (m, 2H), 2.3 (s, 3H).

3-{4-[2-(5-Methyl-2-phenyl-oxazol-4-yl)-ethoxy]-phenyl}-2-thiophen-2-yl-propionic acid ethyl ester (90) was prepared in the following manner.

### (a) 3-{4-[2-(5-Methyl-2-phenyl-oxazol-4-yl)-ethoxy]-phenyl}-2-thiophen-2-yl-propionic acid ethyl ester (90).

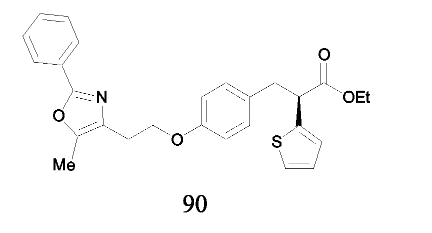

The benzyl group in ethyl 3-(p-benzyloxyphenyl)-2-thiophenepropionate (89)) was removed with TMSI using acetonitrile as a solvent. General Procedure B was followed to prepare the title compound. To a 0 °C solution of 2-(5-methyl-2-phenyl-oxazol-4-yl)ethanol and the resulting phenol, and triphenyl phosphine in 10 mL THF was added DEAD (1.00 mL, 6.4 mmole). The mixture was then stirred at ambient temperature for 20 hours. The solvent was removed and 3-{4-[2-(5-Methyl-2-phenyl-oxazol-4-yl)-ethoxy]-phenyl}-2-thiophen-2-yl-propionic acid ethyl ester (90) was isolated by column chromatography (2.1 g, 84%).

### (b) Ethyl 3-(p-benzyloxyphenyl)-2-thiophenepropionate (89).

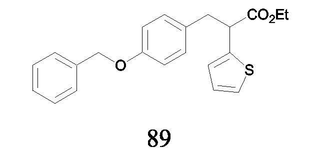

To a solution of ethyl 2-thiophene acetate (1.5 mL, 9.99 mmol) in 50 mL of THF under nitrogen was added LiHMDS (10 mL, 1.0M in THF) dropwise at-40°C. After the mixture was stirred for 30min., compound 3 in THF (50 mL) was added. The resulting mixture was stirred at ambient temperature for another 20 hours. The solvent was removed and the product was collected by column chromatography (2.9 g, 81 %).

### (c) p-Benzyloxybenzyl bromide (88).

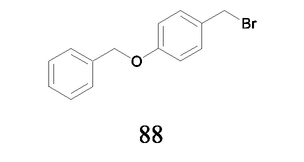

To a solution of 4.6 g of PBr₃ in 5 mL of dry THF at -5 °0 was added a solution 0.66 mL of pyridine in 1.3 mL of THF, followed by a solution of 2 in 75 mL of THF. The mixture was then stirred at ambient temperature for 20 hours, TLC and MS indicated the reaction was completed. The reaction was diluted with THF (100 mL) and filtered through celite, the product obtained (12 g, 87%) was used in the next step without further purification.

### Example 33

### 3-{4-[2-(5-Methyl-2-phenyl-oxazol-4-yl)-ethoxy]-phenyl}-2-pyridin-3-yl-propionic acid (92).

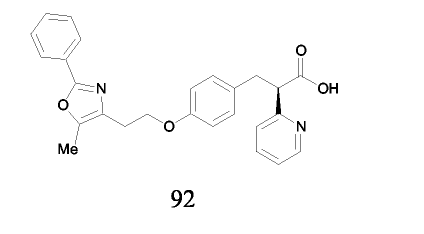

This compound was synthesized in the same manner as in Exampple 32 except ethyl 2-thiophene acetate was replaced by methyl 2-2-pyridin-3-yl acetate. Mp: 74.4-75.80 °C, MS(M+1):471, Anal. Calcd for C₂₆H₂₄O₄N₂: C, 66.32; H, 5.49, N, 5.95. Found: C, 66.34; H, 5.86, N, 5.81. ¹H NMR(DMSO-D₆) δ 8.37-6.73 (m, 13H), 4.1 (t, 2H), 3.8 (t, 2H), 2.95 (m, 1H), 2.80 (m, 2H), 2.3 (s, 3H).

### Example 34

### 3-{4-[2-(5-Methyl-2-phenyl-oxazol-4-yl)-ethoxy]-phenyl}-2-phenylpropionic acid (93).

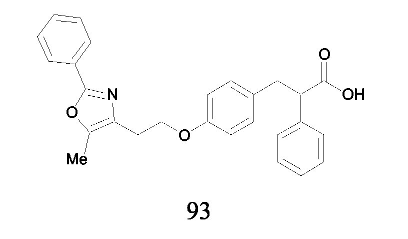

This compound was synthesized in the same manner as example 32 except ethyl 2-thiophene acetate was replaced by methyl 2-phenyl acetate. Mp: 177.9°C to 179.1°C, MS(M+1):417. Anal. Calcd for C₂₅H₂₄O₄N₂: C, 72.10; H, 5.81, N, 6.73. Found: C, 73.41; H, 6.04, N, 6.64. ¹H NMR(CDCl₃) δ 6.6-7.9 (m, 15H), 4.3 (t, 2H), 3.7 (m, 2H), 3.41 (m, 1H), 3.2 (m, 2H), 2.3 (s, 3H).

### Example 35

### 3- {4-[2-(5-Methyl-2-phenyl-oxazol-4-yl)-ethoxy]-phenyl}-2-(3-triflorophenyl)-propionic acid (94).

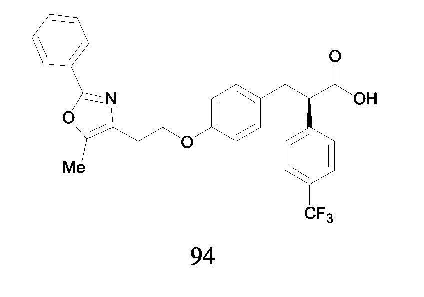

This compound was synthesized in the same manner as example 32 except ethyl 2-thiophene acetate was replaced by methyl 2-(3-triflorophenyl) acetate. Mp: 118.8°C to 110°C, MS(M+1): 496.2,; Anal. Calcd for C₂₈H₂₄O₄NF₃ HCl: C, 65.00; H, 4.79, N, 2.71. Found: C, 65.17; H, 4.37, N, 2.32. ¹H NMR(DMSO-D₆) δ 6.65-7.9 (m, 14H), 4.1 (t, 2H), 4.0 (m, 2H), 3.35 (m, 1H), 3.2 (m, 2H), 2.3 (s, 3H).

### Example 36

### 3-{4-[2-(5-Methyl-2-phenyl-oxazol-4-yl)-ethoxy]-phenyl}-2-phenylpropionic acid (R) (95).

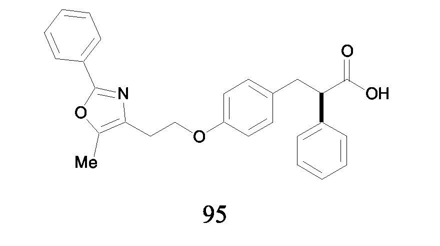

To a solution of 2-phenylacetic acid (10 g, 73.4 mmol) in oxalyl chloride (7 mL, 80.2 mmol) and 200 mL of CH₂Cl₂, was added 2 drops of DMF and stirred at ambient temperature for 2 hours, the solvent was removed and the residue was redisollved in 50 mL of THF. To this solution was added dropwise a solution of lithium (R)-(+)-4-benzyl-2-oxazolidinone (6.58 g, 37.1 mmol) in THF (50 mL). The resulting mixture was stirred at ambient temperature for 17 hours. The solvent was removed and the residue was treated with saturated NH₄Cl and extracted into CH₂Cl₂. The organic layer was washed with brine, and the solvent was removed to give 10.5gm of oil (97%). Mp: 124.1°C to 125°C, MS(M+1): 428.1; Anal. Calcd for C₂₇H₂₅O₄N: C, 75.86; H, 5.89, N, 3.28. Found: C, 75.84; H, 5.91, N, 3.24. ¹H NMR(DMSO-D₆) δ 12.5 (br s, 1H), 7.05-6.75 (m, 14H), 4.1 (t, 2H), 3.72 (t, 2H), 3.15 (m, 1H), 2.7-2.88 (m, 2H), 2.3 (s, 3H).

### Example 37

### 2-(4-Methoxy-phenyl)-3-{4-[2-(5-methyl-2-phenyl-oxazol-4-yl)-ethoxy]-phenyl}-propionic acid (96).

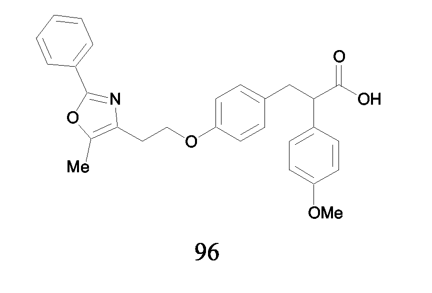

This compound was synthesized in the same manner as example 32 except that ethyl 2-thiopheneacetate was replaced by methyl p-methoxyphenylacetate. Mp: 114.7°C to 116.6°C, MS(M+1): 458.1; Anal. Calcd for C₂₈H₂₇O₅N: C, 73.51; H, 5.95, N, 3.06. Found: C, 73.31; H, 5.76, N, 2.89. ¹H NMR (DMSO-D₆) δ 7.85-6.70 (m, 13H), 3.65 (s,3H), 4.1 (t, 2H), 3.1 (m, 1H), 2.85 (m, 2H), 2.7-2.88 (m, 2H), 2.25 (s, 3H).

### Example 38

### 3-{4-[2-(5-Methyl-2-phenyl-oxazol-4-yl)-ethylamino]-phenyl}-2-pyrrol-1-yl-propionic acid (100).

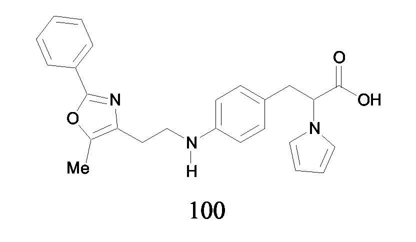

Compounds 99 (0.213 g, 1.06 mmol) and 97 (0.285 g, 1.17 mmol) were mixed in 20 mL of CH₂Cl₂ together with NaBH(OAc)₃ (0.337 mg, 1.6 mmol) and HOAc (0.7 mL) at 0 °C for 24 hours. The reaction was then diluted with CH₂Cl₂ and washed with saturated NaHCO₃. The organic layer was concentrated and purified by chromatography (4:1 EtOAc:Hexanes) to provide the product as a solid (0.15 g, 35%). Mp: 85.1°C to 87.4°C, MS(M+1): 416.1; Anal. Calcd for C₂₅H₂₅O₃N₃: C, 70.42; H, 5.98, N, 9.85. Found: C, 70.36; H, 5.97, N, 9.65. ¹H NMR(DMSO-D₆) δ 7.9-5.9 (m, 13H), 4.79 (t, 2H), 2.89-3.5 (m, 3H), 2.6 (t, 2H), 2.2 (s, 3H).

Compounds 99 and 98 were prepared in the following manner.

### (a) 3-(4-aminophenyl)-2-pyrrolopropionic acid methyl ester (99).

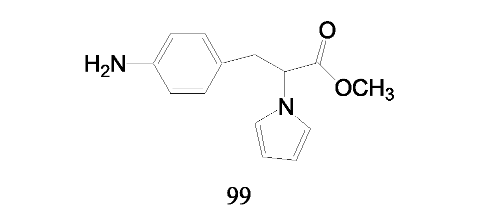

The nitro group in compound 98 was reduced to an amino group with Raney nickel in MeOH, which was used without further purification.

### (b) 3-(4-nitrophenyl)-2-pyrrolopropionic acid methyl ester (98).

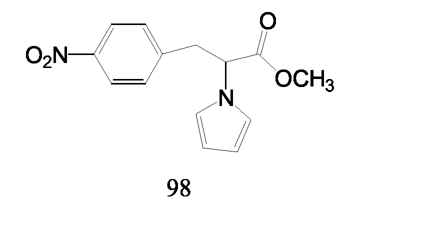

A mixture of 4-nitrophenylalanine (2.03 g, 9.66 mmol), 2,5-dimethoxytetrahydrofuran (2.15 mL, 16.6 mmol), and sodium acetate (1.603 g, 19.5 mmol) in 10 mL of water and 10 mL acetic acid was heated to 100 0°C for 20 minutes. The black solid was partitioned between EtOAc and H₂O. The organic layer was dried and the solvent was removed. 0.6g of pure compound (3-(4-nitrophenyl)-2-pyrrolopropionic acid) was obtained by column chromatography (5% MeOH/CH₂Cl₂). The product was treated with TMSCHN₂ to provide the methyl ester (98).

### (c) 2-(5-methyl-2-phenyl-oxazol-4-yl)acetaldehyde (97).

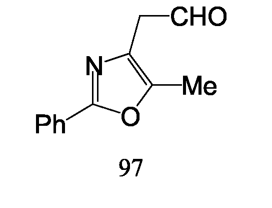

2-(5-methyl-2-phenyl-oxazol-4-yl)ethanol (0.5 g, 2.5 mmol) was added to a solution of ([1,1,1-triacetoxy-1,1-dihydro-1,2-benziodoxol-3(1H)-one (Dess-Martin reagent) in 10 mL of CH₂Cl₂. The mixture was stirred at ambient temperature for one hr. TLC and MS showed the reaction was completed, and the aldehyde 97 was isolated as an oil.

### Example 39

### 2-Methyl-3-{4-[2-(5-methyl-2-phenyl-oxazol-4-yl)-ethoxy]-phenyl}-2-phenyl-propionic acid (101).

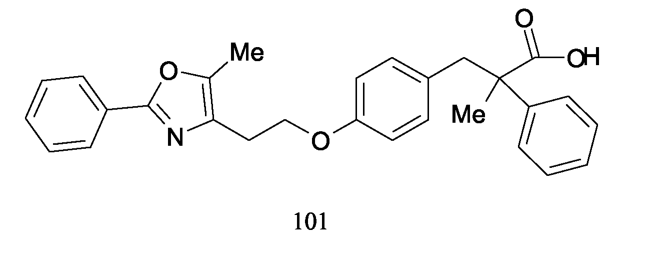

This compound was synthesized in the same manner as example 32 except that ethyl 2-thiopheneacetate was replaced by methyl 2,2-methylphenylacetate. Mp: 178°C to 179°C, MS(M+1): 442.1; Anal. Calcd for C₂₈H₂₇O₅N₁ 1/3 H₂O: C, 75.08; H, 6.18, N, 3.12. Found: C, 75.21; H, 6.34, N, 2.77. ¹H NMR(DMSO-D₆) δ 7.9-6.5 (m, 14H), 4.1 (t, 2H), 2.85 (t, 2H), 2.45 (s, 3H), 2.3 (s, 2H), 1.25 (s, 3H).

### Example 40

### 2,2-Dimethyl-3-{4-[2-(5-methyl-2-phenyl-oxazol-4-yl)-ethoxy]-phenyl}-propionic acid (102).

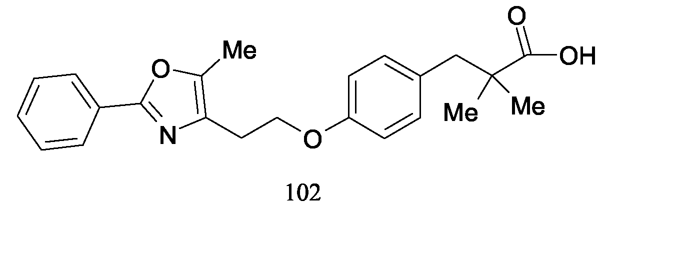

This compound was synthesized in the same manner as example 32 except that ethyl 2-thiopheneacetate was replaced by methyl 2,2-dimethylacetate. Mp: 133°C to 134°C, MS(M+1): 380.1; Anal. Calcd for C₂₃H₂₅O₄N: C, 72.80; H, 6.64, N, 3.69. Found: C, 72.60; H, 6.66, N, 3.50. ¹H NMR (DMSO-D₆) δ 7.85 (m, 2H), 7.45 (m, 3H), 6.95 (d, 2H), 4.15 (t, 2H), 2.85 (t, 2H), 2.65 (s, 2H), 2.3 (s, 3H), 0.99 (s, 3H).

### Example 41

### 2-Fluoro-3-{4-[2-(5-methyl-2-phenyl-oxazol-4-yl)-ethoxy]-phenyl}-2-phenyl-propionic acid (103).

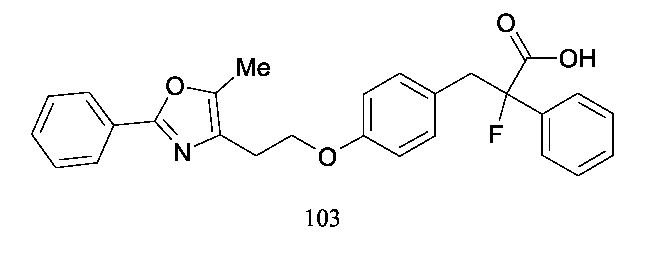

This compound was synthesized in the same manner as example 32 except that ethyl 2-thiopheneacetate was replaced by methyl 2,2-fluorophenylacetate. Mp: 163-165 °C, MS(M-1): 444.0; Anal. Calcd for C₂₇H₂₄O₄NF: C, 72.80; H, 5.43, N, 3.14. Found: C, 72.50; H, 5.56, N, 3.06. ¹H NMR(DMSO-D₆) δ 7.9-6.72 (m, 14H), 4.10 (t, 2H), 3.5 (t, 2H), 2.85 (t, 2H), 2.3 (s, 3H).

### Example 42

### 3-{4-[2-(5-Methyl-2-phenyl-oxazol-4-yl)-ethoxy]-phenyl}-2-(2-oxopyrrolidin-1-yl)-propionic acid (104).

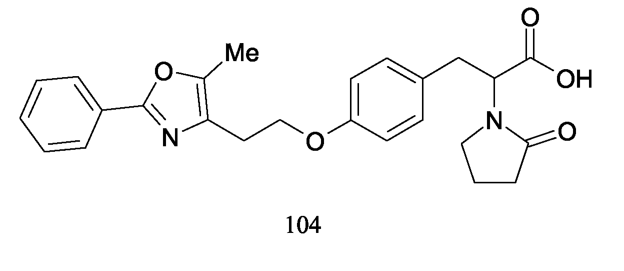

This compound was synthesized in the same manner as example 32 except that ethyl 2-thiopheneacetate was replaced by methyl 2-(2-oxo-pyrrolidin-1-ylphenyl) acetate. MS(M-1):435.1; Anal. Calcd for C₂₅H₂₆O₅N₂ H₂O: C, 66.30; H, 6.19, N, 6.19. Found: C, 66.26; H, 6.19, N, 6.19. ¹H NMR(DMSO-D₆) δ 7.88 (d, 2H), 7.4 (m, 3H), 7.05 (d, 2H), 6.75 (d, 2H), 4.60 (m, 1H), 4.1 (t, 2H), 3.1-2.75 (m, 4H), 2.3 (s, 3H), 2.1-1.65 (m, 6H).

### Example 43

### 2-Ethyl-5-(2-{4-[2-(5-methyl-2-phenyl-oxazol-4-yl)-ethoxy]-phenyl}-1-phenyl-ethyl)-1,3,4-oxadiazole (105).

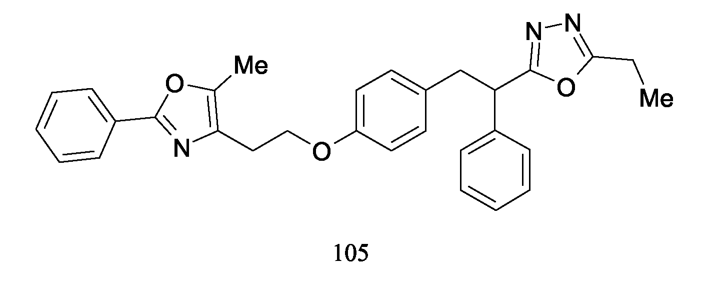

To a 0°C of 3-{4-[2-(5-Methyl-2-phenyl-oxazol-4-yl)-ethoxy]-phenyl}-2-phenyl-propionic acid (93) (0.197 g, 0.46 mmol) in 3 mL THF under N₂ was added 4-methylmorpholine (0.084 mL, 0.65 mmol), followed by isobutylchlorformate (0.084 mL, 0.65 mmol). The reaction mixture was stirred at 0 °C for 30 min., then filtered into a 0 °C solution of hydrazine (0.07 mL, 2.23 mmol) in 3 mL THF. The mixture was stirred for 50 min. at 0°C. EtOAc (10 mL), water, and NH₄Cl were then added. The organic portion was separated and evaporated to a white solid. The solid was redissolved in 4 mL dioxane. To the resulting solution was addred triethylorthopropionate (0.28 mL, 1.39 mmol), and methanesulfonic acid (0.06 mL, 0.09 mmol). The mixture was heated at 105°C for 15 min, then cooled to ambient temperature. EtOAc was added, then washed with saturated NaHCO₃ and brine. The solvent was evaporated and the residue was triturated with Et₂O and hexanes. A white solid was obtained (105). Mp: 110°C, MS(M+1):480.2; Anal. Calcd for C₃₀H₂₉O₃N₃ 1/2H₂O: C, 73.97; H, 6.31, N, 8.40. Found: C, 73.68; H, 6.14, N, 8.59. ¹H NMR (CDCl₃) δ 7.95 (d, 2H), 7.45-7.2 (m, 8H). 6.95 (d, 2H), (d, 2H), 6.6.7 (d, 2H), 4.35 (t, 1H), 4.15 (t, 2H), 3.5 (m, 1H), 3.2 (m, 1H), 2.95 (t, 2H), 2.75 (m, 2H), 3.25 (s, 3H), 1.3 (t, 3H).

### Example 44

### 3- {4-[3-(5-Methyl-2-phenyl-oxazol-4-yl)-propionyl]-phenyl}-2-pyrrol-1-yl-propionic acid (107).

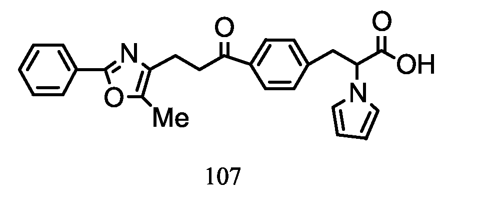

According to General Procedure A. LiOH (0.24 g, 10 mmole) in 10 mL H₂O was added to a solution of 8 in 20 mL of THF/Methanol (1/1) and stirred for 17 hours. The solvent was removed and the residue was redissolved in H₂O, this was acidified with 1N HCl, the product was collect by filtration, total weight 0.36 g (56%). MP: 90°C to 92°C, MS (M+1) 429.

3- {4-[3-(5-Methyl-2-phenyl-oxazol-4-yl)-propionyl]-phenyl}-2-pyrrol-1-yl-propionic acid ethyl ester (106) was prepared in the following manner.

### (a) 3-{4-[3-(5-Methyl-2-phenyl-oxazol-4-yl)-propionyl]-phenyl}-2-pyrrol-1-yl-propionic acid ethyl ester (106).

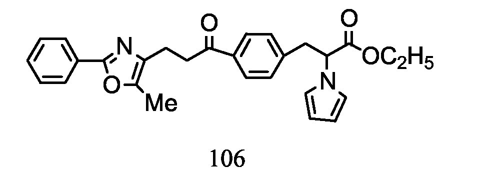

Oxalyl chloride (0.5 mL) was added to a solution of 2-(5-methyl-2-phenyloxazol-4-yl)acetic acid (See Example 31). (0.7 g, 2.88 mmole) in 10 mL of CH₂Cl₂ with a drop of DMF, and stirred at room temperature for one hour, the solvent was evaporated, and the residue was redissolved in CH₂Cl₂ (20 mL). Ethyl 3-phenyl-2-pyrrolopropionate (See Example 31) was added then added, followed by AlCl₃ (0.77 g, 5.75 mmole), the mixture was stirred for 17 hours, water and EtOAc was added, the organic layer was washed with brine, sat. NaHCO₃. The pure product was isolated by column chromatography (2/1 = hexane/EtOAc), it weight 0.62 g (45%).

### Example 45

### 3-{4-[2-(5-Methyl-2-phenyl-oxazol-4-yl)-ethoxy]-phenyl}-2-pyridin-3-yl-propionic acid (108).

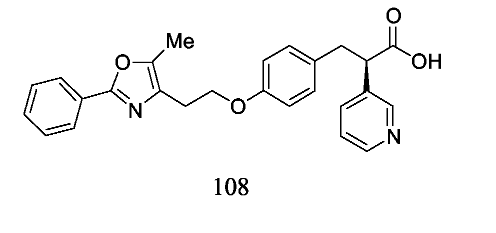

This compound was synthesized in the same manner as Example 32 except ethyl 2-thiophene acetate was replaced by methyl 2-2-pyridin-3-yl acetate. Mp: 74.4°C to 75.80°C, MS(M+1):471, Anal. Calcd for C₂₆H₂₄O₄N₂: C, 66.32; H, 5.49, N, 5.95. Found: C, 66.34; H, 5.86, N, 5.81. ¹H NMR(DMSO-D₆) δ 8.37-6.73 (m, 13 H), 4.1 (t, 2H), 3.8 (t, 2H), 2.95 (m, 1H), 2.80 (m, 2H), 2.3 (s, 3H).

### Example 46

### 3-{4-[2-(5-Methyl-2-phenyl-oxazol-4-yl)-.ethoxy]-phenyl}-2-phenylpropionic acid (110).

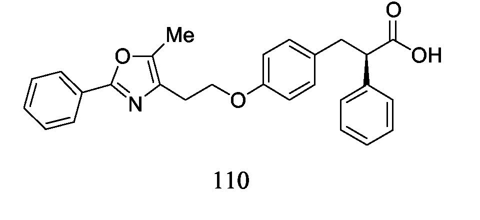

This compound was synthesized in the same manner as Example 32 from the corresponding methyl ester except compound 108 was used in the coupling reaction. Mp: 177.9°C to 179.1°C, MS(M+1): 417. Anal. Calcd for C₂₅H₂₄O₄N₂: C, 72.10; H, 5.81, N, 6.73. Found: C, 73.41; H, 6.04, N, 6.64. ¹H NMR(CDCl₃) δ 6.6-7.9 (m, 15H), 4.3 (t, 2H), 3.7 (m, 2H), 3.41 (m, 1H), 3.2 (m, 2H), 2.3 (s, 3H).

Methyl-2-phenyl-3-(p-hydroxy)phenyl propionate (109 was prepared in the following manner.

### (a) Methyl-2-phenyl-3-(p-hydroxy)phenyl propionate (109).

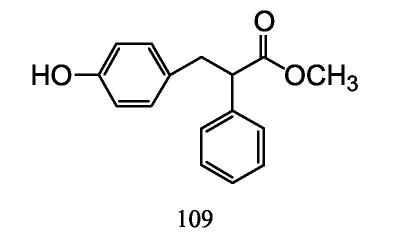

Prepared from 2-phenyl-3-(p-hydroxy) phenyl propionic acid using trimethylsilyl azide as the esterfying agent.

### Example 47

### 3- {4-[2-(5-Methyl-2-phenyl-oxazol-4-yl)-ethoxy]-phenyl}-2-(3-triflorophenyl)-propionic acid (111).

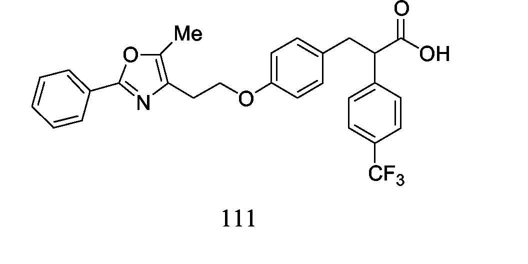

This compound was synthesized in the same manner as Example 32 except ethyl 2-thiophene acetate was replaced by methyl 2-(3-triflorophenyl) acetate. Mp: 118.8°C to 110°C, MS(M+1): 496.2; Anal. Calcd for C₂₈H₂₄O₄NF₃ 0.6HCl: C, 65.00; H, 4.79, N, 2.71. Found: C, 65.17; H, 4.37, N, 2.32. ¹H NMR(DMSO-D₆) δ 6.65-7.9 (m, 14H), 4.1 (t, 2H), 4.0 (m, 2H), 3.35 (m, 1H), 3.2 (m, 2H), 2.3 (s, 3H).

### Example 48

### (R)-3-{4-[2-(5-Methyl-2-phenyl-oxazol-4-yl)-ethoxy]-phenyl}-2-phenylpropionic acid (113).

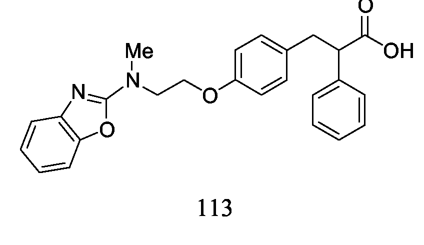

Synthesized in the same manner as example 32 except that ethyl 2-thiophene acetate was replaced by compound 112. Mp: 124.1°C to 125°C, MS(M+1): 428.1; Anal. Calcd for C₂₇H₂₅O₄N: C, 75.86; H, 5.89, N, 3.28. Found: C, 75.84; H, 5.91, N, 3.24. ¹H NMR(DMSO-D₆) δ 12.5 (b, 1H), 7.06-6.75 (m, 14H), 4.1 (t, 2H), 3.72 (t, 2H), 3.15 (m, 1H), 2.7-2.88 (m, 2H), 2.3 (s, 3H).

Compound 112 was prepared in the following manner.

### (a) Compound (112).

To a solution of 2-phenylacetic acid (10 g, 73.4 mmol) in oxalyl chloride (7 mL, 80.2 mmol) and 200 mL of CH₂Cl₂, was added 2 drops of DMF and stirred at ambient temperature for 2 hours. The solvent was removed and the residue was redisolved in 50 mL of THF. To this solution was added dropwise a solution of lithium (R)-(+)-4-benzyl-2-oxazolidinone (6.58 g, 37.1 mmol) in THF (50 mL). The mixture was again stirred at ambient temperature for 17 hours. The solvent was removed and the residue was treated with saturated NH₄Cl and extracted into CH₂Cl₂. The CH₂Cl₂ layer was washed with brine, dried over MgSO₄, and concentrated *in vacuo,* leaving 10.5g of oil (97% yield).

### Example 49

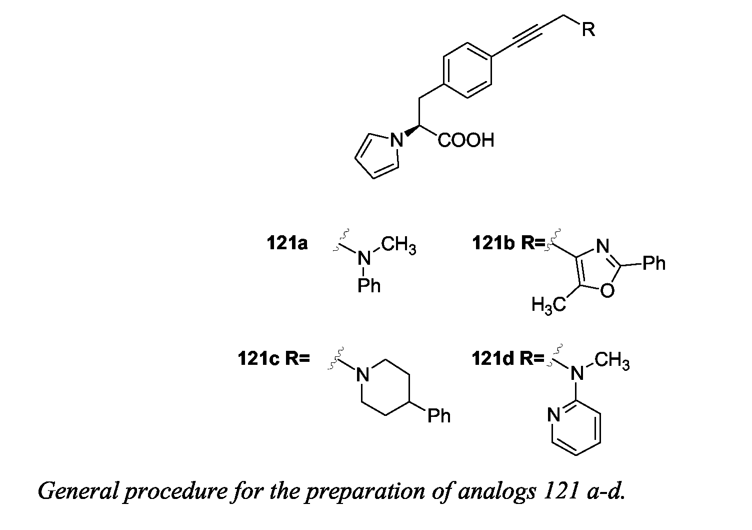

(*S*)-3-{4-[3-(Methyl-phenyl-amino)-prop-1-ynyl]-phenyl}-2-pyrrol-1-yl-propionic acid (121a). Ester 120a (0.82 g, 2.201 mmol) was dissolved in THF:H₂O (40:10 mL) and LiOH·H₂O (0.138 g, 3.301 mmol) was added and the mixture was stirred at room temperature for 3.5 hours. The solvent was removed and the residue was diluted with water, acidified with 10% HCl. The mixture was extracted with CHCl₃ (3 x 50 mL). The combined organic extracts were dried over MgSO₄, filtered and the solvent removed. Purification by chromatography on silica gel eluting with 0-2% MeOH in CHCl₃ containing formic acid (0-0.1 %) gave pure 129a as a light brown solid (0.727 g, 92%): Mp 55°C to 60°C; ¹H NMR (CDCl₃, 400 MHz) δ 7.29-7.25 (m, 2 H), 7.21 (d, *J*= 8.0 Hz, 2 H), 6.92 (d, *J*= 8.0 Hz, 2 H), 6.88 (d, *J*= 8.4 Hz, 2 H), 6.83 (t, *J*= 7.4 Hz, 1 H), 6.65 (t, *J*= 2.0 Hz, 2 H), 6.13 (t, *J=* 2.0 Hz, 2 H), 4.72 (dd, *J=* 9.2 and 6.0 Hz, 1 H), 4.23 (s, 2 H), 3.41-3.20 (m, 2 H), 3.00 (s, 3 H); CIMS *m*/*z* 359 (M + H)⁺; IR 3418, 2960, 1726, 1597, 1272 cm⁻¹. Anal. calcd for C₂₃H₂₂N₂O₂·0.3 H₂O: C, 75.93; H, 6.26; N, 7.74. Found: C, 75.73; H, 6.19; N, 7.53.

(*S*)-3-{4-[3-(5-Methyl-2-phenyl-oxazol-4-yl)-prop-1-ynyl]-phenyl}-2-pyrrol-1-yl-propionic acid (121b). Prepared from methyl ester 120b (0.337 g, 0.794 mmol) by the general procedure described for 129a. Purification by chromatography on silica gel eluting with hexanes:ethyl acetate (2:1) followed by hexanes:ethyl acetate (2:1) containing 0.2% formic acid gave acid 128b as a pale yellow solid (0.231 g, 71%): mp 178°C to 180°C; ¹H NMR (CDCl₃, 400 MHz) δ 7.78-7.76 (m, 2 H), 7.22 (m, 3 H), 7.05 (d, *J=* 8.0 Hz,2 H), 6.72 (d, *J=* 8.4 Hz, 2 H), 6.46 (t, *J=* 2.0 Hz, 2 H), 5.86 (t, *J=* 2.0 Hz, 2 H), 4.48 (dd, *J=* 9.6 and 5.6 Hz, 1 H), 3.50 (s, 2 H), 3.27-2.97 (m, 2 H), 2.26 (s, 3 H); CIMS *m*/*z* 409 (M - H)⁺. Anal. calcd for C₂₆H₂₂N₂O₃: C, 76.08; H, 5.40; N, 6.82. Found: C, 75.77; H, 5.45; N, 6.70.

(*S*)-3-{4-[3-(4-Phenyl-piperidin-1-yl)-prop-1-ynyl]-phenyl}-2-pyrrol-1-yl-propionic acid (121c). Prepared from ester 120c (0.344 g, 0.806 mmol) following the general procedure described for 129a. Purification by chromatography eluting with 0-8% MeOH in CHCl₃ containing 0.1 % formic acid gave pure 129c as a white solid (0.307 g, 92%): Mp 201°C-203°C; ¹H NMR (CDCl₃, 400 MHz) δ 7.33-21 (m, 5 H), 7.17 (d, *J*= 7.3 Hz, 2 H), 7.01 (d, *J*= 8.3 Hz, 2 H), 6.70 (t, *J*= 2.2 Hz, 2 H), 6.08 (t, J= 2.2 Hz, 2 H), 4.68 (dd, J= 8.8 and 6.1 Hz, 1 H), 3.91 (s, 2 H), 3.48-3.20 (m, 4 H), 2.98 (t, *J=* 11.5 Hz, 2 H), 2.61 (t, *J=* 11.9 Hz, 1 H), 2.25-2.13 (m, 2 H), 1.96 (d, *J=* 12.7 Hz, 2 H); CIMS *m*/*z* 413 (M + H)⁺. Anal. calcd for C₂₇H₂₈N₂O₂·0.9 H₂O: C, 75.64; H, 7.01; N, 6.53. Found: C, 75.29; H, 6.75; N, 6.44.

(*S*)-3-{4-[3-(Methyl-pyridin-2-yl-amino)-prop-1-ynyl]-phenyl}-2-pyrrol-1-yl-propionic acid (121d). Prepared from ester 120d (0.110 g, 0.294 mmol) by the general procedure described above. Acid 129d was obtained as a yellow solid (0.098 g, 93%): ¹H NMR (CDCl₃, 400 MHz) δ 8.02 (m, 1 H), 7.51-7.46 (m, 1 H), 7.14 (d, *J=* 8.3 Hz, 2 H), 6.82 (d, *J=* 8.0 Hz, 2 H), 6.62-6.54 (m, 2 H), 6.58 (t, *J=* 2.0 Hz, 2 H), 6.00 (t, *J*= 2.2 Hz, 2 H), 4.61 (dd, *J*= 9.4 and 5.6 Hz, 1 H), 4.37 (s, 2 H), 3.35-3.10 (m, 2 H), 3.05 (s, 3 H); CIMS *m*/*z* 360 (M + H)⁺. Anal. calcd for C₂₂H₂₁N₃O₂·0.5C₄H₈O₂: C, 71.44; H, 6.25; N, 10.41. Found: C, 71.14; H, 6.07; N, 10.20.

The ester starting materials were prepared in the following manner.

### (a) (120 a-d).

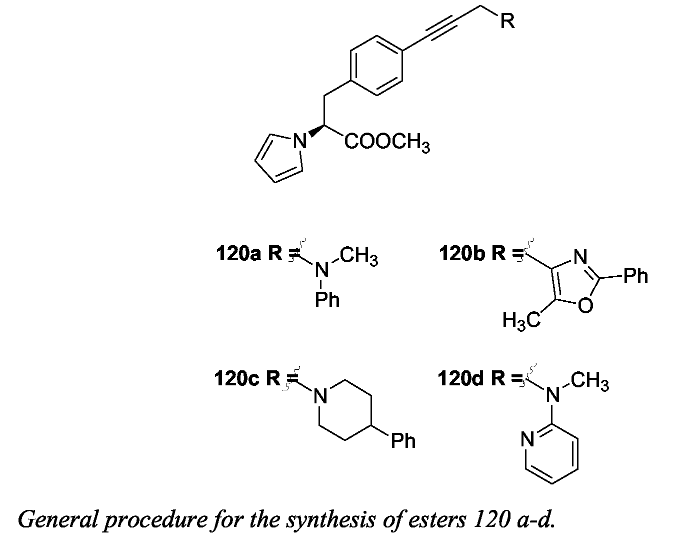

(*S*)-3-{4-[3-(Methyl-phenyl-amino)-prop-1-ynyl]-phenyl}-2-pyrrol-1-yl-propionic acid methyl ester (120a). Triflate 119 (1.66 g, 4.399 mmol) and Pd(PPh₃)₄ (0.356 g, 0.308 mmol) were dissolved in dry DMF (10 mL). A solution of *N*-methyl-*N*-prop-2-ynyl aniline (Magnus, P.; Ladlow, M.; Elliot, *J*.; Sook Kim, C. *J. Chem. Soc. Chem. Comm*. **1989**, 518-519) (1.27 g, 8.798 mmol) in DMF (2 mL) was added, followed by triethylamine (1.84 mL, 13.197 mmol). Nitrogen was passed through the reaction mixture for 0.5 hr. CuI (0.167 g, 0.880 mmol) was added and the mixture heated at 45-50 °C for 6 h under nitrogen. At this time additional Pd(PPh₃)₄ (0.356 g, 0.308 mmol) was incorporated. Heating was continued for 14 h and additional *N*-methyl-*N*-prop-2-ynyl aniline (0.635 g, 4.399 mmol) and catalyst (0.254 g, 0.220 mmol) were added. The mixture was heated for another 12 hr. At this time the mixture was allowed to cooled and diluted with water (150 mL) and Et₂O (100 mL). The phases were separated and the aqueous phase was extracted with Et₂O (4 × 80 mL). The combined organic extracts were washed with water and brine. Activated carbon was added and the mixture was boiled for 15-20 min. It was dried under MgSO₄, filtered and the solvent removed. Purification by flash chromatography on silica gel eluting with 60%-0% petroleum ether in dichloromethane followed by a second chromatographic purification eluting with hexanes:ethyl acetate (7:1 to 5:1) gave pure 11 as a thick oil (1.256 g, 77%): ¹H NMR (CDCl₃, 400 MHz) δ 7.29-7.21 (m, 4 H), 7.23 (d, *J*= 8.0 Hz, 2 H), 6.92-6.66 (m, 1 H), 6.89 (d, *J*= 8.4 Hz, 2 H), 6.66 (t, *J*= 2.4 Hz, 2 H), 6.12 (t, *J*= 2.4 Hz, 2 H), 6.68 (dd, *J*= 9.2 and 6.4 Hz, 1 H), 4.24 (s, 2 H), 3.67 (s, 3 H), 3.39-3.18 (m, 2 H), 3.02 (s, 3 H); CIMS *mlz* 373 (M + H)⁺.

(*S*)-3-{4-[3-(5-Methyl-2-phenyl-oxazol-4-yl)-prop-1-ynyl]-phenyl}-2-pyrrol-1-yl-propionic acid methyl ester (120b). This compound was prepared from triflate 119 (1.47 g, 3.9 mmol) and 5-methyl-2-phenyl-4-prop-2-ynyloxazole 125 (1.0 g, 5.070 mmol) following the general procedure described for 128a, except that no additional catalyst or 127 was added and the reaction was carried out at 90°C. Purification by chromatography eluting with hexanes:ethyl acetate (5:1 to 4:1) gave pure 128b as a thick oil (1.20, 73%): ¹H NMR (CDCl₃, 400 MHz) δ 7.99 (d, *J*= 8.4 Hz, 1 H), 7.98 (d, *J*= 7.2 Hz, 1 H), 7.45-7.39 (m, 3 H), 7.29 (d, *J*= 8.0 Hz, 2 H), 6.92 (d, *J*= 8.4 Hz, 2 H), 6.68 (t, *J*= 2.0 Hz, 2 H), 6.13 (t, *J*= 2.0 Hz, 2 H), 4.70 (dd, *J*= 8.8 and 6.4 Hz, 1 H), 3.71 (s, 2 H), 3.69 (s, 3 H), 3.40-3.20 (m, 2 H), 2.46 (s, 3 H); CIMS *m*/*z* 425 (M + H)⁺.

(*S*)-3-{4-[3-(4-Phenyl-piperidin-1-yl)-prop-1-ynyl]-phenyl}-2-pyrrol-1-yl-propionic acid methyl ester (120c). This compound was prepared from triflate 119 (0.500 g, 1.325 mmol) and *N*-prop-2-ynyl-4-phenylpiperidine (Lambert, S. *J*.; Kabalka, G. W.; Knapp, F. F. *Jr*.; Srivastava, P. C. *J*. *Org. Chem*. **1991,** *56*, 3707-3711) (0.396 g, 1.987 mmol) following the general procedure described for 128a with the exception that the reaction was carried out at 85°C. Additional catalyst (5 mol%) and *N*-prop-2-ynyl-4-phenylpiperidine (0.132 g, 0.662 mmol) were added after 20 hours. Purification by flash chromatography on silica gel eluting with 35%-45% ethyl acetate in hexanes afforded pure 128c as a thick oil (0.344 g, 61%): ¹H NMR (CDCl₃, 400 MHz) δ 7.32-7.17 (m, 5 H), 7.31 (d, *J=* 8.4 Hz, 2 H), 6.93 (d, *J=* 8.0 Hz, 2 H), 6.69 (t, *J=* 2.0 Hz, 2 H), 6.14 (t, *J=* 2.0 Hz, 2 H), 4.71 (dd, *J=* 9.2 and 6.4 Hz, 1 H), 3.70 (s, 3 H), 3.53 (s, 2 H), 3.41- 3.21 (m, 2 H), 3.08 (bd, *J=* 11.6 Hz, 2 H), 2.55-2.46 (m, 1 H), 2.36 (t, *J=* 11.2 Hz, 1 H), 2.35 (t, *J=* 11.2 Hz, 1 H), 1.91-1.79 (m, 4 H); CIMS *m*/*z* 427 (M + H)⁺.

(*S*)-3-{4-[3-(Methyl-pyridin-2-yl-amino)-prop-1-ynyl]-phenyl}-2-pyrrol-1-yl-propionic acid methyl ester (120d). This compound was prepared from triflate 119 (0.526 g, 1.394 mmol) and 2-(*N*-methyl-*N*-prop-2-ynyl)pyridine (see 126, below) (0.407 g, 2.788 mmol) following the general procedure described for 128a, with the exception that piperidine was used as solvent instead of DMF. Purification by chromatography on silica gel eluting with hexanes:ethyl acetate (4:1) gave 128d as a yellowish thick oil (0.132 g, 25.3%): ¹H NMR (CDCl₃, 400 MHz) δ 8.19-8.17 (m, 1 H), 7.48-7.43 (m, 1 H), 7.22 (d, *J*= 8.0 Hz, 2 H), 6.87 (d, *J*= 8.0 Hz, 2 H), 6.64 (t, *J*= 2.0 Hz, 2 H), 6.59 (d, *J*= 7.6 Hz, 1 H), 6.58 (d, *J*= 6.4 Hz, 1 H), 6.09 (t, *J=* 2.0 Hz, 2 H), 4.66 (dd, *J=* 9.0 and 6.4 Hz, 1 H), 4.54 (s, 2 H), 3.65 (s, 3 H), 3.36-3.16 (m, 2 H), 3.09 (s, 3 H); CIMS *m*/*z* 374 (M + H)⁺.

### (b) Triflate 119, used in the coupling action above, was prepared in the following manner.

(*S*)-2-Pyrrol-1-yl-3-[(4-trifluoromethanesulfonyloxy)phenyl]-propionic acid methyl ester (119).

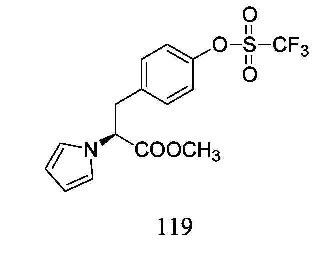

A mixture of phenol 1 (See Example 1) (6.64 g, 27.088 mmol) and *N*-phenyltrifluoromethanesulfonimide (10.47 g, 27.9 mmol) in CH₂Cl₂ (70 mL) under nitrogen was cooled at 0°C. Triethylamine (4.15 mL, 29.8 mmol) was added slowly. The mixture was stirred at 0°C for 1 hr. Then the temperature was allowed to reach room temperature slowly and stirred at this temperature for 2.5 hr. The mixture was diluted with Et₂O (70 mL) and washed with water, 1 N NaOH, and brine. The organic phase was dried over MgSO₄, filtered, and the solvent removed. Purification by flash chromatography on silica gel eluting with hexanes:ethyl acetate (5:1) afforded 127 as a thick oil (9.55 g, 93%) which solidified upon cooling and trituration to give a white solid: [α]²⁵_{D} -93.6° (c = 5, CHCl₃); ¹H NMR (CDCl₃, 400 MHz) δ 7.13 (d, *J=* 8.8 Hz, 2 H), 7.02 (d, *J=* 8.8 Hz, 2 H), 6.66 (t, *J=* 2.1 Hz, 2 H), 6.14 (t, *J*=2.1 Hz, 2 H), 4.69 (dd, *J=* 9.3 and 5.9 Hz, 1 H), 3.72 (s, 3 H), 3.43-3.25 (m, 2 H); IR 1731, 1426, 1203, 1136 cm⁻¹; CIMS *m*/*z* 378 (M + H)⁺. Anal. calcd for C₁₅H₁₄F₃NO₅S: C, 47.75; H, 3.74; N, 3.71. Found: C, 47.83; H, 3.64; N, 3.54.

### (c) The alkynes that were used in the coupling reaction above were prepared in the following manner.

2-(*N*-Methyl-*N*-prop-2-ynyl)pyridine (118).

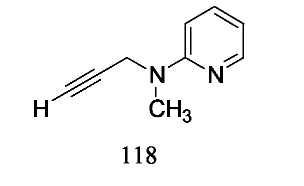

Sodium hydride (0.467 g, 11.673 mmol) was suspended in DMF (10 mL) under nitrogen and stirred in an ice bath. 2-(Methylamino)pyridine (1 mL, 9.728 mmol) was added and the mixture stirred at 0°C for 45 min. An 80% solution of propargyl bromide in toluene (1.19 mL, 10.7 mmol) was then incorporated. The mixture was allowed to reach room temperature and stirred overnight. The mixture was diluted with water and extracted with Et₂O. The combined organic extracts were washed with water and brine, dried over MgSO₄, filtered, and the solvent removed. Purification by flash chromatography on silica gel eluting with hexanes:ethyl acetate (8:1) afforded 126 as an oil (0.867 g, 61%): ¹H NMR (CDCl₃, 400 MHz) δ 8.17 (dd, *J=* 4.6 and 1.7 Hz, 1 H), 7.46 (dt, *J=* 8.5 and 2.0 Hz, 1 H), 6.58 (m, 2 H), 4.35 (d, *J =* 2.4 Hz, 2 H), 3.04 (s, 3 H), 2.11 (t, *J =* 2.4 Hz, 1 H); CIMS *m*/*z* 147 (M + H)⁺.

5-Methyl-2-phenyl-4-prop-2-ynyloxazole (117).

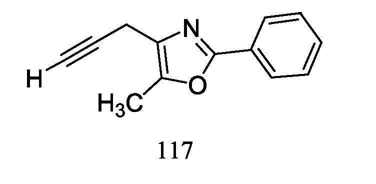

According to the procedure of Hulin (Hulin, B.; Newton, L. S.; Lewis, D. M.; Genereux, P. E.; Gibbs, M.; Clark, D. A. *J. Med. Chem*. **1996,** *39*, 3897-3907). Alkyne 7 (3.01 g, 10.472 mmol) was dissolved in MeOH (150 mL) and treated with 10% KOH (10 mL). The mixture was stirred at room temperature for 4.5 hours. At this time the solvents were removed and the residue diluted with water and acidified to pH~2 with 6 M HCl. The solid that precipitated was separated by vacuum filtration and dried. The filtrate was extracted with ethyl acetate (3 x 40 mL) and the combined organic extracts were dried over MgSO₄, filtered, and the solvent removed. The solid obtained from the previous steps (2.19 g) was treated with TFA (16 mL) and TFAA (8 mL) at 35-40 °C overnight, as previously described by Hulin and collaborators, to give oxazole 125 after purification by flash chromatography over silica gel eluting with hexanes:ethyl acetate (10:1 to 9:1). Oxazole 125 was obtained as an off-white solid (1.89 g, 94%): ¹H NMR (CDCl₃, 400 MHz) δ 7.96-7.99 (m, 2 H), 7.37-7.44 (m, 3 H), 3.50 (d, *J*= 2.8 Hz, 2 H), 2.41 (s, 3 H), 2.12 (t, *J*= 2.8 Hz, 1 H); CIMS *m*/*z* 198 (M + H)⁺.

*N*-[(1-acetyl-4-(trimethylsilyl)but-3-ynyl]benzamide (116).

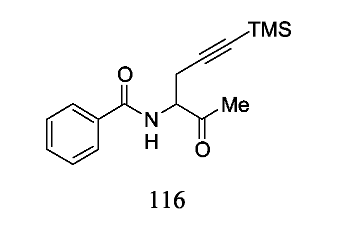

Amide 123 (2.55 g, 14.4 mmol) was dissolved in THF (150 mL) and cooled to -78 °C under nitrogen. A 1.0 M solution of LHMDS in THF (14.4 mL, 14.4 mmol) was added and the mixture stirred for 0.5 hr. A solution of 3-bromo-1-(trimethylsilyl)-1-propyne (2.6 mL, 18.7 mmol) in THF (15 mL) was added. The mixture was allowed to warm to room temperature and stirred overnight. The mixture was diluted with water and the phases were separated. The aqueous phase was extracted with ethyl acetate (3 x 50 mL) and the combined organic extracts were dried over MgSO₄, filtered, and the solvent removed. Purification by flash chromatography on silica gel eluting with hexanes:ethyl acetate (3:1) gave amide 124 as white solid (3.01 g, 73%): ¹H NMR (CDCl₃, 400 MHz) δ 7.68 (d, *J*= 7.2 Hz, 2 H), 7.40 (t, *J*= 7.2 Hz, 1 H), 7.32 (t, *J* = 7.2 Hz, 2 H), 7.02 (bd, *J* = 6.4 Hz, 1 H), 4.71 (q, *J*= 5.2 Hz, 1 H), 2.78 (d, *J*= 5.6 Hz, 2 H), 2.20 (s, 3 H), 0.01 (s, 9 H); CIMS *m*/*z* 288 (M + H)⁺; IR 3396, 2961, 2174, 1722, 1644, 1481, 1250 cm⁻¹. Anal. calcd for C₁₆H₂₁NO₂Si: C, 66.86; H, 7.36; N, 4.87. Found: C, 67.15; H, 7.49; N, 4.72.

Benzamidoacetone (115).

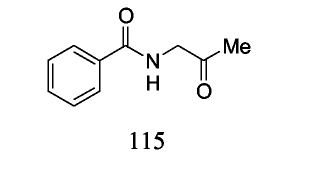

According to the procedure of Ellinger (Ellinger, L. P.; Goldberg, A. A. *J. Chem. Soc*. **1949**, 263). *N*-(2-Hydroxypropyl)benzamide (123) (3.0 g, 16.7 mmol) was dissolved in CH₂Cl₂ (60 mL) and PDC (9.42 g, 25.0 mmol) was added. The mixture was stirred at room temperature for 24 and more PDC (9.42 g, 25.0 mmol) was added. Stirring was continued for 24 hours. The mixture was diluted with ethyl acetate and filtered through a celite pad. The residue was then passed through a shoroom temperature silica gel column eluting with ethyl acetate. The solvent was removed and the residue was purified by flash chromatography on silica gel eluting with hexanes:ethyl acetate (1:1) containing MeOH (0 to 4%). This purification afforded amide 122 as a white solid (2.21 g, 75%): ¹H NMR (CDCl₃, 400 MHz) δ 7.76 (dd, *J=* 7.0 and 1.5 Hz, 2 H), 7.48-7.36 (m, 3 H), 6.96 (bs, 1 H), 4.30 (d, *J=* 4.6 Hz, 2 H), 2.21 (s, 3 H); CIMS *m*/*z* 178 (M + H)⁺.

*N*-(2-Hydroxypropyl)benzamide (114).

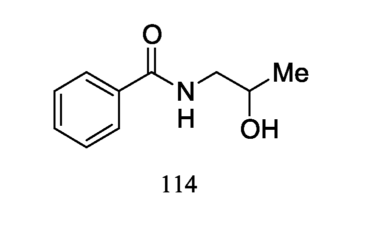

According to the procedure of Arai (Arai, K.; Tamura, S.; Masumizu, T.; Kawai, K.-I.; Naka*J*ima, S.; Ueda, A. *Can. J. Chem.* **1990,** *68,* 903-907). A solution of DL-1-amino-2-propanol (3.4 mL, 43.2 mmol) and triethylamine (16.4 mL, 117.9 mmol) in CH₂Cl₂ (60 mL) under nitrogen was cooled at -78°C. Benzoyl chloride (4.6 mL, 39.3 mmol) was added dropwise. The mixture was allowed to warm slowly and stirred at room temperature overnight. It was then diluted with CH₂Cl₂ (100 mL) and washed with cold 5% HCl and brine. The phases were separated and the aqueous phase was extracted with CH₂Cl₂ (2 x 50 mL). The combined organic extracts were dried over MgSO₄, filtered, and the solvent removed. The residue was dried under vacuum to give amide 5 as a pale yellow solid (6.21 g, 88%): ¹H NMR (CDCl₃, 400 MHz) δ 7.75 (d, *J*= 6.8 Hz, 2 H), 7.49-7.39 (m, 3 H), 6.57 (bs, 1 H), 4.00 (m, 1 H), 3.67-3.61 (m, 1 H), 3.31-3.24 (m, 1 H), 1.22 (d, *J*= 6.3 Hz, 3 H).

### Example 50

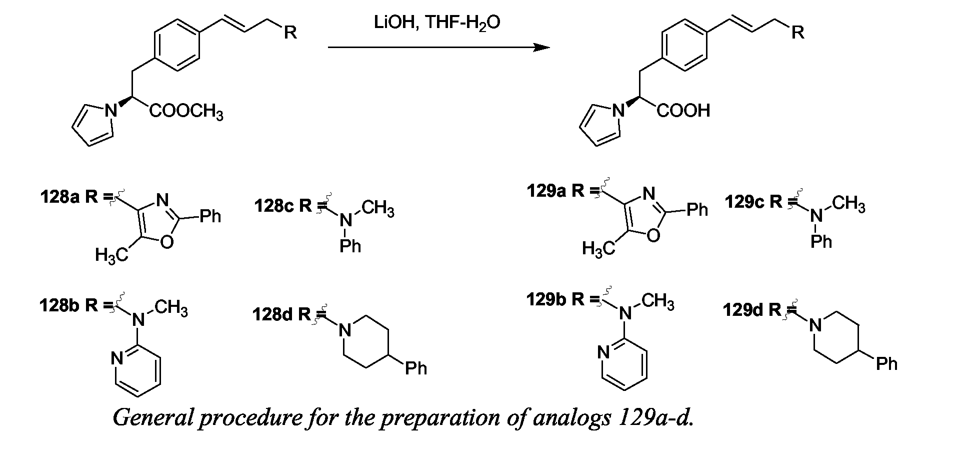

(*S*)-3-{4-[(*E*)-3-(5-Methyl-2-phenyl-oxazol-4-yl)-propenyl]-phenyl}-2-pyrrol-1-yl-propionic acid (129a). Prepared from ester 128a (0.140 g, 0.328 mmol) following the general procedure. Purification by chromatography on silica gel eluting with hexanes:ethyl acetate (2:1) containing formic acid (0-0.2%) gave acid 138a as an off-white solid (0.085 g, 63%): Mp 132°C-133°C; ¹H NMR (CDCl₃, 400 MHz) δ 7.95-7.92 (m, 2 H), 7.41-7.39 (m, 3 H), 7.19 (d, *J*= 8.0 Hz, 2 H), 6.90 (d, *J*= 8.4 Hz, 2 H), 6.65 (t, *J*= 2.0 Hz, 2 H), 6.37 (d, *J*= 15.6 Hz, 1 H), 6.25 (dt, *J*= 16.0 and 6.4 Hz, 1 H), 6.13 (t, *J*= 2.0 Hz, 2 H), 4.57 (dd, *J*= 8.8 and 6.0 Hz, 1 H), 3.39 (d, *J=* 6.8 Hz, 2 H), 3.38-3.15 (m, 2 H), 2.33 (s, 3 H); CIMS *m*/*z* 413 (M + H)⁺. Anal. calcd for C₂₆H₂₄N₂O₃·0.1 H₂O: C, 75.38; H, 5.89; N, 6.76. Found: C, 75.58; H, 6.21; N, 6.37.

(S)-3-{4-[(*E*)-3-(Methyl-pyridin-2-yl-amino)-propenyl]-phenyl}-2-pyrrol-1-yl-propionic acid (129b). Prepared from ester 128b (0.481 g, 1.281 mmol) following the general procedure described above. Purification by chromatography on silica gel gel eluting with 0-15% MeOH in CHCl₃ followed by THF gave pure 138b (0.123 g, 26%): Mp 155°C-165°C; ¹H NMR (CD₃OD, 400 MHz) δ 7.98 (dd, *J=* 5.2 and 1.0 Hz, 1 H), 7.52-7.47 (m, 1 H), 7.13 (d, *J=* 8.4 Hz, 2 H), 6.91 (d, *J=* 8.4 Hz, 2 H), 6.66 (t, *J*= 2.0 Hz, 2 H), 6.56 (dd, *J*= 6.4 and 5.2 Hz, 1 H), 6.38 (d, *J*= 15.6 Hz, 1 H), 6.14 (dt, *J*= 16.0 and 5.6 Hz, 1 H), 5.92 (t, *J*= 2.0 Hz, 2 H), 4.58 (dd, *J*= 9.6 and 5.6 Hz, 1 H), 4.22 (dd, *J*= 5.6 and 1.2 Hz, 2 H), 3.34-3.08 (m, 2 H), 3.02 (s, 3 H); CIMS *m*/*z* 362 (M + H)⁺.

(*S*)-3-{4-[(*E*)-3-(Methyl-phenyl-amino)-propenyl]-phenyl}-2-pyrrol-1-yl-propionic acid (129c). Ester 128c (0.877 g, 2.342 mmol) was dissolved in THF:H₂O (40:10 mL) and LiOH·H₂O (0.147 g, 3.513 mmol) was added and the mixture was stirred at room temperature for 3.5 hours. The solvent was removed and the residue was diluted with water, acidified with 10% HCl. The mixture was extracted with CHCl₃ (3 x 50 mL). The combined organic extracts were dried over MgSO₄, filtered and the solvent removed. Purification by chromatography on silica gel eluting with 0-3% MeOH in CHCl₃ containing 0.1% formic acid gave pure 138c as brownish solid (0.280 g, 33%): mp 85°C-90°C; ¹H NMR (CDCl₃, 400 MHz) δ ***E*****-isomer:** 7.26-7.18 (m, 3 H), 6.92 (d, *J=* 8.4 Hz, 2 H), 6.81-6.69 (m, 4 H), 6.68 (t, *J=* 2 Hz, 2 H), 2 H), 6.44 (d, *J=* 15.6 Hz, 1 H), 6.14 (t, *J=* 2.0 Hz, 2 H), 4.73 (dd, *J*= 9.2 and 6.0 Hz, 1 H), 4.03 (dd, *J*= 5.6 and 1.2 Hz, 2 H), 3.48-3.24 (m, 2 H), 2.97 (s, 3 H); CIMS *m*/*z* 361 (M + H)⁺. Anal. calcd for C₂₃H₂₄N₂O₂·0.9 H₂O: C, 74.41; H, 6.84; N, 7.55. Found: C, 74.03; H, 6.62; N, 7.36.

(*S*)-3-{4-[(*E*)-3-(4-Phenyl-piperidin-1-yl)-propenyl]-phenyl}-2-pyrrol-1-yl-propionic acid (129d). Prepared from ester 128d (1.772 g, 4.135 mmol) following the general procedure. Purification by chromatography on silica gel eluting with 0-12% MeOH in CHCl₃ containing formic acid (0%-0.1%) gave acid 138d as a pale yellow foam (1.38 g, 80%): Mp 126°C-130°C; ¹H NMR (CDCl₃, 400 MHz) δ 7.30-7.18 (m, 5 H), 7.17 (d, *J*= 7.1 Hz, 2 H), 6.95 (d, *J*= 8.1 Hz, 2 H), 6.57 (d, *J*= 16.0 Hz, 1 H), 6.55 (t, *J*= 2.0 Hz, 2 H), 6.26 (dt, *J*= 15.6 and 7.3 Hz, 1 H), 6.02 (t, *J*= 2.0 Hz, 2 H), 4.56 (dd, *J*= 8.8 and 6.1 Hz, 1 H), 3.69-3.49 (m, 4 H), 3.42-3.12 (m, 2 H), 2.70-2.62 (m, 3 H), 2.33-2.26 (m, 2 H), 1.91 (m, 2 H); CIMS *m*/*z* 415 (M + H)⁺. Anal. calcd for C₂₇H₃₀N₂O₂·1.0CH₂O₂: C, 73.02; H, 7.00; N, 6.08. Found: C, 72.85; H, 7.05; N, 6.06.

The ester starting materials were prepared in the following manner. Esters 128a-b.

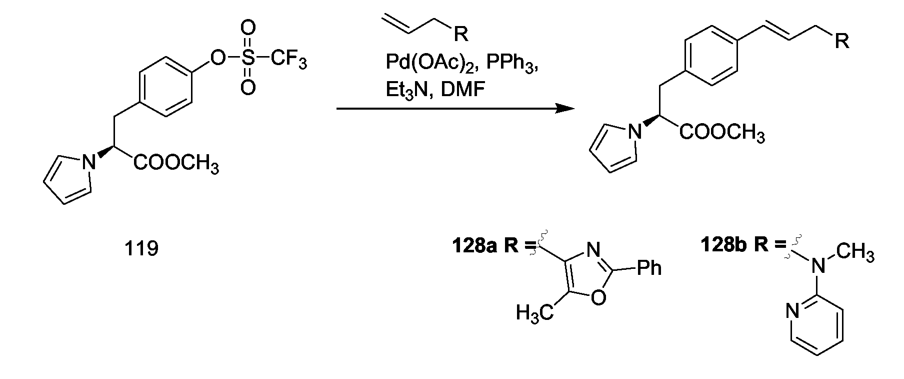

(*S*)-3-{4-[(*E*)-3-(5-Methyl-2-phenyl-oxazol-4-yl)-propenyl]-phenyl}-2-pyrrol-1-yl-propionic acid methyl ester (128a). A mixture of triflate 119 (Example 49) (1.5 g, 3.975 mmol), 5-methyl-2-phenyl-4-prop-2-enyloxazole 135 (1.19 g, 5.962 mmol), Pd(OAc)₂ (0.045 g, 0.199 mmol), PPh₃ (0.114 g, 0.437 mmol), triethylamine (1.10 mL, 7.95 mmol) was dissolved in DMF (15 mL). Nitrogen was passed through the mixture for 20-25 min. The reaction mixture was heated at 90°C under nitrogen for 44 hours. The mixture was allowed to cool and filtered through a celite pad, washing with ethyl ether. Water was added and the phases were separated. The aqueous phase was extracted with ethyl ether (4 x 60 mL). The combined organic extracts were washed with water, brine, dried over MgSO₄, filtered and the solvent removed. Purification by chromatography on silica gel eluting with 20%-25% ethyl acetate in hexanes afforded 137a as a thick oil (0.668, 39%): ¹H NMR (CDCl₃, 400 MHz) δ 8.00-7.97 (m, 2 H), 7.44-7.39 (m, 3 H), 7.22 (d, *J*= 8.0 Hz, 2 H), 6.91 (d, *J*= 8.0 Hz, 2 H), 6.69 (t, *J*= 2.0 Hz, 2 H), 6.42 (d, *J*= 14.8 Hz, 1 H), 6.32 (dt, *J*= 15.6 and 6.4 Hz, 1 H), 6.13 (t, *J*= 2.0 Hz, 2 H), 4.71 (dd, *J*= 8.8 and 6.4 Hz, 1 H), 3.68 (s, 3 H), 3.42 (d, *J*= 6.4 Hz, 2 H), 3.39-3.18 (m, 2 H), 2.34 (s, 3 H); CIMS *m*/*z* 427 (M + H)⁺.

(S)-3-{4-[(*E*)-3-(Methyl-pyridin-2-yl-aimino)-propenyl]-phenyl}-2-pyrrol-1-yl-propionic acid methyl ester (128b). This compound was prepared from triflate 119 (1.0 g, 5.30 mmol) and 2-(*N*-methyl-*N*-prop-2-enyl)pyridine 136 (0.785 g, 5.300 mmol) following the procedure described for 137a. Additional Pd(OAc)₂ (5 mol%) was added after 16 hr. The reaction was completed after 24 hours. Purification by chromatography eluting with 20%-25% ethyl acetate in hexanes gave 137b as a thick oil (0.600, 60%): ¹H NMR (CDCl₃, 400 MHz) δ 8.18-8.16 (m, 1 H), 7.47-7.42 (m, 1 H), 7.21 (d, *J*= 8.4 Hz, 2 H), 6.92 (d, *J*= 8.0 Hz, 2 H), 6.69 (t, *J*= 2.0 Hz, 2 H), 6.67-6.51 (m, 2 H), 6.42 (d, *J*= 15.6 Hz, 1 H), 6.18 (dt, *J*= 16.0 and 5.6 Hz, 1 H), 6.13 (t, *J*= 2.0 Hz, 2 H), 4.71 (dd, *J*= 8.6 and 6.0 Hz, 1 H), 4.30 (dd, *J*= 5.6 and 1.2 Hz, 2 H), 3.66 (s, 3 H), 3.39-3.19 (m, 2 H), 3.05 (s, 3 H); CIMS *m*/*z* 376 (M + H)⁺.

2-(*N*-Methyl-*N*-prop-2-enyl)pyridine (127).

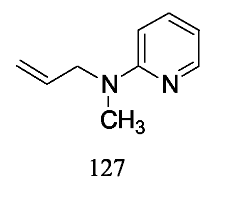

Sodium hydride (0.82 g, 20.4 mmol) was suspended in DMF (10 mL) under nitrogen and stirred in an ice bathr. 2-(Methylamino)pyridine (1.5 mL, 14.6 mmol) was added. The ice bath was removed and the mixture stirred at room temperature for 0.5 hr. The mixture was cooled back in an ice bath and allyl bromide (1.9 mL, 21.9 mmol) was added. The mixture was allowed to reach room temperature and stirred overnight. The mixture was diluted with water and extracted with Et₂O. The combined organic extracts were washed with water and brine, dried over MgSO₄, filtered, and the solvent removed. Purification by flash chromatography on silica gel eluting with hexanes:ethyl acetate (20:1) afforded 127 as an oil (1.74 g, 80%): ¹H NMR (CDCl₃, 400 MHz) δ 8.15 (m, 1 H), 7.42 (m, 1 H), 6.53 (dd, *J*= 6.8 and 4.8 Hz, 1 H), 6.48 (d, *J*=8.4 Hz, 1 H), 5.89-5.79 (m, 1 H), 5.14 (m, 1 H), 4.14 (d, *J=* 5.1 Hz, 2 H), 3.03 (s, 3 H); CIMS *m*/*z* 149 (M + H)⁺.

5-Methyl-2-phenyl-4-prop-2-enyloxazole (126).

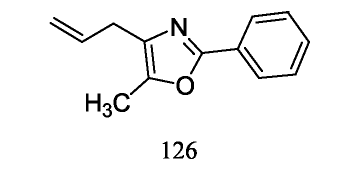

Amide 125 (2.00 g, 9.205 mmol) was dissolved in TFA (16 mL) and TFAA (8 mL) was added. The mixture was heated at 35°C-40°C for 16 hours. The mixture was allowed to cool and the solvents removed under reduced pressure. The residue was diluted with saturated NaHCO₃ (50 mL) and solid NaHCO₃ was added to neutralize the mixture. It then was extracted with ethyl acetate (3 x 60 mL). The combined organic extracts were washed with brine, dried over MgSO₄, filtered and the solvent removed. Purification by flash chromatography on silica gel eluting with hexanes:ethyl acetate (15:1) gave 135 (1.751 g, 95.5%): ¹H NMR (CDCl₃, 400 MHz) δ 7.98 (d, *J*= 7.8 Hz, 2 H), 7.43-7.35 (m, 3 H), 6.03-5.93 (m, 1 H), 5.13 (dq, *J*= 16.9 and 1.7 Hz, 1 H), 5.09 (dq, *J*= 10.0 and 1.5 Hz, 1 H), 3.29 (d, *J*= 6.3 Hz, 2 H), 2.32 (s, 3 H); CIMS *m*/*z* 200 (M + H)⁺; IR 3070, 2924, 1638, 1450 cm⁻¹.

*N*-(1-Acetylbut-3-enyl)benzamide (125).

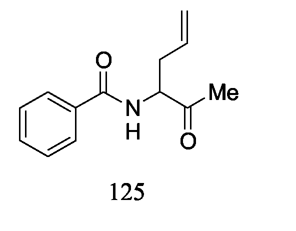

Benzamidoacetone (See Example 49) (2.098 g, 11.839 mmol) was dissolved in THF (120 mL) and cooled to -78°C under nitrogen. A 1.0 M solution of LHMDS in THF (11.9 mL, 11.9 mmol) was added and the mixture stirred for 40 minutes. A solution of allyl bromide (1.33 mL, 15.39 mmol) in THF (10 mL) was added. The mixture was allowed to warm to room temperature and stirred overnight. The mixture was diluted with brine and the phases were separated. The aqueous phase was extracted with ethyl acetate (3 x 50 mL) and the combined organic extracts were dried over MgSO₄, filtered, and the solvent removed. Purification by flash chromatography on silica gel eluting with hexanes:ethyl acetate (2:1 to 1:1) gave amide 9 (2.019 g, 78.5%): ¹H NMR (CDCl₃, 400 MHz) δ 7.79 (d, *J=* 7.1 Hz, 2 H), 7.51-7.41 (m, 3 H), 6.95 (bd, *J=* 5.4 Hz, 1 H), 5.74-5.64 (m, 1 H), 5.17-5.12 (m, 2 H), 4.88 (dt, *J=* 6.8 and 5.4 Hz, 1 H), 2.85-2.78 (m, 1 H), 2.61-2.54 (m, 1 H), 2.28 (s, 3 H); CIMS *m*/*z* 218 (M + H)⁺; IR 3263, 3081, 1719, 1632, 1556 cm⁻¹. Anal. calcd for C₁₃H₁₅NO₂: C, 71.87; H, 6.96; N, 6.45. Found: C, 71.91; H, 7.03; N, 6.52.

### (a-2) Esters 128c-d were prepared in the following manner.

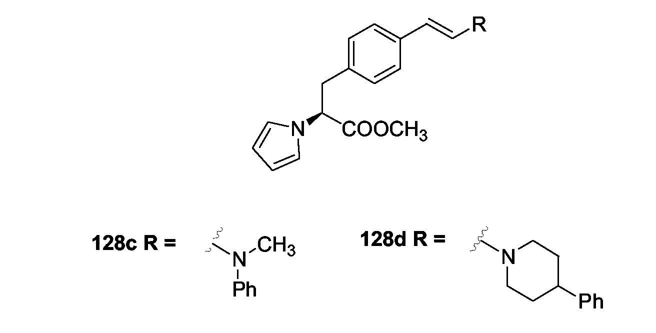

(*S*)-3-{4-[(*E*)-3-(Methyl-phenyl-amino)-propenyl]-phenyl}-2-pyrrol-1-yl-propionic acid methyl ester (128c). Triflate 127 (0.98 g, 2.592 mmol), boronate ester 124 (0.850 g, 3.111 mmol), K₂CO₃ (0.716 g, 5.184 mmol) were stirred in dry toluene (25 mL). Nitrogen was passed through the mixture for 0.5 hr. Pd(PPh₃)₄ (0.149 g, 0.129 mmol) was added and the reaction mixture was heated at 85°C-90°C for 24 hours. The mixture was allowed to cool, diluted with ethyl acetate (70 mL) and washed with sat NaHCO₃, water and brine. The organic extracts were dried over MgSO₄, filtered and the solvent removed. Purification by flash chromatography on silica gel eluting with 50-0% petroleum ether in dichloromethane followed by a second chromatographic purification eluting with hexanes:ethyl acetate (8:1 to 5:1) gave ester 137c as a 94:6 *E:Z* mixture of isomers (0.819 g, 84%). **E-isomer:** ¹H NMR (CDCl₃, 400 MHz) δ 7.23-7.20 (m, 2 H), 7.21 (d, *J*= 8.0 Hz, 2 H), 6.92 (d, *J*= 8.0 Hz, 2 H), 6.77-6.70 (m, 3 H), 6.69 (t, *J*= 2.0 Hz, 2 H), 6.44 (d, *J*= 16.0 Hz, 1 H), 6.19 (dt, *J*= 15.6 and 5.6 Hz, 1 H), 6.13 (t, *J*= 2.0 Hz, 2 H), 4.71 (dd, *J*= 9.2 and 6.4 Hz, 1 H), 4.05 (dd, *J*= 5.6 and 1.2 Hz, 2 H), 3.69 (s, 3 H), 3.40-3.19 (m, 2 H), 2.95 (s, 3 H); CIMS *m*/*z* 375 (M + H)⁺.

(*S*)-3-{4-[(*E*)-3-(4-Phenyl-piperidin-1-yl)-propenyl]-phenyl}-2-pyrrol-1-yl-propionic acid methyl ester (128d). This compound was prepared from triflate 127 (2.0 g, 5.296 mmol), and boronate ester 124 (2.6 g, 7.944 mmol) following the procedure described for 128c. Purification by flash chromatography eluting with 33-45% ethyl acetate in hexanes containing 1% Et₃N gave 128d (E-isomer, 1.801 g, 79%) and *E:Z* mixture (0.217 g, 9.9%) as oils. E-isomer: ¹H NMR (CDCl₃, 400 MHz) δ 7.50-7.15 (m, 7 H), 6.95 (d, *J*= 8.4 Hz, 2 H), 6.69 (t, *J*= 2.0 Hz, 2 H), 6.45 (d, *J*= 16.0 Hz, 1 H), 6.27 (dt, *J*= 15.6 and 6.8 Hz, 1 H), 6.13 (t, *J* = 2.0 Hz, 2 H), 4.71 (dd, *J*= 8.8 and 6.4 Hz, 1 H), 3.68 (s, 3 H), 3.40-3.19 (m, 2 H), 3.16 (d, *J*= 6.8 Hz, 2 H), 3.08 (d, *J*= 11.6 Hz, 2 H), 2.51-2.46 (m, 1 H), 2.07 (t, *J*= 11.2 Hz, 1 H), 2.06 (t, *J*= 11.2 Hz, 1 H), 1.84-1.77 (m, 4 H); CIMS *m*/*z* 429 (M + H)⁺.

Methyl-phenyl-[2-(4,4,5,5-tetramethyl-[1,3,2]dioxaborolan-2-yl)-vinyl]-amine (124).

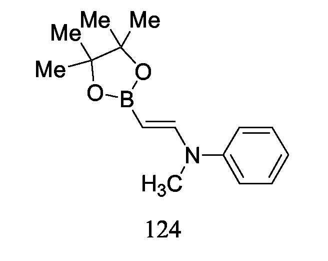

A mixture of *N*-methyl-*N*-prop-2-ynyl aniline (1.0 mL, 6.873 mmol) and pinacolborane (1.2 mL, 8.247 mmol) in CH₂Cl₂ (5 mL) was added to Cp₂ZrHCl (0.088 g, 0.343 mmol) at 0°C. The mixture was allowed to warm at room temperature and stirred for 8 days under nitrogen. At this time, the mixture was diluted with Et₂O (50 mL) and washed with water (30 mL). The phases were separated and the aqueous phase was extracted with Et₂O (2 x 30 mL). The combined organic extracts were dried over MgSO₄, filtered and the solvent removed. Purification by chromatography on silica gel eluting with hexanes: ethyl acetate (20:1 to 10:1) afforded 124 as an off-white solid (0.99 g, 53%): ¹H NMR (CDCl₃, 400 MHz) δ 7.21-7.17 (m, 2 H), 6.69-6.64 (m, 3 H), 6.60 (dt, *J=* 18.0 and 4.4 Hz, 1 H), 5.55 (dt, *J*= 18.0 and 1.6 Hz, 1 H), 3.99 (dd, *J*= 4.4 and 1.6 Hz, 2 H), 2.94 (s, 3 H), 1.25 (s, 12 H); CIMS *m*/*z* 274 (M + H)⁺.

(*S*)-3-{4-[(*E*)-3-(Methyl-phenyl-amino)-propenyl]-phenyl}-2-pyrrol-1-yl-propionic acid methyl ester (123). This compound was prepared from triflate 119 (2.0 g, 5.296 mmol), and boronate ester 124 (2.6 g, 7.944 mmol) following the procedure described for 137c. Purification by flash chromatography eluting with 33-45% ethyl acetate in hexanes containing 1% Et₃N gave 123 (*E*-isomer, 1.801 g, 79%) and *E:Z* mixture (0.217 g, 9.9%) as oils. ***E-isomer:*** ¹H NMR (CDCl₃, 400 MHz) δ 7.50-7.15 (m, 7 H), 6.95 (d, *J=* 8.4 Hz, 2 H), 6.69 (t, *J=* 2.0 Hz, 2 H), 6.45 (d, *J*= 16.0 Hz, 1 H), 6.27 (dt, *J*= 15.6 and 6.8 Hz, 1 H), 6.13 (t, *J*= 2.0 Hz, 2 H), 4.71 (dd, *J*= 8.8 and 6.4 Hz, 1 H), 3.68 (s, 3 H), 3.40-3.19 (m, 2 H), 3.16 (d, *J*= 6.8 Hz, 2 H), 3.08 (d, *J*= 11.6 Hz, 2 H), 2.51-2.46 (m, 1 H), 2.07 (t, *J*= 11.2 Hz, 1 H), 2.06 (t, *J*= 11.2 Hz, 1 H), 1.84-1.77 (m, 4 H); CIMS m/z 429 (M + H)⁺.

4-Phenyl-1-[2-(4,4,5,5-tetramethyl-[1,3,2]dioxaborolan-2-yl)-vinyl]-piperidine (130).

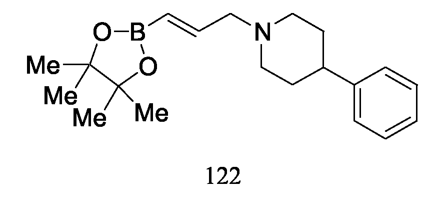

A mixture of *N*-prop-2-ynyl-4-phenylpiperidine (1.5 g, 7.526 mmol) and pinacolborane (1.64 mL, 11.289 mmol) in CH₂Cl₂ (8 mL) was added to Cp₂ZrHCl (0.194 g, 0.753 mmol) at 0°C. The mixture was allowed to warm at room temperature and stirred for 48 hours under nitrogen. At this time, the mixture was diluted with Et₂O (50 mL) and water (30 mL) was added carefully. The phases were separated and the organic extracts were dried over MgSO₄, filtered and the solvent removed to give 18 as a solid (2.46 g, 100%): ¹H NMR (CDCl₃, 400 MHz) δ 7.29-7.18 (m, 5 H), 6.68 (dt, *J*= 17.6 and 6.0 Hz, 1 H), 5.62 (dt, *J*= 18.0 and 1.6 Hz, 1 H), 3.11 (dd, *J=* 6.4 and 1.6 Hz, 2 H), 3.04 (d, *J=* 12.0 Hz, 2 H), 2.51-2.44 (m, 1 H), 2.10-2.03 (m, 2 H), 1.84-1.79 (m, 4 H), 1.26 (s, 12 H); CIMS *m*/*z* 328 (M+H)⁺.

### Example 51

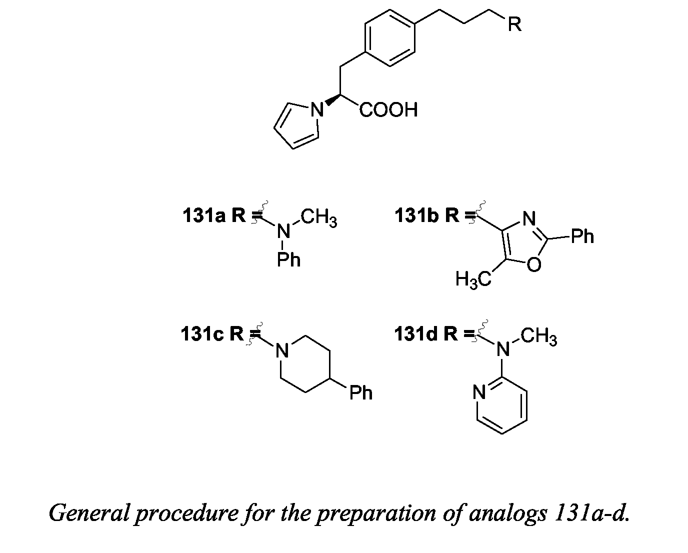

(*S*)-3-{4-[3-(Methyl-phenyl-amino)-propyl]-phenyl}-2-pyrrol-1-yl-propionic acid (131a). Ester 130a (0.223 g, 0.592 mmol) was dissolved in THF:H₂O (10:4 mL) and LiOH·H₂O (0.037 g, 0.888 mmol) was added and the mixture was stirred at room temperature for 3.5 hours. The solvent was removed and the residue was diluted with water, acidified with 10% HCl. The mixture was extracted with CHCl₃ (3 x 50 mL). The combined organic extracts were dried over MgSO₄, filtered and the solvent removed. Purification by chromatography on silica gel eluting with 0-5% MeOH in CHCl₃ containing formic acid (0-0.1%) gave pure 140a as pale brown solid (0.190 g, 88%): ¹H NMR (CDCl₃, 400 MHz) δ 7.33 (m, 2 H), 7.12-7.06 (m, 3 H), 6.95 (d, *J*= 8.3 Hz, 2 H), 6.91 (d, *J*= 8.3 Hz, 2 H), 6.69 (t, *J*= 2.0 Hz, 2 H), 6.10 (t, *J*= 2.0 Hz, 2 H), 4.73 (dd, *J*= 9.1 and 6.3 Hz, 1 H), 3.42-3.18 (m, 4 H), 2.52 (t, *J*= 7.3 Hz, 2 H), 1.88-1.81 (m, 2 H). Anal. calcd for C₂₃H₂₆N₂O₂·0.1 H₂O: C, 75.84; H, 7.25; N, 7.69. Found: C, 75.73; H, 7.40; N, 7.50.

(*S*)-3-{4-[3-(5-Methyl-2-phenyl-oxazol-4-yl)-propyl]-phenyl}-2-pyrrol-1-yl-propionic acid (131b). Prepared from ester 130b (0.243 g, 0.567 mmol) following the general procedure. Purification by flash chromatography eluting with 33-50% ethyl acetate in hexanes containing formic acid (0-0.2%) gave pure 140b as a light yellow solid (0.206 g, 88%): mp 167°C-168°C; ¹H NMR (CDCl₃, 400 MHz) δ 7.94 (m, 2 H), 7.40 (m, 3 H), 7.04 (d, *J=* 7.8 Hz, 2 H), 6.93 (d, *J*= 7.8 Hz, 2 H), 6.71 (bs, 2 H), 6.14 (bs, 2 H), 4.72 (m, 1 H), 3.39-3.16 (m, 2 H), 2.58 (t, *J=* 7.3 Hz, 2 H), 2.47 (t, *J=* 7.3 Hz, 2 H), 2.26 (s, 3 H), 1.92 (qn, *J=* 7.3 Hz, 2 H); CIMS *m*/*z* 415 (M + H)⁺.

(*S*)-3-{4-[3-(4-Phenyl-piperidin-1-yl)-propyl]-phenyl}-2-pyrrol-1-yl-propionic acid (131c). Prepared from ester 131c (0.248 g, 0.576 mmol) by the general procedure described above. Purification by chromatography on silica gel eluting with 0-9% MeOH in CHCl₃ containing formic acid (0-0.1 %) gave pure 139c as a white foam (0.137 g, 57%): mp 95°C-100°C; ¹H NMR (CDCl₃, 400 MHz) δ 7.29-7.16 (m, 3 H), 7.14 (d, *J=* 8.3 Hz, 2 H), 6.98 (d, *J=* 8.3 Hz, 2 H), 6.95 (d, *J*= 8.0 Hz, 2 H), 6.67 (t, *J*= 2.0 Hz, 2 H), 6.04 (t, *J*= 2.0 Hz, 2 H), 4.64 (t, *J*= 7.5 Hz, 1 H), 3.50 (m, 1 H), 3.45 (m, 1 H), 3.41-3.09 (m, 2 H), 2.82-2.78 (m, 2 H), 2.57-2.33 (m, 4 H), 2.21 (m, 2 H), 2.03-1.99 (m, 2 H), 1.84 (m, 2 H); CIMS *m*/*z* 417 (M + H)⁺. Anal. calcd for C₂₇H₃₂N₂O₂·1.0 H₂O: C, 74.62; H, 7.89; N, 6.45. Found: C, 74.23; H, 7.63; N, 6.25.

(*S*)-3-{4-[3-(Methyl-pyridin-2-yl-amino)-propyl]-phenyl}-2-pyrrol-1-yl-propionic acid (131d). Prepared from ester 131d (0.378 g, 1.001 mmol) by the general procedure. Purification by flash chromatography eluting with 0-15% MeOH in CHCl₃ gave 142d as a white solid (0.280 g, 77%): mp 66°C-68°C; ¹H NMR (CD₃OD, 400 MHz) δ 7.90-7.85 (m, 1 H), 7.82 (d, *J*= 6.0 Hz, 1 H), 7.01 (t, *J =* 8.0 Hz, 3 H), 6.94 (d, *J =* 8.0 Hz, 2 H), 6.87 (t, *J* = 6.8 Hz, 1 H), 6.66 (t, *J* = 2.0 Hz, 2 H), 5.96 (t, *J*= 2.0 Hz, 2 H), 4.80 (dd, *J*= 10.0 and 5.6 Hz, 1 H), 3.55 (t, *J*= 7.6 Hz, 2 H), 3.37-3.15 (m, 2 H), 3.12 (s, 3 H), 2.62 (t, *J*= 7.6 Hz, 2 H), 1.94 (qn, *J*= 7.2 Hz, 2 H); CIMS *m*/*z* 364 (M + H)⁺. Anal. calcd for C₂₂H₂₅N₃O₂·0.3 CHCl₃: C, 67.08; N, 6.39; N, 10.52. Found: C, 67.30; H, 6.39; N, 10.21.

### (a) The esters 130a-c were prepared in the following manner.

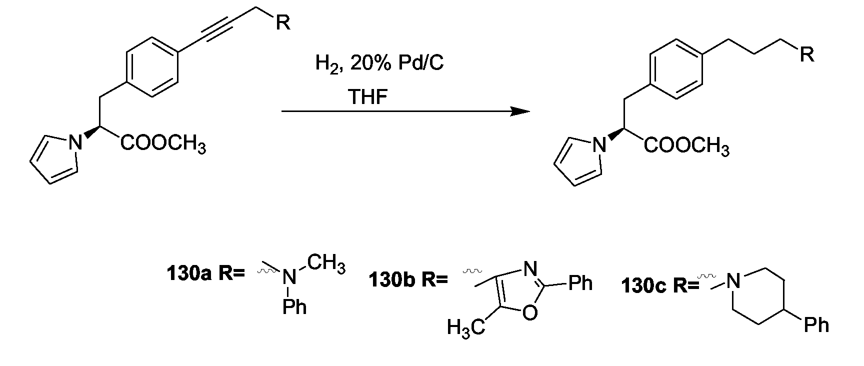

(*S*)-3-{4-[3-(Methyl-phenyl-amino)-propyl]-phenyl}-2-pyrrol-1-yl-propionic acid methyl ester (130a). Alkyne 120a (0.648 g, 1.739 mmol) was dissolved in MeOH (50 mL) and 20% Pd/C (0.050 g) was added. The mixture was hydrogenated at room temperature for 18 hr. The mixture was filtered through celite and the solvent removed. Purification by flash chromatography eluting with hexanes:ethyl acetate (6:1) gave 130a as an oil (0.439 g, 67%): ¹H NMR (CDCl₃, 400 MHz) δ 7.20 (t, *J=* 8.0 Hz, 2 H), 7.05 (d, *J=* 7.6 Hz, 2 H), 6.92 (d, *J=* 8.0 Hz, 2 H), 6.71 (t, *J=* 2.0 Hz, 2 H), 6.65 (t, *J=* 7.8 Hz, 3 H), 6.14 (t, *J=* 2.0 Hz, 2 H), 4.73 (dd, *J*= 8.8 and 6.4 Hz, 1 H), 3.69 (s, 3 H), 3.41-3.20 (m, 2 H), 3.30 (t, J = 7.4 Hz, 2 H), 2.90 (s, 3 H), 2.59 (t, *J*= 7.6 Hz, 2 H), 1.86 (qn, *J*= 7.6 Hz, 2 H); CIMS *m*/*z* 377 (M + H)⁺.

(*S*)-3-{4-[3-(5-Methyl-2-phenyl-oxazol-4-yl)-propyl]-phenyl}-2-pyrrol-1-yl-propionic acid methyl ester (130b). This compound was prepared from alkyne 120b (0.249 g, 0.586 mmol) following the general procedure described for 130a with the exception that the reaction was carried out in THF. The crude product was used for subsequent transformations. Ester 130b was obtained as an oil (0.243, 96.8%): ¹H NMR (CDCl₃, 400 MHz) δ 7.91 (dd, *J=* 8.0 and 1.6 Hz, 2 H), 7.37-7.32 (m, 3 H), 6.99 (d, *J=* 8.4 Hz, 2 H), 6.85 (d, *J=* 8.0 Hz, 2 H), 6.64 (t, *J=* 2.0 Hz, 2 H), 6.07 (t, *J*= 2.0 Hz, 2 H), 4.65 (dd, *J*= 8.8 and 6.8 Hz, 1 H), 3.62 (s, 3 H), 3.33-3.11 (m, 2 H), 2.54 (t, *J*= 7.6 Hz, 2 H), 2.41 (t, *J*= 7.6 Hz, 2 H), 2.19 (s, 3 H), 1.89 (qn, *J*= 7.6 Hz, 2 H); CIMS *m*/*z* 429 (M + H)⁺.

(*S*)-3-{4-[3-(4-Phenyl-piperidin-1-yl)-propyl]-phenyl}-2-pyrrol-1-yl-propionic acid methyl ester (130c). This compound was prepared from alkyne 120c (0.408 g, 0.956 mmol) following the procedure described for 130a. The reaction was carried out in THF. Purification was achieved by chromatography eluting with 33-45% ethyl acetate in hexanes containing 1.0% Et₃N to give 130c as an oil (0.262 g, 64%): ¹H NMR (CDCl₃, 400 MHz) δ 7.24-7.10 (m, 5 H), 7.00 (d, *J*= 8.0 Hz, 2 H), 6.85 (d, *J*= 8.0 Hz, 2 H), 6.65 (t, *J*= 2.0 Hz, 2 H), 6.08 (t, *J*= 2.0 Hz, 2 H), 4.66 (dd, *J*= 8.8 and 6.4 Hz, 1 H), 3.62 (s, 3 H), 3.34-3.12 (m, 2 H), 2,98 (d, *J*= 11.6 Hz, 2 H), 2.52 (t, *J*= 7.6 Hz, 2 H), 2.42 (m, 1 H), 2.31 (t, *J*= 7.6 Hz, 2 H), 1.99-1.93 (m, 2 H), 1.80-1.72 (m, 6 H); CIMS *m*/*z* 431 (M + H)⁺.

(*S*)-3-{4-[3-(Methyl-pyridin-2-yl-amino)-propyl]-phenyl}-2-pyrrol-1-yl-propionic acid methyl ester (130d).

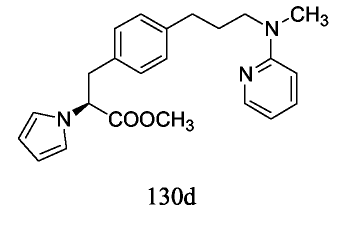

Alkene 128b (0.430 g, 1.145 mmol) was dissolved in THF (16 mL). 5% Pd/C (0.050 g) was added and the mixture was hydrogenated at room temperature for 24 hours. Additional catalyst (0.050 g) was added and the reaction was continued for another 24 hours. The catalyst was filtered off and the solvent removed to give ester 130d as a thick oil (0.378 g, 87%): ¹H NMR (CDCl₃, 400 MHz) δ 8.08-8.06 (m, 1 H), 7.36-7.31 (m, 1 H), 6.99 (d, *J*= 8.0 Hz, 2 H), 6.85 (d, *J*= 8.0 Hz, 2 H), 6.64 (t, *J*= 2.0 Hz, 2 H), 6.43 (dd, *J*= 6.4 and 5.4 Hz, 1 H), 6.33 (d, *J*= 8.8 Hz, 6.07 (t, *J*= 2.0 Hz, 2 H), 6.65 (dd, *J*= 8.8 and 6.8 Hz, 1 H), 3.62 (s, 3 H), 3.45 (t, *J*= 7.4 Hz, 2 H), 3.33-3.12 (m, 2 H), 2.94 (s, 3 H), 2.53 (t, *J*= 7.6 Hz, 2 H), 1.81 (qn, *J*= 7.6 Hz, 2 H); CIMS *m*/*z* 378 (M + H)⁺.

### Example 52

### S)-2-Methyl-3-{4-[2-(5-methyl-2-phenyl-oxazol-4-yl)-ethoxy]-phenyl}-2-pyrrol-1-yl-propionic acid methyl ester (134).

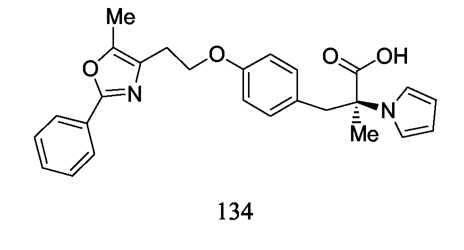

To a stirred solution of (S)-2-Methyl-3-{4-[2-(5-methyl-2-phenyl-oxazol-4-yl)-ethoxy]-phenyl}-2-pyrrol-1-yl-propionic acid methyl ester (3.33 g, 7.5mmol) in 2-methoxyethanol (20 mL) and water (5 mL) was added Lithium Hydroxide (180 mg, 7.5 mmol) The reaction was stirred at room temperature overnight. The reaction mixture was poured into water and acidified with dilute Hydrochloric acid. The reaction was extracted with ethyl acetate and the organic layer dried (MgSO4) and purified by chromatography. Recrystallisation from ethyl acetate and hexane gave the product as white crystals.Yield = 1.3g,40% Mp 149.5°C-150.5°C. 500 MHz ¹H NMR (CDCl₃) δ 7.94 (m, 2H), 7.41 (m, 3H), 6.76 (m, 2H), 6.70 (d, *J*=9 Hz) 6.65 (d, *J*=9 Hz) 6.18 (m, 2H) 4.15 (t, 2H, *J=* 6.8 Hz), 3.36 (d, 1H, *J*=13.9) 3.29 (d, 1H *J*=13.9) 2.96 (t, 2H *J*=6.8Hz), 2.36 (s, 3H), 1.62 (s, 3H); MS *m*/*z* 431 (M+1), 429 (M-1) Anal. Calc'd for C26H26N2O4 C, 72.54: H 6.09; N 6.51 found: C, 72.17; H,6.15; N 6.50

(S)-2-Methyl-3-{4-[2-(5-methyl-2-phenyl-oxazol-4-yl)-ethoxy]-phenyl}-2 pyrrol-1-yl-propionic acid methyl ester was prepared in the following manner.

### (a) (S)-2-Methyl-3-{4-[2-(5-methyl-2-phenyl-oxazol-4-yl)-ethoxy]-phenyl]-2-pyrrol-1-yl-propionic acid methyl ester (133).

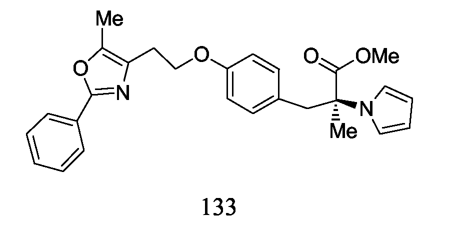

To a stirred solution of of (S)-3-(4-Hydroxy-phenyl)-2-methyl-2-pyrrol-1-yl-propionic acid methyl ester described in example 1 (2.44g, 9.4mmol) in anhydrous THF (25mL) was added triphenyl phosphine (2.47g, 9.4mmol) and 2-(5-methyl-2-phenyloxazol-4-yl) ethanol (1.911g, 9.4mmol). A solution of DIAD (1.85mL, 9.4mmol) in anhydrous THF (10mL) was added dropwise and the reaction mixture stirred at room temperature for 48hr. The solvent was then removed under reduced pressure to give a yellow oil. The reaction mixture was purified by chromatography eluting with 1:10 to 1:1 EtOAc: Hexane. To give the desired ester as an opaque oil (3.33g, 80%). MS *m*/*z* 445 (M+1).

### (b) (S)-3-(4-Hydroxy-phenyl)-2-methyl-2-pyrrol-1-yl-propionic acid methyl ester (132).

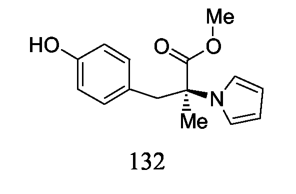

To a stirred, cooled (0°C) solution of methanol (20mL) was added dropwise thionyl chloride (2.06mL, 28.2mmol) and the reaction stirred for 5 minutes. α-methyl-L-tyrosine (5g, 25.6mmol) was added and the reaction mixture warmed to room temperature. The reaction was then heated at reflux for 6 hours and cooled to room temperature. The solvent was removed in vacuo. The crude methyl ester was dissolved in water (50mL) acetic acid (50mL) and sodium acetate (3.36g, 40.9mmol), 2,5 dimethoxy tetrahyrdrofuran (5.29mL, 40.9mmol) added. The reaction mixture was heated at 90°C for 15 minutes, poured into water (200mL) and extracted with ethyl acetate (3x100mL). The organic layer was dried over MgSO₄ and the solvent was removed under reduced pressure. The residue was purified by chromatography (using ethyl acetate hexanes 1:10 to 1:1) to give the desired pyrrole (5.38g, 81% g) as a clear oil. 500 MHz ¹NMR (CDCl₃) δ 6.71 (m, 2H), 6.63 (d, 2H *J*= 8.4Hz), 6.57 (d, 2H *J*= 8.4Hz), 6.17 (m, 2H) 3.34 (s, 3H), 3.36 (d, 1H, *J*= 13.9,) 3.25 (d, 1H, *J*= 13.9) 1.6 (s, 3H); MS *m*/*z* 258 (M-1).

### Example 53

### 3-{5-[2-(5-Methyl-2-phenyl-oxazol-4-yl)-ethoxy]-indol-1-yl}-propionic acid (140).

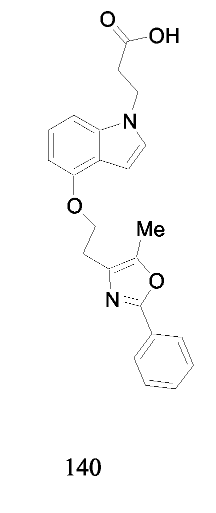

A mixture of 4-{5-[2-(5-Methyl-2-phenyl-oxazol-4-yl)-ethoxy]-indol-1-yl}-propionic acid methyl ester (136, 0.81 g, 2.00 mmol) and LiOH (240 mg, 10.0 mmol) in DME (16 mL) and water (4 mL) was stirred at room temperature overnight. The reaction was diluted with water, acidified with 2N HCl, extracted with ethyl acetate, dried over MgSOₛ, and concentrated under reduced pressure. The solid was recrystallized from EtOAc/hexanes to yield the desired propionic acid (560 mg, 72%). 400 MHz ¹H NMR (DMSO-*d*₆) δ 7.86 (m, 2H), 7.43 (m, 3H), 7.31 (d, 1H, *J=* 8.8 Hz), 7.23 (d, 1H, *J=* 2.9 Hz), 7.01 (d, 1H, *J=* 2.4 Hz), 6.71 (dd, 1H, *J=* 8.8, 2.4 Hz), 6.24 (d, 1H, *J=* 2.9 Hz), 4.28 (t, 2H, *J=* 6.8 Hz), 4.14 (t, 2H, *J*= 6.6 Hz), 2.88 (t, 2H, *J*= 6.6 Hz), 2.65 (t, 2H, *J*= 6.8 Hz), 2.32 (s, 3H). MS m/z 391 (M+1).

4-{5-[2-(5-Methyl-2-phenyl-oxazol-4-yl)-ethoxy]-indol-1-yl}-propionic acid methyl ester was prepared in the following manner.

### (a) 4-{5-[2-(5-Methyl-2-phenyl-oxazol-4-yl)-ethoxy]-indol-1-yl}-propionic acid methyl ester (139).

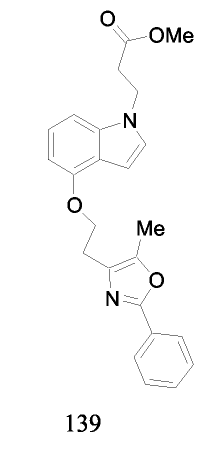

A mixture of 3-{4-[2-(5-Methyl-2-phenyl-oxazol-4-yl)-ethoxy]-indol-1-yl}-propionic acid methyl ester (0.88g, 2.18 mmol) and LiOH (260 mg, 10.9 mmol) in 2-methoxyethanol (16 mL) and water (4 mL) was stirred at room temperature overnight. The reaction was diluted with water, acidified with 2N HCl, extracted with ethyl acetate, dried over MgSOₛ, and concentrated under reduced pressure. The solid was recrystallized from EtOAc/hexanes to yield the desired propionic acid (400 mg, 47%). 400 MHz ¹H NMR (DMSO-*d*₆) δ 7.86 (m, 2H), 7.43 (m, 3H), 7.16 (d, 1H, *J=* 3.2 Hz), 6.99 (m, 2H), 6.50 (d, 1H, *J=* 7.3 Hz), 6.30 (d, 1H, *J=* 2.7 Hz), 4.29 (t, 2H, *J=* 6.8 Hz), 4.24 (t, 2H, *J=* 6.4 Hz), 2.94 (t, 2H, *J=* 6.4 Hx), 2.65 (t, 2H, *J=* 6.8 Hz), 2.35 (s, 3H). MS *m*/*z* 391 (M+1).

### (b) 3-(4-Hydroxy-indol-1-yl)-propionic acid methyl ester (138)

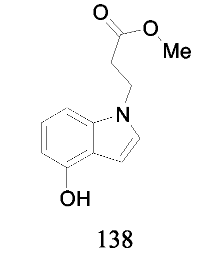

A mixture of 3-(4-Hydroxy-indol-1-yl)-propionic acid methyl ester (0.56g, 2.55 mmol), cesium carbonate (1.66g, 5.11 mmol) and toluene-4-sulfonic acid 2-(5-methyl-2-phenyl-oxazol-4-yl)-ethyl ester (1.63g, 4.57 mmol) in DMF (25 mL) was heated to 50°C overnight. The reaction was diluted with water, acidified with 2N HCl, extracted with ethyl acetate, dried over MgSOₛ, and concentrated under reduced pressure.. The crude oil was chromatographed eluting with 20%-25% EtOAc (with 20% HOAc) in hexanes to yield the desired propionic acid (0.88g, 85%). 400 MHz ¹H NMR (DMSO-*d*₆) δ 7.86 (m, 2H), 7.44 (m, 3H), 7.14 (d, 1H, *J*= 3.2 Hz), 6.99 (m, 2H), 6.50 (dd, 1H, *J*= 7.1, 1.0 Hz), 6.30 (d, *J*= 3.2 Hz), 4.33 (t, 2H, *J=* 6.8 Hz), 4.24 (t, 2H, *J=* 6.4 Hz), 3.49 (s, 3H), 2.94 (t, 2H, *J=* 6.4 Hz), 2.74 (t, 2H, *J=* 6.8 Hz), 2.35 (s, 3H). MS *m*/*z* 405 (M+1).

### (c) 3-(4-Benzyloxy-indol-1-yl)-propionic acid methyl ester (137).

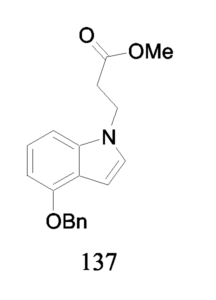

A mixture of 3-(4-Benzyloxy-indol-1-yl)-propionic acid methyl ester (1.52g, 4.91 mmol) and 20% Pd/C (160mg) in methanol (25 mL) and THF (25 mL) was stirred under an H₂ atmosphere for 3 days. The reaction was filtered and concentrated to yield the crude deprotected product. MS m/z 220 (M+1).

### (d) 3-(4-Benzyloxy-indol-1-yl)-propionic acid (136).

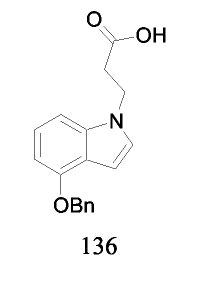

A solution of 4-Benzyloxyindole (1.61g, 7.21 mmol) and potassium hydroxide (2.02g, 36.1 mmol) in DMSO (12 mL) was stirred at room temperature for 1.5 hours. Ethyl 3-bromopropionate (1.11 mL, 8.65 mmol) was added and the mixture stirred overnight. The reaction was diluted with water, acidified with 2N HC1, extracted with ethyl acetate, dried over MgSOₛ, and concentrated under reduced pressure. The crude oil was chromatographed eluting with 25%-30% EtOAc (with 20% HOAc) in hexanes to yield the desired propionic acid (1.65g, 77%). 400 MHz ¹H NMR (DMSO-*d*₆) δ 7.44 (m, 2H), 7.35 (m, 2H), 7.27 (m, 1H), 7.18 (d, 1H, *J*= 3.2 Hz), 7.04 (d, 1H, *J*= 8.3 Hz), 6.97 (m, 1H), 6.55 (d, 1H, *J*= 7.3 Hz), 6.39 (m, 1H), 5.16 (s, 2H), 4.30 (t, 2H, *J*= 6.8 Hz), 2.67 (t, 2H, *J*= 6.8 Hz). MS *m*/*z* 296 (M+1).

### (e) Toluene-4-sulfonic acid 2-(5-methyl-2-phenyl-oxazol-4-yl)-ethyl ester (135).

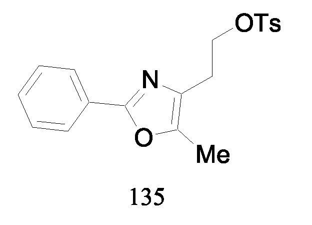

A solution of 2-(5-Methyl-2-phenyl-oxazol-4-yl)-ethanol (2g, 9.84 mmol), *p*-toluenesulfonyl chloride (2.25g, 11.8 mmol) and DMAP (10 mg) in dichloromethane (40 mL) was cooled to 0°C. Triethylamine (2.75 mL, 19.7 mmol) was added dropwise. The reaction was stirred at 0°C for 3 hours, then warmed to room temperature overnight. The solution was cooled to 0°C, diluted with water (25 mL) and neutralized with 1N HCl. The mixture was extracted with dichloromethane, washed with saturated NaHCO₃ and brine, dried with MgSO₄, and concentrated to yield a brown solid in near quantitative yield. 400 MHz ¹H NMR (DMSO-*d*₆) δ 6.83 (m, 2H), 6.66 (m, 4H), 6.11 (m, 2H), 4.65 (dd, 1H, *J=* 8.8, 6.6 Hz), 3.66 (s, 3H), 3.30 (dd, 1H, *J=* 13.9, 6.6 Hz), 3.15 (dd, 1H, *J=* 13.9, 8.8 Hz). MS *m*/*z* 246 (M+1).

### Example 53a

### 4-{5-[2-(5-Methyl-2-phenyl-oxazol-4-yl)-ethoxy]-indol-1-yl}-propionic acid (140a).

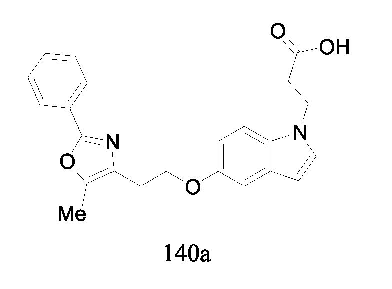

Prepared as in Example 53 from the corresponding 3-(5-Benzyloxy-indol-1-yl)-propionic acid. Thus, a mixture of 3-{5-[2-(5-Methyl-2-phenyl-oxazol-4-yl)-ethoxy]-indol-1-yl]-propionic acid methyl ester (0.81g, 2.00 mmol) and LiOH (240 mg, 10.0 mmol) in 2-methoxyethanol (16 mL) and water (4 mL) was stirred at room temperature overnight. The reaction was diluted with water, acidified with 2N HCl, extracted with ethyl acetate, dried over MgSOₛ, and concentrated under reduced pressure. The solid was recrystallized from EtOAc/hexanes to yield the desired propionic acid (560 mg, 72%). 400 MHz ¹H NMR (DMSO-*d*₆) δ 7.86 (m, 2H), 7.43 (m, 3H), 7.31 (d, 1H, *J=* 8.8 Hz), 7.23 (d, 1H, *J=* 2.9 Hz), 7.01 (d, 1H, *J=* 2.4 Hz), 6.71 (dd, 1H, *J=* 8.8, 2.4 Hz), 6.24 (d, 1H, *J=* 2.9 Hz), 4.28 (t, 2H, *J* = 6.8 Hz), 4.14 (t, 2H, *J=* 6.6 Hz), 2.88 (t, 2H, *J=* 6.6 Hz), 2.65 (t, 2H, *J=* 6.8 Hz), 2.32 (s, 3H). MS *m*/*z* 391 (M+1).

4-{5-[2-(5-Methyl-2-phenyl-oxazol-4-yl)-ethoxy]-indol-1-yl}-propionic acid (139a). A mixture of 3-(5-Hydroxy-indol-1-yl)-propionic acid methyl ester (1.14g, 5.20 mmol), cesium carbonate (3.39g, 10.4 mmol) and toluene-4-sulfonic acid 2-(5-methyl-2-phenyl-oxazol-4-yl)-ethyl ester (3.27g, 9.14 mmol) in DMF (50 mL) was heated to 50°C overnight. The reaction was diluted with water, acidified with 2N HCl, extracted with ethyl acetate, dried over MgSOₛ, and concentrated under reduced pressure.. The crude oil was chromatographed eluting with 25%-30% EtOAc (with 20% HOAc) in hexanes to yield the desired propionic acid (0.81g, 39%). 400 MHz ¹H NMR (DMSO-*d*₆) δ 7.86 (, m, 2H), 7.44 (m, 3H), 7.31 (d, 1H, 9.0 Hz), 7.21 (d, 1H, 2.9 Hz), 7.01 (d, 1H, 2.2 Hz), 6.71 (dd, 1H, 9.0, 2.2 Hz), 6.24 (m, 1H), 4.32 (t, 2H, *J=* 6.8 Hz), 4.14 (t, 2H, *J=* 6.8 Hz), 3.50 (s, 3H), 2.88 (t, 2H, *J=* 6.8 Hz), 2.75 (t, 2H, *J=* 6.8 Hz), 2.32 (s, 3H). MS *m*/*z* 405 (M+1).

3-(5-Hydroxy-indol-1-yl)-propionic acid methyl ester (138a). A mixture of 3-(5-Benzyloxy-indol-1-yl)-propionic acid methyl ester (2.38g, 7.69 mmol) and 20% Pd/C (145mg) in methanol (25 mL) and THF (25 mL) was stirred under an H₂ atmosphere for 18 hours. The reaction was filtered and concentrated to yield the deprotected product in near quantitative yield. 400 MHz ¹H NMR (DMSO-*d*₆) δ 7.19 (d, 1H, *J*= 8.8 Hz), 7.15 (d, 1H, *J*= 2.6 Hz), 6.78 (1H, d, *J*= 2.6 Hz), 6.58 (dd, 1H, *J*= 8.8, 2.4 Hz), 6.15 (d, 1H, *J*= 2.4 Hz), 4.28 (t, 2H, *J*= 6.8 Hz), 3.51 (s, 3H), 2.74 (t, 2H, *J*= 6.8 Hz). MS *m*/*z* 220 (M+1).

3-(5-Benzyloxy-indol-1-yl)-propionic acid methyl ester (137a). TMS-diazomethane (6.65 mL, 13.3 mmol, 2.0 M in hexanes) was added to a solution of 3-(5-Benzyloxy-indol-1-yl)-propionic acid (2.27g, 8.87 mmol) in toluene (72 mL) and methanol (18 mL). The reaction stirred at room temperature for 30 minutes, quenced with acetic acid and concentrated. The desired ester as a brown oil was obtained in near quantitative yield. 400 MHz ¹H NMR (DMSO-*d*₆) δ 7.41 (m, 2H), 7.33 (m, 3H), 7.25 (m, 2H), 7.06 (d, 1H, *J=* 2.4 Hz), 6.80 (dd, 1H, *J=* 9.0, 2.4 Hz), 6.25 (d, 1H, *J=* 2.4 Hz), 5.03 (s, 2H), 4.33 (t, 2H, *J=* 6.7 Hz), 3.50 (s, 3H), 2.75 (t, 2H, *J=* 6.7 Hz). MS *m*/*z* 310 (M+1).

3-(5-Benzyloxy-indol-1-yl)-propionic acid (136a). A solution of 5-Benzyloxyindole (4g, 17.9 mmol) and potassium hydroxide (5.03g, 89.6 mmol) in DMSO (30 mL) was stirred at room temperature for 1.5 hours. Ethyl 3-bromopropionate (2.75 mL, 21.5 mmol) was added and the mixture stirred for 3 hours. The reaction was diluted with water, acidified with 2N HCl, extracted with ethyl acetate, dried over MgSOₛ, and concentrated under reduced pressure. The crude oil was chromatographed eluting with 25%-30% EtOAc (with 20% HOAc) in hexanes to yield the desired propionic acid (2.56g, 49%). 400 MHz ¹H NMR (DMSO-*d*₆) δ 7.40 (m, 2H), 7.33 (m, 3H), 7.25 (m, 2H), 7.06 (d, 1H, *J*= 2.4 Hz), 6.80 (dd, 1H, *J*= 8.8, 2.4 Hz), 6.24 (dd, 1H, *J=* 2.9, 0.7 Hz), 5.03 (s, 2H), 4.29 (t, 2H, *J*= 6.7 Hz), 2.66 (t, 2H, *J*= 6.7 Hz). MS *m*/*z* 324 (M+1).

### Example 54

### (S)-2-(2-{4-[2-(5-Methyl-2-phenyl-oxazol-4-yl)-ethoxy]-phenyl}-1-pyrrol-1-yl-ethyl)-5-propyl-1,3,4-oxadiazole (147).

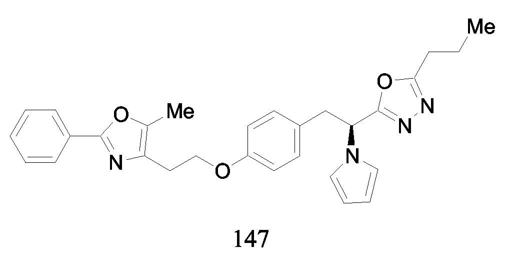

(S)-3-(4-[2-(5-Methyl-2-phenyl-oxazol-4-yl)-ethoxy]-phenyl}-2-pyrrol-1-yl-propionic acid hydrazide (147, 2.95g, 6.85mmol) was dissolved in dioxane (20ml) and trimethyl orthobutyrate (3.29mL, 20.5mmol), and methane sulfonic acid (100µL, 1.37mmol) were added. The reaction mixture was heated at 105°C for 15 minutes then poured into water and extracted with ethyl acetate (2x100ml). The organic layer was washed with sodium bicarbonate, brine and dried over MgSO₄. The solvent was removed under vacuum and the residual oil purified by chromatography using 1:10 to 1:1 ethyl acetate: hexane. (S)-2-(2-{4-[2-(5-Methyl-2-phenyl-oxazol-4-yl)-ethoxy]-phenyl}-1-pyrrol-1-yl-ethyl)-5-propyl-1,3,4-oxadiazole was isolated as a clear oil. Yield = 0.80g, 24% ¹H NMR (400MHz) δ (CDCl₃) 7.97 (2H, m), 7.41 (3H, m), 6.88 (2H dd, J=2.2, 5.85) 6.74 (2H, dd, J=1.95, 6.59 Hz), 6.69 (2H, t, J=2.2Hz), 6.13 (2H, t, J=2.2Hz), 5.33 (1H, dd, J = 6.83, 8.78Hz), 4.18 (2H, t, J=6.83), 3.52 (1H, dd, J=6.59, 14.16Hz, ), 3.45 (1H, dd, J = 8.78, 14.16Hz). 2.95 (2H, t, J=6.59Hz) 2.77 (2H, m) 2.35 (3H, s), 1.76 (2H, sex, J=7.32), 0.97 (3H, t, J=7.32), MS *m*/*z* 483 (M+1).

(S)-3-(4-[2-(5-Methyl-2-phenyl-oxazol-4-yl)-ethoxy]-phenyl}-2-pyrrol-1-yl-propionic acid hydrazide (146) was prepared in the following manner.

### (a) (S)-3-(4-[2-(5-Methyl-2-phenyl-oxazol-4-yl)-ethoxy]-phenyl}-2-pyrrol-1-yl-propionic acid hydrazide (146).

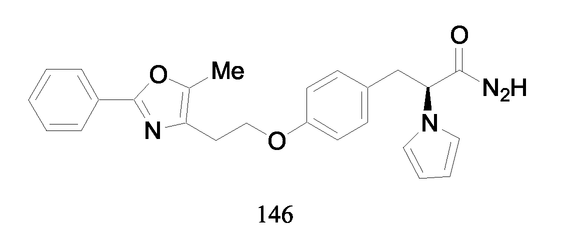

(S)-3-{4-[2-(5-Methyl-2-phenyl-oxazol-4-yl)-ethoxy]-phenyl}-2-pyrrol-1-yl-propionic acid prepared as described in example 1 (5g, 12 mmol) was dissolved in THF (60mL) and 4-methyl morpholine (1.70mL, 15.5 mmol) was added followed by isobutyl chloroformate (2.22mL, 17.2mmol). The resulting suspension was stirred for 30 minutes at 10°C and then filtered into a cooled (0°C) solution of hydrazine (1.88mL, 60mmol) in THF (40mL). After stirring for 45 minutes the mixture was poured into ethyl acetate and, washed with water, followed by a saturated solution of ammonium chloride and then brine. The organic layer was separated and dried over MgSO₄. Removal of the solvent under reduced pressure provided the desired monoacyl hydrazide as a clear oil used without further purification. ¹H NMR (400MHz) δ (CDCl₃) 7.96 (2H, m), 7.40 (3H, m), 6.88 (2H d, J=8.78) 6.73 (2H, dd, J=1.95, 6.59 Hz), 6.62 (2H, t, J=2.2Hz), 6.16 (2H, t, J=2.2Hz), 5.59 (1H, dd, J = 4.63, 10.49Hz), 4.16 (2H, t, J=6.59), 3.57 (1H, dd, J=4.63, 6.59, 14.4Hz, ), 3.15 (1H, dd, J = 14.40, 10.49Hz). 2.93 ( 2H, t, J=6.59Hz) 2.33 (3H, s) MS *m*/*z* 431 (M+1).

### Example 55

### (S)-2-Ethyl-5-(2-{4-[2-(5-methyl-2-phenyl-oxazol-4-yl)-ethoxy]-phenyl}-1-pyrrol-1-yl-ethyl)-1,3,4-oxadiazole (148)

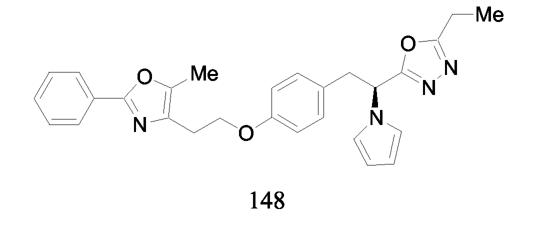

(S)-3-(4-[2-(5-Methyl-2-phenyl-oxazol-4-yl)-ethoxy]-phenyl}-2-pyrrol-1-yl-propionic acid hydrazide (146, 2.89 g, 6.72 mmol) was dissolved in dioxane (20 ml) and triethyl propionate (4.06 mL, 20.2 mmol), and methane sulfonic acid (98 µL, 1.34 mmol) were added. The reaction mixture was heated at 105°C for 15 minutes then poured into water and extracted with ethyl acetate (2x100 mL). The organic layer was washed with sodium bicarbonate, brine and dried over MgSO₄. The solvent was removed under vacuum and the residual oil purified by chromatography using 1:10 to 1:1 ethyl acetate: hexane. 2-Ethyl-5-(2-{4-[2-(5-methyl-2-phenyl-oxazol-4-yl)-ethoxy]-phenyl}-1-pyrrol-1-yl-ethyl)-1,3,4-oxadiazole was isolated as a clear oil. Yield = 0.82g, 26% some of the oil was dissolved in ethyl ether and evaporated slowly to give clear crystals Yield = 58mg, m.p. 96.5-98°C, C28H28N4O3 required C71.78, H6.02, N11.96 found C71.68, H6.15, N11.89. 73431x34 mw= 468.54

### Example 56

### (S)-2-{4-[2-(5-Methyl-2-phenyl-oxazol-4-yl)-ethoxy]-phenyl]-1-pyrrol-1-yl-ethyl)-1,3,4-oxadiazole (149).

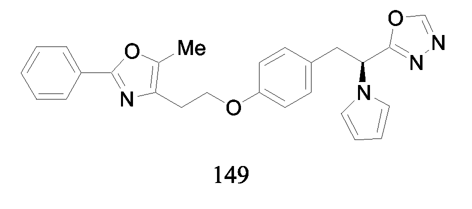

(S)-3-(4-[2-(5-Methyl-2-phenyl-oxazol-4-yl)-ethoxy]-phenyl}-2-pyrrol-1-yl-propionic acid hydrazide (146, 1.22g, 2.85mmol) was dissolved in dioxane (20ml) and trimethyl orthoformate (1.42mL, 8.56 mmol) and methane sulfonic acid (42, 0.57 mmol) added. The reaction mixture was heated at 105°C for 15 minutes then poured into water and extracted with ethyl acetate (2x100ml). The organic layer was washed with sodium bicarbonate, brine and dried over MgSO₄. The solvent was removed under vacuum and the residual oil purified by chromatography using 1:10 to 1:1 ethyl acetate: hexane. (S)-2-{4-[2-(5-Methyl-2-phenyl-oxazol-4-yl)-ethoxy]-phenyl}-1-pyrrol-1-yl-ethyl)-1,3,4-oxadiazole was isolated as a clear tacky oil. Yield = 401mg, 32% ¹H NMR (400MHz) δ (CDCl₃) 8.34 (1H, s), 7.96 (2H, m), 7.41 (3H, m), 6.88 (2H d, J=8.79) 6.73 (2H, m), 6.62 (2H, t, J=2.2Hz), 6.16 (2H, t, J=2.2Hz), 5.42 (1H, dd, J = 6.83, 8.78 Hz), 4.18 (2H, t, J=6.59), 3.57 (1H, dd, J=6.6, 13.9Hz, ), 3.15 (1H, dd, J = 8.54, 13.9Hz). 2.95 (2H, t, J=6.59Hz) 2.35 (3H, s) MS *m*/*z* 431 (M+1)

### Example 57

### (S)-3-Methyl-5-(2-{4-[2-(5-methyl-2-phenyl-oxazol-4-yl)-ethoxy]-phenyl}-1-(S)-pyrrol-1-yl-ethyl)-4H-1,2,4-triazole (151).

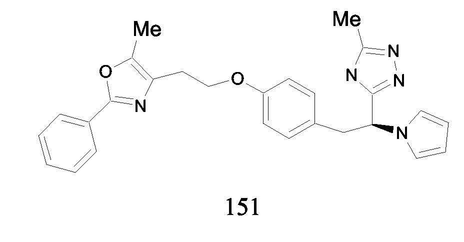

(S)-3-{4-[2-(5-Methyl-2-phenyl-oxazol-4-yl)-ethoxy]-phenyl}-2-pyrrol-1-yl-propionic acid N'-(1-imino-ethyl)-hydrazide (150, 0.860g, 1. 82mmol) was suspended in para xylene (15ml) and heated to 120°C in a sealed tube for 24h. The product was isolated by chromatography 1:1 ethyl acetate:hexane to give the desired product as a white foam. Yield = 684mg, 76%.¹H NMR (400MHz) δ (CDCl₃) 7.96 (2H, m), 7.40 (3H, m), 6.87 (2H d, J=8.54) 6.77 (2H, t, J=1.95), 6.11 (2H, d, J=8.54Hz), 6.11 (2H, t, J=1.95Hz), 5.25 (1H, dd, J = 6.34, 9.27 Hz), 4.16 (2H, t, J=6.83), 3.51 (1H, dd, J=6.1, 14.16Hz, ), 3.38 (1H, dd, J = 9.27, 14.16Hz) 2.93 (2H, t, J=6.59Hz) 2.42 (3H, s) 2.34 (3H, s) MS *m*/*z* 454 (M+1)

(S)-3-{4-[2-(5-Methyl-2-phenyl-oxazol-4-yl)-ethoxy]-phenyl}-2-pyrrol-1-yl-propionic acid N'-(1-imino-ethyl)-hydrazide (150) was prepared in the following manner.

### (a) (S)-3-{4-[2-(5-Methyl-2-phenyl-oxazol-4-yl)-ethoxy]-phenyl}-2-pyrrol-1-yl-propionic acid N'-(1-imino-ethyl)-hydrazide (150)

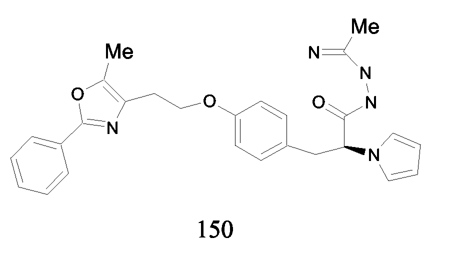

(S)-3-(4-[2-(5-Methyl-2-phenyl-oxazol-4-yl)-ethoxy]-phenyl}-2-pyrrol-1-yl-propionic acid hydrazide (146, 1.22 g, 2.83 mmol) was dissolved in THF (20ml) and cooled to 0°C. Ethyl acetimidate hydrochloride (420 mg, 3.40 mmol) and triethyl amine (513 µL, 3.68 mmol) were added and the reaction mixture stirred at 0°C for 1.5 hours follwed by stirring at room temperature overnight. The reaction mixture was purified by chromatography 1:20 to 1:4 methanol to dichloromethane The desired product was obtained as a pink solid. Yield = 860mg, 64% ¹H NMR (400MHz) δ (D6DMSO) 7.85 (2H, m), 7.42 (3H, m), 6.95 (2H d, J=8.54), 6.90 (2H, t, J=8.30), 6.73 (4H, m), 5.85 (1H, m), 4.08 (2H, t, J=6.83), 3.11-2.93 (4H, m) 2.83 (2H, m) 2.43 (3H, s) MS *m*/*z* 472 (M+1)

### Example 58

### 2-(3-Bromo-pyrrol-1-yl)-2-methyl-3-{4-[2-(5-methyl-2-phenyl-oxazol-4-yl)-ethoxy]-phenyl}-propionic acid (154)

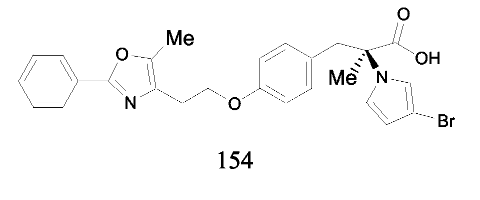

(S)-2-(3-Bromo-pyrrol-1-yl)-2-methyl-3-{4-[2-(5-methyl-2-phenyl-oxazol-4-yl)-ethoxy]-phenyl}-propionic acid methyl ester (153, 1.46g, 2.79mmol) was hydrolysed as described in procedure A. The desired product was isolated by chromatography 1;1 ethyl acetate : hexane Yield = 0.633, 45% mp 158-159°C ¹H NMR (400MHz) δ (CDCl₃) 8.06 (2H, m), 7.46 (3H, m), 6.75-6.61 (6H, m), 6.15 (1H, m), 4.21 (2H, t, J=6.1), 3.25 (2H, m) 3.05 (2H, t, J=6.1) 2.41 (3H, s), 1.58 (3H, s) MS m/z 511 (M+1).

(S)-2-(3-Bromo-pyrrol-1-yl)-2-methyl-3-{4-[2-(5-methyl-2-phenyl-oxazol-4-yl)-ethoxy]-phenyl}-propionic acid methyl ester (153) was prepared in the following manner.

### (a) (S)-2-(3-Bromo-pyrrol-1-yl)-2-methyl-3-{4-[2-(5-methyl-2-phenyl-oxazol-4-yl)-ethoxy]-phenyl}-propionic acid methyl ester (153).

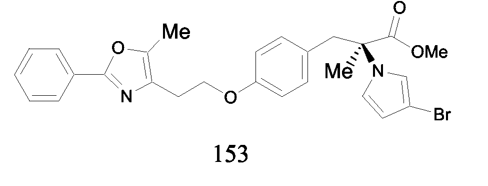

(S)-2-(3-Bromo-pyrrol-1-yl)-3-(4-hydroxy-phenyl)-2-methyl-propionic acid methyl ester (152, 4.01g, 11.8 mmol) was coupled as described in procedure B. The ester was isolated via chromatography 1:10 to 1:1 ethyl acetate:hexanes as an oil Yield = 1.46g, 24% ¹H NMR (400MHz) δ (CDCl₃) 7.96 (2H, m), 7.40 (3H, m), 6.75-6.57 (6H, m), 6.15 (1H, m), 4.18 (2H, m), 3.73 (3H, s) 3.21 (2H, m), 2.35 (3H, s), 1.54 (3H, s) MS *m*/*z* 525 (M+1),

### (b) (S)-2-(3-Bromo-pyrrol-1-yl)-3-(4-hydroxy-phenyl)-2-methyl-propionic acid methyl ester (152).

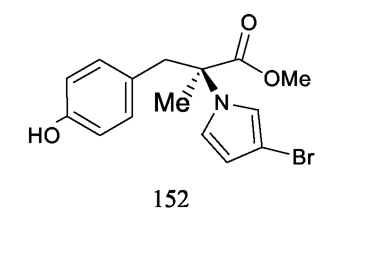

(S)-3-(4-Hydroxy-phenyl)-2-methyl-2-pyrrol-1-yl-propionic acid methyl ester (5.4g 20.8mmol) was dissolved in dichloromethane, (50mL) and cooled to - 78°C. N-Bromosuccinimide (4.08g, 22.9mmol) was added and the reaction warmed to room temperature and stirred for 3h. The solvent was removed *in vacuo* and the residue adsorbed onto silica gel. Chromatography using 1:10 to 1:1 ethyl acetate:hexane gave the desired product as a gum. Yield = 6.44g, 91%, ¹H NMR (400MHz) δ (CDCl₃) 6.71-6.58 (6H, m), 6.16 (1H, m), 3.75 (3H, s) 3.31-3.18 (2H, m), 1.55 (3H, s) MS *m*/*z* 525 (M+1).

### Example 59

### 3,3,3-Trifluoro-2-{4-[2-(5-methyl-2-phenyl-oxazol-4-yl)-ethoxy]-benzyl}-2-pyrrol-1-yl-propionic acid (159).

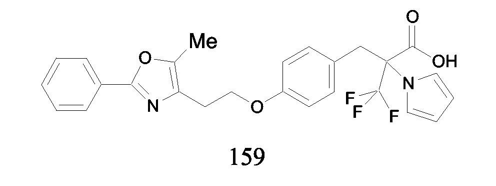

3,3,3Trifluoro-2-{4-[2-(5-methyl-2-phenyl-oxazol-4-yl)-ethoxy]-benzyl}-2-pyrrol-1-yl-propionic acid ethyl ester (158, 0.83g, 13.3mmol) was hydrolysed as described in procedure B. the product was recrystallised from hot ethyl acetate to give 2.03g, 31% m.p. 201-203°C ¹H NMR (400MHz) δ (D₆DMSO) 7.85 (2H, m), 7.42 (3H, m), 7.71 (2H, d J=8.78), 6.93 (2H, br s), 6.79 (2H, d, J=8.78), 6.06 (2H, t, J=2.19) 4.12 (2H, t, J=6.59), 3.74 (1H, d, J=14.39Hz), 3.62 (1H, d, J = 14.64) 2.85 (2H, t, J=6.58Hz) 2.29 (3H, s) MS *m*/*z* 485 (M+1)

3,3,3-Trifluoro-2-{4-[2-(5-methyl-2-phenyl-oxazol-4-yl)-ethoxy]-benzyl}-2-pyrrol-1-yl-propionic acid ethyl ester (158) was prepared in the following manner.

### (a) 3,3,3-Trifluoro-2-{4-[2-(5-methyl-2-phenyl-oxazol-4-yl)-ethoxy]-benzyl}-2-pyrrol-1-yl-propionic acid ethyl ester (158)

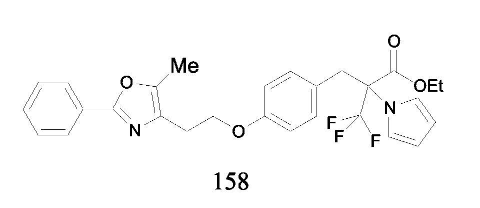

3,3,3-Trifluoro-2-(4-hydroxy-benzyl)-2-pyrrol-1-yl-propionic acid ethyl ester (157, 4.92g, 15mmol) was coupled as described in procedure B. The product was purified using chromatography to give the desired product. Yield=6.83g, 88% ¹H NMR (400MHz) (CDCl₃) 8.05 (2H, m), 7.44 (3H, m), 6.98 (2H, d J=8.54), 6.81 (2H, br s), 6.75 (2H, dd, J=2.19, 6.06), 6.23 (2H, t, J=2.19) 4.22 (4H, m), 3.67 (2H, 2d, J=14.39, Hz), 3.01 (2H, t, J=6.58Hz) 2.38 (3H, s) 1.21 (3H, m) MS *m*/*z* 513 (M+1)

### (b) 3,3,3-Trifluoro-2-(4-hydroxy-benzyl)-2-pyrrol-1-yl-propionic acid ethyl ester (157).

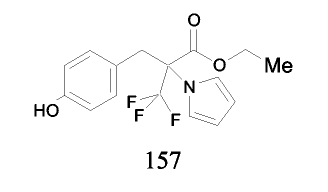

2-(4-Benzyloxy-benzyl)-2-tert-butoxycarbonylamino-3,3,3-trifluoropropionic acid ethyl ester (156, 10.72g) was dissolved in THF and Hydrogenated using Palladium on Carbon. The product 2-tert-Butoxycarbonylamino-3,3,3-trifluoro-2-(4-hydroxy-benzyl)-propionic acid ethyl ester (156a) was used crude in the next step.

Thionyl Chloride (3.46mL, 47.4mmol) was added to methanol (100mL) at 0°C. 2-tert-Butoxycarbonylamino-3,3,3-trifluoro-2-(4-hydroxy-benzyl)-propionic acid ethyl ester (8.94g, 23.7mmol) was added and the reaction mixture allowed to reach room temperature and stirred for 24h.. The solvent was removed in vacuo and the crude product 2-Amino-3,3,3-trifluoro-2-(4-hydroxy-benzyl)-propionic acid ethyl ester hydrochloride used directly in the next step. MS *m*/*z* 278 (M+1)

2-Amino-3,3,3-trifluoro-2-(4-hydroxy-benzyl)-propionic acid ethyl ester hydrochloride (156a, 7.13g, 22.7 mmol) was dissolved in water (40ml) and acetic acid (40mL). Sodium acetate (2.98g, 36.3mmol) and 2,5 dimethoxy tetrahydrofuran (4.7mL, 36.3 mmol) were added and the reaction mixture heated at 90°C for 20minutes until no starting material was observed by mass spectrum analysis. The reaction mixture was poured into water, extracted with ethyl acetate and neutralized using sodium carbonate. The organic layer was dried MgSO4 and the solvent removed in vacuo. The residue was purified by Chromatography 1:10 to 1:1 ethyl acetate:hexane to give the desired product 3,3,3-Trifluoro-2-(4-hydroxy-benzyl)-2-pyrrol-1-yl-propionic acid ethyl ester as a white solid. Yield = 4.95g, 66%, ¹H NMR (400MHz) (CDCl₃) 6.98 (2H, m), 6.83 (2H, br s), 6.75 (2H, m), 6.24 (2H, t, J=2.44) 4.24 (2H, m), 3.70 (1H, d, J=14.64 Hz), 3.66 (1H, d, J=14.64) 1.21 (3H, t, J=7.32) MS *m*/*z* 328 (M+1)

### (c) 2-(4-Benzyloxy-benzyl)-2-tert-butoxycarbonylamino-3,3,3-trifluoro-propionic acid ethyl ester (156).

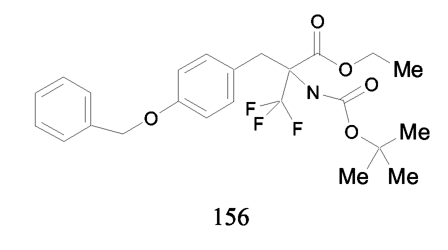

Magnesium turnings (2.82g, 0.16mol) in toluene (5ml) was stirred at room temperature. 4-Benzyloxy benzyl chloride (11.3g, 48.5mmol) in THF (100ml) was added dropwise with careful control of the Grignard formation. Once all the chloride had been added the reaction was stirred until no more color change was observed. The Grignard reagent was transferred via cannular to a solution of 2-tert-Butoxycarbonylimino-3,3,3-trifluoro-propionic acid ethyl ester (155, 29.4mmol) in THF (50ml) at -78°C. The reaction was then warmed to 0°C and stirred for 10minutes. This was quenched with saturated aqueous ammonium chloride and the product extracted with ethyl acetate. The organic layer was dried MgSO4 and the solvent removed in vacuo. The desired product was isolated using chromatography 1:10 to 1:1 ethyl acetate:hexane. 2-(4-Benzyloxy-benzyl)-2-tert-butoxycarbonylamino-3,3,3-trifluoro-propionic acid ethyl ester was obtained as a white solid. Yield = 10.72g, 78% (3 steps) ¹H NMR (400MHz) (CDCl₃) 7.43-7.32 (5H, m), 7.11 (2H, d, J=8.78), 6.87 (2H, d, J=8.78), 5.52 (1H br s), 5.02 (2H, s), 4.30 (2H, m), 3.70 (2H, d, J=13.9Hz), 1.46, (9H, s), 1.33 (3H, t, J=7.32) MS *m*/*z* 466 (M-1)

### (d) 2-tert-Butoxycarbonylimino-3,3,3-trifluoro-propionic acid ethyl ester (155).

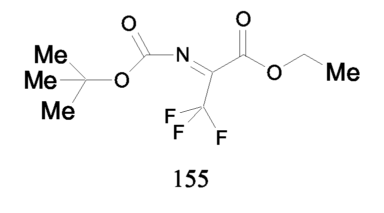

Ethyl 3,3,3 trifluoropyruvate (5g, 29.4mmol) was dissolved in dichloromethane and t-Butyl carbamate added (3.44g, 29.4mmol). The reaction mixture was stirred at room temperature for 16h. The solvent was removed under vacuum to give a white solid. The solid was dissolved in ether (50mL) and trifluoroacetic anhydride (4.57mL, 32.34mmol) was added dropwise over 30 minutes with vigorous stirring. After stirring for an additional 30 minutes Pyridine (5.23mL, 64.7mmol) was added dropwise and the reaction stirred for an additional 2h. Hexane (100ml) was added and the reaction filtered rapidly after cooling to - 78°C. The isolated product 2-tert-Butoxycarbonylimino-3,3,3-trifluoro-propionic acid ethyl ester was stored under vacuum and used without further purification. ¹H NMR (400MHz) (CDCl₃) 4.30 (2H, q, J=7.32), 1.57, (9H, s), 1.37 (3H, t, J=7.32) MS *m*/*z* 269 (M)

### Example 60

### 2-(4-Benzyloxy-indol-1-yl)-propionic acid (160).

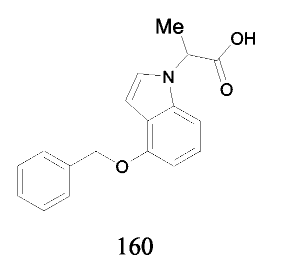

Prepared as in Example 53 using methyl 2-bromopropionate. Mp 143°C-146°C, CHN; CALC'D: C, 73.20; H, 5.80; N, 4.74; FOUND: C, 72.95; H, 5.86: N, 4.62

### Example 61

### 3-(1-{4-[3-(5-Methyl-2-phenyl-oxazol-4-yl)-propyl]-benzyl}-1H-pyrrol-2-yl)-propionic acid (165).

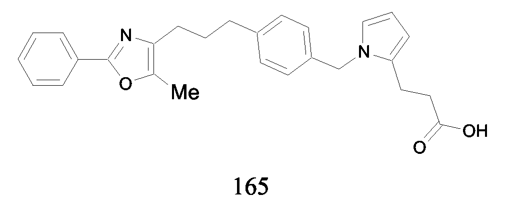

A mixture of 3-(1-{4-[3-(5-Methyl-2-phenyl-oxazol-4-yl)-propyl]-benzyl}-1H-pyrrol-2-yl)-propionic acid methyl ester (164, 0.44g, 1.48 mmol) and Lithium hydroxide (175 mg, 7.39 mmol) in 2-methoxyethanol (12 mL) and water (3 mL) was stirred at room temperature overnight. The reaction was diluted with water, acidified, extracted with ethyl acetate, dried over MgSO₄, and concentrated under reduced pressure. The crude solid was recrystallized from EtOAc/hexanes to yield the desired acid. 400 MHz 1H NMR (DMSO-*d*6) δ 7.84 (m, 2H), 7.43 (m, 3H), 7.11 (d, 2H, *J=* 8.1 Hz), 6.88 (d, 2H, *J=* 8.1 Hz), 6.67 (m, 1H), 5.88 (m, 1H), 5.75 (dd, 1H, *J=* 3.4, 2.7 Hz), 5.00 (s, 2H), 2.55 (m, 4H), 2.38 (m, 4H), 2.23 (s, 3H), 1.82 (m, 2H), mp 139-141.

3-(1-{4-[3-(5-Methyl-2-phenyl-oxazol-4-yl)-propyl]-benzyl}-1H-pyrrol-2-yl)-propionic acid methyl ester (164) was prepared in the following manner.

### (a) 3-(1-{4-[3-(5-Methyl-2-phenyl-oxazol-4-yl)-propyl]-benzyl}-1H-pyrrol-2-yl)-propionic acid methyl ester (164).

A mixture of 3-(1-{4-[3-(5-Methyl-2-phenyl-oxazol-4-yl)-propyl]-benzyl]-1H-pyrrol-2-yl)-acrylic acid methyl ester (163, 0.47g, 1.07 mmol) and 10% Pd/C (59 mg) in methanol (40 mL) and THF (10 mL) was stirred under an H2 atmosphere for 17 hours. The reaction was filtered and concentrated to yield the desired product.

### (b) 3-(1-{4-[3-(5-Methyl-2-phenyl-oxazol-4-yl)-propyl]-benzyl]-1H-pyrrol-2-yl)-acrylic acid methyl ester (163).

Sodium hydride (60%, 85 mg, 2.10 mmol) was added to a solution of 3-(1H-Pyrrol-2-yl)-acrylic acid methyl ester (162, 265 mg, 1.75 mmol) in dimethylformamide (15 mL) and the mixture was stirred at room temperature for 15 minutes. 4-[3-(4-Chloromethyl-phenyl)-propyl]-5-methyl-2-phenyl-oxazole (161, 1.14g, 3.50 mmol) was added and the reaction heated to 60°C overnight. The reaction was diluted with water, acidified, extracted with ethyl acetate, dried over MgSOs, and concentrated under reduced pressure. A mixture of the product and starting material was taken on to the next step.

### (c) 3-(1H-Pyrrol-2-yl)-acrylic acid methyl ester (162).

A solution of pyrrole-2-carboxaldehyde (2.5g, 26.3 mmol) and methyl (triphenylphosphoranylidine)-acetate (9.23g, 27.6 mmol) in toluene (250 mL) was heated to 45°C overnight. The reaction was concentrated under reduced pressure. The crude oil was chromatographed eluting with 20%-30% EtOAc in hexanes to yield the desired product (2.70g, 68%).

### (d) 4-[3-(4-Chloromethyl-phenyl)-propyl]-5-methyl-2-phenyl-oxazole (161).

A solution of {4-[3-(5-Methyl-2-phenyl-oxazol-4-yl)-propyl]-phenyl}-methanol (2.0g, 6.51 mmol) in dichloromethane (25 mL) was cooled to 0°C. Methanesulfanyl chloride (550µL, 7.16 mmol) and triethylamine (1.81 mL, 13.0 mmol) were added and the mixture warmed to room temperature overnight. The reaction was diluted with saturated NH₄Cl solution, extracted with CH₂Cl₂, washed with brine, dried over MgSO₄, and concentrated under reduced pressure to yield the desired product.

### Example 62

### (1-[2-(5-Methyl-2-phenyl-oxazol-4-yl)-ethyl]-1H-indol-5-yloxy)-phenylacetic acid (169).

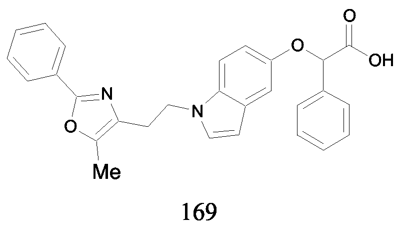

A mixture of {1-[2-(5-Methyl-2-phenyl-oxazol-4-yl)-ethyl]-1H-indol-5-yloxy}-phenyl-acetic acid methyl ester (168, 1.44g, 3.09 mmol) and LiOH (370 mg, 15.4 mmol) in 2-methoxyethanol (24 mL) and water (6 mL) was stirred at room temperature overnight. The reaction was diluted with water, acidified with 2N HCl, extracted with ethyl acetate, dried over MgSO₄, and concentrated under reduced pressure. The crude oil was chromatographed eluting with 30% EtOAc (with 20% HOAc) in hexanes. The resulting solid was recrystallized from EtOAc/hexanes to yield the desired acid (140 mg). 400 MHz 1H NMR (DMSO-*d*6) δ 7.88 (m, 2H), 7.49 (m, 5H), 7.35 (m, 3H), 7.26 (d, 1H, *J=* 9.0 Hz), 7.17 (d, 1H, *J=* 2.9 Hz), 7.00 (d, 1H, *J=* 2.4 Hz), 6.77 (dd, 1H, *J=* 9.0, 2.4 Hz), 6.25 (d, 1H, *J=* 2.9 Hz), 5.68 (s, 1H), 4.34 (t, 2H, *J=* 6.6 Hz), 2.87 (t, 2H, *J=* 6.6 Hz), 1.87 (s, 3H), mp 176-178.

{1-[2-(5-Methyl-2-phenyl-oxazol-4-yl)-ethyl]-1H-indol-5-yloxy}-phenylacetic acid methyl ester (168) was prepared in the following manner.

### (a) {1-[2-(5-Methyl-2-phenyl-oxazol-4-yl)-ethyl]-1H-indol-5-yloxy]-phenyl-acetic acid methyl ester (168).

A mixture of 1-[2-(5-Methyl-2-phenyl-oxazol-4-yl)-ethyl]-1H-indol-5-ol (167, 1.16g, 3.64 mmol), cesium carbonate (2.37g, 7.29 mmol) and methyl α-bromophenylacetate (1.15 mL, 7.29 mmol) in DMF (35 mL) was heated to 55°C overnight. The reaction was diluted with water, acidified with 2N HCl, extracted with ethyl acetate, dried over MgSOs, and concentrated under reduced pressure.. The crude oil was chromatographed eluting with 15%-25% EtOAc (with 20% HOAc) in hexanes to yield the desired product (1.44g, 85%).

### (b) 1-[2-(5-Methyl-2-phenyl-oxazol-4-yl)-ethyl]-1H-indol-5-ol (167).

A mixture of 3-(5-Benzyloxy-indol-1-yl)-propionic acid methyl ester (166, 1.93g, 4.72 mmol) and 20% Pd/C (360 mg) in ethanol (40 mL) and THF (10 mL) was stirred under an H2 atmosphere for 18 hours. The reaction was filtered and concentrated. Purified by chromatography to yield the deprotected product in near quantitative yield.

### (c) 5-Benzyloxy-1-[2-(5-methyl-2-phenyl-oxazol-4-yl)-ethyl]-1H-indole (166).

5-Benzyloxyindole (2.0 g, 8.96 mmol) was dissolved in dimethylformamide (90 mL) and 60% sodium hydride (540 mg, 13.4 mmol) was added. Toluene-4-sulfonic acid 2-(5-methyl-2-phenyl-oxazol-4-yl)-ethyl ester (600 µL, 5.37 mmol) was added. The reaction was stirred at room temperature overnight. The reaction was diluted with water, acidified, extracted with ethyl acetate, dried over MgSOs, and concentrated under reduced pressure. Purified using chromatography. Yield 1.93 g (60%).

### Example 63

### 2-(1-Methyl-1H-indol-3-yl)-3-{4-[2-(5-methyl-2-phenyl-oxazol-4-yl)-ethoxy]-phenyl}-propionic acid (173)

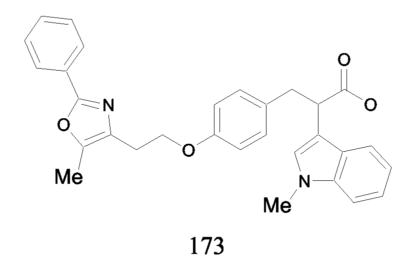

2-(1-Methyl-1H-indol-3-yl)-3-{4-[2-(5-methyl-2-phenyl-oxazol-4-yl)-ethoxy]-phenyl}-propionic acid methyl ester (172, 250 mg, 0.51 mmol) was dissolved in 2-methoxyethanol (4 mL) and H₂O (1mL). LiOH monohydrate (60 mg, 2.53 mmol) was added in one portion and the suspension stirred overnight. The reaction was diluted with water, extracted with ethyl acetate, dried over MgSO₄, and concentrated under reduced pressure to yield the desired product. Near quantitative yield. 400 MHz 1H NMR (DMSO-*d*6) δ 7.85 (m, 2H), 7.58 (d, 1H, *J=* 7.8 Hz), 7.44 (m, 3H), 7.32 (t, 1H, *J=* 6.6 Hz), 7.17 (s, 1H), 7.08 (m, 3H), 6.96 (m, 1H), 6.75 (dd, 2H, *J*= 8.5, 6.6 Hz), 4.10 (m, 2H), 3.97 (m, 1H), 3.67 (s, 3H), 2.95 (m, 2H), 2.84 (t, 2H, *J*= 6.3 Hz), 2.29 (s, 3H). MS *m*/*z* 481 (M+1).

2-(1-Methyl-1H-indol-3-yl)-3-{4-[2-(5-methyl-2-phenyl-oxazol-4-yl)-ethoxy]-phenyl}-propionic acid methyl ester (172) was prepared in the following manner.

### (a) 2-(1-Methyl-1H-indol-3-yl)-3-{4-[2-(5-methyl-2-phenyl-oxazol-4-yl)-ethoxy]-phenyl}-propionic acid methyl ester (172).

A solution of (1-Methyl-1H-indol-3-yl)-acetic acid methyl ester (171, 450 mg, 2.21 mmol) in tetrahydrofuran (14 mL) was cooled to 0°C. Lithium bis(trimethylsilyl)amide (1.0 M in THF, 2.32 mL, 2.32 mmol) was added and the mixture stirred at 0°C for 1 hour. A solution of 4-[2-(4-Chloromethyl-phenoxy)-ethyl]-5-methyl-2-phenyl-oxazole (835 mg, 2.55 mmol) in THF (4 mL) was added and the reaction warmed to room temperature and stirred for 6 hours. The reaction was diluted with water, extracted with ethyl acetate, dried over MgSOs, and concentrated under reduced pressure. The crude oil was chromatographed eluting with 15%-20% EtOAc (with 20% HOAc) in hexanes to yield the desired product (250mg, 23%).

### (b) 4-[2-(4-Chloromethyl-phenoxy)-ethyl]-5-methyl-2-phenyl-oxazole (171).

A solution of {4-[2-(5-Methyl-2-phenyl-oxazol-4-yl)-ethoxy]-phenyl}-methanol (170, 2.0g, 6.46 mmol) in dichloromethane (25 mL) was cooled to 0°C. Methanesulfanyl chloride (550µL, 7.16 mmol) and triethylamine (1.80 mL, 12.9 mmol) were added and the mixture warmed to room temperature overnight. The reaction was diluted with saturated NH₄Cl solution, extracted with CH₂Cl₂, washed with brine, dried over MgSO₄, and concentrated under reduced pressure to yield the desired product.

### (c) (1-Methyl-1H-indol-3-yl)-acetic acid methyl ester (170).

TMS-diazomethane (7.93 mL, 15.9 mmol, 2.0 M in hexanes) was added to a solution of 1-methyl-3-indoleacetic acid (2.0g, 10.6 mmol) in toluene (80 mL) and methanol (20 mL). The reaction stirred at room temperature for 30 minutes, quenced with acetic acid and concentrated. The desired ester was obtained in near quantitative yield.

### Example 64

### 3-(4-[3-(5-Methyl-2-phenyl-oxazol-4-yl)-propoxy]-indol-1-yl)-propionic acid (174).

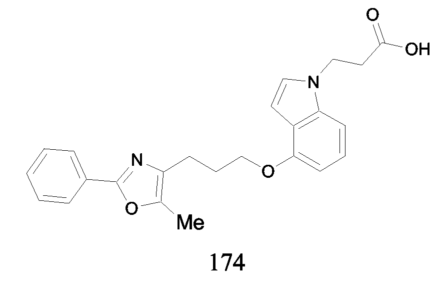

Prepared as described for Example 53 using 4-(3-Bromo-propyl)-5-methyl-2-phenyl-oxazole. m.p. 133-134, CHN (theor): 71.27% C, 5.98% H, 6.93% N. Found: 71.19% C, 5.92% H, 6.93% N.

### Example 65

### 2-(4-[2-(5-Methyl-2-phenyl-oxazol-4-yl)-ethoxy]-indol-1-yl)-propionic acid (175).

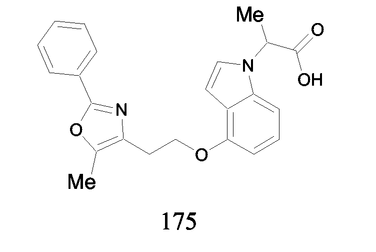

Prepared as described for Example 53 using methyl 2-bromopropionate. Mp 157°C-159°C, CHN (theor.): 70.75% C, 5.68% H, 7.17% N. Found: 70.82% C, 5.74% H, 7.13% N.

### Example 67

### 3-(4-[2-(2-Trifluoromethyl-phenyl)-ethoxy]-indol-1-yl)-propionic acid (176)

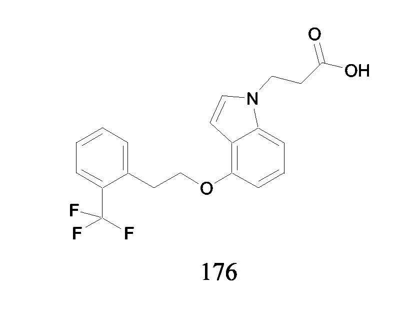

Prepared as described for Example 53 except the alkylation on the indole oxygen was completed in the following manner. 3-(4-Hydroxy-indol-1-yl)-propionic acid methyl ester (1.73g, 7.89 mmol), triphenylphosphine (2.07g, 7.89 mmol), and 2-(2-Trifluoromethyl-phenyl)-ethanol (1.25 mL, 7.89 mmol) were dissolved in tetrahydrofuran (35 mL). Diethyl azodicarboxylate (1.28 mL in 5 mL THF, 7.89 mmol) was added dropwise and the reaction stirred at room temperature overnight. The mixture was concentrated and purified by chromatography. Yield = 290 mg, 10%. m.p.=126-129, THEORY: 63.66% C, 4.81% H, 3.71% N. ACTUAL: 63.68% C, 4.97% H, 3.71% N.

### Example 67

### (4-[3-(5-Methyl-2-phenyl-oxazol-4-yl)-propoxy]-indol-1-yl)-acetic acid (177).

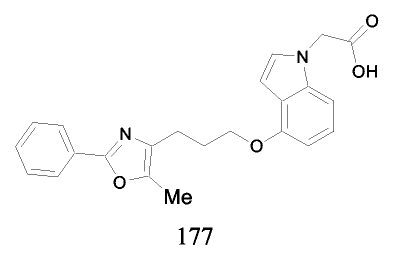

Prepared from 4-(3-Bromo-propyl)-5-methyl-2-phenyl-oxazole as described in Examople 69. CHN: THEORY: 70.75% C, 5.68% H, 7.17% N. ACTUAL: 69.37% C, 5.59% H, 6.82% N. MS *m*/*z* 391 (M+1).

### Example 68

### 4-[2-(5-Methyl-2-phenyl-oxazol-4-yl)-ethoxy]-indol-1-yl}-acetic acid (178).

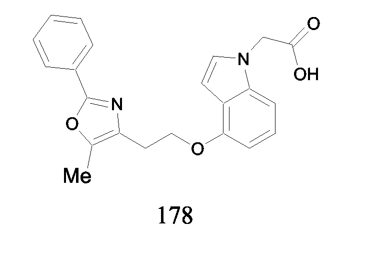

4-Benzyloxyindole (1.0 g, 4.48 mmol) was dissolved in dimethylformamide (45 mL) and 60% sodium hydride 180 mg, 4.48 mmol) was added. Ethyl bromoacetate (600 µL, 5.37 mmol) was added. The reaction was stirred at room temperature overnight. The reaction was diluted with water, extracted with ethyl acetate, dried over MgSOs, and concentrated under reduced pressure. Near quantitative yield. The compound was then prepared as described for Example 53. MW=376.41, mp. 193-196, CHN: THEORY: 70.20% C, 5.36% H, 7.44% N. ACTUAL: 69.89% C, 5.33% H, 7.39% N.

### Example 69

### 4-{4-[2-(5-Methyl-2-phenyl-oxazol-4-yl)-ethoxy]-indol-1-yl}-butyric acid (179).

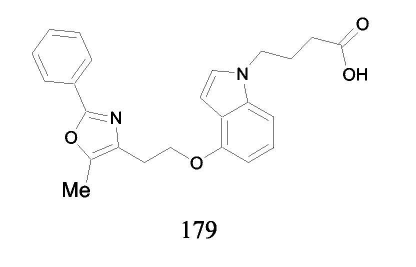

4-Benzyloxyindole (2.0 g, 8.96 mmol) was dissolved in toluene (5 mL) and 60% sodium hydride (360 mg, 8.96 mmol) was added. The mixture was heated to 100°C for 20 minutes. The residue was taken up in dimethylformamide (20 mL) and γ-butyrolactone (1.38 mL, 17.9 mmol) was added. The reaction was heated to 150°C for 72 hours. The reaction was diluted with water, acidified with 2N HCl, extracted with ethyl acetate, dried over MgSOs, and concentrated under reduced pressure. The compound was then prepared as described for PD#0333941-0000. 73140x27, mw=404.4, mp 151-154; CHN; THEORY: 71.27% C, 5.98% H, 6.93% N; ACTUAL: 71.01% C, 6.02% H, 6.83% N.

### Example 70

### 3-(3-Fluoro-4-[2-(5-methyl-2-phenyl-oxazol-4-yl)-ethoxy]-phenyl}-2-(S)-pyrrol-1-yl-propionic acid (180).

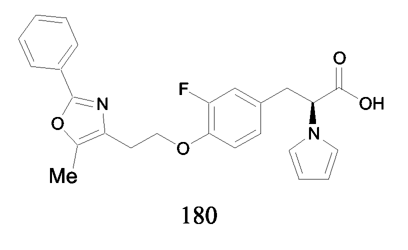

Prepared as described for Example 1 using 5-fluoro-L-tyrosine. 73140x37 MW=434.4, mp=154-155, CHN; THEORY: 69.11% C, 5.34% H, 6.45% N; ACTUAL: 69.09% C, 5.25% H, 6.26% N

### Example 71

### 3-{4-[2-(5-Methyl-2-phenyl-oxazol-4-yl)-ethoxy]-phenyl}-2-pyridin-4-yl-propionic acid (181).

The title compound was synthesized as described in Example 12 using ethyl 4-pyridyl acetate as starting material (45 mg, 7%). ¹H NMR d6-DMSO δ 2.30 (s, 3H) 2.85 (m, 3H) 3.02 (m, 2H) 4.11 (t, 2H) 6.80 (d, 2H) 7.06 (d, 2H) 7.44 (m, 3H) 7.65 (d, 2H) 7.84 (d, 2H)8.63 (s, 2H)...MS(M-44)(minus carboxylate): 385.1. Anal. Calcd for C₂₆H₂₄O₄N₂·1.67 H₂O: C, 68.05; H, 5.82; N, 6.11. Found: C, 68.03; H, 6.00; N, 6.11.

### Example 72

### 2-Methyl-5-(2-{4-[2-(5-methyl-2-phenyl-oxazol-4-yl)-ethoxy]-phenyl}-1-phenyl-ethyl)-2H-tetrazole (186a) and 1-Methyl-5-(2-{4-[2-(5-methyl-2-phenyl-oxazol-4-yl)-ethoxy]-phenyl]-1-phenyl-ethyl)-1H-tetrazole (186b).

A mixture of 5-(2-{4-[2-(5-methyl-2-phenyl-oxazol-4-yl)-ethoxy]-phenyl]-1-phenyl-ethyl)-1H-tetrazole (185a) and 5-(2-{4-[2-(5-methyl-2-phenyl-oxazol-4-yl)-ethoxy]-phenyl]-1-phenyl-ethyl)-2H-tetrazole (185b) (0.23 g, 0.51 mmol), methyliodide (0.20 mL, 3.2 mmol), TEA (0.22 mL, 1.6 mmol) was refluxed in acetonitrile for 1 hour. The reaction was cooled and concentrated. The residue was dissolved in EtOAc, washed with water, dried (MgSO₄). The products were isolated by column chromatography (25% to 35% EtOAc/hex)

2-methyl tetrazole (186a): front running spot (13%). C₂₈H₂₇N₅O₂ Mass Calc. = 465.555; M+1(obs)=466.2. HPLC: c-18 column, 87%, 20/80 (CH₃CN/H₂O) to 90/10 over 20 min, rt=18.876 min, 254 nm. Lack of NOE between H35/H23 confirmed regiochemistry.

1-methyl tetrazole (186b): bottom running spot (24%). C₂₈H₂₇N₅O₂ Mass Calc. = 465.555; M+1(obs)=466.2. HPLC: c-18 column, 85%, 20/80 (CH₃CN/H₂O) to 90/10 over 20 min, rt=17.438 min, 254 nm. Presence of NOE between H35/H23 confirmed regiochemistry.

5-(2-{4-[2-(5-methyl-2-phenyl-oxazol-4-yl)-ethoxy]-phenyl]-1-phenylethyl)-1H-tetrazole (185a) and 5-(2-{4-[2-(5-methyl-2-phenyl-oxazol-4-yl)-ethoxy]-phenyl}-1-phenyl-ethyl)-2H-tetrazole (185b) were prepared in the following manner.

### (a) 5-(2-{4-[2-(5-methyl-2-phenyl-oxazol-4-yl)-ethoxy]-phenyl}-1-phenyl-ethyl)-1H-tetrazole (185a) and 5-(2-{4-[2-(5-methyl-2-phenyl-oxazol-4-yl)-ethoxy]-phenyl}-1-phenyl-ethyl)-2H-tetrazole (185b).

3-{4-[2-(5-methyl-2-phenyl-oxazol-4-yl)-ethoxy]-phenyl-propionitrile (184, 1.81 g, 4.47 mmol) was refluxed with azidotributyltin (2.3 mL, 8.4 mmol) in 20 mL dioxane for 25 hours. The reaction was concentrated and the residue dissolved in Et₂O. HCl gas was bubbled through the solution. The solution was concentrated and triturated with hexanes and the hexanes layer decanted. The remaining residue was purified by column chromatography (25% to 100% EtOAc/hex) to obtain a mixture of 5-(2-{4-[2-(5-methyl-2-phenyl-oxazol-4-yl)-ethoxy]-phenyl}-1-phenyl-ethyl)-1H-tetrazole (185a) and 5-(2-{4-[2-(5-methyl-2-phenyl-oxazol-4-yl)-ethoxy]-phenyl}-1-phenyl-ethyl)-2H-tetrazole (185b) (23% recovered starting material, 14% product).

### (b) 3-{4-[2-(5-methyl-2-phenyl-oxazol-4-yl)-ethoxy]-phenyl-propionitrile (184).

The title compound was prepared according to General Procedure B using 3-(4-hydroxy-phenyl)-2-phenyl-propionitrile (183) and 2-(5-methyl-2-phenyloxazol-4-yl) ethanol as starting materials.

### (c) 3-(4-Hydroxy-phenyl)-2-phenyl-propionitrile (183).

The benzyl group in 3-(4-Benzyloxy-phenyl)-2-phenyl-propionitrile (182) was removed by hydrogenolysis using 10% Pd/C as a catalyst.

### (d) 3-(4-Benzyloxy-phenyl)-2-phenyl-propionitrile (182).

This compound was synthesized in the same manner as Compound 89 using benzyl cyanide (2.4 mL, 21 mmol) and compound 88 (5.85 g, 21 mmol) as starting materials.

### Example 73

### 2-Methyl-3-{4-[2-(5-methyl-2-phenyl-oxazol-4-yl)-ethoxy]-phenyl}-2-thiophen-2-yl-propionic acid (189).

This compound was prepared as described in Example 32 using 2-thiophen-3-yl-propionic acid ethyl ester (188) and compound 88 as the starting materials. Mp: 165-166°C. Anal. Calcd for C₂₆H₂₅O₄NS: C, 69.78; H, 5.63; N, 3.13. Found: C, 69.65; H, 5.88; N, 3.00.

2-Thiophen-2-yl-propionic acid ethyl ester was prepared in the following manner.

### (a) 2-Thiophen-2-yl-propionic acid ethyl ester (188).

This compound was prepared as described for compound 89, using ethyl 2-thiophene acetate and methyliodide as starting materials.

### Example 74

### 2-Methyl-3-{4-[2-(5-methyl-2-phenyl-oxazol-4-yl)-ethoxy]-phenyl}-2-thiophen-3-yl-propionic acid (191).

This compound was prepared as described in Example 32 using 2-thiophen-3-yl-propionic acid ethyl ester (191) and compound 88 as the starting materials. Mp: 165-166°C. Anal. Calcd for C₂₆H₂₅O₄NS: C, 69.78; H, 5.63; N, 3.13. Found: C, 69.65; H, 5.88; N, 3.00.

2-Thiophen-3-yl-propionic acid ethyl ester (190) was prepared in the following manner.

### (a) 2-Thiophen-3-yl-propionic acid ethyl ester (190).

This compound was prepared as described for compound 89, using ethyl 3-thiophene acetate and methyliodide as starting materials.

### Example 75

### N-(2,2-Dimethyl-3-{4-[2-(5-methyl-2-phenyl-oxazol-4-yl)-ethoxy]-phenyl}-propionyl)-methanesulfonamide (192).

Compound 102 (0.493 g, 1.3 mmol), 4-DMAP (0.169 g, 1.4 mmol), EDC (0.266 g, 1.4 mmol) and methanesulfonamide (0.132g, 1.4 mmol) were combined in 10 mL CH₂Cl₂ and stirred at ambient temperature overnight. The reaction was washed with 1N HCl and H₂O, dried (MgSO₄). Column chromatography (25% to 40% EtOAc/Hex) was used to isolate the pure compound which was recrystallized from Et₂O/hex (73 mg, 12%). Mp: 112-113°C. Anal. Calcd for C₂₄H₂₈O₅N₂S: C, 63.14; H, 6.18; N, 6.14. Found: C, 63.46; H, 6.20; N, 6.09.

### Example 76

### N-(2-Fluoro-3-{4-[2-(5-methyl-2-phenyl-oxazol-4-yl)-ethoxy]-phenyl}-2-phenyl-propionyl)-methanesulfonamide (193).

Compound 103 (1.003 g, 2.3 mmol) was subjected to the same conditions as above for PD 0333437. (32 mg, 2%). Mp: 118-120°C. Anal. Calcd for C₂₈H₂₇O₅N₂SF: C, 64.35; H, 5.21; N, 5.36. Found: C, 64.02; H, 5.00; N, 5.15.

### Example 77

### 3-{3-[2-(5-Methyl-2-phenyl-oxazol-4-yl)-ethoxy]-isoxazol-5-yl}-2-pyrrol-1-yl-propionic acid (199).

3-{3-[2-(5-Methyl-2-phenyl-oxazol-4-yl)-ethoxy]-isoxazol-5-yl}-2-pyrrol-1-yl-propionic acid methyl ester (198, 0.2 g, 0.5 mmol) was hydrolyzed by using General Procedure A. (0.16 g, 81%). Mp: 143-144°C. Anal. Calcd for C₂₂H₂₁O₅N₃·0.17 H₂O: C, 64.32; H, 5.20; N, 10.23. Found: C, 64.37; H, 5.24; N, 9.79. C₂₂H₂₁N₃O₅ Mass Calc. = 407.426; M+1(obs) = 408.2.

3-{3-[2-(5-Methyl-2-phenyl-oxazol-4-yl)-ethoxy]-isoxazol-5-yl}-2-pyrrol-1-yl-propionic acid methyl ester (198) was prepared in the following manner.

### (a) 3-{3-[2-(5-Methyl-2-phenyl-oxazol-4-yl)-ethoxy]-isoxazol-5-yl}-2-pyrrol-1-yl-propionic acid methyl ester (198).

The title compound was synthesized as described for Compound 89 using Pyrrol-2-yl-acetic acid methyl ester (197, 0.139 g , 1 mmol) and 5-Bromomethyl-3-[2-(5-methyl-2-phenyl-oxazol-4-yl)-ethoxy]-isoxazole (196, 0.359 g, 0.99 mmol) as starting materials. (0.20 g, 48%).

### (b) Pyrrol-2-yl-acetic acid methyl ester (197).

General procedure C (for synthesis of compound 1) was used to synthesize pyrrol-2-yl-acetic acid methyl ester using glycine methyl ester hydrochloride (1.26 g, 10 mmol) as the starting material, heating only until the reaction starts to yellow (7-10 min). (1.01 g, 73%)

### (c) 5-Bromomethyl-3-[2-(5-methyl-2-phenyl-oxazol-4-yl)-ethoxy]-isoxazole (196).

Carbon tetrabromide (1.99 g, 6 mmol) was added to a 0°C solution of {3-[2-(195, 5-Methyl-2-phenyl-oxazol-4-yl)-ethoxy]-oxazol-5-yl}-methanol (0.91 g, 3 mmol) and Ph₃P (1.99 g, 7.6 mmol) in 15 mL THF. The reaction was stirred at 0°C for 1 hour. The reaction was concentrated and isolated by column chromatography (15% to 20% EtOAc/hex). (0.62 g, 57%)

### (d) {3-[2-(5-Methyl-2-phenyl-oxazol-4-yl)-ethoxy]-oxazol-5-yl}-methanol (195).

Sodium borohydride (0.20 g, 5.3mmol) was added in portions to a suspension of 3-[2-(5-Methyl-2-phenyl-oxazol-4-yl)-ethoxy]-oxazole-5-carboxylic acid methyl ester (194, 0.57 g, 1.7 mmol) in 15 mL MeOH at 0°C. The reaction was allowed to warm to ambient temperature for 2 hours. The reaction was quenched with water and the solvent removed. The residue was dissolved in Et₂O, washed with water and brine, dried (MgSO₄) and concentrated to give a white solid. (0.47 g, 90%)

### (e) 3-[2-(5-Methyl-2-phenyl-oxazol-4-yl)-ethoxy]-oxazole-5-carboxylic acid methyl ester (194).

General Procedure B was used to synthesize this compound using methyl 3-hydroxy-5-isoxazole carboxylate (1.423 g, 10 mmol) and 2-(5-methyl-2-phenyl-oxazol-4-yl)ethanol (2.141 g, 10.5 mmol) as starting materials. (2.71 g, 83%)

### Example 78

### 2-(5-Methyl-2-phenyl-oxazol-4-yl)-3-{4-[3-(5-methyl-2-phenyl-oxazol-4-yl)-propyl]-phenyl}-propionic acid (204).

General Procedure A was used to hydrolyze 3-{4-[3-(5-Methyl-2-phenyl-oxazol-r-yl)-propel]-phenyl}-2-pyrrol-1-yl-propionic acid methyl ester (203) to provide the final product. (0.54 g, 80%). C₃₂H₃₀N₂O₄ Mass Calc. = 506.601; M+1(obs) = 507.3. Anal. Calc'd for C₃₂H₃₀O₄N₂ 0.17 H₂O: C, 75.35; H, 5.95; N, 5.49. Found: C, 75.19; H, 6.06; N, 5.28.

3- {4-[3-(5-Methyl-2-phenyl-oxazol-r-yl)-propel]-phenyl}-2-pyrrol-1-yl-propionic acid methyl ester (203) was prepared in the following manner.

### (a) 3-{4-[3-(5-Methyl-2-phenyl-oxazol-r-yl)-propel]-phenyl}-2-pyrrol-1-yl-propionic acid methyl ester (203).

The title compound was synthesized as described for compound 89 using methyl 2-(5-methyl-2-phenyl-1,3-oxazol-4-yl)acetate (0.55 g, 2.3 mmol) and 4-[3-(4-Bromomethyl-phenyl)-propyl]-5-methyl-2-phenyl-oxazole (202, 0.78 g, 2.1 mmol) as starting materials.

### (b) 4-[3-(4-Bromomethyl-phenyl)-propyl]-5-methyl-2-phenyl-oxazole (202).

Carbon tetrabromide (2.65 g, 8 mmol) was added to a 0°C solution of {4-[3-(5-Methyl-2-phenyl-oxazol-4-yl)-propyl]-phenyl}-methanol (202, 1.25 g, 4 mmol) and Ph₃P (2.67 g, 10 mmol) in 40 mL THF. The reaction was stirred at 0°C for 1 hour. The reaction was concentrated and isolated by column chromatography (5% EtOAc/hex). (0.83 g, 53%).

### (c) {4-[3-(5-Methyl-2-phenyl-oxazol-4-yl)-propyl]-phenyl}-methanol (202).

(4-[3-(5-Methyl-2-phenyl-oxazol-4-yl)-prop-1ynyl]-phenyl}-methanol (201, 6.63 g, 21.8 mmol) is reduced using Raney nickel in 1:1 MeOH/THF. The catalyst is filtered off and the filtrate concentrated to obtain a pale yellow solid. (6.45 g, 100%).

### (d) {4-[3-(5-Methyl-2-phenyl-oxazol-4-yl)-prop-1ynyl]-phenyl}-methanol (201).

5-Methyl-2-phenyl-4-prop-2-ynyloxazole (compound 117, 11.74 g, 60 mmol) in 50 mL pyrrolidine was added to a suspension of 4-iodobenzyl alcohol (10.71 g, 46 mmol), tetrakis(triphenylphosphine) palladium (0) (5.30 g, 4.6 mmol) and copper (I) iodide (0.431 g, 2.3 mmol) in 50 mL pyrrolidine. The reaction was complete after alkyne addition. The reaction was concentrated and the residue dissolved in EtOAc. The organic layer was washed with sat'd NH₄Cl and brine, filtered, dried (MgSO₄). The crude material was triturated with CH₂Cl₂ and filtered. This process is repeated twice to obtain two crops of desired material which was recrystallized from EtOAc/hex. (6.63 g, 48%)

### Example 79

### 2-(5-Methyl-2-phenyl-oxazol-4-yl)-3-{4-[2-(5-methyl-2-phenyl-oxazol-4-yl)-ethoxy]-phenyl}-propionic acid (205).

This compound was synthesized as described for Example 32 using methyl 2-(5-methyl-2-phenyl-1,3-oxazol-4-yl)acetate and compound 88 as starting materials. Mp: 120°C. MS(M+1):509.2.

### Example 80

### 3-{4-[3-(5-Methyl-2-phenyloxazol-4-yl)-propyl]-phenyl}-2-phenylpropionic acid (211).

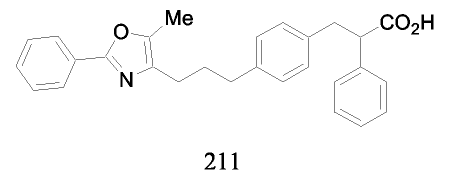

A solution of 3-{4-[3-(5-Methyl-2-phenyloxazol-4-yl)-propyl]-phenyl}-2-phenylpropionic acid (210, 0.86 g, 1.96 mmol) in MeOH was treated with excess LiOH (1.0 M) and stirred at ambient temperature. After completion, the reaction was diluted with sat. NH₄Cl and extracted with EtOAc. The combined organic extracts were dried with brine followed by Na₂SO₄ and chromatographed (5 to 60 % EtOAc in hexanes) to provide 120 mg of the title compound. MS *m*/*z* 426 (M+H)⁺.

3-{4-[3-(5-Methyl-2-phenyloxazol-4-yl)-propyl]-phenyl}-2-phenylpropionic acid methyl ester (210) was prepared in the following manner.

### (a) 3-{4-[3-(5-Methyl-2-phenyloxazol-4-yl)-propyl]-phenyl}-2-phenylpropionic acid methyl ester (210).

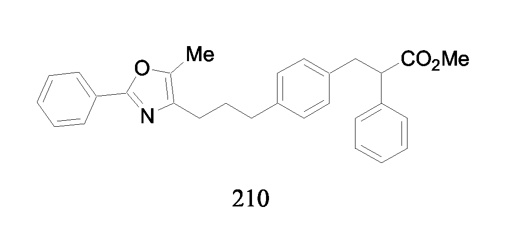

A solution of 3-{4-[3-(5-methyl-2-phenyloxazol-4-yl)-prop-1-ynyl]-phenyl}-2-phenylpropionic acid methyl ester (209, 0.89 g, 2.0 mmol) in MeOH (50 mL) with 20 % Pd/C (0.13 g) was stirred under an atmosphere of H₂ for 13.5 h. Filtration provided the desired compound in 96 % yield.

### (b) 3-{4-[3-(5-Methyl-2-phenyloxazol-4-yl)-prop-1-ynyl]-phenyl}-2-phenylpropionic acid methyl ester (208).

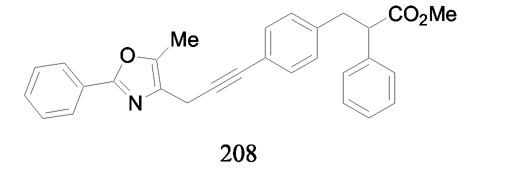

A mixture of 5-methyl-2-phenyl-4-prop-ynyloxazole (1.0, 5.07 mmol), 2-phenyl-3-(4-trifluoromethanesulfonylphenyl)-propionic acid methyl ester (207, 1.52 g, 3.9 mmol), and tetrakis(triphenylphosphine)palladium(0) (0.32 g, 0.27 mmol) were combined in 3 mL DMF. The mixture was purged with nitrogen before adding TEA (0.163 mL, 1.17 mmol) and CuI (15 mg, 0.078 mmol). After stirring at 80 °C for 18 h, the mixture was diluted with EtOAc and washed with water and brine, dried over Na₂SO₄, and chromatographed (5 % EtOAc in hexanes) to give 0.89 g of the title compound.

### (c) 2-Phenyl-3-(4-trifluoromethanesulfonylphenyl)-propionic acid methyl ester (207).

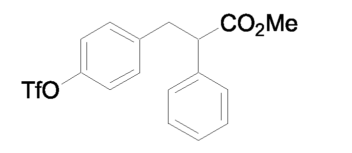

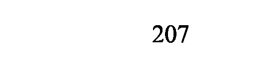

A solution of 2-phenyl-3-(4-hydroxyphenyl)-propionic acid methyl ester (2.0 g, 7.8 mmol) in CH₂Cl₂ (25 mL) was purged with nitrogen. TEA (1.2 mL, 8.6 mmol) was added, and the mixture was cooled to 0 °C. Trifluoromethanesulfonic anhydride (1.38 mL, 8.2 mmol) was added, and the reaction was allowed to warm to ambient temperature. After 18 h, the reaction was diluted with EtOAc, washed with 5 % NaHCO₃, water, and brine, dried over Na₂SO₄, and chromatographed (10 % EtOAc in hexanes) to provide 2.84 g of the title compound.

### Example 81

### 3-{6-[2-(5-Methyl-2-phenyl-oxazol-4-yl)-ethoxy]-pyridin-3-yl}-2-pyrrol-1-yl-propionic acid (216).

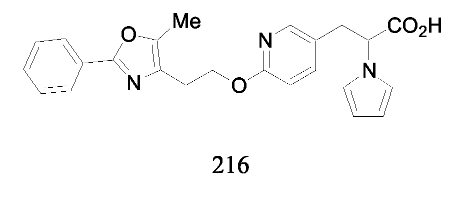

A solution of 5-bromomethyl-2-[2-(5-methyl-2-phenyl-oxazol-4-yl)-ethoxy]-pyridine (215, 0.73 g, 1.96 mmol) and pyrrole-1-yl-acetic acid methyl ester (0.54 g, 3.91 mmol) in 20 mL anhydrous THF was purged with dry nitrogen and treated with lithium bis(trimethylsilyl)amide (1.0 M in THF, 3.9 mL). After stirring at room temperature for 18 h, the reaction was diluted with EtOAc and washed with sat. NH₄Cl. TLC and MS of the organic layer indicate hydrolysis of some of the methyl ester. The organic layer was washed with brine and evaporated to dryness. The resultant oil was dissolved in MeOH and treated with excess LiOH (1 M in water). After stirring 18 h at room temperature, the mixture was evaporated to dryness and partitioned between EtOAc and pH 7 buffer. Upon washing the organic layer with brine, the product precipitated. The solid was collected, washed with water and ether, and dried under vacuum to constant weight to provide 287 mg (34 %) of the title compound. MS m/z 465 (M+H)⁺.

5-Bromomethyl-2-[2-(5-methyl-2-phenyl-oxazol-4-yl)-ethoxy]-pyridine (215) was prepared in the following manner.

### (a) 5-Bromomethyl-2-[2-(5-methyl-2-phenyl-oxazol-4-yl)-ethoxy]-pyridine (215).

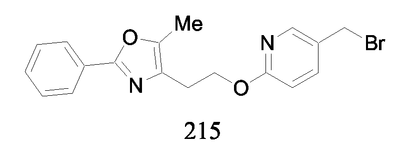

{6-[2-(5-Methyl-2-phenyl-oxazol-4-yl)-ethoxy]-pyridin-3-yl}-methanol (214, 1.50 g, 4.83 mmol) was dissolved in anhydrous CH₂Cl₂ (40 mL) and purged with dry nitrogen and cooled to 0 °C. Triphenylphosphine (1.34 g, 5.12 mmol) was added, followed by N-bromosuccinimide (0.95 g, 5.36 mmol). After 30 min, the reaction mixture was concentrated and chromatographed (5 to 25 % EtOAc in hexanes) to give 1.19g (66 %) of the bromide.

### (b) {6-[2-(5-Methyl-2-phenyl-oxazol-4-yl)-ethoxy]-pyridin-3-yl}-methanol (214).

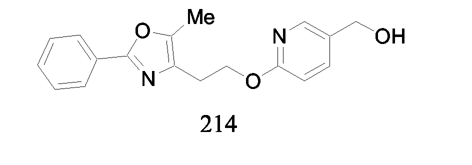

To a nitrogen-purged solution of 6-[2-(5-Methyl-2-phenyl-oxazol-4-yl)-ethoxy]-nicotinic acid methyl ester (213, 1.87 g, 5.53 mmol) in anhydrous ether (50 mL) was added lithium aluminum hydride (1.0 M in THF, 11.1 mL) over about 5 min. The bath was removed, and the reaction was allowed to stir at ambient temperature for 4 h. HCl (1.0 M) was added, and the reaction was stirred at room temperature overnight. Na₂CO₃ (5 %) was added to pH 8. The mixture was extracted with EtOAc. The combined organic extracts were washed with water and brine, dried over anhydrous Na₂SO₄, and evaporated to provide the title compound in 62 % yield.

### (c) 6-[2-(5-Methyl-2-phenyl-oxazol-4-yl)-ethoxy]-nicotinic acid methyl ester (213).

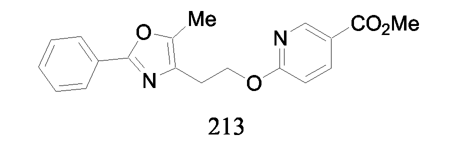

6-Hydroxynicotinic acid methyl ester (212, 3.47 g, 22.7 mmol), 2-(5-methyl-2-phenyl-oxazol-4-yl)-ethanol (5.53 g, 27.2 mmol), and triphenylphosphine (7.1 g, 27.2 mmol) were combined in anhydrous THF (100 mL), purged with dry nitrogen, and cooled to 0 °C. DEAD (5.9 g, 34.0 mmol) was added over 5 min, and the mixture was allowed to slowly warm to ambient temperature overnight. The THF was removed under reduced pressure, and the residue was partitioned between EtOAc and 5 % NaHCO₃. The organic layer wa washed with 5 % NaHCO₃, water, and brine, dried over anhydrous Na₂SO₄, and chromatographed (5 to 40 % EtOAc in hexanes) to provide 2.98 g (39 %) of the title compound.

### (d) 6-Hydroxynicotinic acid methyl ester (212).

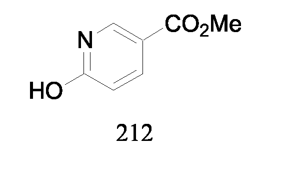

A suspension of 6-hydroxynicotinic acid (10.0 g, 71.9 mmol) in anhydrous CH₂Cl₂ (80 mL, plus 3 drops of DMF) was purged with nitrogen, cooled to 0 °C, and treated with oxalyl chloride (7.53 mL, 86.3 mmol). After 18 h at room temperature, excess methanol was cautiously added. The reaction was evaporated and chromatographed (20 % EtOAc in hexanes) to provide 2.74 g (25 %) of the title compound.

### Example 82

### 3-{6-[2-(5-Methyl-2-phenyl-xoazol-4-yl)-ethoxy]-yridin-3-yl}-2-phenylpropionic acid methyl ester (217).

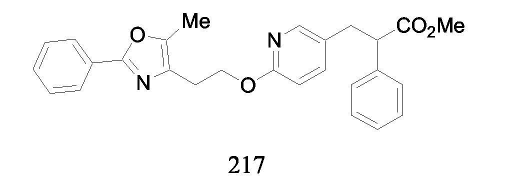

The title compound was prepared in a manner analogous to PD-341440-0000 utilizing methyl phenylacetate in 79 % yield. MS m/z 443 (M+H)⁺.

### Example 83

### 3-{6-[2-(5-Methyl-2-phenyl-xoazol-4-yl)-ethoxy]-yridin-3-yl}-2-phenylpropionic acid (218).

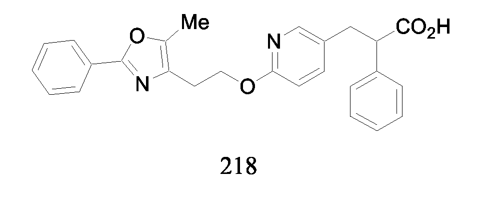

A solution of 3-{6-[2-(5-Methyl-2-phenyl-oxazol-4-yl)-ethoxy]-yridin-3-yl}-2-phenylpropionic acid methyl ester (0.52 g, 1.18 mmol) in 10 mL MeOH was treated with excess 1 M LiOH. After completion, the reaction was diluted with sat. NH₄Cl and extracted with EtOAc. The combined organic extracts were dried with brine followed by Na₂SO₄ and chromatographed (5 to 60 % EtOAc in hexanes) to provide 323 mg of the title compound. MS *m*/*z* 429 (M+H)⁺.

### Example 84

### 3-{6-[2-(5-Methyl-2-phenyl-oxazol-4-yl)-ethoxy]-pyridin-3-yl}-2-thiophen-2-yl-propionic acid (219).

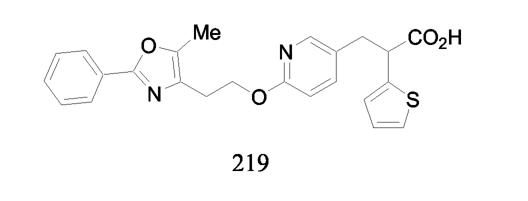

The title compound (155 mg) was prepared in a manner analogous to PD-341440-0000 utilizing methyl thiophen-2-ylacetate. MS *m*/*z* 435 (M+H)⁺.

### Example 85

### 3-{4-[2-(5-Methyl-2-phenyl-oxazol-4-yl)-ethoxy]-phenyl}-2-phenylacrylic acid (222).

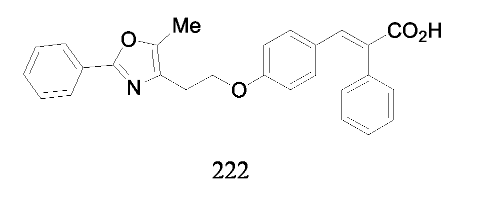

A solution of 3-{4-[2-(5-methyl-2-phenyl-oxazol-4-yl)-ethoxy]-phenyl}-2-phenyl-acrylic acid methyl ester (221, 0.31 g, 0.71 mmol) in 10 mL MeOH was treated with excess 1 M LiOH. After completion, the reaction was diluted with sat. NH₄Cl and extracted with EtOAc. The combined organic extracts were dried with brine followed by Na₂SO₄ and chromatographed (5 to 60 % EtOAc in hexanes) to provide 241 mg of the title compound. MS *m*/*z* 426 (M+H)⁺.

3-{4-[2-(5-Methyl-2-phenyl-oxazol-4-yl)-ethoxy]-phenyl}-2-phenylacrylic acid methyl ester (221) was prepared in the following manner.

### (a) 3-{4-[2-(5-Methyl-2-phenyl-oxazol-4-yl)-ethoxy]-phenyl}-2-phenyl-acrylic acid methyl ester (221)

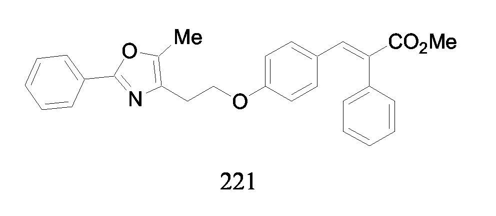

Triphenylphosphine (0.57 g, 2.17 mmol), 3-(4-hydroxyphenyl)-2-phenylacrylic acid methyl ester (220, 0.46g, 1.81 mmol), and 2-(5-methyl-2-phenyloxazol-4-yl)-ethanol (0.44 g, 2.17 mmol) were combined in THF (10 mL), purged with nitrogen, and cooled to 0 °C. DEAD (0.43 mL, 2.71 mmol) was added, and the mixture was allowed to warm to ambient temperature overnight. The reaction was diluted with EtOAc and washed with 5 % NaHCO₃, water, and brine, dried over Na₂SO₄, and chromatographed (0 to 60 % EtOAc in hexanes) to provide 0.31 g of the title compound.

### (b) 3-(4-Hydroxyphenyl)-2-phenylacrylic acid methyl ester (220).

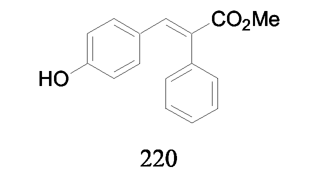

A suspension of 3-(4-hydroxyphenyl)-2-phenylacrylic acid (2.5 g, 10.4 mmol) in MeOH (50 mL) was treated with H₂SO₄ (1 mL) and refluxed 72 h. Most of the solvent was removed under reduced pressure, and the residue was diluted with EtOAc and washed with 5 % NaHCO₃, water, and brine. Chromatography (5 to 60 % EtOAc in hexanes) provided 0.46 g of the title compound as a white solid.

### Example 87

### {4-[2-(5-methyl-2-phenyl-oxazol-4-yl)-ethoxy]-benzylamino}-phenylacetic acid (223).

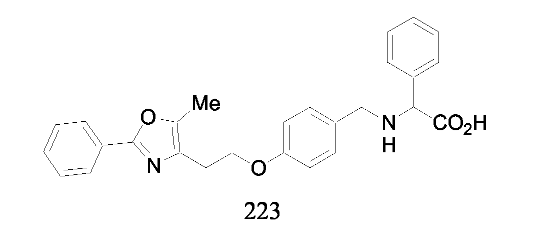

A solution of {4-[2-(5-methyl-2-phenyl-oxazol-4-yl)-ethoxy]-benzylamino}-phenylacetic acid methyl ester (0.30 g, 0.66 mmol) in 10 mL MeOH was treated with excess 1 M LiOH. After completion, the reaction was diluted with sat. NH₄Cl and extracted with EtOAc. The combined organic extracts were dried with brine followed by Na₂SO₄ and chromatographed (5 to 6 % EtOAc in hexanes) to provide the title compound in 71 % yield. MS *m*/*z* 443 (M+H)⁺.

### Example 87

### {4-[2-(5-methyl-2-phenyl-oxazol-4-yl)-ethoxy]-benzylamino}-phenylacetic acid methyl ester (226).

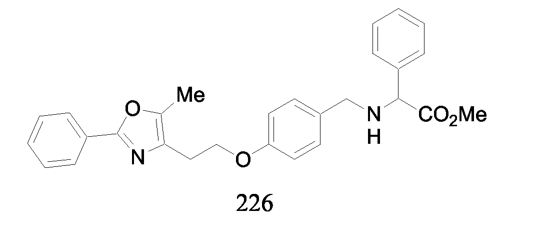

Triphenylphosphine (2.00 g, 7.65 mmol), (4-hydroxybenzylamino)-phenylacetic acid methyl ester (225, 1.73 g, 6.38 mmol), and 2-(5-methyl-2-phenyloxazol-4-yl)-ethanol (1.56 g, 7.65 mmol) were combined in THF (25 mL), purged with nitrogen, and cooled to 0 °C. DEAD (1.20 mL, 7.65 mmol) was added, and the mixture was allowed to warm to ambient temperature overnight. The reaction was diluted with EtOAc and washed with 5 % NaHCO₃, water, and brine, dried over Na₂SO₄, and chromatographed (0 to 60 % EtOAc in hexanes) to provide 1.52 g of the title compound. MS *m*/*z* 457 (M+H)⁺.

(4-Hydroxybenzylamino)-phenylacetic acid methyl ester (225) was prepared in the following manner.

### (a) (4-Hydroxybenzylamino)-phenylacetic acid methyl ester (225).

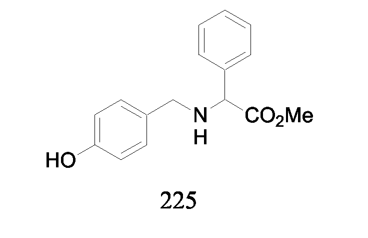

Phenylglycine methyl ester (224, 1.63 g, 9.87 mmol) and 4-hydroxybenzaldehyde (0.80 g, 6.58 mmol) were combined in 1 % HOAc in MeOH (100 mL). Sodium cyanoborohydride (0.62 g, 9.87 mmol) was added, and the mixture was allowed to stir at room temperature. After 18 hours, the reaction was concentrated under reduced pressure and partitioned between EtOAc and 5 % NaHCO₃. The organic layer was washer with water and brine, dried over Na₂SO₄, and chromatographed (0 to 50 % EtOAc in hexanes) to provide the title compound in 97 % yield.

### (b) Phenylglycine methyl ester (224).

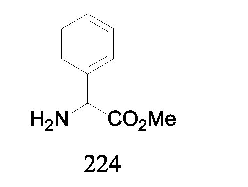

Phenylglycine (10.0 g, 66.2 mmol) was dissolved in 100 mL MeOH with 2 mL H₂SO₄ and refluxed overnight. The reaction was concentrated under reduced pressure and partitioned between ether and water. Solid NaHCO₃ was added to pH 7. The aqueous layer was extracted with ether and EtOAc. The combined organic extracts were washed with water and brine, and dried over Na₂SO₄ to provide 1.63 g of the title compound.

### Example 88

### Methyl-{4-[2-(5-methyl-2-phenyl-oxazol-4-yl)-ethoxy]-benzylamino}-phenylacetic acid (230).

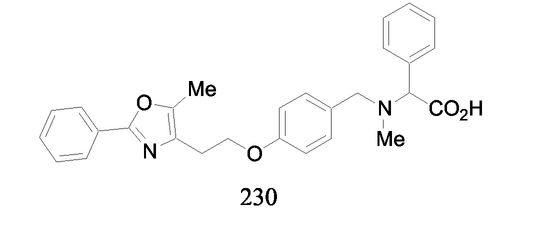

A solution of methyl-{4-[2-(5-methyl-2-phenyl-oxazol-4-yl)-ethoxy]-benzylamino}-phenylacetic acid methyl ester (229, 0.18 g, 0.38 mmol) in 5 mL MeOH was treated with excess 1 M LiOH. After completion, the reaction was diluted with sat. NH₄Cl and extracted with EtOAc. The combined organic extracts were dried with brine followed by Na₂SO₄ and chromatographed (5 to 60 % EtOAc in hexanes) to provide the title compound in 62 % yield. MS m/z 457 (M+H)⁺.

Methyl- {4-[2-(5-methyl-2-phenyl-oxazol-4-yl)-ethoxy]-benzylamino}-phenylacetic acid methyl ester (229) was prepared in the following manner.

### (a) Methyl- {4-[2-(5-methyl-2-phenyl-oxazol-4-yl)-ethoxy]-benzylamino}-phenylacetic acid methyl ester (229).

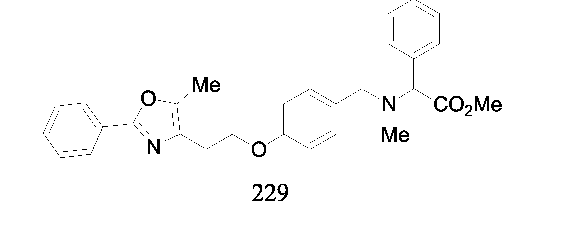

A solution of {4-[2-(5-methyl-2-phenyl-oxazol-4-yl)-ethoxy]-benzylamino}-phenylacetic acid methyl ester (228, 0.30 g, 0.66 mmol), formaldehyde (37 % in water, 0.26 mL, 3.10 mmol), and HOAc (0.056 mL, 0.99 mmol) in CH₃CN (5 mL) was treated with sodium cyanoborohydride (45 mg, 0.72 mmol). After 18 h, the product was purified as in the preparation of (4-hydroxybenzylamino)-phenylacetic acid methyl ester to give the title compound in 65 % yield. MS *mlz* 471 (M+H)⁺.

### (b) Acetyl- {4-[2-(5-methyl-2-phenyl-oxazol-4-yl)-ethoxy]-benzylamino}-phenylacetic acid (228).

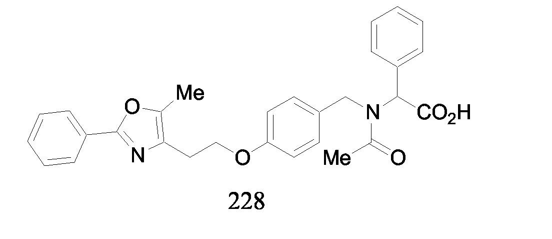

A solution of acetyl-{4-[2-(5-methyl-2-phenyl-oxazol-4-yl)-ethoxy]-benzylamino}-phenylacetic acid methyl ester (227, 0.25 g, 0.50 mmol) in 5 mL MeOH was treated with excess 1 M LiOH. After completion, the reaction was diluted with sat. NH₄Cl and extracted with EtOAc. The combined organic extracts were dried with brine followed by Na₂SO₄ and chromatographed (5 to 60 % EtOAc in hexanes) to provide the title compound in 55 % yield. MS *m*/*z* 485 (M+H)⁺.

### (c) Acetyl-{4-[2-(5-methyl-2-phenyl-oxazol-4-yl)-ethoxy]-benzylamino}-phenylacetic acid methyl ester (227).

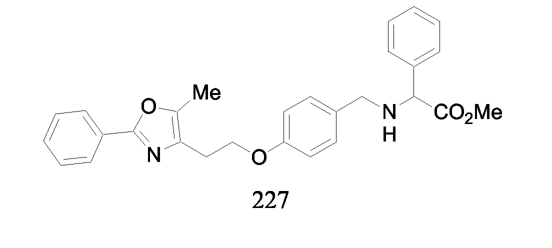

A solution of {4-[2-(5-methyl-2-phenyl-oxazol-4-yl)-ethoxy]-benzylamino}-phenylacetic acid methyl ester (0.30 g, 0.66 mmol) and triethylamine (0.10 mL, 0.72 mmol) in CH₂Cl₂ (5 mL) was treated with acetyl chloride (0.050 mL, 0.69 mmol). After 18 h, the reaction mixture was evaporated and chromatographed (5 to 20 % EtOAc in hexanes) to give the title compound in 85 % yield. MS *m*/*z* 499 (M+H)⁺.

### Example 89

### 6-[2-(5-Methyl-2-phenyl-oxazol-4-yl)-ethoxy]-naphthalene-2-carboxylic acid (231).

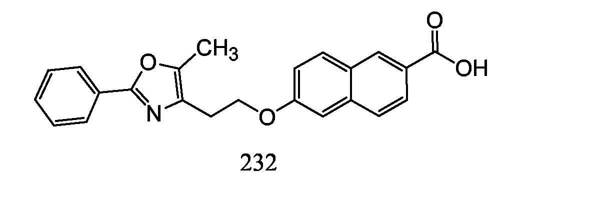

A solution of 6-[2-(5-Methyl-2-phenyl-oxazol-4-yl)-ethoxy]-naphthalene-2-carboxylic acid methyl ester (231) in MeOH (5 mL) was treated with excess LiOH (1.0 M) and stirred at ambient temperature. After completion, the reaction was diluted with sat. NH₄Cl and extracted with EtOAc. The combined organic extracts were dried with brine followed by Na₂SO₄ and chromatographed (5 to 60 % EtOAc in hexanes) to provide 131 mg of the title compound. MS *m*/*z* 374 (M+H)⁺.

6-[2-(5-Methyl-2-phenyl-oxazol-4-yl)-ethoxy]-naphthalene-2-carboxylic acid methyl ester (231) was prepared in the following manner.

### (a) 6-[2-(5-Methyl-2-phenyl-oxazol-4-yl)-ethoxy]-naphthalene-2-carboxylic acid methyl ester (231).

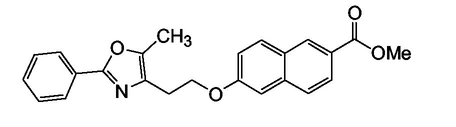

Triphenylphosphine (1.65 g, 6.29 mmol), 6-hydroxynaphthalene-2-carboxylic acid methyl ester (1.06 g, 5.24 mmol), and 2-(5-methyl-2-phenyloxazol-4-yl)-ethanol (1.28 g, 6.29 mmol) were combined in THF (25 mL), purged with nitrogen, and cooled to 0 °C. DEAD (1.00 mL, 6.29 mmol) was added, and the mixture was allowed to warm to ambient temperature overnight. The reaction was diluted with EtOAc and washed with 5 % NaHCO₃, water, and brine, dried over Na₂SO₄, and chromatographed (0 to 60 % EtOAc in hexanes) to provide 0.58 g of the title compound.

### Example 90

### 3-{5-[3-(5-Methyl-2-phenyl-oxazol-4-yl)-propyl]-naphthalen-1-yl}-2-pyrrol-1-yl-propionic acid (237).

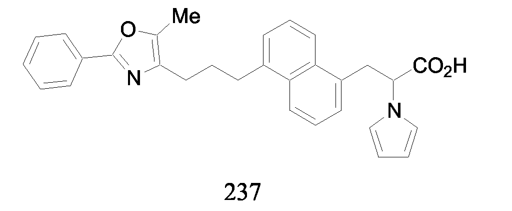

A solution of 3-{5-[3-(5-methyl-2-phenyl-oxazol-4-yl)-propyl]-naphthalen-1-yl}-2-pyrrol-1-yl-propionic acid methyl ester (236, 0.15 g, 0.31 mmol) in methanol was treated with excess LiOH (1 M in water). After stirring at ambient temperature overnight, the reaction mixture was diluted with water and washed with ether. The aqueous layer was acidified with 1 M HCl and extracted with EtOAc. The combined extracts were washed with brine, dried over Na₂SO₄, and evaporated to provide 84 mg (56 %) of the title compound.

3-{5-[3-(5-methyl-2-phenyl-oxazol-4-yl)-propyl]-naphthalen-l-yl}-2-pyrrol-1-yl-propionic acid methyl ester (236) was prepared in the following manner.

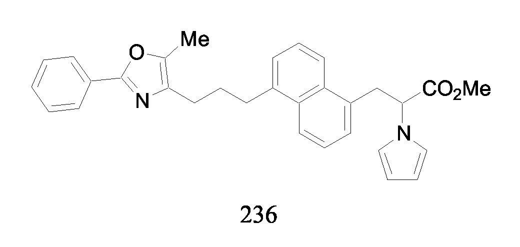

### (a) 3-{5-[3-(5-methyl-2-phenyl-oxazol-4-yl)-propyl]-naphthalen-1-yl}-2-pyrrol-1-yl-propionic acid methyl ester (236).

A solution of 4-allyl-5-methyl-2-phenyl-oxazole (0.38 g, 1.89 mmol) was purged with nitrogen, cooled to 0 °C, and treated with 9-borabicyclo[3.3.1]nonane (0.5 M in THF, 7.6 mL). The reaction was allowed to slowly warm to ambient temperature overnight. The 9-BBN adduct solution was transferred to a purged mixture of 3-(5-bromo-naphthalen-1-yl)-2-pyrrol-1-yl-propionic acid methyl ester (235, 0.52 g, 1.45 mmol), [1,1'-bis(diphenylphosphino)ferrocene]dichloro palladium(II) complex with dichloromethane (1:1) (0.10 g, 0.15 mmol), cesium carbonate (1.32 g, 4.06 mmol), triphenylarsine (44 mg, 0.15 mmol), water (0.31 mL, 17.4 mmol), and DMF (4 mL). The reaction stirred at room temperature 24 h before cooling to 0 °C and adding NaOAc (3.0 M, 6 mL) and H₂O₂ (30 %, 3 mL). After 2 h, the mixture was diluted with water and extracted with a 7:1 mixture of ether/EtOAc. The combined organic extracts were washed with water and brine, dried over anhydrous Na₂SO₄, evaporated, and flashed (5 to 50 % EtOAc in hexanes) to give 0.15 g (22 %) of the title compound.

### (b) 3-(5-bromo-naphthalen-1-yl)-2-pyrrol-1-yl-propionic acid methyl ester (235).

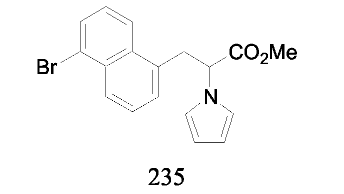

A solution of 1-bromo-5-bromomethyl-naphthalene (234, 1.05 g, 3.50 mmol) and pyrrole-1-yl-acetic acid methyl ester (0.97 g, 7.00 mmol) in anhydrous THF (20 mL) was purged with nitrogen and treated with LiN(TMS)₂ (1.0 M, 7.0 mL). After about 30 min at ambient temperature, the reaction was heated to 50 °C for 48 h. The reaction mixture was concentrated and chromatographed (5 to 50 % EtOAc in hexanes) to provide 0.80 g (64 %) of the title compound.

### (c) 1-bromo-5-bromomethyl-naphthalene (234).

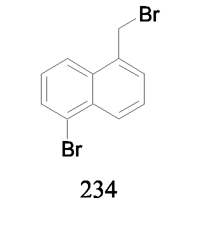

A solution of (5-bromo-naphthalen-1-yl)-methanol (233, 1.13 g, 4.77 mmol) in anhydrous CH₂Cl₂ (20 mL) was purged with nitrogen and cooled to 0 °C. Triphenylphosphine (1.33 g, 5.05 mmol) was added followed by NBS (0.94 g, 5.29 mmol). After 30 min at 0 °C, the mixture was concentrated and chromatographed twice (5 to 25 % EtOAc in hexanes) to give 1.05 g (73 %) of the title compound.

### (d) (5-bromo-naphthalen-1-yl)-methanol (233).

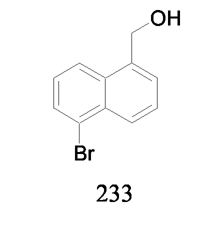

A solution of 5-bromonaphthalene-1-carboxylic acid (2.50 g, 9.96 mmol, PD-59348, Lot P) in anhydrous THF (100 mL) was purged with nitrogen. Borane (1.0 M in THF, 20 mL) was added, and the reaction was refluxed for 6 h. The reaction was cooled to ambient temperature, treated with 10 mL MeOH, and stirred at ambient temperature overnight. The mixture was concentrated, dissolved in ether, washed with 1 M NaOH, water, and brine., dried over anhydrous Na₂SO₄. The crude product was purified by crystallization from CH₂Cl₂/hexanes to provide 1.5 g (64 %) of the title compound.

### Example 91

### 3-{4-[3-(5-Methyl-2-phenyl-oxazol-4-yl)-propyl]-phenyl}-2-1,2,3-triazol-1-yl-propionic acid (242).

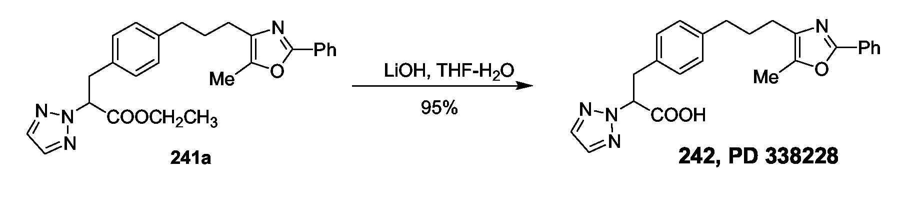

GENERAL METHOD 91. 3-{4-[3-(5-Methyl-2-phenyl-oxazol-4-yl)-propyl]-phenyl}-2-1,2,3-triazol-2-yl-propionic acid ethyl ester (241a) (2.37 g, 5.331 mmol) was dissolved in THF (60 mL) and water was added (15 mL). Lithium hydroxide monohydrate (0.335 g, 8.00 mmol) was incorporated and the mixture was stirred at room temperature for 1 h. The organic solvent was removed in the rotary evaporator at room temperature. The aqueous residue was diluted with water and extracted with Et₂O (2 x 45 mL). A flow of air was passed through the aqueous phase to remove residual ether. The aqueous phase was then acidified with 10% HC1. The precipitated solid was separated by vacuum filtration and washed with water. The solid was dried on air overnight and then at 45 °C for 14 h to give the title compound as an off-white solid (2.11 g, 95%): mp (softens or melts between 50-85 °C, forms another solid that melts at 153-153.5 °C); ¹H NMR (CDCl₃, 400 MHz) δ 7.98-7.96 (m, 2 H), 7.62 (s, 2 H), 7.43-7.40 (m, 3 H), 7.03 (d, *J*= 8.1 Hz, 2 H), 6.98 (d, *J*= 8.1 Hz, 2 H), 5.52 (dd, *J*= 10.4 and 5.0 Hz, 1 H), 3.65-3.49 (m, 2 H), 2.58 (t, *J*= 7.6 Hz, 2 H), 2.48 (t, *J*= 7.6 Hz, 2 H), 2.27 (s, 3 H), 1.93 (m, *J*= 7.6 Hz, 2 H); CIMS *m*/*z* 417 (M+1). HPLC: purity: 100%; column: symmetry C₁₈, 4.6 x 150 mm, 5µM; mobile phase: A: water + 0.1% TFA, B: acetonitrile + 0.1% TFA; retention time: 15.914 min; wavelength: 254 nm.

3-{4-[3-(5-Methyl-2-phenyl-oxazol-4-yl)-propyl]-phenyl}-2-1,2,3-triazol-2-yl-propionic acid ethyl ester (241a) was prepared in the following manner.

### (a) 3-{4-[3-(5-Methyl-2-phenyl-oxazol-4-yl)-propyl]-phenyl}-2-1,2,3-triazol-2-yl-propionic acid ethyl ester (241a).

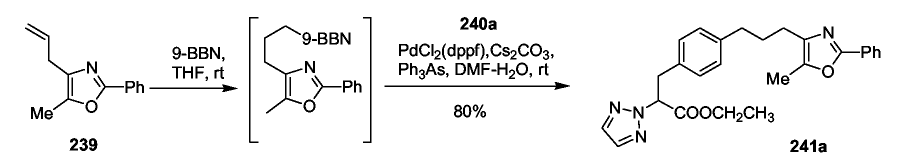

GENERAL METHOD 92-a. A solution of 5-methyl-2-phenyl-4-prop-2-enyloxazole (compound 239) (1.716 g, 8.614 mmol) in dry THF (25 mL) was added to a 0.5 M solution of 9-BBN in THF (34.45 mL) at 0 °C under a nitrogen atmosphere. The ice bath was removed and the mixture was stirred at room temperature overnight. The 9-BBN adduct was then added to a flask containing bromide 238a (2.148 g, 6.626 mmol), PdCl₂(dppf) (0.485 g, 0.662 mmol), Cs₂CO₃ (3.88 g, 11.927 mmol), Ph₃As (0.203 g, 0.662 mmol), water (1.4 mL, 79.5 mmol) and DMF (15 mL). The reaction mixture was stirred at room temperature under a nitrogen atmosphere for 2 days. At the end of this time, the mixture was cooled in an ice bath and 3 M NaOAc (16 mL) was added followed by 30% H₂O₂ (8 mL). Stirring was continued for 2 h, allowing the reaction to warm to room temperature slowly. Water (100 mL) was added followed by Et₂O and EtOAc. The phases were separated and the aqueous phase was extracted with Et₂O-EtOAc (60:10 mL x 4). The combined organic extracts were washed with brine, dried over magnesium sulfate and the solvent removed. Purification by column chromatography on silica gel eluting with EtOAc in hexanes (0 to 22%) afforded ester 241a as a thick oil (2.37 g, 80%): ¹H NMR (CDCl₃, 400 MHz) δ 7.98-7.95 (m, 2 H), 7.61 (s, 2 H), 7.44-7.38 (m, 3 H), 7.03 (d, *J*= 8.3 Hz, 2 H), 7.00 (d, *J*= 8.3 Hz, 2 H), 5.50 (dd, *J*= 10.2 and 5.6 Hz, 1 H), 4.18 (q, *J*= 7.1 Hz, 2 H), 3.71-3.57 (m, 2 H), 2.58 (t, *J*= 7.6 Hz, 2 H), 2.46 (t, *J*= 7.6 Hz, 2 H), 2.24 (s, 3 H), 1.93 (m, *J*= 7.6 Hz, 2 H), 1.19 (t, *J*= 7.1 Hz, 3 H); CIMS *m*/*z* 445 (M+1).

### (b) Bromide 240a (PD 0341554).

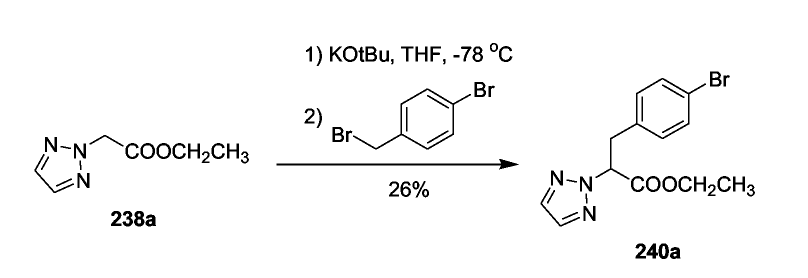

General Method 91-b. Ester 238a (3.0 g, 19.336 mmol) was dissolved in dry THF (60 mL) and cooled to -78°C under a nitrogen atmosphere. A 1.0 M solution of potassium *tert*-butoxide in THF (20.3 mL, 20.3 mmol) was added. The mixture was stirred at -78 °C for 45 min. A solution of 4-bromobenzyl bromide (5.56 g, 22.236 mmol) in THF (20 mL) was added. The reaction was allowed to reach room temperature slowly and then stirred at room temperature for 6 days. The mixture was quenched with water (60 mL) and diluted with Et₂O and EtOAc. The phases were separated and the aqueous phase was extracted with Et₂O-EtOAc (50:5 mL x 3). The combined organic extracts were washed with brine, dried over magnesium sulfate and the solvent removed. Purification by column chromatography on silica gel eluting with EtOAc in hexanes (0 to 16%) afforded the title compound as an oil (1.668 g, 26%): ¹H NMR (CDCl₃, 400 MHz) δ 7.62 (s, 2 H), 7.32 (d, *J=* 8.5 Hz, 2 H), 6.97 (d, *J=* 8.5 Hz, 2 H), 5.48 (dd, *J*= 10.4 and 5.4 Hz, 1 H), 4.19 (q, *J=* 7.1 Hz, 2 H), 3.70-3.55 (m, 2 H), 1.20 (t, *J*= 7.1 Hz, 3 H); CIMS *m*/*z* 324 (M)⁺.

### (c) 2-(2H-1,2,3-Triazol-2-yl)ethyl acetate (238a).

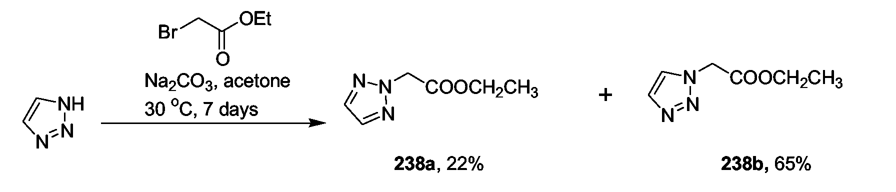

The title compound was prepared following the procedure described by Kume et. al.(Kume, M.; Kubota, T.; Kimura, Y.; Nakashimizu, H.; Motokawa, K.; Nakano, M. *J. Antibiotics* **1993,** *46,* 177-192.) Following this procedure, starting from 1*H*-1,2,3-triazole (18.17 g, 0.263 mol), ester 238a was obtained as a liquid (9.2 g, 22%) after column chromatography on silica gel eluting with EtOAc in hexanes (0 to 55%): ¹H NMR (CDCl₃, 400 MHz) δ 7.68 (s, 2 H), 5.23 (s, 2 H), 4.24 (q, *J*= 7.1 Hz, 2 H), 1.27 (t, *J*= 7.1 Hz, 3 H); CIMS *m*/*z* 156 (M+1).

From the same purification, the *N*-1 alkylated product 23 8b was obtained as the major product (26.45 g, 65%): ¹H NMR (CDCl₃, 400 MHz) δ 7.75 (d, *J*= 1.0 Hz, 1 H), 7.71 (d, *J*= 1.0 Hz, 1 H), 5.19 (s, 2 H), 4.25 (q, *J*= 7.1 Hz, 2 H), 1.28 (t, *J*= 7.1 Hz, 3 H); CIMS *m*/*z* 156 (M+1).

### Example 92

### 3-{4-[3-(5-Methyl-2-phenyl-oxazol-4-yl)-propyl]-phenyl}-2-1,2,3-triazol-1-yl-propionic acid (245).

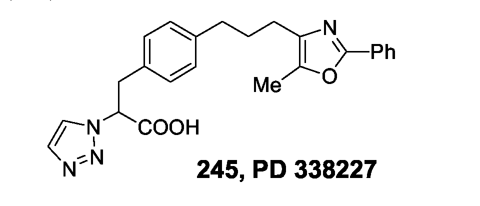

Prepared from ester 244 (1.0 g, 2.249 mmol) following General Method 92. The title compound was obtained as white solid (0.854 g, 91%): mp 162-163.5 °C; ¹H NMR (CDCl₃, 400 MHz) δ 7.93 (m, 2 H), 7.66 (s, 1 H), 7.63 (s, 1 H), 7.41 (m, 3 H), 7.01 (d, J = 8.0 Hz, 2 H), 6.92 (d, J = 8.0 Hz, 2 H), 5.62 (dd, J = 8.3 and 6.1 Hz, 1 H), 3.46-3.35 (m, 2 H), 2.56 (t, J = 7.3 Hz, 2 H), 2.48 (t, J = 7.3 Hz, 2 H), 2.28 (s, 3 H), 1.89 (m, 2 H); CIMS *m*/*z* 417 (M+1).

3-{4-[3-(5-Methyl-2-phenyl-oxazol-4-yl)-propyl]-phenyl}-2-1,2,3-triazol-1-yl-propionic acid methyl ester (244) was prepared in the following manner.

### (a) 3-{4-[3-(5-Methyl-2-phenyl-oxazol-4-yl)-propyl]-phenyl}-2-1,2,3-triazol-1-yl-propionic acid methyl ester (244).

The title compound was prepared from bromide 243 (1.2 g, 3.702 mmol) using the General Method 92a. Ester 244b was obtained as a thick oil (1.00 g, 61%): ¹H NMR (CDCl₃, 400 MHz) δ 7.98 (m, 2 H), 7.66 (s, 1 H), 7.63 (s, 1 H), 7.44-7.38 (m, 3 H), 7.06 (d, J = 8.0 Hz, 2 H), 6.93 (d, J = 8.0 Hz, 2 H), 5.59 (dd, J = 8.3 and 6.6 Hz, 1 H), 4.19 (q, *J* = 7.3 Hz, 2 H), 3.49-3.38 (m, 2 H), 2.61 (t, J = 7.6 Hz, 2 H), 2.48 (t, J = 7.3 Hz, 2 H), 2.27 (s, 3 H), 1.96 (m, 2 H), 1.21 (t, J = 7.1 Hz, 3 H); CIMS *m*/*z* 445 (M+1).

### (b) Bromide 243

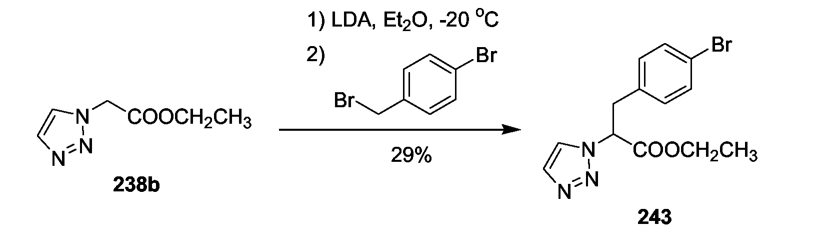

A solution of diisopropylamine (2.0 mL, 14.18 mmol) in Et₂O (30 mL) was cooled at -20 °C under a nitrogen atmosphere. A 1.6 M solution of BuLi in hexanes (9.7 mL, 15.47 mmol) was added. The mixture was stirred for 25 min. A solution of ester 238b (2.0 g, 12.89 mmol) in Et₂O (20 mL) was added. The mixture was stirred for 30 min. A solution of 4-bromobenzylbromide (3.70 g, 14.82 mmol) in Et₂O (20 mL) was incorporated. The reaction mixture was kept at -20 °C for 2.5 h and then allowed to warm to room temperature slowly. Stirring was continued at room temperature for 24 h. At this time, the mixture was quenched with water and diluted with Et₂O. The phases were separated and the aqueous layer was extracted with Et₂O (3 x 40 mL). The combined organic extracts were washed with brine, dried over magnesium sulfate and the solvent removed. Purification by column chromatography over silica gel afforded bromide 243 as a pale yellow oil (1.21 g, 29%): ¹H NMR (CDCl₃, 400 MHz) δ 7.67 (d, J = 0.7 Hz, 1 H), 7.63 (d, J = 1.0 Hz, 1 H), 7.35 (d, J = 8.5 Hz, 2 H), 6.89 (d, J = 8.5 Hz, 2 H), 5.54 (dd, J = 8.7 and 6.5 Hz, 1 H), 4.21 (q, J = 7.1 Hz, 2 H), 3.51-3.39 (m, 2 H), 1.22 (t, J = 7.3 Hz, 3 H); CIMS *m*/*z* 324 (M)⁺.

### Example 93

### 3-{4-[3-(5-Methyl-2-phenyl-oxazol-4-yl)-propyl]-phenyl}-2-pyrazol-1-yl-propionic acid (249).

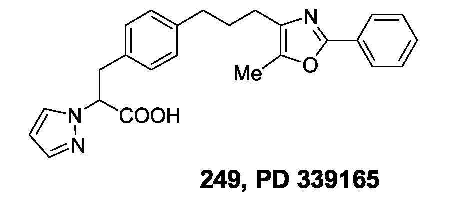

The title compound was prepared from 248 (0.735 g, 1.657 mmol) by the General Method 92. PD 0339165 was obtained as a yellowish solid (0.602 g, 87%): mp 75-77 °C; ¹H NMR (CDCl₃, 400 MHz) δ 7.96 (m, 2 H), 7.61 (d, J = 2.0 Hz, 1 H), 7.40 (m, 3 H), 7.09 (d, J = 2.0 Hz, 1 H), 7.02 (d, J = 8.0 Hz, 2 H), 6.80 (d, J = 8.0 Hz, 2 H), 6.19 (t, J = 2.2 Hz, 1 H), 5.02 (dd, J = 10.0 and 4.6 Hz, 1 H), 3.45-3.27 (m, 2 H), 2.59 (t, J = 7.6 Hz, 2 H), 2.47 (t, J = 7.6 Hz, 2 H), 2.27 (s, 3 H), 1.93 (m, 2 H); CIMS *m*/*z* 416 (M+1). Anal. calcd for C₂₅H₂₅N₃O₃·0.4 H₂O: C, 71.04; H, 6.15; N, 9.94. Found: C, 70.76; H, 6.08; N, 9.91.

3-{4-[3-(5-Methyl-2-phenyl-oxazol-4-yl)-propyl]-phenyl}-2-pyrazol-1-yl-propionic acid ethyl ester (248) was prepared in the following manner.

### (a) 3-{4-[3-(5-Methyl-2-phenyl-oxazol-4-yl)-propyl]-phenyl}-2-pyrazol-1-yl-propionic acid ethyl ester (4c).

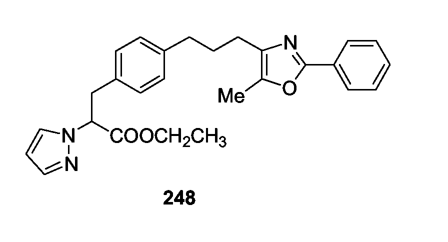

Prepared from bromide 247 (0.760 g, 2.351 mmol) by the General Method 95. Purification by column chromatography afforded 248 (0.735 g, 70%): ¹H NMR (CDCl₃, 400 MHz) δ 7.96 (m, 2 H), 7.52 (d, J = 1.5 Hz, 1 H), 7.44-7.38 (m, 4 H), 7.05 (d, J = 8.0 Hz, 2 H), 6.94 (d, J = 8.0 Hz, 2 H), 6.22 (t, J = 2.1 Hz, 1 H), 5.11 (t, J = 7.7 Hz, 1 H), 4.16 (q, J = 7.1 Hz, 2 H), 2.60 (t, J = 7.6 Hz, 2 H), 2.47 (t, J = 7.3 Hz, 2 H), 2.26 (s, 3 H), 1.95 (m, 2 H), 1.18 (t, J = 7.1 Hz, 3 H); CIMS *m*/*z* 444 (M+1).

### (b) Pyrazol-1-yl acetic acid ethyl ester (247).

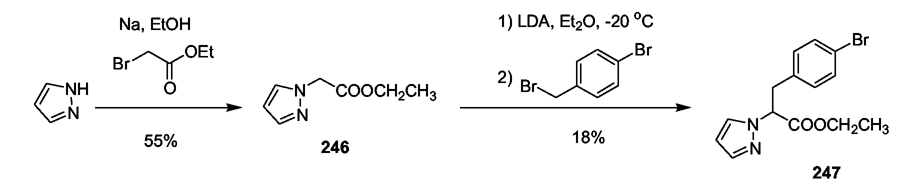

Sodium (3.7 g, 161 mmol) was dissolved in ethanol (150 mL) with ice cooling. Pyrazole (10 g, 146 mmol) was added. Ethyl bromoacetate (32 mL, 292 mmol) was added dropwise. The ice bath was removed after the addition was finished and the mixture stirred at room temperature for 5 days. The solvent was removed and the residue diluted with cold 6 N HCl and extracted with Et₂O (2 x 50 mL). The aqueous layer was neutralized with solid sodium carbonate, then it was extracted with chloroform. The combined organic extracts were washed with brine, dried over magnesium sulfate and the solvent removed. Purification by distillation afforded 247 as an oil (12.495 g, 55%): bp 65-80 °C at 2 mm Hg; ¹H NMR (CDCl₃, 400 MHz) δ 7.55 (d, *J* = 1.7 Hz, 1 H), 7.47 (d, *J* = 2.2 Hz, 1 H), 6.32 (t, *J* = 2.0 Hz, 1 H), 4.92 (s, 2 H), 4.22 (q, *J* = 7.1 Hz, 2 H), 1.27 (t, *J* = 7.1 Hz, 3 H); CIMS *m*/*z* 155 (M+1).

**Bromide 246.** The title compound was prepared from ester 246 (2.0 g, 12.97 mmol) by General Method 92-b. Bromide 247 was obtained as a thick oil (0.768 g, 18%): ¹H NMR (CDCl₃, 400 MHz) δ 7.53 (d, *J*= 1.7 Hz, 1 H), 7.36 (d, *J* = 2.0 Hz, 1 H), 7.33 (d, *J*= 8.5 Hz, 2 H), 6.88 (d, *J*= 8.3 Hz, 2 H), 5.07-5.03 (m, 1 H), 4.18 (q, *J*= 7.0 Hz, 2 H), 3.49-3.39 (m, 2 H), 1.20 (t, *J*= 7.1 Hz, 3 H); CIMS *m*/*z* 323 (M)⁺.

### Example 94

### 3-{4-[3-(5-Methyl-2-phenyl-oxazol-4-yl)-propyl]-phenyl}-2-1,2,4-triazol-1-yl-propionic acid (253).

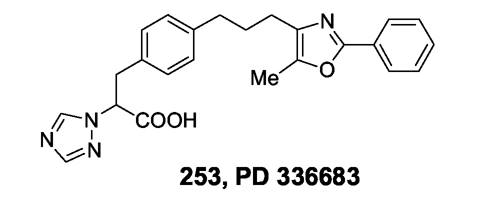

Prepared from ester 252 (0.492 g, 1.106 mmol) by the General Method 92. The title compound was obtained as a white solid (0.33 g, 72%): mp 169-171 °C; ¹H NMR (CDCl₃, 400 MHz) δ 8.16 (s, 1 H), 8.00 (m, 2 H), 7.98 (s, 1 H), 7.45-7.40 (m,3 H), 7.01 (d, J = 8.0 Hz, 2 H), 6.87 (d, J = 8.0 Hz, 2 H), 5.24 (t, J = 7.1 Hz, 1 H), 3.42 (d, J = 7.1 Hz, 2 H), 2.57 (t, J = 7.6 Hz, 2 H), 2.52 (t, J = 7.6 Hz, 2 H), 2.28 (s, 3 H), 1.93 (m, 2 H); CIMS *m*/*z* 417 (M+1).

3-{4-[3-(5-Methyl-2-phenyl-oxazol-4-yl)-propyl]-phenyl}-2-1,2,4-triazol-1-yl-propionic acid ethyl ester (252) was prepared in the following manner.

### (a) 3-{4-[3-(5-Methyl-2-phenyl-oxazol-4-yl)-propyl]-phenyl}-2-1,2,4-triazol-1-yl-propionic acid ethyl ester (252).

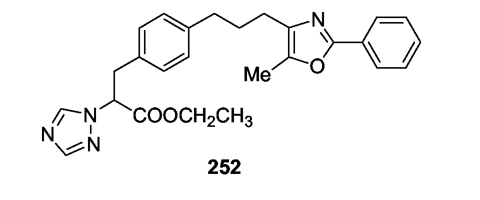

Prepared from bromide 251 (0.440 g, 1.357 mmol) by the General Method 95-a. The title compound was obtained as a thick oil (0.492 g, 82%): ¹H NMR (CDCl₃, 400 MHz) δ 8.08 (m, 2 H), 8.00 (s, 1 H), 7.94 (s, 1 H), 7.48-7.41 (m, 3 H), 7.06 (d, J = 8.0 Hz, 2 H), 6.88 (d, J = 8.0 Hz, 2 H), 5.17 (dd, J = 8.0 and 6.6 Hz, 1 H), 4.21 (q, J = 7.1 Hz, 2 H), 3.43 (d, J = 7.3 Hz, 2 H), 2.62 (t, J = 7.6 Hz, 2 H), 2.53 (d, J = 7.3 Hz, 2 H), 2.28 (s, 3 H), 1.23 (t, J = 7.1 Hz, 3 H); CIMS *m*/*z* 445 (M+1).

### (b) Ethyl 2-1,2,4-Triazol-1-yl acetate (250) and Bromide (251).

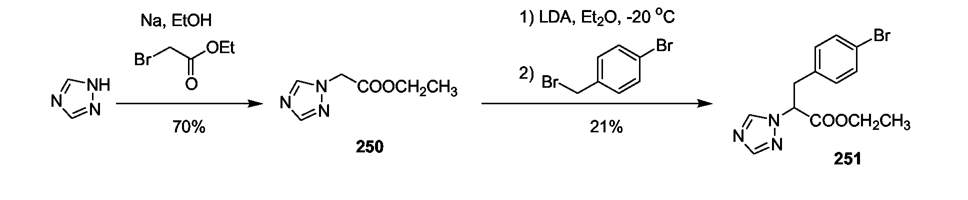

Ester 250 was prepared following the procedure described by Ainsworth and Jones (Ainsworth, C.; Jones, R. G. *J. Am. Chem. Soc.* **1955,** 77, 621-624). ¹H NMR (CDCl₃, 400 MHz) δ 8.19 (s, 1 H), 7.97 (s, 1 H), 4.96 (s, 2 H), 4.25 (q, J = 7.1 Hz, 2 H), 1.28 (t, J = 7.1 Hz, 3 H); CIMS *m*/*z* 156 (M+1).

**Bromide 251.** The title compound was prepared from ester 250 (1.0 g, 6.445 mmol) following the General Method III. Bromide 251 was obtained as a thick oil (0.447 g, 21%): ¹H NMR (CDCl₃, 400 MHz) δ 8.10 (s, 1 H), 7.98 (s, 1 H), 7.35 (d, *J* = 8.3 Hz, 2 H), 6.86 (d, *J* = 8.5 Hz, 2 H), 5.16 (dd, *J* = 8.1 and 6.7 Hz, 1 H), 4.22 (q, J = 7.1 Hz, 2 H), 3.46 (d, J 7.6 Hz, 2 H), 1.23 (t, J = 7.1 Hz, 3 H); CIMS *m*/*z* 324 (M)⁺.

### Example 95

### 5-Methyl-2-phenyl-4-prop-2-enyloxazole (239).

Amide 256 (2.00 g, 9.205 mmol) was dissolved in TFA (16 mL) and TFAA (8 mL) was added. The mixture was heated at 35-40 °C for 16 h. The mixture was allowed to cool and the solvents removed under reduced pressure. The residue was diluted with saturated NaHCO₃ (50 mL) and solid NaHCO₃ was added to neutralize the mixture. It then was extracted with ethyl acetate (3 x 60 mL). The combined organic extracts were washed with brine, dried over MgSO₄, filtered and the solvent removed. Purification by flash chromatography on silica gel eluting with hexanes:ethyl acetate (15:1) gave oxazole 239 (1.75 g, 95%): ¹H NMR (CDCl₃, 400 MHz) δ 7.98 (d, *J=* 7.8 Hz, 2 H), 7.43-7.35 (m, 3 H), 6.03-5.93 (m, 1 H), 5.13 (dq, *J=* 16.9 and 1.7 Hz, 1 H), 5.09 (dq, *J=* 10.0 and 1.5 Hz, 1 H), 3.29 (d, *J=* 6.3 Hz, 2 H), 2.32 (s, 3 H); CIMS *m*/*z* 200 (M + H)⁺; IR 3070, 2924, 1638, 1450 cm⁻¹.

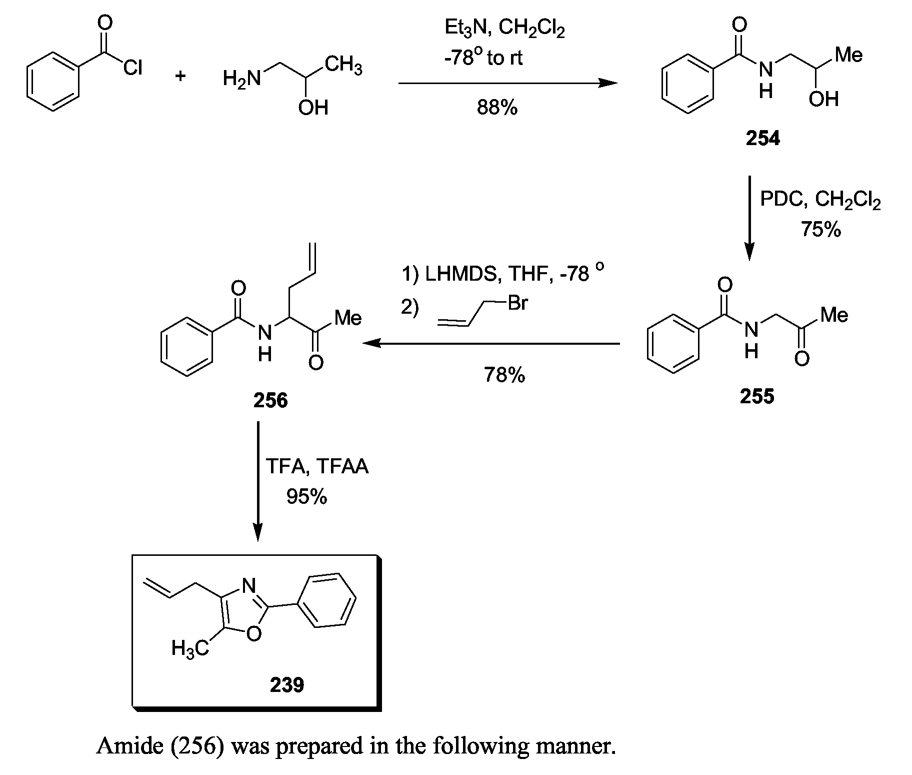

### (a) N-(1-Acetylbut-3-enyl)benzamide (256).

Amide 255 (2.098 g, 11.839 mmol) was dissolved in THF (120 mL) and cooled to -78 °C under nitrogen. A 1.0 M solution of LHMDS in THF (11.9 mL, 11.9 mmol) was added and the mixture stirred for 40 min. A solution of allyl bromide (1.33 mL, 15.39 mmol) in THF (10 mL) was added. The mixture was allowed to warm to room temperature and stirred overnight. The mixture was diluted with brine and the phases were separated. The aqueous phase was extracted with ethyl acetate (3 x 50 mL) and the combined organic extracts were dried over MgSO₄, filtered, and the solvent removed. Purification by flash chromatography on silica gel eluting with hexanes:ethyl acetate (2:1 1 to 1:1) gave amide 256 (2.02 g, 78%): ¹H NMR (CDCl₃, 400 MHz) δ 7.79 (d, *J*= 7.1 Hz, 2 H), 7.51-7.41 (m, 3 H), 6.95 (bd, *J*= 5.4 Hz, 1 H), 5.74-5.64 (m, 1 H), 5.17-5.12 (m, 2 H), 4.88 (dt, *J*= 6.8 and 5.4 Hz, 1 H), 2.85-2.78 (m, 1 H), 2.61-2.54 (m, 1 H), 2.28 (s, 3 H); CIMS *mlz* 218 (M + H)⁺; IR 3263, 3081, 1719, 1632, 1556 cm⁻¹. Anal. calcd for C₁₃H₁₅NO₂: C, 71.87; H, 6.96; N, 6.45. Found: C, 71.91; H, 7.03; N, 6.52.

### (b) Amide (255).

According to the method of Ellinger, L. P.; Goldberg, A. A. *J*. *Chem. Soc.* **1949**, 263. Alcohol 254 (3.0 g, 16.7 mmol) was dissolved in CH₂Cl₂ (60 mL) and PDC (9.42 g, 25.0 mmol) was added. The mixture was stirred at room temperature for 24 and more PDC (9.42 g, 25.0 mmol) was added. Stirring was continued for 24 h. The mixture was diluted with ethyl acetate and filtered through a celite pad. The residue was then passed through a shoroom temperature silica gel column eluting with ethyl acetate. The solvent was removed and the residue was purified by flash chromatography on silica gel eluting with hexanes:ethyl acetate (1:1) containing MeOH (0 to 4%). This purification afforded amide 255 as a white solid (2.21 g, 75%): ¹H NMR (CDCl₃, 400 MHz) δ 7.76 (dd, *J*= 7.0 and 1.5 Hz, 2 H), 7.48-7.36 (m, 3 H), 6.96 (bs, 1 H), 4.30 (d, *J*= 4.6 Hz, 2 H), 2.21 (s, 3 H); CIMS *m*/*z* 178 (M + H)⁺.

### (b-1) An alternative route to benzamidoacetone 255 is shown below.

This route eliminates the PDC oxidation that is tedious to work up.

This method does not require chromatographic purification and affords amide 6 in 63% for the two steps.

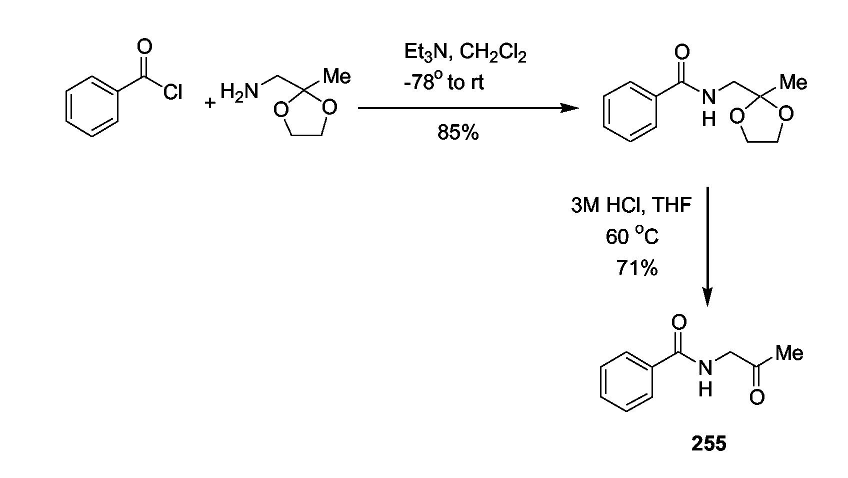

### (c) N-(2-Hydroxypropyl)benzamide (254).

The title compound was prepared according to the method of Arai, K.; Tamura, S.; Masumizu, T.; Kawai, K.-I.; Nakajima, S.; Ueda, A. *Can. J. Chem*. **1990;***68*:903-907. A solution of DL-1-amino-2-propanol (3.4 mL, 43.2 mmol) and triethylamine (16.4 mL, 117.9 mmol) in CH₂Cl₂ (60 mL) under nitrogen was cooled at -78 °C. Benzoyl chloride (4.6 mL, 39.3 mmol) was added dropwise. The mixture was allowed to warm slowly and stirred at room temperature overnight. It was then diluted with CH₂Cl₂ (100 mL) and washed with cold 5% HCl and brine. The phases were separated and the aqueous phase was extracted with CH₂Cl₂ (2 x 50 mL). The combined organic extracts were dried over MgSO₄, filtered, and the solvent removed. The residue was dried under vacuum to give amide 254 as a pale yellow solid (6.21 g, 88%): ¹H NMR (CDCl₃, 400 MHz) δ 7.75 (d, *J*= 6.8 Hz, 2 H), 7.49-7.39 (m, 3 H), 6.57 (bs, 1 H), 4.00 (m, 1 H), 3.67-3.61 (m, 1 H), 3.31-3.24 (m, 1 H), 1.22 (d, *J*= 6.3 Hz, 3 H).

### Example 96

### 3-{4-[3-(5-Methyl-2-phenyl-oxazol-4-yl)-prop-1-ynyl]-phenyl}-2-pyridin-3-yl-propionic acid (261).

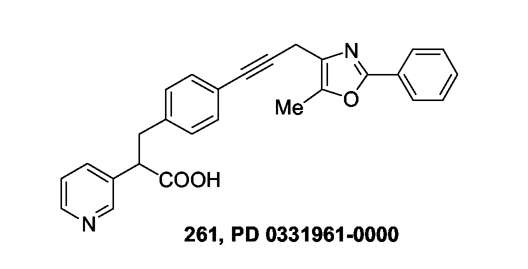

General Procedure 96. 3-{4-[3-(5-Methyl-2-phenyl-oxazol-4-yl)-prop-1-ynyl]-phenyl}-2-pyridin-3-yl-propionic acid methyl ester 4a (0.284 g, 0.630 mmol) was dissolved in THF (15 mL) and water was added (5 mL). Lithium hydroxide monohydrate (0.040 g, 0.945 mmol) was incorporated and the mixture was stirred at room temperature for 1 h. The organic solvent was removed in the rotary evaporator at room temperature. The aqueous residue was diluted with water and extracted with Et₂O (2 x 45 mL). The aqueous phase was then acidified with 10% HCl. The product was extracted with CH₂Cl₂-CHCl₃. The extracts were dried over magnesium sulfate, filtered and the solvent removed. Purification by column chromatography on silica gel eluting with methanol in chloroform (0 to 10%) afforded the title compound as a solid (0.100 g, 37%): mp 115-117 °C; CIMS *m*/*z* 423.1 (M+1).

3-{4-[3-(5-Methyl-2-phenyl-oxazol-4-yl)-prop-1-ynyl]-phenyl}-2-pyridin-3-yl-propionic acid methyl ester (260) was prepared in the following manner.

### (a) 3-{4-[3-(5-Methyl-2-phenyl-oxazol-4-yl)-prop-1-ynyl]-phenyl]-2-pyridin-3-yl-propionic acid methyl ester (260).

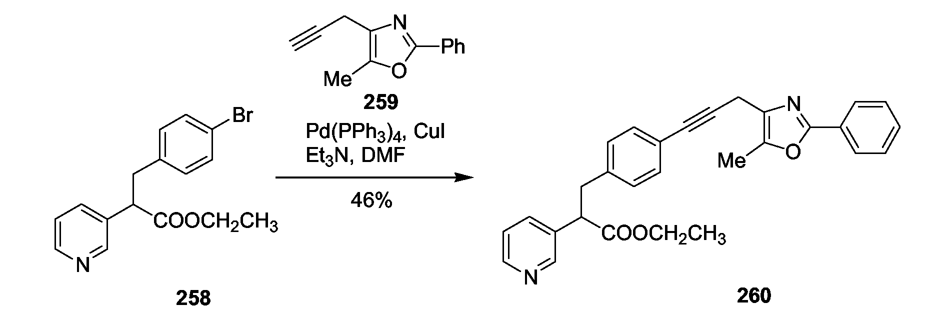

General Procedure 96-a. Bromide 258.354 g, 4.051 mmol) and alkyne 259.04 g, 5.266 mmol) were dissolved in dry DMF (14 mL). Triethylamine (1.7 mL, 12.153 mmol) was added and nitrogen was passed through the reaction mixture for 0.5 h. Pd(PPh₃)₄ (0.328 g, 0.284 mmol) was added followed by CuI (0.154 g, 0.810 mmol). The mixture was heated at 80 °C for 24 h under nitrogen. The mixture was allowed to cool and diluted with water (100 mL) and Et₂O (70 mL). The phases were separated and the aqueous phase was extracted with Et₂O (4 x 60 mL). The combined organic extracts were washed with water and brine. Activated carbon was added and the mixture was boiled for 15-20 min. After cooling to room temperature, the mixture was dired over MgSO₄, filtered and the solvent removed. Purification by flash chromatography on silica gel eluting with ethyl acetate in hexanes (0 to 30%) gave pure 260 as a thick oil (0.838 g, 46%): CIMS *m*/*z* 451 (M + H)⁺.

### (b) 3-(4-Bromo-phenyl)-2-pyridin-3-yl-propionic acid ethyl ester (258).

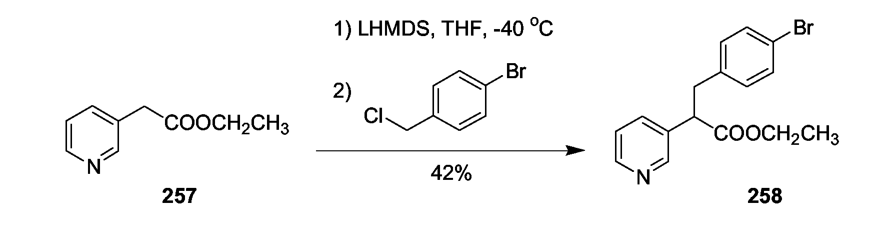

General Procedure 96-b Ester 257.63 g, 9.861 mmol) was dissolved in dry THF (20 mL) and cooled to -78°C under a nitrogen atmosphere. A 1.0 M solution of LHMDS in THF (10.8 mL, 10.8 mmol) was added. The mixture was stirred at - 78 to -40 °C for 1.25 h. A solution of 4-chlorobenzyl bromide (2.43 g, 11.833 mmol) in THF (5 mL) was added. The reaction was allowed to reach room temperature overnight. The mixture was quenched with water (40 mL) and diluted with EtOAc (30 mL). The phases were separated and the aqueous phase was extracted with EtOAc (30 mL x 3). The combined organic extracts were washed with brine, dried over magnesium sulfate and the solvent removed. Purification by column chromatography on silica gel eluting with EtOAc in hexanes (0 to 45%) afforded bromide 258 an oil (1.37 g, 42%): CIMS *m*/*z* 334.0 (M)⁺.

### Example 97

### 3-{4-[3-(5-Methyl-2-phenyl-oxazol-4-yl)-propyl]-phenyl}-2-pyridin-3-yl-propionic acid (264).

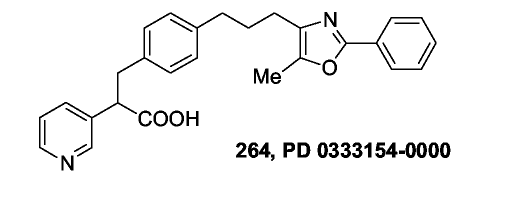

This compound was prepared from 3-{4-[3-(5-Methyl-2-phenyl-oxazol-4-yl)-propyl]-phenyl}-2-pyridin-3-yl-propionic acid ethyl ester 263 following General Procedure 100. The title compound was obtained as a solid in 58% yield: mp 138-141 °C; CIMS *m*/*z* 427.2 (M+1). Anal. calcd for C₂₇H₂₆N₂O₃·1.0 H₂O: C, 72.95; H, 6.35; N, 6.30. Found: C, 72.64; H, 6.32; N, 5.97.

3-{4-[3-(5-Methyl-2-phenyl-oxazol-4-yl)-propyl]-phenyl}-2-pyridin-3-yl-propionic acid ethyl ester (263) was prepared in the following manner.

### (a) 3-{4-[3-(5-Methyl-2-phenyl-oxazol-4-yl)-propyl]-phenyl}-2-pyridin-3-yl-propionic acid ethyl ester (263).

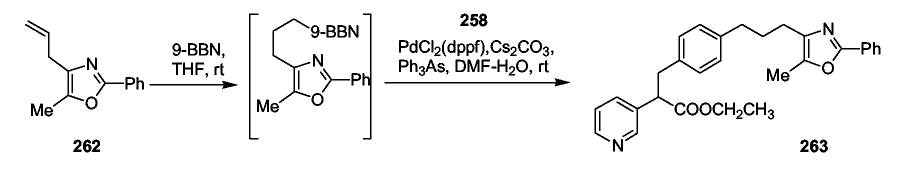

General Procedure 97-a. A solution of 5-methyl-2-phenyl-4-prop-2-enyloxazole (262) (0.20 g, 1.003 mmol) in dry THF (5 mL) was added to a 0.5 M solution of 9-BBN in THF (4 mL, 2.0 mmol) at 0 °C under a nitrogen atmosphere. The ice bath was removed and the mixture was stirred at room temperature overnight. The 9-BBN adduct was then added to a flask containing bromide 258 (0.351 g, 1.053 mmol), PdCl₂(dppf) (0.073 g, 0.1 mmol), Cs₂CO₃ (0.588 g, 1.805 mmol), Ph₃As (0.030 g, 0.1 mmol), water (0.22 mL, 12 mmol) and DMF (5 mL). The reaction mixture was stirred at room temperature under a nitrogen atmosphere overnight. At the end of this time, the mixture was cooled in an ice bath and 3 M NaOAc (6 mL) was added followed by 30% H₂O₂ (3 mL). Stirring was continued for 2 h, allowing the reaction to warm to room temperature slowly. Water (50 mL) was added followed by Et₂O and EtOAc. The phases were separated and the aqueous phase was extracted with Et₂O-EtOAc (30:5 mL x 4). The combined organic extracts were washed with brine, dried over magnesium sulfate and the solvent removed. Purification by column chromatography on silica gel eluting with EtOAc in hexanes (0 to 25%) afforded ester 263 as a thick oil (0.272, 60%): CIMS *m*/*z* 455.2 (M+1).

### Example 98

### 3-{4-[3-(5-Methyl-2-phenyl-oxazol-4-yl)-propyl]-phenyl}-2-pyridin-3-yl-propionic acid (267).

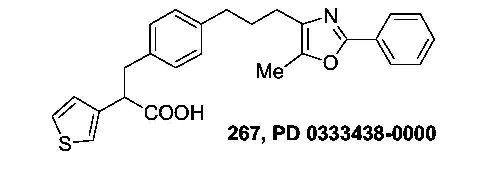

This compound was prepared from 3-{4-[3-(5-Methyl-2-phenyl-oxazol-4-yl)-propyl]-phenyl}-2-thiophen-3-yl-propionic acid ethyl ester (266).. The purification was carried out by chromatography on silica gel eluting with ethyl acetate in hexanes (0 to 45%). The title compound was obtained as a solid in 58% yield: mp 112-114 °C; CIMS *m*/*z* 432.1 (M+1). Anal. calcd for C₂₆H₂₅NO₃S: C, 72.36; H, 5.84; N, 3.25. Found: C, 72.18; H, 5.97; N, 3.04.

3-{4-[3-(5-Methyl-2-phenyl-oxazol-4-yl)-propyl]-phenyl}-2-thiophen-3-yl-propionic acid ethyl ester (266) was prepared in the following manner.

### (a) 3-{4-[3-(5-Methyl-2-phenyl-oxazol-4-yl)-propyl]-phenyl}-2-thiophen-3-yl-propionic acid ethyl ester (267).

This compound was prepared following General Procedure 98-a using bromide 266 for the Suzuki coupling. Ester 267 was obtained as thick oil in 78% yield: CIMS *m*/*z* **460.2** (M+1).

### (b) 3-(4-Bromo-phenyl)-2-thiophen-3-yl-propionic acid ethyl ester (266).

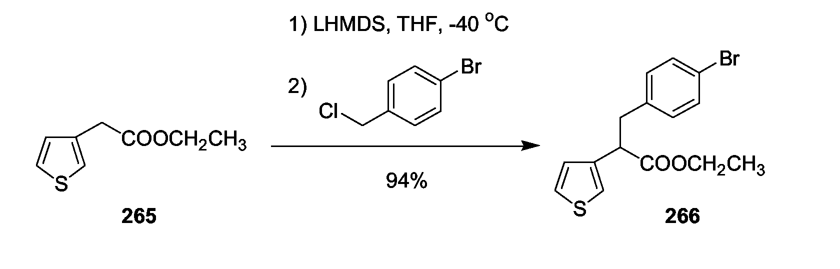

This compound 266 was prepared from ester 265 following General Procedure 98-b and obtained as thick oil in 94% yield: CIMS *m*/*z* 339.0 (M)⁺.

### Example 99

### 2-Biphenyl-4-yl-3-{4-[3-(5-methyl-2-phenyl-oxazol-4-yl)-propyl]-phenyl}-propionic acid (270).

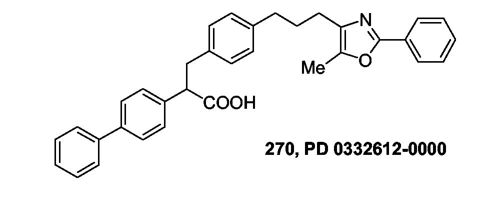

This compound was prepared from 2-Biphenyl-4-yl-3-{4-[3-(5-methyl-2-phenyl-oxazol-4-yl)-propyl]-phenyl}-propionic acid ethyl ester (269) by hydrolysis. The purification was carried out by chromatography on silica gel eluting with ethyl acetate in hexanes (0 to 30%). The title compound was obtained as a solid in 96% yield: mp 158-160 °C; CIMS *m*/*z* 502.3 (M+1). Anal. calcd for C₃₄H₃₁NO₃: C, 81.41; H, 6.23; N, 2.79. Found: C, 81.05; H, 6.42; N, 2.63.

2-Biphenyl-4-yl-3-4-[3-(5-methyl-2-phenyl-oxazol-4-yl)-propyl]-phenyl}-propionic acid ethyl ester (269) was prepared in the following manner.

### (a) 2-Biphenyl-4-yl-3-{4-[3-(5-methyl-2-phenyl-oxazol-4-yl)-propyl]-phenyl]-propionic acid ethyl ester (269).

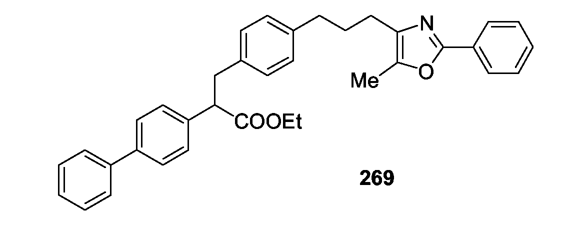

2-Biphenyl-4-yl-3-{4-[3-(5-methyl-2-phenyl-oxazol-4-yl)-prop-1-ynyl]-phenyl}-propionic acid ethyl ester (268, See Example 104) (0.325 g, 0.618 mmol) was dissolved in THF (50 mL) and hydrogenated over Raney Nickel (1.0 g) overnight. The mixture was filtered off and the solvent removed. Purification by chromatography on silica gel eluting with ethyl acetate in hexanes gave **6c** as clear oil (0.254 g, 77%): CIMS *m*/*z* 530.2 (M+1).

### Example 100

### 2-Biphenyl-4-yl-3-{4-[3-(5-methyl-2-phenyl-oxazol-4-yl)-prop-1-ynyl]-phenyl}-propionic acid (273).

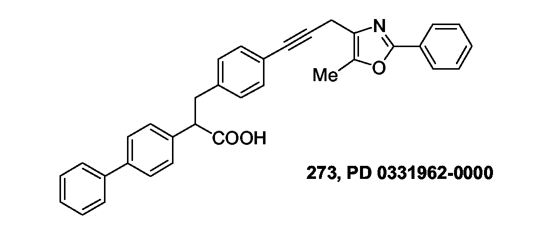

This compound was prepared from ester 272. The reaction mixture was heated at 80 °C for 7 h. The purification was carried out by chromatography on silica gel eluting with methanol in chloroform (0 to 6%). The title compound was obtained as an orange solid in 54% yield: mp 163-165 °C; CIMS m/z 498.2 (M+1).

2-Biphenyl-4-yl-3-{4-[3-(5-methyl-2-phenyl-oxazol-4-yl)-prop-1-ynyl]-phenyl}-propionic acid ethyl ester (268) was prepared in the following manner.

### (a) 2-Biphenyl-4-yl-3-{4-[3-(5-methyl-2-phenyl-oxazol-4-yl)-prop-1-ynyl]-phenyl}-propionic acid ethyl ester (268)

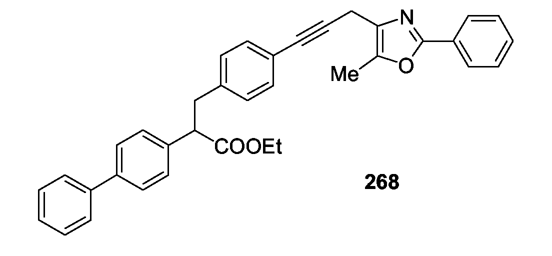

This compound was obtained from bromide 271 by following the General Method II. Ester 268 was obtained as a thick orange oil in 37% yield: CIMS m/z 526 (M+1).

### (b) 2-Biphenyl-3-(4-Bromo-phenyl)-propionic acid ethyl ester (271).

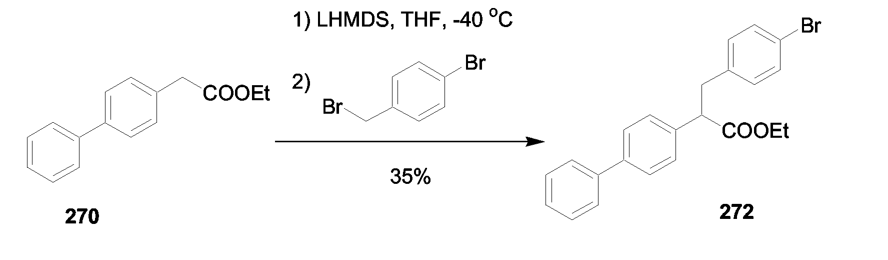

This compound was prepared from 27. It was obtained as a white solid in 35% yield: CIMS *m*/*z* 409.0 (M)⁺.

### Example 101

### 2-Biphenyl-3-yl-3-{4-[3-(5-methyl-2-phenyl-oxazol-4-yl)-prop-1-ynyl]-phenyl]-propionic acid (278).

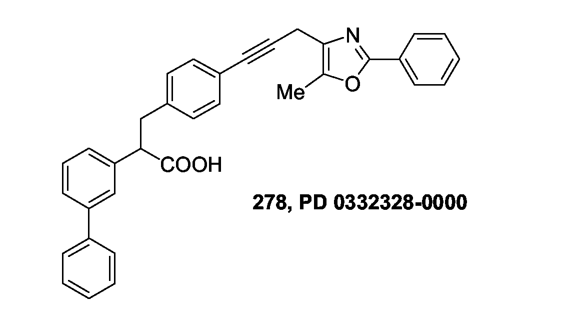

This compound was prepared from ester 277. The purification was carried out by chromatography on silica gel eluting with ethyl acetate in hexanes (0 to 45%). The title compound was obtained as a solid in 96% yield: mp 105-107 °C; CIMS *m*/*z* 498.2 (M+1). Anal. calcd for C₃₄H₂₇NO₃·1.3 H₂O: C, 78.38; H, 5.73; N, 2.69. Found: C, 78.22; H, 5.57; M, 2.59.

2-Biphenyl-3-yl-3-{4-[3-(5-methyl-2-phenyl-oxazol-4-yl)-prop-1-ynyl]-phenyl]-propionic acid methyl ester (277) was prepared in the following manner.

### (a) 2-Biphenyl-3-yl-3-{4-[3-(5-methyl-2-phenyl-oxazol-4-yl)-prop-1-ynyl]-phenyl}-propionic acid methyl ester (277).

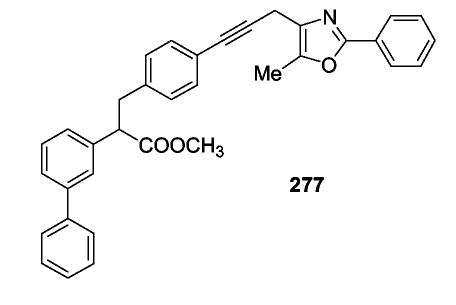

This compound was obtained from bromide 276 following General Procedure 100-a. Ester 277 was obtained as a thick orange oil in 12% yield: CIMS m/z 512.2 (M+1).

### (b) 2-Biphenyl-3-yl-3-(4-Bromo-phenyl)-propionic acid methyl ester (276).

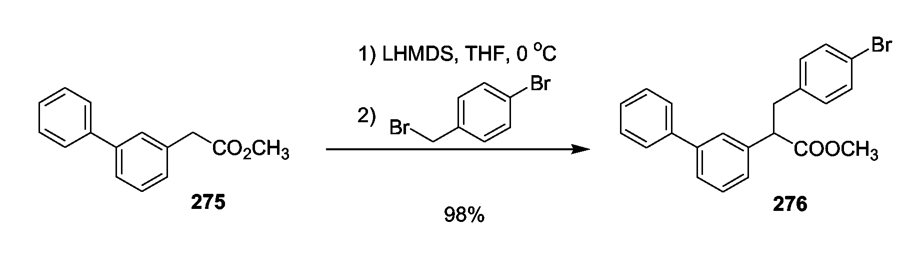

This compound was prepared from ester 275. It was obtained as a thick, colorless oil in 98% yield: CIMS m/z 395.1 (M)⁺.

### (c) 2-biphenyl-3yl-acetic acid methyl ester (275).

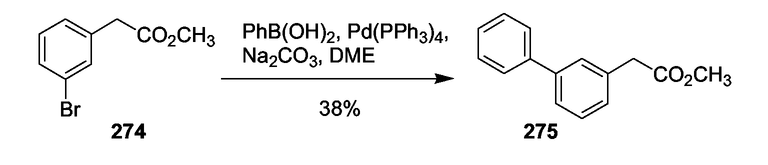

Bromide 274 (1.872 g, 8.172 mmol) and phenylboronic acid (1.49 g, 12.258 mmol) were dissolved in DME. Sodium carbonate (1.73 g, 16.344 mmol) was added. Nitrogen was bubble into the reaction mixture for 25 min. Pd(PPh₃)₄ (0.471 g, 0.408 mmol) was added and the reaction mixture was heated at 90 °C for 40 h. Mixture cooled at rt, diluted with ethyl acetate (30 mL) and water (30 mL) was added. Phases separated. Aqueous phase extracted with ethyl acetate (3 x 35 mL). The combined extracts were dried over magnesium sulfate, filtered and the solvent removed. The residue was purified by column chromatography on silica gel eluting with ethyl acetate in hexanes (0 to 5 %). Ester 275 was obtained as a thick oil (0.706 g, 38%): CIMS *m*/*z* 227.0 (M+1).

### (d) 2-(3-Bromo-phenyl)-acetic acid methyl ester (274).

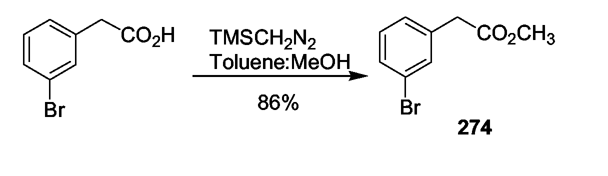

2-(3-Bromo-phenyl)-acetic acid (5.00 g, 23.25 mmol) was dissolved in toluene/MeOH (10:5 mL). The solution was cooled in an ice bath and 2.0 M solution of trimethylsilyldiazomethane in hexanes (14 mL, 28 mmol) was added dropwise. The mixture was stirred at room temperature overnight. The mixture was quenched with acetic acid and stirred for 0.5 h. Solvents removed and the residue purified by column chromatography on silica gel eluting with ethyl acetate in hexanes (0 to 5 %). Ester 274 was obtained as a clear oil (4.59 g, 86%): CIMS *m*/*z* 228.9 (M)⁺.

### Example 102

### 2-Biphenyl-3-yl-3-{4-[3-(5-methyl-2-phenyl-oxazol-4-yl)-propyl]-phenyl}-propionic acid (280).

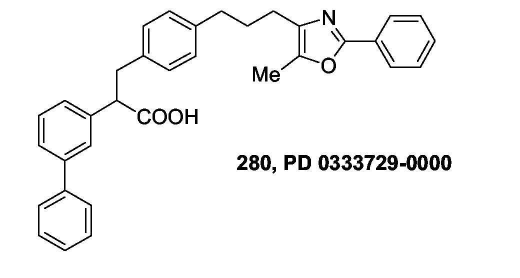

This compound was prepared from ester 279 following General Procedure 100. Purification was carried out by chromatography on silica gel eluting with ethyl acetate in hexanes (0 to 30%). The title compound was obtained as a solid in 69% yield: mp 75-80 °C; CIMS *m*/*z* 502.3 (M+1). Anal. calcd for C₃₄H₃₁NO₃·0.1 H₂O: C, 81.12; H, 6.25; N, 2.78. Found: C, 80.80; H, 6.33; N, 2.69.

2-Biphenyl-3-yl-3-{4-[3-(5-methyl-2-phenyl-oxazol-4-yl)-propyl]-phenyl}-propionic acid methyl ester (279) was prepared in the following manner.

### (a) 2-Biphenyl-3-yl-3-{4-[3-(5-methyl-2-phenyl-oxazol-4-yl)-propyl]-phenyl]-propionic acid methyl ester (279).

This compound was prepared following Example 104 using bromide 272 for the Suzuki coupling. Ester 279 was obtained as a thick oil in 37% yield: CIMS *m*/*z* 516.3 (M+1).

### Example 103

### 2-(5-Methyl-isoxazol-3-yl)-3-{4-[3-(5-methyl-2-phenyl-oxazol-4-yl)-propyl]-phenyl}-propionic acid (284).

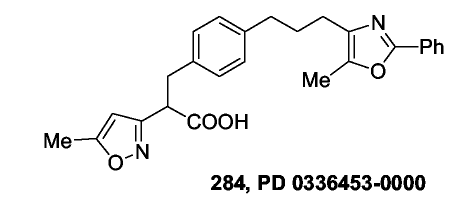

This compound was prepared from ester 3-(4-Bromo-phenyl)-2-(5-Methyl-isoxazol-3-yl)-propionic acid ethyl ester (283) by LiOH-mediated hydrolysis. The purification was carried out by chromatography on silica gel eluting with ethyl acetate in hexanes (0 to 30%). The title compound was obtained as a solid in 69% yield: mp 75-80 °C; CIMS *m*/*z* 502.3 (M+1). Anal. calcd for C₃₄H₃₁NO₃·0.1 H₂O: C, 81.12; H, 6.25; N, 2.78. Found: C, 80.80; H, 6.33; N, 2.69.

2-(5-Methyl-isoxazol-3-yl)-3-{4-[3-(5-methyl-2-phenyl-oxazol-4-yl)-propyl]-phenyl}-propionic acid ethyl ester (283) (PD 0335776-0000) was prepared in the following manner.

### (a) 2-(5-Methyl-isoxazol-3-yl)-3-{4-[3-(5-methyl-2-phenyl-oxazol-4-yl)-propyl]-phenyl}-propionic acid ethyl ester (283).

This compound was prepared following General Procedure 100-a using bromide 282 for the Suzuki coupling. Ester 283 was obtained as thick oil in 84% yield: CIMS *m*/*z* 459.3 (M+1).

### (b) 3-(4-Bromo-phenyl)-2-(5-Methyl-isoxazol-3-yl)-propionic acid ethyl ester (282).

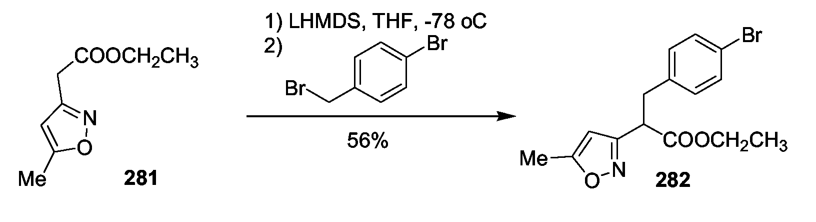

This compound was prepared from ester 281 following General Procedure 97-b and was obtained as a thick oil in 56% yield: CIMS *m*/*z* 338.0 (M)⁺.

### Example 104

### 2-(3-Methyl-isoxazol-5-yl)-3-{4-[3-(5-methyl-2-phenyl-oxazol-4-yl)-propyl]-phenyl}-propionic acid (288).

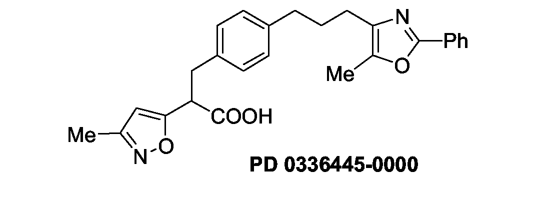

This compound was prepared from 2-(3-Methyl-isoxazol-5-yl)-3-{4-[3-(5-methyl-2-phenyl-oxazol-4-yl)-propyl]-phenyl}-propionic acid methyl ester (287) following General Procedure 96. The purification was carried out by chromatography on silica gel eluting with methanol in chloroform (0 to 5%). The title compound was obtained as an off-white solid in 85% yield: mp 133-135 °C; CIMS *m*/*z* 431.2 (M+1). Anal. calcd for C₂₆H₂₆N₂O₄: C, 72.54; H, 6.09; N, 6.51. Found: C, 72.32; H, 6.28; N, 6.41.

2-(3-Methyl-isoxazol-5-yl)-3-{4-[3-(5-methyl-2-phenyl-oxazol-4-yl)-propyl]-phenyl}-propionic acid methyl ester (287) was prepared in the following manner.

### (a) 2-(3-Methyl-isoxazol-5-yl)-3-{4-[3-(5-methyl-2-phenyl-oxazol-4-yl)-propyl]-phenyl}-propionic acid methyl ester (287).

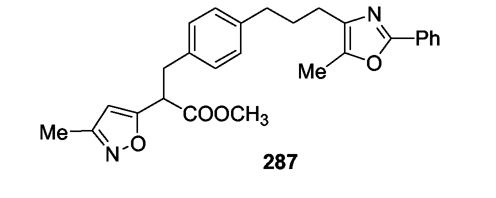

This compound was prepared following General Procedure 96-a using bromide 286 for the Suzuki coupling. 2-(3-Methyl-isoxazol-5-yl)-3-{4-[3-(5-methyl-2-phenyl-oxazol-4-yl)-propyl]-phenyl}-propionic acid methyl ester (287) was obtained as thick oil in 60% yield: CIMS *m*/*z* 459.3 (M+1).

### (b) 3-(4-Bromo-phenyl)-2-(3-Methyl-isoxazol-5-yl)-propionic acid ethyl ester (286).

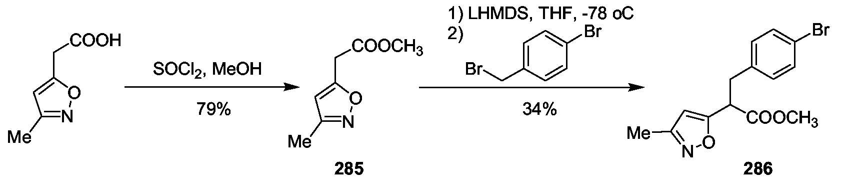

This compound was prepared from ester 285 following General Procedure 96-b and was obtained as a thick oil in 34% yield: CIMS *m*/*z* 324.0 (M)⁺.

### (c) 3-Methyl-isoxazol-5-yl-acetic acid methyl ester (285).

3-Methyl-isoxazol-5-yl-acetic acid (3.0 g, 21.257 mmol) was dissolved in MeOH (50 mL) and cooled in an ice bath. Thionyl chloride (2.3 mL, 31.9 mmol) was added and the ice bath removed. The mixture was heated at 40 °C for 24 h. The solvent was removed and the residue purified by distillation. The title compound was obtained as a clear oil (2.62 g, 79%); bp 81-82 °C (at 0.7 mmHg); CIMS *m*/*z* 154.1 (M+1).

### Example 105

### (S)-3-(4-{[(5-Methyl-2-phenyl-oxazol-4-ylmethyl)-amino]-methyl}-phenyl)-2-pyrrol-1-yl-propionic acid (294).

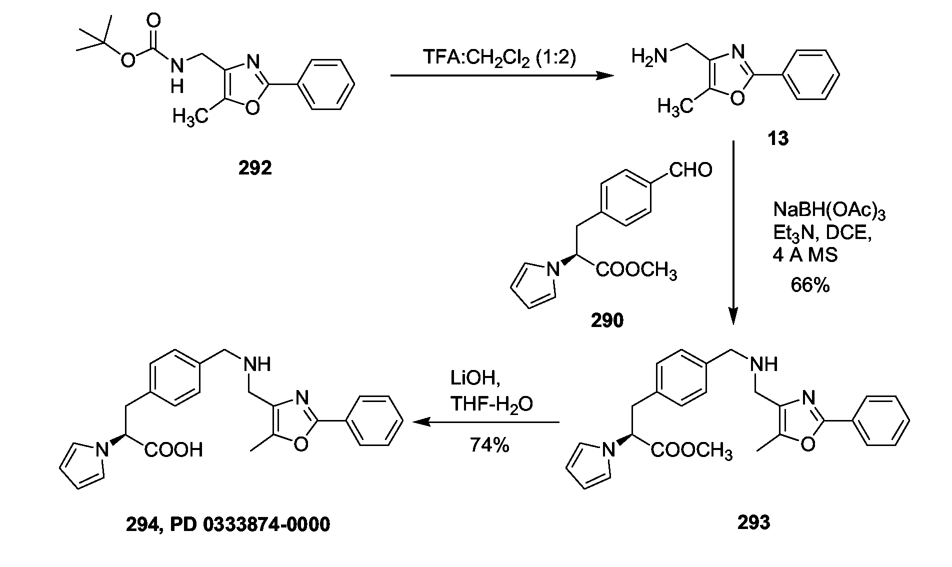

This compound was prepared from ester 293 following General Procedue 100. The purification was carried out by chromatography on silica gel eluting with methanol in chloroform (0 to 5%). The title compound was obtained as an off-white solid in 74% yield: mp 163-165 °C; CIMS *m*/*z* 416.2 (M+1). Anal. calcd for C₂₅H₂₅N₃O₃·0.42 CHCl₃: C, 65.57; H, 5.50; N, 9.02. Found: C, 65.26; H, 5.81; N, 8.86.

(*S*)-3-(4-{[(5-Methyl-2-phenyl-oxazol-4-ylmethyl)-amino]-methyl}-phenyl)-2-pyrrol-1-yl-propionic acid methyl ester (293) was prepared in the following manner.

### (a) (S)-3-(4-{[(5-Methyl-2-phenyl-oxazol-4-ylmethyl)-amino]-methyl}-phenyl)-2-pyrrol-1-yl-propionic acid methyl ester (293).

Carbamate 292 (0.326 g, 1.130 mmol) was dissolved in dichloromethane (6 mL) and cooled in an ice bath. TFA (3 mL) was added and the mixture was stirred for 10 min and the ice bath was removed. The reaction mixture was stirred at room temperature for 3 h. The solvents were removed and the residue dried under high vacuum. The residue was then dissolved in 1,2-dichloroethane (15 mL) and Et₃N (0.39 mL, 2.825 mmol) added. After 5 min, aldehyde 290 (0.215 g, 0.837 mmol) was added, followed by NaBH(OAc)₃ (0.283 g, 1.339 mmol) after 10 min. The mixture was stirred at room temperature for 18 h. Additional NaBH(OAc)₃ (0.300 g) was added and stirring continued for 5 h. The mixture was diluted with dichloromethane and filtered through a celite pad. Saturated NaHCO₃ was added and the phases were separated. The aqueous phase was extracted with dichloromethane (3 x 25 mL). The combined extracts were dried over magnesium sulfate, filtered and the solvent removed. The solvents were removed and the residue was purified by column chromatography on silica gel eluting with methanol in chloroform (0 to 5 %). Ester 293 was obtained as a thick oil (0.236 g, 66%): CIMS *m*/*z* 430.2 (M+1).

### (b) Preparation of (5-Methyl-2-phenyl-oxazol-4-ylmethyl)-carbamic acid tert-butyl ester (292).

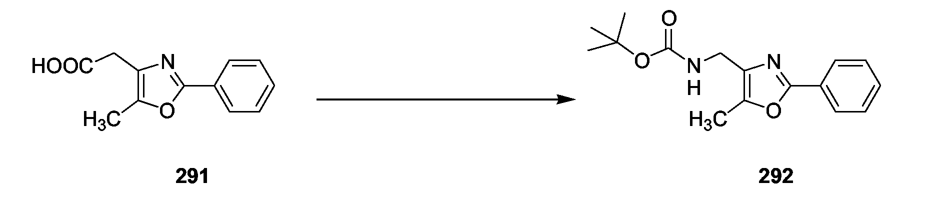

Acid 291 (3.0 g, 13.8 mmol) was dissolved in dichloromethane (70 mL) under nitrogen. The mixture was cooled in an ice bath and then oxalyl chloride (1.7 mL, 19.32 mmol) was added, followed by DMF (0.053 mL, 0.69 mmol). The ice bath was removed after 20 min and the reaction mixture stirred at room temperature for 5 h. The solvent was removed. The residue was dissolved in acetone and cooled at 0 °C. A solution of sodium azide (1.19 g, 18.354 mmol) in water (4.2 mL) was added. The mixture was stirred at 0 °C for 1 h, then it was diluted with water and extracted with toluene (4 x 35 mL). The combined extracts were dried over magnesium sulfate, filtered and the solvent removed. The residue was dissolved in toluene (80 mL) and tert-BuOH (2.64 mL, 27.6 mmol) was added. The solution was heated at reflux for 18 h. The solvents were removed and the residue was purified by column chromatography on silica gel eluting with ethyl acetate in hexanes (0 to 25 %). Carbamate 292 was obtained as a solid (2.33 g, 58%): CIMS *m*/*z* 289.1 (M+1).

### (c) (S)-2-Pyrrol-1-yl-3-(4-formyl-phenyl)-propionic acid methyl ester (290).

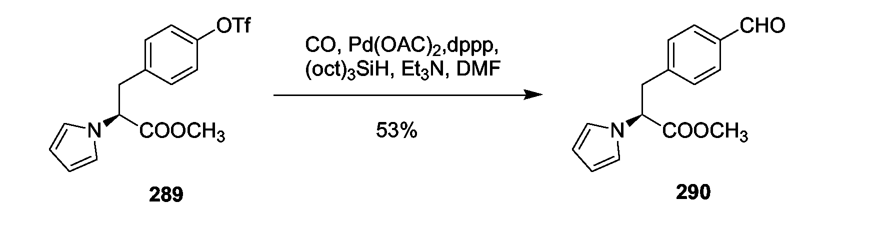

A mixture of triflate 289 (3.0 g, 7.950 mmol), Pd(OAc)₂ (0.065 g, 0.289 mmol), dppp (0.122 g, 0.295 mmol), Et₃N (0.92 mL, 6.60 mmol), (octyl)₃SiH (2.38 mL, 5.298 mmol) in DMF (45 mL) was stirred and heated at 70 °C under 500 psi of CO for 14 h. The solvent was removed and the residue was diluted with Et₂O (50 mL) and washed with water (70 mL). The aqueous phase was extracted with Et₂O (4 x 50 mL). The combined extracts were dried over magnesium sulfate, filtered and the solvent removed. The residue was purified by column chromatography on silica gel eluting with ethyl acetate in hexanes (0 to 25 %). The title compound 290 was obtained as an orange solid (1.11 g, 54%): CIMS m/z 258.1 (M+1).

### Example 106

### (S)-3-(4-{[Acetyl-(5-methyl-2-phenyl-oxazol-4-ylmethyl)-amino]-methyl}-phenyl)-2-pyrrol-1-yl-propionic acid (296).

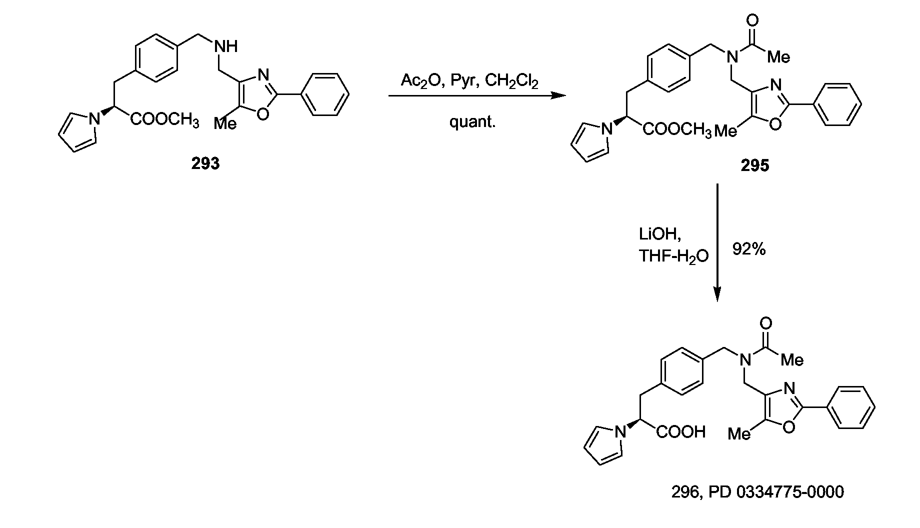

Ester 295 (0.268 g, 0.570 mmol) was dissolved in THF (15 mL) and water was added (5 mL). Lithium hydroxide monohydrate (0.036 g, 0.885 mmol) was incorporated and the mixture was stirred at room temperature for 2.5 h. The organic solvent was removed in the rotary evaporator at room temperature. The aqueous residue was diluted with water and cooled in an ice bath. It was then acidified with 10% HCl. The solid that was obtained was separated by filtration, washed with water, and dried under high vacuum at 50 °C for 12 h to give the title compound as a yellowish-pale brown solid (0.241 g, 92%): mp 97-100 °C; CIMS *m*/*z* 458.2 (M+1). Anal. calcd for C₂₇H₂₇N₃O₄·0.5 H₂O: C, 69.51; H, 6.05; N, 9.01. Found: C, 69.32; H, 6.02; N, 8.73.

Preparation of (*S*)-3-(4-{[Acetyl-(5-methyl-2-phenyl-oxazol-4-ylmethyl)-amino]-methyl}-phenyl)-2-pyrrol-1-yl-propionic acid methyl ester (295) was prepared in the following manner.

### (a) (S)-3-(4-{[Acetyl-(5-methyl-2-phenyl-oxazol-4-ylmethyl)-amino]-methyl}-phenyl)-2-pyrrol-1-yl-propionic acid methyl ester (15).

(*S*)-3-(4-{[(5-Methyl-2-phenyl-oxazol-4-ylmethyl)-amino]-methyl}-phenyl)-2-pyrrol-1-yl-propionic acid methyl ester (293) (0.245 g, 0.570 mmol) was dissolved in dichloromethane (10 mL) and cooled in an ice bath. Pyridine (0.115 mL, 1.425 mmol) was added followed by Ac₂O (0.107 mL, 1.14 mmol). The ice bath was removed after 30 min and the mixture stirred at room temperature for 20 h. dichloromethane (50 mL) was added and the solution was washed with 10% HCl, sat. NaHCO₃, sat. NaCl. The organic phase was dried over magnesium sulfate, filtered and the solvent removed. The solvents were removed and the residue was dried under high vacuum overnight to give a pale yellow oil (0.295 g): CIMS *m*/*z* 472 (M+1).

### Example 107

### (S)-3-(4-{[Methyl-(5-methyl-2-phenyl-oxazol-4-ylmethyl)-amino]-methyl}-phenyl)-2-pyrrol-1-yl-propionic acid (298).

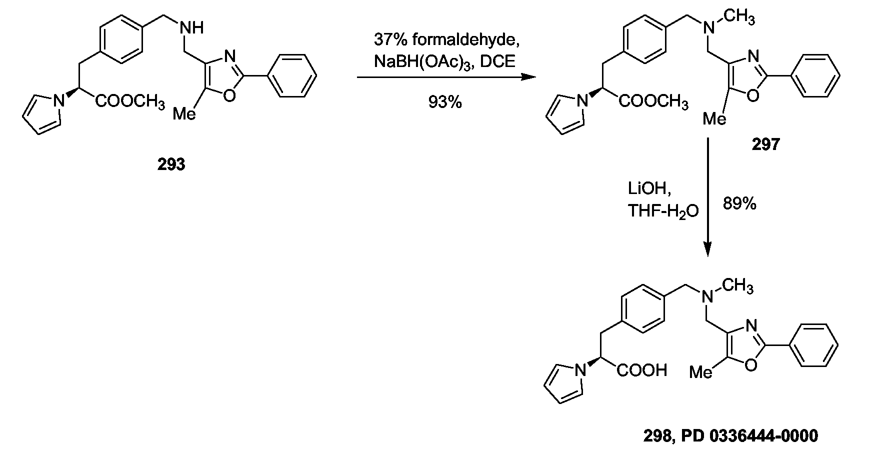

This compound was prepared from ester (*S*)-3-(4-{[Methyl-(5-methyl-2-phenyl-oxazol-4-ylmethyl)-amino]-methyl}-phenyl)-2-pyrrol-1-yl-propionic acid methyl ester (297) following General Procedure 96. The purification was carried out by chromatography on silica gel eluting with methanol in chloroform (0 to 10%). The title compound was obtained as a solid in 89% yield: mp 133-135 °C; CIMS *m*/*z* 430.3 (M+1). Anal. calcd for C₂₆H₂₇N₃O₃·1.71 H₂O: C, 67.84; H, 6.66; N, 9.13. Found: C, 67.85; H, 6.39; N, 8.73.

(*S*)-3-(4-{[Methyl-(5-methyl-2-phenyl-oxazol-4-ylmethyl)-amino]-methyl}-phenyl)-2-pyrrol-1-yl-propionic acid methyl ester (297) was prepared in the following manner.

### (a) (S)-3-(4-{[Methyl-(5-methyl-2-phenyl-oxazol-4-ylmethyl)-amino]-methyl}-phenyl)-2-pyrrol-1-yl-propionic acid methyl ester (16).

(*S*)-3-(4-{[(5-Methyl-2-phenyl-oxazol-4-ylmethyl)-amino]-methyl}-phenyl)-2-pyrrol-1-yl-propionic acid methyl ester (293) (0.432 g, 1.006 mmol) was dissolved in 1,2-dichloroethane (10 mL) and 37% formaldehyde solution (0.090 mL, 1.106 mmol) was added, followed by NaBH(OAc)₃ (0.300 g, 1.408 mmol). The mixture was stirred at room temperature for 24 h under nitrogen. Sat. NaHCO₃ (30 mL) was added and the mixture was stirred for 20 min. At this time, it was diluted with dichloromethane and the phases were separated. The aqueous phase was extracted with dichloromethane (3 x 30 mL). The combined extracts were dried over magnesium sulfate, filtered and the solvent removed. The solvents were removed and the residue was purified by column chromatography on silica gel eluting with methanol in chloroform (0 to 3%). Ester 16 was obtained as a thick pale yellow oil (0.417 g, 93%): CIMS *m*/*z* 444.2 (M+1).

### Example 108

### (S)-3-(4-{[2-(5-Methyl-2-phenyl-oxazol-4-yl)-ethylamino]-methyl}-phenyl)-2-pyrrol-1-yl-propionic acid (304).

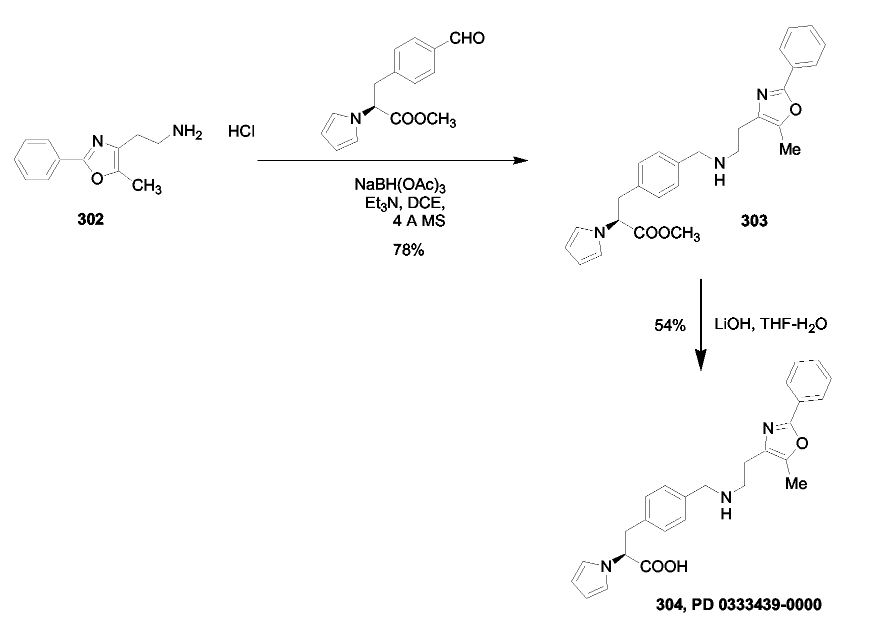

This compound was prepared from (*S*)-3-(4-{[2-(5-Methyl-2-phenyl-oxazol-4-yl)-ethylamino]-methyl}-phenyl)-2-pyrrol-1-yl-propionic acid methyl ester (303) following General Procedure 97. The purification was carried out by chromatography on silica gel eluting with methanol in chloroform (0 to 12%). The title compound was obtained as a white solid in 54% yield: mp 208-210 °C; CIMS *mlz* 430.2 (M+1). Anal. calcd for C₂₆H₂₇N₃O₃·0.6 H₂O: C, 70.92; H, 6.46; N, 9.54. Found: C, 70.60; H, 6.34; N, 9.48.

(*S*)-3-(4-{[2-(5-Methyl-2-phenyl-oxazol-4-yl)-ethylamino]-methyl}-phenyl)-2-pyrrol-1-yl-propionic acid methyl ester (303) was prepared in the following manner.

### (a) (S)-3-(4-{[2-(5-Methyl-2-phenyl-oxazol-4-yl)-ethylamino]-methyl}-phenyl)-2-pyrrol-1-yl-propionic acid methyl ester (303).

Amine 302 (0.392 g, 1.642 mmol) was suspended in 1,2-dichloroethane (15mL). Et₃N (0.252 mL, 1.806 mmol) added and the mixture stirred for 10 min. A solution of aldehyde 290 (0.352 g, 1.368 mmol) in 1,2-dichloroethane (15mL) was incorporated. After 10 min, NaBH(OAc)₃ (0.405 g, 1.915 mmol) was added. The mixture was stirred at room temperature for 18 h. Additional NaBH(OAc)₃ (0.290 g) and 4A MS (0.4 g) were added and stirring continued for 12 h. More NaBH(OAc)₃ (0.150 g) was added and stirring continued for 3 days. The mixture was diluted with dichloromethane and filtered through a celite pad. Saturated NaHCO₃ was added and the phases were separated. The aqueous phase was extracted with dichloromethane (3 x 25 mL). The combined extracts were dried over magnesium sulfate, filtered and the solvent removed. The solvents were removed and the residue was purified by column chromatography on silica gel eluting with methanol in chloroform (0 to 5 %). Ester **21** was obtained as a yellow oil (0.461 g, 76%): CIMS *m*/*z* 444.2 (M+1).

### (b) 2-(5-Methyl-2-phenyl-oxazol-4-yl)-ethylamine hydrochloride (302).

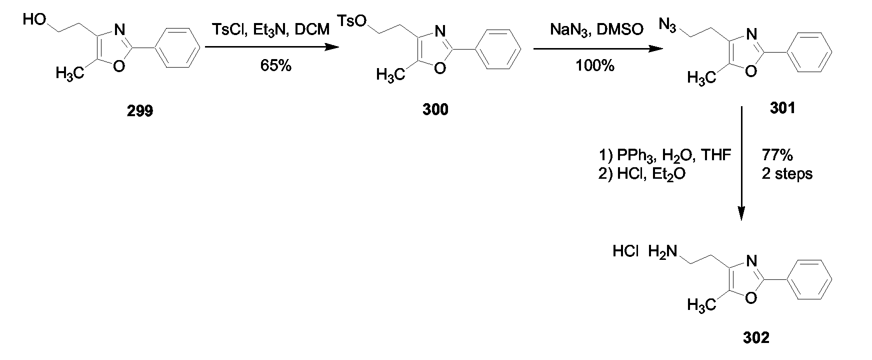

Alcohol 299 (3.87 g, 19.04 mmol) was dissolved in dichloromethane (60 mL) and cooled to 0 °C. TsCl (4.35 g, 22.85 mmol) was added followed by Et₃N (5.3 mL, 38.08 mmol). The mixture was allowed to warm to room temperature overnight. Then, it was diluted with dichloromethane (100 mL) and the solution washed with cold 5% HCl (2 x 50 mL), sat. NaHCO₃ (50 mL), dried over magnesium sulfate, filtered and the solvent removed. The solvents were removed and the residue was purified by column chromatography on silica gel eluting with ethyl acetate in hexanes (0 to 30%). Tosylate 300 was obtained as a yellowish solid (4.41 g, 65%): CIMS *m*/*z* 358.1 (M+1).

Tosylate 300 (4.41 g, 12.34 mmol) was dissolved in DMSO and NaN₃ (1.04 g, 16.04 mmol) was added. The mixture was stirred at room temperature for 3 days. The solution was diluted with water (150 mL) and extracted with Et₂O (5 x 100 mL). The combined extracts were dried over magnesium sulfate, filtered and the solvent removed. Azide 301 was obtained as an orange oil (2.86 g): CIMS *m*/*z* 229.1 (M+1).

Azide 301 (2.86 g, 12.53 mmol) was dissolved in THF (50 mL) and PPh₃ (6.57 g, 25.06 mmol) was added followed by water (2.25 mL, 125.3 mmol). The mixture was stirred at room temperature for 20 h. The solvents were removed, the residue dissolved in Et₂O and the solution extracted with 10% HCl (3 x 20 mL). The combined aqueous extracts were washed with Et₂O. The pH of the solution was adjusted to 14 with sodium hydroxide pellets. The mixture was extracted with Et₂O (3 x 30 mL).). The combined extracts were dried over magnesium sulfate, filtered and the solvent removed to leave a small volume. A 1.0 N solution of HCl in Et₂O was added dropwise. The solid that precipitated was separated by filtration, washed with Et₂O and dried overnight to give amine 302 as the hydrochloride salt. Amine 302 was obtained as an off-white solid (2.316 g, 77%): CIMS *m*/*z* 203.1 (M+1).

### Example 109

### (S)-3-[4-({Benzyl-[2-(5-methyl-2-phenyl-oxazol-4-yl)-ethyl]-amino}-methyl)-phenyl]-2-pyrrol-1-yl-propionic acid (306).

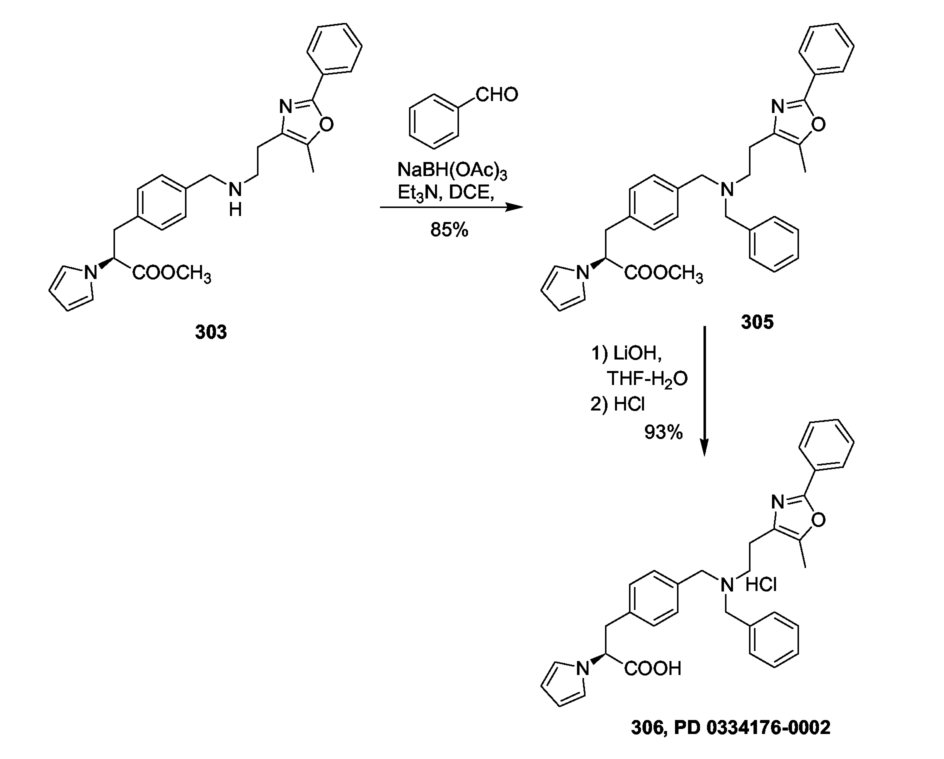

This compound was prepared from (*S*)-3-[4-({Benzyl-[2-(5-methyl-2-phenyl-oxazol-4-yl)-ethyl]-amino}-methyl)-phenyl]-2-pyrrol-1-yl-propionic acid methyl ester (22). following General Procedure 96. A solid was obtained after acidification with 10% HCl. The solid was separated by filtration, washed with water and dried under high vacuum to give an off-white solid (93%): mp 110-113 °C; CIMS *m*/*z* 520.3 (M+1). Anal. calcd for C₃₃H₃₃N₃O₃·HCl: C, 71.27; H, 6.16; N, 7.56. Found: C, 71.22; H, 6.26; N, 7.42.

(*S*)-3-[4-({Benzyl-[2-(5-methyl-2-phenyl-oxazol-4-yl)-ethyl]-amino}-methyl)-phenyl]-2-pyrrol-1-yl-propionic acid methyl ester (305) was prepared in the following manner.

This compound was prepared from (*S*)-3-(4-{[2-(5-Methyl-2-phenyl-oxazol-4-yl)-ethylamino]-methyl}-phenyl)-2-pyrrol-1-yl-propionic acid methyl ester (303) and benzaldehyde following Example 112, step a, and was obtained as an oil in 85% yield: CIMS *m*/*z* 534.3 (M+1).

### Example 110

### (S)-3-[4-({Benzoyl-[2-(5-methyl-2-phenyl-oxazol-4-yl)-ethyl]-amino}-methyl)-phenyl]-2-pyrrol-1-yl-propionic acid (308).

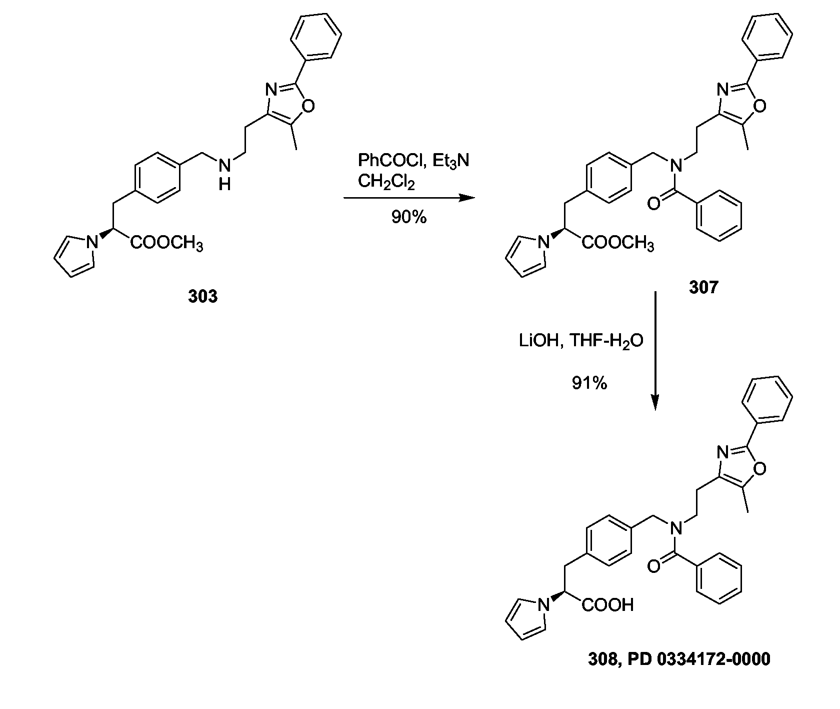

This compound was prepared from (*S*)-3-[4-({Benzoyl-[2-(5-methyl-2-phenyl-oxazol-4-yl)-ethyl]-amino}-methyl)-phenyl]-2-pyrrol-1-yl-propionic acid methyl ester (307). The title compound was obtained as a white solid in 91%): mp 105-110 °C; CIMS *m*/*z* 534.2 (M+1). Anal. calcd for C₃₃H₃₁N₃O₄·0.2 H₂O: C, 73.78; H, 5.89; N, 7.82. Found: C, 73.46; H, 5.76; N, 7.70.

(*S*)-3-[4-({Benzoyl-[2-(5-methyl-2-phenyl-oxazol-4-yl)-ethyl]-amino}-methyl)-phenyl]-2-pyrrol-1-yl-propionic acid methyl ester (307) was prepared in the following manner.

### (a) (S)-3-[4-({Benzoyl-[2-(5-methyl-2-phenyl-oxazol-4-yl)-ethyl]-amino}-methyl)-phenyl]-2-pyrrol-1-yl-propionic acid methyl ester (307).

(*S*)-3-(4-{[2-(5-Methyl-2-phenyl-oxazol-4-yl)-ethylamino]-methyl}-phenyl)-2-pyrrol-1-yl-propionic acid methyl ester (303) (0.286 g, 0.645 mmol) was dissolved in dichloromethane (10 mL) and cooled in an ice bath. Et₃N (0.180 mL, 1.29 mmol) was added followed by PhCOCI (0.082 mL, 0.709 mmol). The ice bath was removed after 30 min and the mixture stirred at room temperature for 20 h. dichloromethane (50 mL) was added and the solution was washed with 10% HCl, sat. NaHCO₃, sat. NaCl. The organic phase was dried over magnesium sulfate, filtered and the solvent removed. The solvents were removed and the residue was purified by column chromatography on silica gel eluting with ethyl acetate in hexanes (0 to 45%). The title compound was obtained as a clear oil (0.319 g, 90%): CIMS *m*/*z* 548.2 (M+1).

### Example 111

### (S)-3-[4-({Acetyl-[2-(5-methyl-2-phenyl-oxazol-4-yl)-ethyl]-amino}-methyl)-phenyl]-2-pyrrol-1-yl-propionic acid (311).

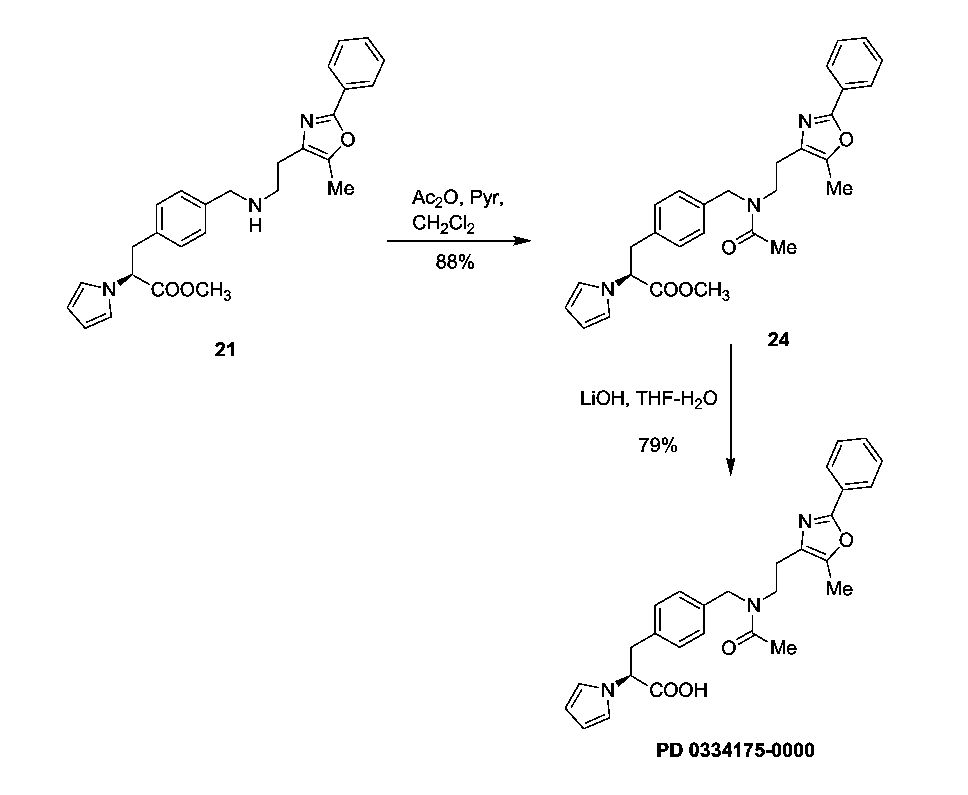

This compound was prepared from (*S*)- 3-[4-({Acetyl-[2-(5-methyl-2-phenyl-oxazol-4-yl)-ethyl]-amino}-methyl)-phenyl]-2-pyrrol-1-yl-propionic acid methyl ester (310) following General Procedure 97. The purification was carried out by chromatography on silica gel eluting with methanol in chloroform (0 to 10%). The title compound was obtained as a white solid in 79% yield: mp 82-87°C; CIMS m/z 472.2 (M+1). Anal. calcd for C₂₈H₂₉N₃O₄·0.4 H₂O: C, 70.25; H, 6.27; N, 8.78. Found: C, 69.92; H, 6.16; N, 8.47.

(*S*)-3-[4-({Acetyl-[2-(5-methyl-2-phenyl-oxazol-4-yl)-ethyl]-amino}-methyl)-phenyl]-2-pyrrol-1-yl-propionic acid methyl ester (310) was prepared in the following manner.

### (a) (S)-3-[4-({Acetyl-[2-(5-methyl-2-phenyl-oxazol-4-yl)-ethyl]-amino}-methyl)-phenyl]-2-pyrrol-1-yl-propionic acid methyl ester (310).

(*S*)-3-(4-{[2-(5-Methyl-2-phenyl-oxazol-4-yl)-ethylamino]-methyl}-phenyl)-2-pyrrol-1-yl-propionic acid methyl ester (303) (0.388 g, 0.875 mmol) was dissolved in dichloromethane (10 mL) and cooled in an ice bath. Pyridine (0.177 mL, 2.187 mmol) was added followed by Ac₂O (0.165 mL, 1.75 mmol). The ice bath was removed after 30 min and the mixture stirred at room temperature for 20 h. dichloromethane (50 mL) was added and the solution was washed with 10% HCl, sat. NaHCO₃, sat. NaCl. The organic phase was dried over magnesium sulfate, filtered and the solvent removed. The residue was purified by column chromatography on silica gel eluting with ethyl acetates in hexanes (0 to 40%). The title compound was obtained as a pale yellow oil (0.376 g, 88%): CIMS *m*/*z* 486.3 (M+1).

### Example 112

### 4-{4-[3-(5-Methyl-2-phenyl-oxazol-4-yl)-propyl]-phenyl}-2-pyrrol-1-yl-butyric acid (312).

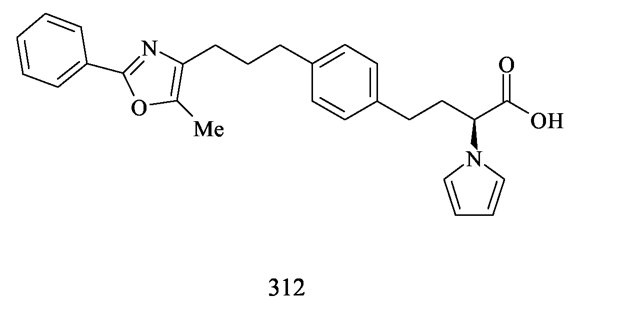

4-{4-[3-(5-Methyl-2-phenyl-oxazol-4-yl)-propyl]-phenyl}-2-pyrrol-1-yl-butyric acid methyl ester was hydrolyzed as in general procedure A to give carboxylic acid 312 in 90% yield. (δ) NMR (CHCl₃) 7.86 (2H, m); 7.33 (3H, m); 7.03 (2H, d, J=8.1Hz); 6.97 (2H, d, J=8.8Hz), 6.68 (2H, m); 6.14 (2H, m); 4.46 (1H, m); 2.56 (3H, m); 2.44 (5H, m); 2.22 (3H, s); 1.88 (2H, m). CIMS *m*/*z* 429.3 (M)⁺.

4-{4-[3-(5-Methyl-2-phenyl-oxazol-4-yl)-propyl]-phenyl}-2-pyrrol-1-yl-butyric acid methyl ester was prepared in the following manner.

### (a) 4-{4-[3-(5-Methyl-2-phenyl-oxazol-4-yl)-propyl]-phenyl}-2-pyrrol-1-yl-butyric acid methyl ester (311).

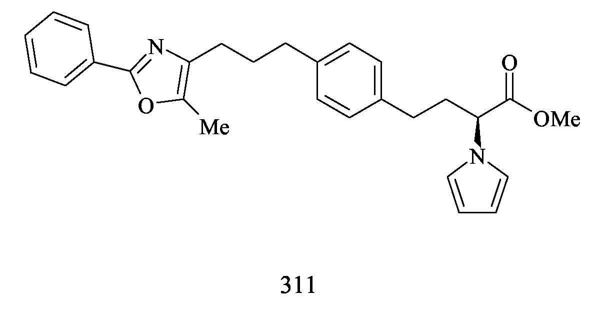

The title compound was prepared as in the procedure outlined for the preparation of esters 120 a-d in 38% yield. 2-Pyrrol-1-yl-4-(4-trifluoromethanesulfonyl-phenyl)-butyric acid methyl ester was used as the triflate component. (δ) NMR (CHCl₃) 8.00 (2H, m); 7.47 (3H, m); 7.10 (2H, d, J=7.6Hz); 7.62 (2H, d, J=7.6Hz); 6.74 (2H, s); 6.21 (2H, s); 4.50 (1H, m); 3.69 (3H, s); 2.58 (6H, m); 2.37 (2H0; 2.31 (3H, s); 1.99 (2H, m). CIMS *m*/*z* 44.3 (M)⁺.

### Example 116

### 2-Pyrrol-1-yl-4-(4-trifluoromethanesulfonyl-phenyl)-butyric acid methyl ester (314).

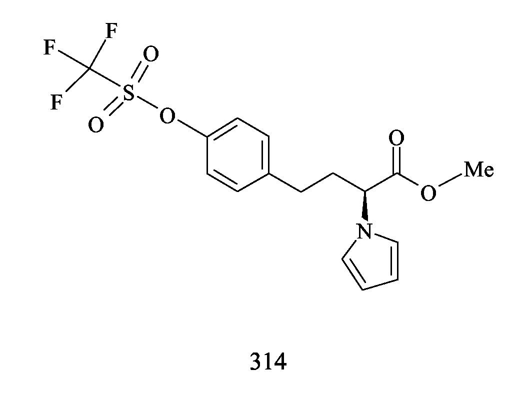

The title compound was prepared as described in the synthesis of (*S*)-2-Pyrrol-1-yl-3-[(4-trifluoromethanesulfonyloxy)phenyl]-propionic acid methyl ester (119) from homo-tyrosine (CAS 141899-12-9) via esterification and pyrrole condensation as described in the preparation of pyrrolotyrosine methyl ester (1). (δ) NMR (CHCl₃) 7.18 (4H, s); 6.71 (2H, s); 6.21 (2H, s); 4.51 (1H, m); 3.70 (3H, s); 2.45 (4H, s). CIMS *m*/*z* 391.9 (M)⁺.

### Example 117

### 3- {1-[3-(5-Methyl-2-phenyl-oxazol-4-yl)-propyl]-piperidin-4-yl}-2-pyrrol-1-yl-propionic acid (318).

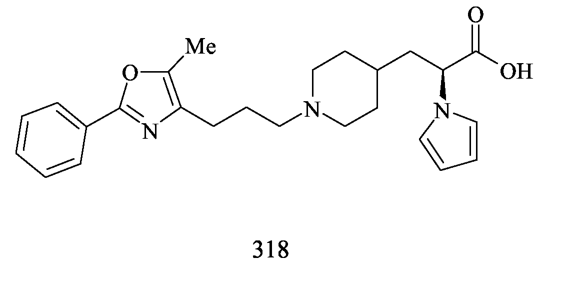

The title compound was prepared from 3-{1-[3-(5-Methyl-2-phenyl-oxazol-4-yl)-propyl]-piperidin-4-yl}-2-pyrrol-1-yl-propionic acid methyl ester (317) via general procedure A in 43% yield. NMR (δ;CHCl₃); 7.9 (2H, m); 7.40 (3H, m); 6.65 (2H, s); 6.13 (2H, s); 6.01 (1H, m); 4.61 (1H, m); 3.50 (2H, m); 2.91 (2H, m); 2.59 (2H, m); 2.29 (3H, m); 2.27 (3H, s); 2.05 (3H, m); 1.85 (1H, m); 1.55 (1H, m). CIMS *m*/*z* 422.3 (M)⁺.

3- {1-[3-(5-Methyl-2-phenyl-oxazol-4-yl)-propyl]-piperidin-4-yl}-2-pyrrol-1-yl-propionic acid methyl ester (317) was prepared in the following manner.

### (a) 3-{1-[3-(5-Methyl-2-phenyl-oxazol-4-yl)-propyl]-piperidin-4-yl}-2-pyrrol-1-yl-propionic acid methyl ester (317).

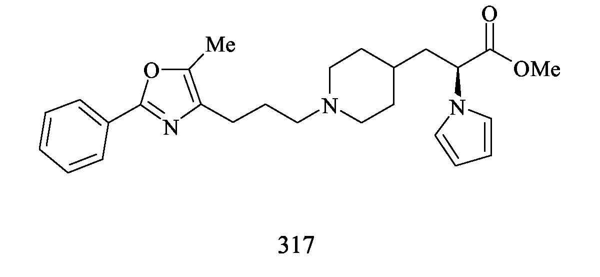

The title compound was prepared from the TFA salt of the amino ester (316) by the pyrrole formation reaction as in Example 1 in 28% yield. NMR (δ;CHCl₃); 7.95 (2H, m); 7.45 (3H, m); 6.70 (2H, s); 6.16 (2H, s); 4.66 (1H, m); 3.71 (3H, s); 2.52 (2H, m); 2.31 (3H, s); 2.03 (3H, m); 1.59 (3H, m); 1.21 (6H, m). CIMS *m*/*z* 436.3 (M)⁺.

### (b) 2-Amino-3-{1-[3-(5-methyl-2-phenyl-oxazol-4-yl)-propyl]-piperidin-4-yl}-propionic acid methyl ester trifluoroacetic acid salt (316).

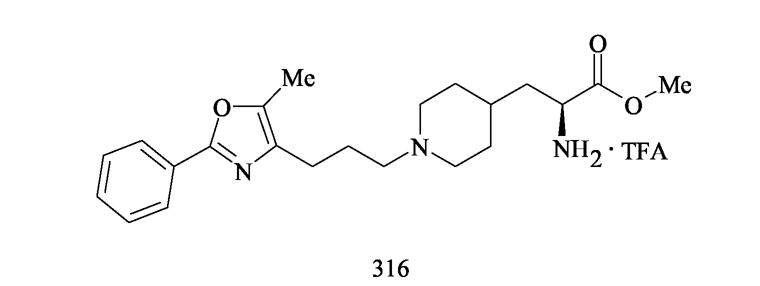

Prepared by treating 2-tert-Butoxycarbonylamino-3-{1-[3-(5-methyl-2-phenyl-oxazol-4-yl)-propyl]-piperidin-4-yl}-propionic acid methyl ester (315) with 5 equiv of trifluoroacetic acid in dichloromethane for 24 hours at 23°C. Removal of solvent gave the trifluoroacetic acid salt which was used without further purification.

### (c) 2-tert-Butoxycarbonylamino-3-{1-[3-(5-methyl-2-phenyl-oxazol-4-yl)-propyl]-piperidin-4-yl}-propionic acid methyl ester

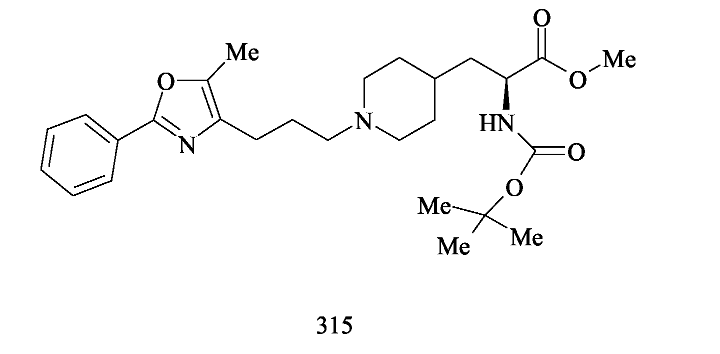

The title compound was prepared by combining 4-(3-Bromo-propyl)-5-methyl-2-phenyl-oxazole (1.21 g, 4.6 mmol), 2-tert-Butoxycarbonylamino-3-piperidin-4-yl-propionic acid methyl ester (315, 1 g, 3.67 mmol) and Cs₂Co₃ (1.79 g, 5.5 mmol) in acetonitrile (30 mL) at 23°C under N₂ atm. The mixture was stirred vigorously for 2 days after which the reaction was filtered, and solvent removed *in vacuo.* The residue was chromatographed (SiO₂) with 30% EtOAc/Hexanes and gave 525 mg (30% yield) as a pale yellow oil. NMR (δ;CHCl₃); 7.86 (2H, m); 7.43 (3H, m); 4.35 (1H, m); 3.72 (3H, s); 2.51 (3H, m); 2.40 (4H, m); 2.32 (3H, s); 1.51 (5H, m); 1.40 (9H, s). CIMS *m*/*z* 486.4 (M)⁺.

### (d) 2-tert-Butoxycarbonylamino-3-piperidin-4-yl-propionic acid methyl ester (315, CAS 256925-70-9).

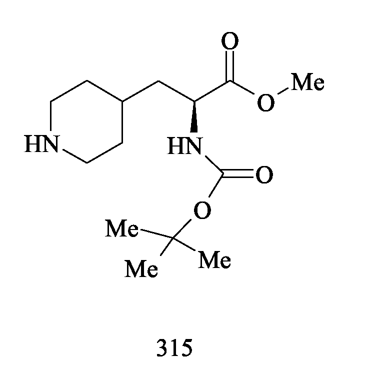

The title compound was prepared as in the esterification of pyrrolotyrosine (1). Used without further purification.

### Example 118

### 3-{3-Methoxy-4-[3-(5-methyl-2-phenyl-oxazol-4-yl)-propenyl]-phenyl}-2-pyrrol-1-yl-propionic acid (321).

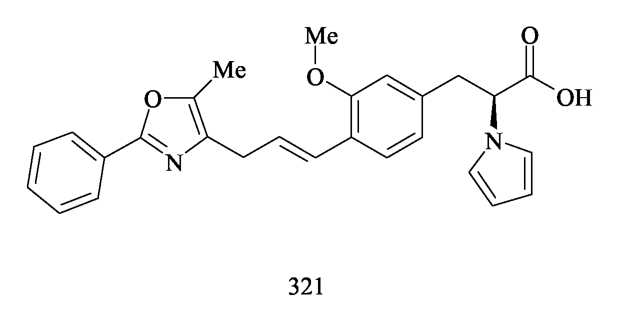

Prepared from 3-{3-Methoxy-4-[3-(5-methyl-2-phenyl-oxazol-4-yl)-propenyl]-phenyl}-2-pyrrol-1-yl-propionic acid methyl ester (320) utilizing general hydrolysis procedure A in 57% yield as a beige foam. NMR (δ;CHCl₃); 8.02 (2H, m); 7.41 (4H, m); 7.29 (1H, d, J=7.8Hz); 6.73 (1H, d, J=16Hz); 6.64 (2H, m); 6.60 (1H, d, J=7.8Hz); 6.32 (1H, m); 6.19 (1H, s); 6.14 (2H, s); 4.60 (1H, m); 3.63 (3H, s); 3.45 (2H, d, J=6.8Hz); 3.25 (2H, m); 2.03 (3H, s). CIMS *m*/*z* 441.3 (M)⁻.

3-{3-Methoxy-4-[3-(5-methyl-2-phenyl-oxazol-4-yl)-propenyl]-phenyl]-2-pyrrol-1-yl-propionic acid methyl ester (320) was prepared in the following manner.

### (a) 3-{3-Methoxy-4-[3-(5-methyl-2-phenyl-oxazol-4-yl)-propenyl]-phenyl}-2-pyrrol-1-yl-propionic acid methyl ester (320).

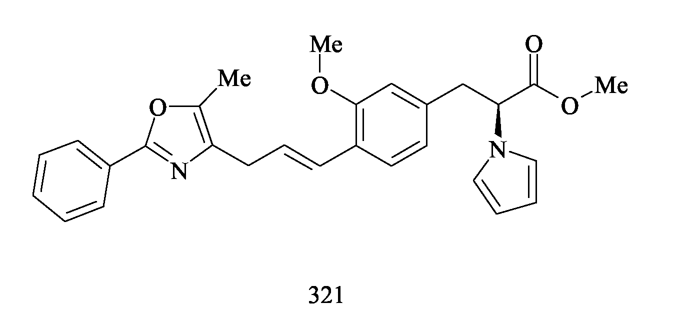

The title compound was prepared as in the Pd coupling procedure used to prepare esters (120) a-d from 3-(3-Methoxy-4-trifluoromethanesulfonyloxyphenyl)-2-pyrrol-1-yl-propionic acid methyl ester and organo borane (117) in 7% yield as a beige foam. NMR (δ;CHCl₃); 8.00 (2H, d, J=8.0Hz); 7.44 (3H, m); 7.23 (1H, d, J=7.1Hz); 6.74 (1H, d, J=16Hz); 6.69 (2H, m); 6.61 (1H, d, J=7.8Hz); 6.30 (1H, m); 6.25 (1H, s); 6.14 (2H, m); 4.70 (1H, m); 3.75 (1H, m); 3.71 (3H, s); 3.66 (3H, s); 3.45 (2H, d, J=6.6Hz); 3.25 (2H, m); 2.40 (3H, s). CIMS *m*/*z* 457.3 (M)⁺.

### (b) 3-(3-Methoxy-4-trifluoromethanesulfonyloxy-phenyl)-2-pyrrol-1-yl-propionic acid methyl ester (319).

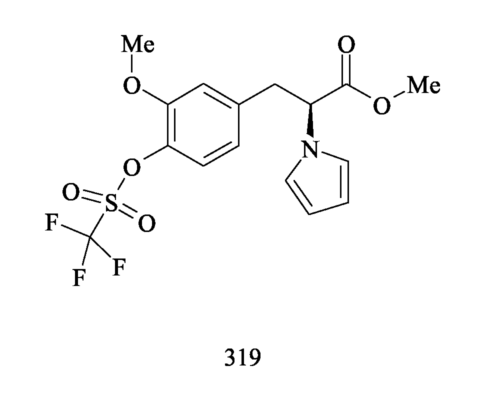

The title compound was prepared using the general procedure for the synthesis of triflate (119). Obtained the product as a light brown oil in 57% yield from 3-(4-Hydroxy-3-methoxy-phenyl)-2-pyrrol-1-yl-propionic acid methyl ester. Microanalysis CHN Theoretical; C=47.18, H=3.96, N=3.44; Observed; C=47.19, H=3.64; N=3.42.

### (c) 3-(4-Hydroxy-3-methoxy-phenyl)-2-pyrrol-1-yl-propionic acid methyl ester. (CAS256925-70-9)

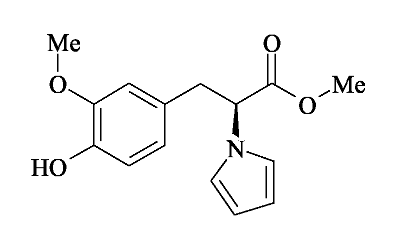

The title compound was obtained from 2-Amino-3-(4-hydroxy-3-methoxyphenyl)-propionic acid methyl ester as in the formation ofpyrrolotyrosine (1) in 69% yield as a yellow oil. CIMS *m*/*z* 276.1 (M)⁺.

### Example 119

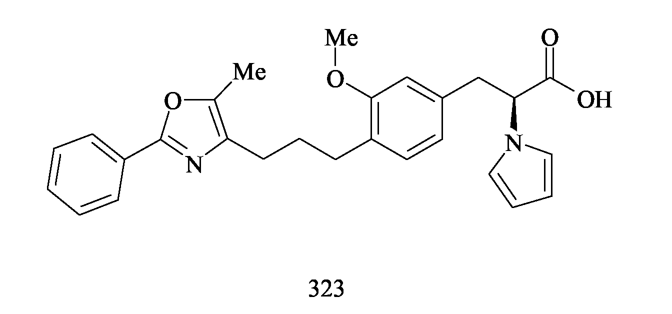

The title compound was obtained in 92% yield from the corresponding methyl ester by general procedure A. Microanalysis CHN theoretical; 72.95, H=6.35, N=6.30; Observed C=72.94, H=6.42, N=6.00. M.P. 120-121°C.

3-{3-Methoxy-4-[3-(5-methyl-2-phenyl-oxazol-4-yl)-propyl]-phenyl}-2-pyrrol-1-yl-propionic acid methyl ester (322) was obtained in the following manner.

### (a) 3-{3-Methoxy-4-[3-(5-methyl-2-phenyl-oxazol-4-yl)-propyl]-phenyl}-2-pyrrol-1-yl-propionic acid methyl ester (322).

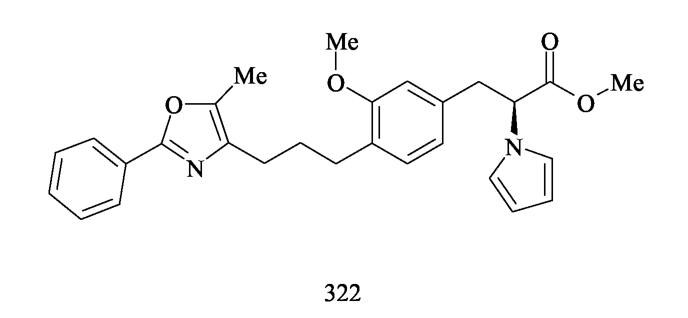

The title compound was obtained via general hydrogenation procedure used to prepare esters 130 a-c. Microanalysis CHN theoretical C=73.34, H=6.59; N=6.11; Observed; C=73.29, H=6.53; N=6.03.

### Example 120

### 3-{3-Methoxy-4-[2-(5-methyl-2-phenyl-oxazol-4-yl)-ethoxy]-phenyl}-2-pyrrol-1-yl-propionic acid (325).

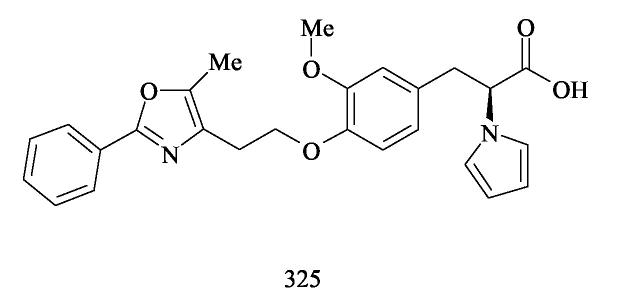

The title compound was obtained from 3-{3-Methoxy-4-[2-(5-methyl-2-phenyl-oxazol-4-yl)-ethoxy]-phenyl}-2-pyrrol-1-yl-propionic acid methyl ester (324) via general hydrolysis procedure A in 86% yield. Microanalysis CHN theoretical C=69.94, H=5.87, N=6.27; Observed C=70.25, H=5.75, N=6.04; M.P.=142-143°C.

3-{3-Methoxy-4-[2-(5-methyl-2-phenyl-oxazol-4-yl)-ethoxy]-phenyl}-2-pyrrol-1-yl-propionic acid methyl ester (324) was obtained in the following manner.

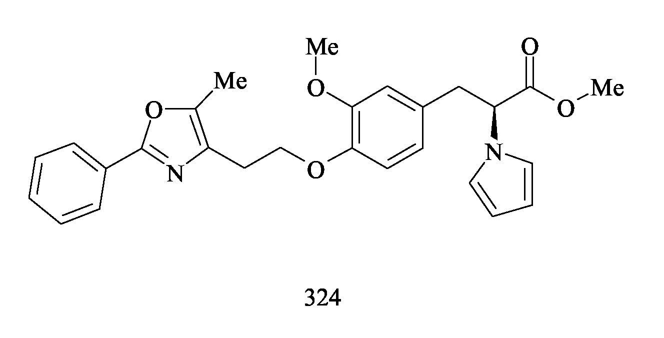

The title compound was obtained from 3-(4-Hydroxy-3-methoxy-phenyl)-2-pyrrol-1-yl-propionic acid methyl ester via general coupling procedure B in 35%. Microanalysis CHN theoretical C=70.42, H=6.13, N=6.08; Observed C=70.42, H=6.03, N=6.04.

### Example 121

### Butane-1-sulfonic acid (3-{4-[3-(5-methyl-2-phenyl-oxazol-4-yl)-propyl]-phenyl}-2-pyrrol-1-yl-propionyl)-amide (326).

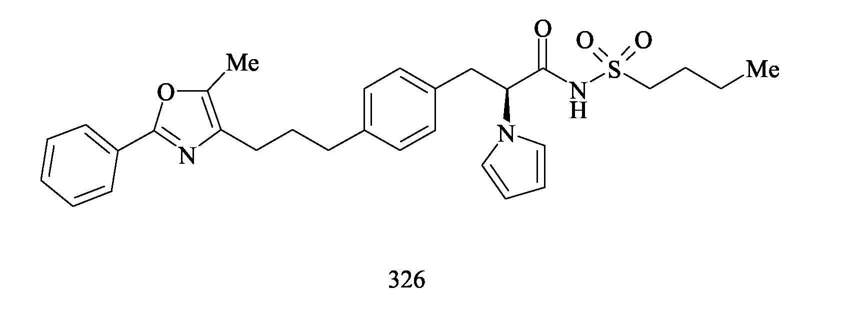

The title compound was obtained from the corresponding acid in 50% yield via the same procedure used to obtain acyl sulfonamide (17). NMR (δ;CHCl₃); 7.96 (2H, m); 7.43 (3H, m); 7.04 (2H, d, J=8Hz); 6.88 (2H, d, J=8.0Hz); 6.61 (2H, m); 6.22 (2H, m); 4.71 (1H, m); 3.35 (1H, m); 3.30 (2H, m); 3.20 (1H, m); 2.60 (2H, m); 2.45 (2H, m); 2.25 (3H, s); 1.95 (2H, m); 1.69 (2H, m); 1.45 (2H, m); 0.95 (3H, m). CIMS *m*/*z* 534.3 (M)⁺.

### Example 122

### N-(3-{4-[3-(5-Methyl-2-phenyl-oxazol-4-yl)-propyl]-phenyl}-2-pyrrol-1-yl-propionyl)-methanesulfonamide (327).

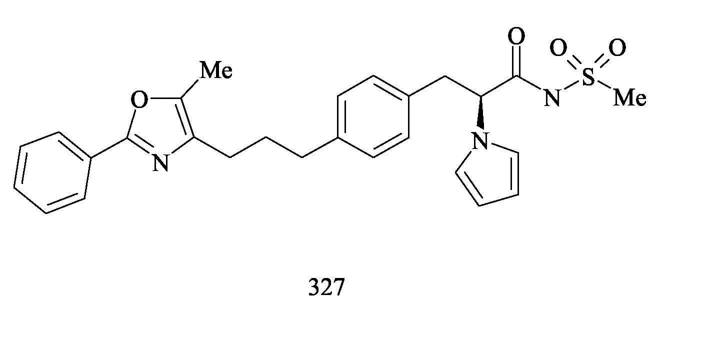

The title compound was obtained as a brown oil from the corresponding acid in 40% yield via the same procedure used to obtain acyl sulfonamide (327). NMR (δ;CHCl₃); 7.96 (2H, m); 7.40 (3H, m); 7.03 (2H, d, J=*.1Hz); 6.89 (2H, d, J=8.1 Hz); 6.65 (1H, s); 6.14 (2H, s); 4.69 (2H, m); 3.49 (1H, m); 3.17 (1H, m); 3.08 (2H, m); 2.58 (2H, t, J=7.3, 8.0Hz); 2.45 (2H, t, J=7.3, 7.6Hz); 2.24 (3H, s); 1.85 (2H, m). CIMS *m*/*z* 492.3 (M)⁺.

### Example 123

### 3-{3-Iodo-4-[2-(5-methyl-2-phenyl-oxazol-4-yl)-ethoxy]-phenyl}-2-pyrrol-1-yl-propionic acid (330).

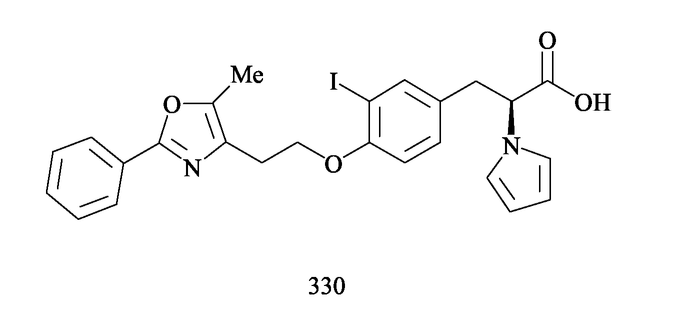

The title compound was obtained from hydrolysis of the corresponding 3-{3-Iodo-4-[2-(5-methyl-2-phenyl-oxazol-4-yl)-ethoxy]-phenyl}-2-pyrrol-1-yl-propionic acid methyl ester (329) via general procedure A. White crystals. 68% yield. Microanalysis CHN Theoretical C=72.95, H=6.35, N=6.30; Observed C=72.71, H=6.44, N=6.29. M.P.=202-203°C.

3-{3-Iodo-4-[2-(5-methyl-2-phenyl-oxazol-4-yl)-ethoxy]-phenyl}-2-pyrrol-1-yl-propionic acid methyl ester (329) was prepared in the following manner.

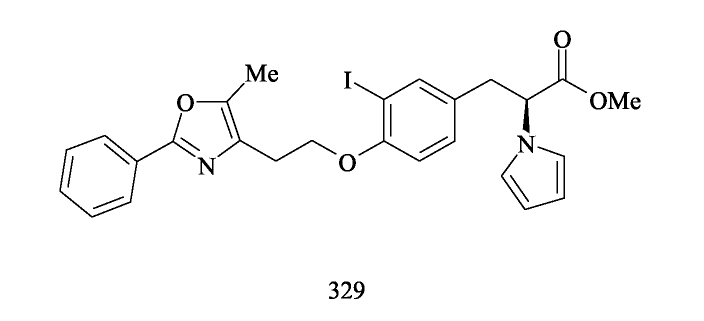

The title compound was obtained via general coupling method B from 3-(4-Hydroxy-3-iodo-phenyl)-2-pyrrol-1-yl-propionic acid methyl ester in 63% yield. CIMS *m*/*z* 557.1 (M)⁺.

### (b) 3-(4-Hydroxy-3-iodo-phenyl)-2-pyrrol-1-yl-propionic acid methyl ester (328).

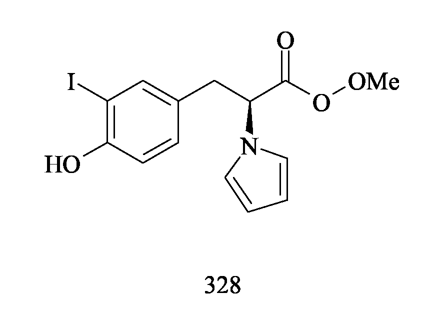

The title compound was obtained in 84% over two steps as in the synthesis ofpyrrolotyrosine methyl ester (1). CIMS *m*/*z* 372.0 (M)⁺.

### Example 124

### 3-{4-[2-(5-Methyl-2-phenyl-oxazol-4-yl)-ethoxy]-3-prop-1-ynyl-phenyl}-2-pyrrol-1-yl-propionic acid (332).

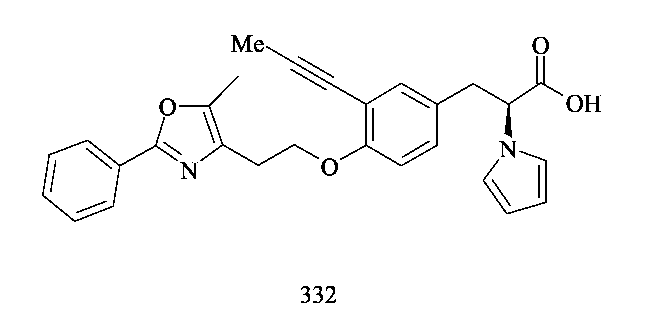

The title compound was obtained from 3-{4-[2-(5-Methyl-2-phenyl-oxazol-4-yl)-ethoxy]-3-prop-1-ynyl-phenyl}-2-pyrrol-1-yl-propionic acid methyl ester. (331, PD0333000-0000) via general hydrolysis procedure A in 66% yield as white crystals from Ethyl acetate M.P.=155-156°C. Microanalysis CHN theoretical: 73.99, H=5.77, N=6.16; Observed C=74.04, H=5.72, H=5.85.

3-{4-[2-(5-Methyl-2-phenyl-oxazol-4-yl)-ethoxy]-3-prop-1-ynyl-phenyl}-2-pyrrol-1-yl-propionic acid methyl ester (331) was prepared in the following manner.

### (a) 3-{4-[2-(5-Methyl-2-phenyl-oxazol-4-yl)-ethoxy]-3-prop-1-ynyl-phenyl}-2-pyrrol-1-yl-propionic acid methyl ester (331).

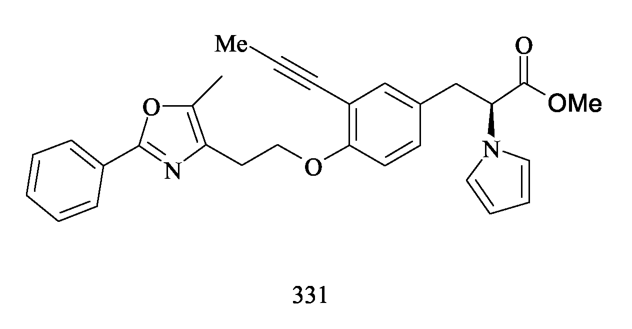

The title compound (1.0 g, 1.8 mol) was dissolved in toluene under N₂ atmosphere at 23°C. To this solution was added tributyl-prop-1-ynyl-stannane (1.10 mL, 3.6 mmol) and Pd(Cl₂(PPh₃)₂ (38 mg, 0.054 mmol) and the flask was heated to reflux for 18 hours. The reaction was washed with aqueous KF (1x 50 mL), water (2x 50 mL), and brine (1x 50 mL). The organic layer was dried over Na₂SO₄ and solvent removed *in vacuo.* The residue was chromatographed with 10-30% EtOAc/Hex to give 400 mg (47% yield) of product as a beige gum. CIMS m/z 469.2 (M).

### Example 125

### 3-{4-[2-(5-Methyl-2-phenyl-oxazol-4-yl)-ethoxy]-3-vinyl-phenyl}-2-pyrrol-1-yl-propionic acid (334).

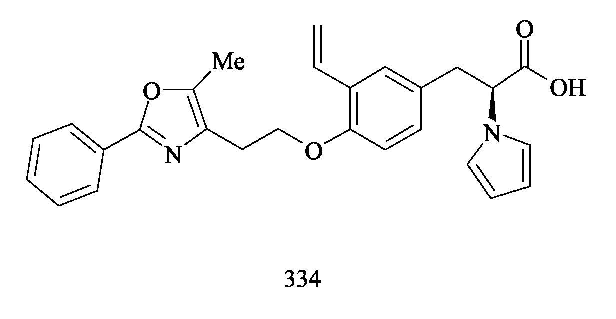

The title compound was obtained from 3-{4-[2-(5-Methyl-2-phenyl-oxazol-4-yl)-ethoxy]-3-vinyl-phenyl}-2-pyrrol-1-yl-propionic acid methyl ester (333) 54% yield utilizing hydrolysis procedure A. NMR (δ;CHCl₃); 7.96 (2H, m); 7.41 (3H, m); 6.99 (1H, s); 6.90 (2H, m); 6.71 (3H, m); 6.15 (2H, s); 5.58 (1H, d, J=17Hz); 5.17 (1H, d, J=11Hz); 4.72 (1H, m); 4.20 (2H, m); 3.36 (1H, m); 3.20 (1H, m); 2.99 (2H, m); 2.35 (3H, s). CIMS *m*/*z* 443.1 (M)⁺.

3-{4-[2-(5-Methyl-2-phenyl-oxazol-4-yl)-ethoxy]-3-vinyl-phenyl}-2-pyrrol-1-yl-propionic acid methyl ester (333) was obtained in the followong manner.

### (a) 3-{4-[2-(5-Methyl-2-phenyl-oxazol-4-yl)-ethoxy]-3-vinyl-phenyl}-2-pyrrol-1-yl-propionic acid methyl ester (333).

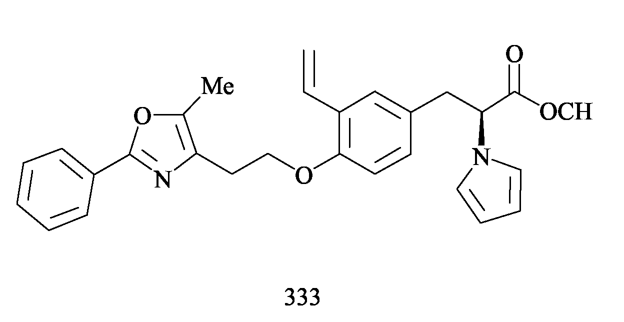

The title compound was obtained in 61 % utilizing vinyl tributyl stannane and a procedure similar to that used in the synthesis of Example 125. MICROANALYSIS CHN theoretical C=73.66, H=6.18, N=6.14. Observed C=73.55, H=5.92, N=5.60 with 0.1 eq. EtOAc as impurity.

### Example 126

### 3-{3-Ethyl-4-[2-(5-methyl-2-phenyl-oxazol-4-yl)-ethoxy]-phenyl}-2-pyrrol-1-yl-propionic acid (336).

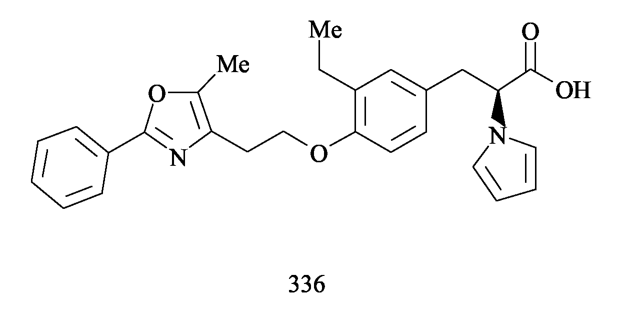

The title compound was obtained from 3-{3-Ethyl-4-[2-(5-methyl-2-phenyl-oxazol-4-yl)-ethoxy]-phenyl}-2-pyrrol-1-yl-propionic acid methyl ester (335) in 68% yield utilizing general hydrolysis procedure A. Microanalysis CHN theoretical C=72.95, H=6.35, N=6.30; Observed 72.71, H=6.44, N=6.29. M.P.=164-165.

3-{3-Ethyl-4-[2-(5-methyl-2-phenyl-oxazol-4-yl)-ethoxy]-phenyl}-2-pyrrol-1-yl-propionic acid methyl ester (335) was prepared in the following manner.

### (a) 3-{3-Ethyl-4-[2-(5-methyl-2-phenyl-oxazol-4-yl)-ethoxy]-phenyl}-2-pyrrol-1-yl-propionic acid methyl ester (335)

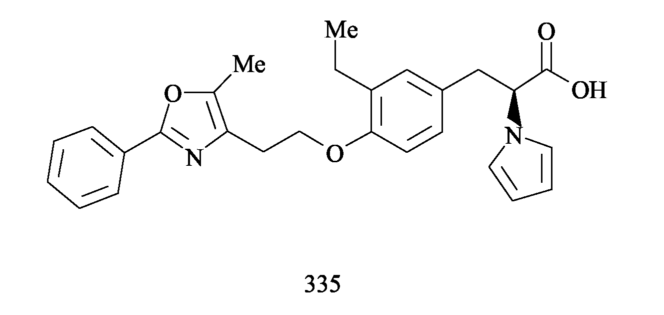

The title compound was obtained from the corresponding vinyl-phenyl derivative via hydrogenation procedure used in the preparation of esters 130a-c in 70% yield as white powder. Microanalysis CHN theoretical C=73.34, H=6.59, N=6.11; Observed 72.96, H=6.60, N=6.10.

### Example 127

### 3-{6-[2-(5-Methyl-2-phenyl-oxazol-4-yl)-ethoxy]-biphenyl-3-yl}-2-pyrrol-1-yl-propionic acid (338).

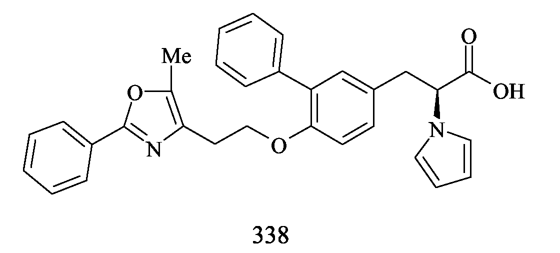

The title compound was obtained from 3-{6-[2-(5-Methyl-2-phenyl-oxazol-4-yl)-ethoxy]-biphenyl-3-yl}-2-pyrrol-1-yl-propionic acid methyl ester (337) via general hydrolysis procedure A in 56% yield as white crystals. M.P. 163-164°C. Microanalysis CHN theoretical C=75.59, H=5.73, N=5.69; Observed 75.39, H=5.83, N=5.43.

3-{6-[2-(5-Methyl-2-phenyl-oxazol-4-yl)-ethoxy]-biphenyl-3-yl}-2-pyrrol-1-yl-propionic acid methyl ester (338) was prepared in the following manner.

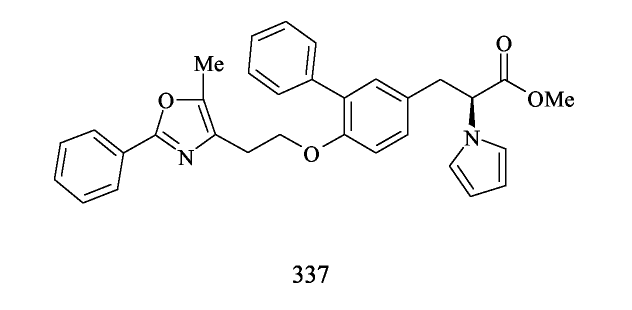

The title compound was obtained in 96% yield from phenyl-boronic acid via a Pd catalyzed procedure similar to that used to obtain the vinyl-phenyl group. CIMS *mlz* 507.2 (M)⁺.

### Example 128

The following illustrates representative pharmaceutical dosage forms, containing a compound of Formula I (Compound 4f), for therapeutic or prophylactic use in humans.

| (i) | Tablet | mg/tablet |
|---|---|---|
| | Invention Compound | 25.0 |
| | Lactose | 50.0 |
| | Corn Starch (for mix) | 10.0 |
| | Corn Starch (paste) | 10.0 |
| | Magnesium Stearate (1%) | 3.0 |
| | | 300.0 |

The biphenylsulfonamide, lactose, and corn starch (for mix) are blended to uniformity. The corn starch (for paste) is suspended in 200 mL of water and heated with stirring to form a paste. The paste is used to granulate the mixed powders. The wet granules are passed through a No. 8 hand screen and dried at 80°C. The dry granules are lubricated with the 1% magnesium stearate and pressed into a tablet. Such tablets can be administered to a human from one to four times a day for treatment of pathogenic bacterial infections.

| (ii) | Tablet | mg/capsule |
|---|---|---|
| | Invention Compound | 10.0 |
| | Colloidal Silicon Dioxide | 1.5 |
| | Lactose | 465.5 |
| | Pregelatinized Starch | 120.0 |
| | Magnesium Stearate (1%) | 3.0 |
| | | 600.0 |

| (iii) | Preparation for Oral Solution | Amount |
|---|---|---|
| | 'Compound 4f' | 400 mg |
| | Sorbitol Soluition (70 % N.F.) | 40 mL |
| | Sodium Benzoate | 20 mg |
| | Saccharin | 5 mg |
| | Cherry Flavor | 20 mg |
| | Distilled Water q.s. | 100 mL |

The sorbitol solution is added to 40 mL of distilled water, and the biphenylsulfonamide is dissolved therein. The saccharin, sodium benzoate, flavor, and dye are added and dissolved. The volume is adjusted to 100 mL with distilled water. Each milliliter of syrup contains 4 mg of invention compound.

### (iv) Parenteral Solution

In a solution of 700 mL of propylene glycol and 200 mL of water for injection is suspended 20 g of 2-Amino-4-cyclopropyl-7-fluoro-5-methyl-6-[3-(1-methylamino-ethyl)-pyrrolidin-1-yl]-pyrido[1,2-*c*]pyrimidine-1,3-dione (Compound 4f). After suspension is complete, the pH is adjusted to 6.5 with 1 N hydrochloric acid, and the volume is made up to 1000 mL with water for injection. The Formulation is sterilized, filled into 5.0 mL ampoules each containing 2.0 mL, and sealed under nitrogen.

| (v) | Injection 1 (1 mg/mL) | Amount |
|---|---|---|
| | Invention Compound | 1.0 |
| | Dibasic Sodium Phosphate | 12.0 |
| | Monobasic Sodium Phosphate | 0.7 |
| | Sodium Chloride | 4.5 |
| | N Sodium hydroxide solution (pH adjustment to 7.0-7.5) | q.s. |
| | Water for injection | q.s. ad 1 mL |

| (vi) | Injection 2 (10 mg/mL) | Amount |
|---|---|---|
| | Invention Compound | 10.0 |
| | Dibasic Sodium Phosphate | 1.1 |
| | Monobasic Sodium Phosphate | 0.3 |
| | Polyethylene glyco 400 | 200.0 |
| | N hydrochloric acid solution (pH adjustment to 7.0-7.5) | q.s. |
| | Water for injection | q.s. ad 1 mL |

| (vii) | Injection 2 (10 mg/mL) | Amount |
|---|---|---|
| | Invention Compound | 20.0 |
| | Oleic Acid | 10.0 |
| | Trichloromonofluoromethane | 5,000.0 |
| | Dichlorodifluoromethane | 10,000.0 |
| | Dichlorotetrafluoroethane | 5,000.0. |

All patents, and patent documents are incorporated by reference herein, as though individually incorporated by reference. The invention has been described with reference to various specific and preferred embodiments and techniques. However, it should be understood that many variations and modifications may be made while remaining within the spirit and scope of the invention.

## Claims

1. A compound of formula (I) 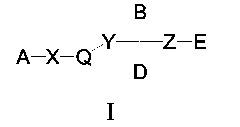 or a pharmaceutically acceptable salt thereof, wherein:
A is 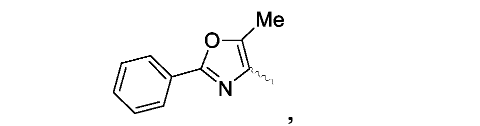
X is 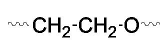 or -CH₂CH₂CH₂- ;
Q is 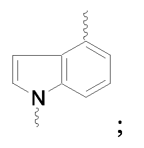
Y is CH₂;
Z is absent;
B is H;
D is H, 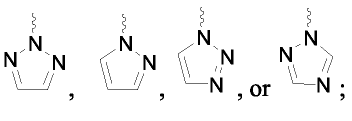 and
E is CO₂H.

2. A compound according to claim 1 which is:

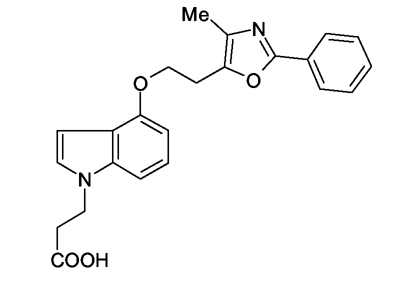

3. A pharmaceutical composition comprising a compound of Claim 1 admixed with a carrier, diluent, or excipient.

4. A compound of claim 1 for use as a medicament.

5. The use of a compound of claim 1 in the preparation of a medicament for treating, preventing or controlling non-insulin dependent diabetes mellitus, obesity, hyperglycemia, hyperlipidemia, hypercholesteremia, atherosclerosis, hypertriglyceridemia, hyperinsulinemia, or insulin resistance syndrome in a mammal.

6. The use of a compound of formula I in the preparation of a medicament for treating, preventing or controlling a condition ameliorated by modulating PPAR activity, lowering blood glucose, or modulating fat cell differentiation in a mammal.
